# EUROPEAN PATENT APPLICATION

(11) **EP 4 242 212 A2**
(43) Date of publication of application: **13.09.2023**
(21) Application number: 23162945.2
(22) Date of filing: 22.03.2019
(51) Int. Cl.: C07D 519/00, C07F 9/09, A61P 35/00, A61K 31/675

(54) **STING MODULATOR COMPOUNDS WITH SULFAMATE LINKAGES, AND METHODS OF MAKING AND USING**

(30) Priority: 23.03.2018 US 201862647227 P; 21.03.2019 US 201962821831 P
(62) Divisional of application: 19721764.9
(71) Applicant: Takeda Pharmaceutical Company Limited, Osaka-shi, Osaka 541-0045 (JP)
(72) Inventor: CIAVARRi, Jeffrey, Cambridge, Massachusetts 02139 (US); ENGLAND, Dylan Bradley, Cambridge, Massachusetts 02139 (US); GIGSTAD, Kenneth M., Cambridge, Massachusetts 02139 (US); GOULD, Alexandra E., Cambridge, Massachusetts 02139 (US); GREENSPAN, Paul, Cambridge, Massachusetts 02139 (US); HU, Yongbo, Cambridge, Massachusetts 02139 (US); HUANG, Shih-Chung, Cambridge, Massachusetts 02139 (US); LANGSTON, Steven Paul, Cambridge, Massachusetts 02139 (US); MIZUTANI, Hirotake, Cambridge, Massachusetts 02139 (US); SHI, Zhan, Cambridge, Massachusetts 02139 (US); VYSKOCIL, Stepan, Cambridge, Massachusetts 02139 (US); XU, He, Cambridge, Massachusetts 02139 (US)
(74) Representative: Harris, Jennifer Lucy

(57) **Abstract**

The present disclosure provides novel compounds of Formula (I) below. Compounds of the disclosure act as STING modulators/agonists. The present disclosure further relates to methods of synthesis of compounds of Formula (I) and methods of using of compounds of Formula (I) for the prophylaxis or treatment of cancer and other STING-related diseases. The compounds have the structure represented by Formula (I): 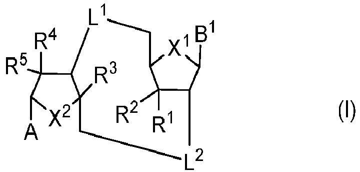 wherein each symbol is as defined in the description, or a pharmaceutically acceptable salt thereof.

## Description

### TECHNICAL FIELD

The present disclosure relates to novel compounds and their use as STING (stimulator of interferon genes) modulators/agonists. The present disclosure also relates to methods of synthesis and methods of using such compounds for the prophylaxis or treatment of cancer and other diseases.

### BACKGROUND

STING is a transmembrane receptor localized to the ER that recognizes and binds cyclic dinucleotides. The natural ligands recognized by STING include bacteria/protozoa-derived cyclic dinucleotides (CDNs), 2',3'-cGAMP synthesized by the upstream cGAS (cyclic GMP-AMP synthase), and the like. See Trends in Immunology 35, 88-93 (2014). It is reported that 2',3'-cGAMP, which is one of natural ligands, is decomposed by ENPP1 (ecto-nucleotide pyrophosphatase/phosphodiesterase), a pyrophosphatase/phosphodiesterase, and that the other CDNs are decomposed by other phosphodiesterases. See Nat Chem Biol 10, 1043-1048 (2014); Cell Res 25, 539-550 (2015); and Biochemistry 55, 837-849 (2016). STING activation by these natural ligands induces the phosphorylation of TBK1 (TANK binding kinase 1) and IRF3 (Interferon regulatory factor 3), leading to the activation of the NFkB and a type-I-interferon (IFN) response, respectively. See Trends in Immunology 35, 88-93 (2014).

The effects of STING on cancer cell growth control were demonstrated using genetically modified mice. It was reported that STING-deficient and IRF3-deficient mice show uncontrolled tumor growth, compared to wild-type mice. See Immunity 41, 830-842 (2014). In addition, it was also reported that the cancer cell growth in a tumor-allografted mouse was suppressed by radiation therapy, but in mice genetically deficient for STING and IFNAR1 (interferon (alpha and beta) receptor 1, receptor of I-type IFN produced by the downstream signal), the effect of the radiation therapy was reduced. See Immunity 41, 843-852 (2014). Taking the above mentioned evidence together, STING is considered to play a critical role in suppressing cancer cell growth. Therefore, STING agonists can be used as an anticancer agent. See e.g. WO 2015/074145, WO 2015/077354, WO 2015/185565, WO 2016/096174, WO 2016/096577, WO 2016/120305, WO 2016/145102, WO 2017/027645, WO 2017/027646, WO 2017/075477, WO 2017/093933, WO 2017/106740, WO 2017/123657, WO 2017/123669, and WO 2017/161349. In addition, the activation of STING can further potentiate the immune effect of traditional vaccine, due to STING's ability to activate both innate and adaptive immunity. See Ther Adv Vaccines 1, 131-143 (2013). As such, STING agonists can also be used as an adjuvant for various vaccines.

### SUMMARY OF THE DISCLOSURE

The present disclosure provides a novel compound of Formula (I) having a STING agonistic activity, which can be useful as an agent for the prophylaxis or treatment of cancer and other diseases.

The present disclosure provides a compound of Formula (I) : 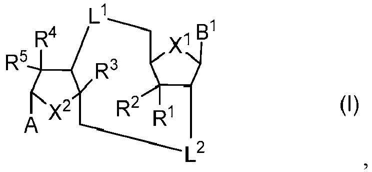 or a pharmaceutically acceptable salt thereof, wherein:
A is B² or -Y-B³, wherein Y is -O-, -S-, -NH-, or -NR^{a}-;
X¹ and X² are independently -O-, -S-, or -CH₂-;
at least one of L¹ and L² is -OS(O₂)NH-, or -NHS(O₂)-O-; and the other is -O-P(O)(R^{c})-O-, -OS(O₂)NH-, or -NHS(O₂)-O-;
R¹ is hydrogen, fluoro, -OH, -NH₂, -OR^{b}, or -NHR^{b};
R² is hydrogen or fluoro;
R³ is hydrogen, or fluoro; R⁴ is hydrogen, fluoro, -OH, -NH₂, -OR^{b}, or -NHR^{b}; or R³ and R⁴ are taken together to form -CH₂O-;
R⁵ is hydrogen or fluoro;
R^{a} is C₁-C₄alkyl;
R^{b} is C₁-C₆alkyl, halo(C₁-C₆)alkyl, or C₃-C₆cycloalkyl;
R^{c} is -OH or -SH;
B¹ and B² are independently an optionally substituted 9- or 10-membered bicyclic heteroaryl ring containing 2-5 heteroatoms selected from N, O, or S; wherein B¹ and B² comprise at least two N atoms in the ring; and
B³ is an optionally substituted 5- or 6-membered monocyclic heteroaryl ring containing 1-4 heteroatoms selected from N, O, or S, or an optionally substituted 9- or 10-membered bicyclic heteroaryl ring containing 1-5 heteroatoms selected from N, O, or S; wherein B³ comprises at least one N atom in the ring, and wherein Y is attached to a carbon atom of B³.

The present disclosure also provides a pharmaceutical composition comprising (a) a compound of Formula (I) or a pharmaceutically acceptable salt thereof, and (b) a pharmaceutically acceptable carrier.

The present disclosure further provides a method of inducing an immune response in a subject, comprising administering to the subject in need thereof a therapeutically effective amount of a compound of Formula (I) or a pharmaceutically acceptable salt thereof.

The present disclosure provides a method of inducing STING-dependent type I interferon production in a subject, comprising administering to the subject in need thereof a therapeutically effective amount of a compound of Formula (I) or a pharmaceutically acceptable salt thereof.

The present disclosure provides a method of treating a cell proliferation disorder in a subject, comprising administering to the subject in need thereof a therapeutically effective amount of a compound of Formula (I) or a pharmaceutically acceptable salt thereof.

The present disclosure also provides a vaccine composition comprising an antigen and a compound of Formula (I) or a pharmaceutically acceptable salt thereof. The present disclosure further provides a method of treating a disease in a subject, comprising administering to the subject in need thereof a therapeutically effective amount of the vaccine composition.

### DETAILED DESCRIPTION OF THE DISCLOSURE

### Definitions

In the present specification, examples of the "halogen atom" include fluorine, chlorine, bromine and iodine.

As used herein, the term "aromatic" includes aryl and heteroaryl groups as described generally below and herein.

The term "aliphatic" or "aliphatic group," as used herein, means a straight-chain or branched C₁₋₁₂ hydrocarbon, or a cyclic C₁₋₁₂ hydrocarbon which is completely saturated or which contains one or more units of unsaturation, but which is not aromatic (also referred to herein as "carbocycle," "cycloaliphatic," "cycloalkyl," or "cycloalkenyl"). For example, suitable aliphatic groups include linear, branched or cyclic alkyl, alkenyl, alkynyl groups and hybrids thereof, such as (cycloalkyl)alkyl, (cycloalkenyl)alkyl, or (cycloalkyl)alkenyl. In various embodiments, aliphatic groups have 1-12, 1-10, 1-8, 1-6, 1-5, 1-4, 1-3, or 1-2 carbon atoms.

The terms "cycloaliphatic," "carbocycle," "carbocyclyl," "carbocyclo," or "carbocyclic," used alone or as part of a larger moiety, refer to a saturated or partially unsaturated cyclic aliphatic ring system having from 3 to about 14 ring carbon atoms.

The term "alkyl," used alone or as part of a larger moiety, refers to a straight or branched chain saturated C₁-C₁₂ hydrocarbon group. In various embodiments, alkyl groups can have 1-12, 1-10, 1-8, 1-6, 1-5, 1-4, 1-3, or 1-2 carbon atoms. Examples of the "C₁-C₆alkyl" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl and 2-ethylbutyl.

The term "cycloalkyl" refers to a saturated ring system of about 3 to about 10 ring carbon atoms. In various embodiments, alkyl groups can have 3-8, 3-7, 3-6, or 3-5 carbon atoms. Examples of the "C₃-C₆cycloalkyl" include cyclopropyl, cyclobutyl, cyclopentyl, and cyclohexyl.

The term "haloalkyl" refers to an alkyl group, as the case may be, which is substituted with one or more halogen atoms. As used herein, the term "halogen" or "halo" means F, Cl, Br, or I. In the present specification, examples of the "halo(C₁-C₆ )alkyl" include a C₁₋₆ alkyl group having 1 to 7, typically 1 to 5, halogen atoms. Specific examples thereof include chloromethyl, difluoromethyl, trichloromethyl, trifluoromethyl, 2-bromoethyl, 2,2,2-trifluoroethyl, tetrafluoroethyl, pentafluoroethyl, 2,2-difluoropropyl, 3,3,3-trifluoropropyl, 4,4,4-trifluorobutyl, 5,5,5-trifluoropentyl, and 6,6,6-trifluorohexyl.

The term "heteroatom" refers to one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2H-pyrrolyl), NH (as in pyrrolidinyl) or NR⁺ (as in N-substituted pyrrolidinyl)).

The terms "aryl" refers to a C₆₋₁₄ aromatic hydrocarbon moiety comprising one to three aromatic rings. In at least one embodiment, the aryl group is a C₆₋₁₀ aryl group. Aryl groups include, without limitation, optionally substituted phenyl, naphthyl, or anthracenyl. In certain embodiments, aryl groups can be optionally substituted as decribed herein. The term "aryl" as used herein, also includes groups in which an aryl ring is fused to one or more cycloaliphatic or heterocyclic rings to form an optionally substituted cyclic structure such as a tetrahydronaphthyl, indenyl, indanyl, or indolinyl ring. The term "aryl" may be used interchangeably with the terms "aryl group," "aryl ring," and "aromatic ring."

The terms "heteroaryl" and "heteroar-" refer to groups having 5 to 14 ring atoms, such as 5, 6, 9, or 10 ring atoms; having 6, 10, or 14 pi electrons shared in a cyclic array; and having, in addition to carbon atoms, from one to five heteroatoms. A heteroaryl group may be mono-, bi-, tri-, or polycyclic, for instance mono-, bi-, or tricyclic, such as mono- or bicyclic. In the context of "heteroar" entities, the term "heteroatom" refers to nitrogen, oxygen, or sulfur, and includes any oxidized form of nitrogen or sulfur, and any quaternized form of a basic nitrogen. For example, a nitrogen atom of a heteroaryl may be a basic nitrogen atom and may also be optionally oxidized to the corresponding N-oxide. When a heteroaryl is substituted by a hydroxy group, it also includes its corresponding tautomer. The terms "heteroaryl" and "heteroar-," as used herein, also include groups in which a heteroaromatic ring is fused to one or more aryl, cycloaliphatic, or heterocycloaliphatic rings. Nonlimiting examples of heteroaryl groups include thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolizinyl, purinyl, naphthyridinyl, pteridinyl, indolyl, isoindolyl, benzothienyl, benzofuranyl, dibenzofuranyl, indazolyl, benzimidazolyl, benzthiazolyl, quinolyl, isoquinolyl, cinnolinyl, phthalazinyl, quinazolinyl, quinoxalinyl, 4H-quinolizinyl, carbazolyl, acridinyl, phenazinyl, phenothiazinyl, phenoxazinyl, tetrahydroquinolinyl, tetrahydroisoquinolinyl, and pyrido[2,3-b]-1,4-oxazin-3(4H)-one. The term "heteroaryl" may be used interchangeably with the terms "heteroaryl ring," "heteroaryl group," or "heteroaromatic." In certain embodiments, heteroaryl groups can be optionally substituted as described herein.

As used herein, the terms "heterocycle," "heterocyclyl," "heterocyclic radical," and "heterocyclic ring" are used interchangeably and refer to a stable 3- to 8-membered monocyclic or 7-10-membered bicyclic heterocyclic moiety that is either saturated or partially unsaturated, and having, in addition to carbon atoms, one or more, for instance one to four, heteroatoms, as defined above. When used in reference to a ring atom of a heterocycle, the term "nitrogen" includes a substituted nitrogen. As an example, in a saturated or partially unsaturated ring having 0-3 heteroatoms selected from oxygen, sulfur or nitrogen, the nitrogen may be N (as in 3,4-dihydro-2H-pyrrolyl), NH (as in pyrrolidinyl), or NR⁺ (as in N-substituted pyrrolidinyl).

A heterocyclic ring can be attached to its pendant group at any heteroatom or carbon atom that results in a stable structure and any of the ring atoms can be optionally substituted. Examples of such saturated or partially unsaturated heterocyclic radicals include, without limitation, tetrahydrofuranyl, tetrahydrothienyl, piperidinyl, decahydroquinolinyl, oxazolidinyl, piperazinyl, dioxanyl, dioxolanyl, diazepinyl, oxazepinyl, thiazepinyl, morpholinyl, and thiamorpholinyl. A heterocyclyl group may be mono-, bi-, tri-, or polycyclic, for instance mono-, bi-, or tricyclic, and such as mono- or bicyclic. The term "heterocyclylalkyl" refers to an alkyl group substituted by a heterocyclyl, and the alkyl and heterocyclyl portions can independently be optionally substituted. Additionally, a heterocyclic ring also includes groups in which the heterocyclic ring is fused to one or more aryl rings.

As used herein, the term "partially unsaturated" refers to a ring moiety that includes at least one double or triple bond between ring atoms. The term "partially unsaturated" is intended to encompass rings having multiple sites of unsaturation, but is not intended to include aromatic (e.g., aryl or heteroaryl) moieties, as herein defined. The term "partially unsaturated" is intended to encompass rings having a tautomer that includes at least one double bond or triple bond between ring atoms. For example, a ring including a carbonyl group exists as an enol tautomer and thus is also considered as "partially unstaturated."

An aryl (including aralkyl, aralkoxy, aryloxyalkyl and the like) or heteroaryl (including heteroaralkyl and heteroarylalkoxy and the like) group can contain one or more substituents and thus can be "optionally substituted." In addition to the substituents defined above and herein, suitable substituents on the unsaturated carbon atom of an aryl or heteroaryl group also include and are generally selected from -halo, -NO₂, -CN, -R^{#}, -C(R^{#})=C(R^{#})₂, -C≡C-R^{#}, -OR^{#}, -SR°, -S(O)R°, -SO₂R°, -SO₃R^{#}, -SO₂N(R^{#})₂, -N(R^{#})₂, -NR^{#}C(O)R^{#}, -NR^{#}C(S)R^{#}, -NR^{#}C(O)N(R^{#})₂, -NR^{#}C(S)N(R^{#})₂, -N(R^{#})C(=NR^{#})-N(R^{#})₂, -N(R^{#})C(=NR^{#})-R°, -NR^{#}CO₂R^{#}, -NR^{#}SO₂R°, -NR^{#}SO₂N(R^{#})₂, -O-C(O)R^{#}, -O-CO₂R^{#}, -OC(O)N(R^{#})₂, -C(O)R^{#}, -C(S)R°, -CO₂R^{#}, -C(O)-C(O)R^{#}, -C(O)N(R^{#})₂, -C(S)N(R^{#})₂, -C(O)N(R^{#})-OR^{#}, -C(O)N(R^{#})C(=NR^{#})-N(R^{#})₂, -N(R^{#})C(=NR^{#})-N(R^{#})-C(O)R^{#}, -C(=NR^{#})-N(R^{#})₂, -C(=NR^{#})-OR^{#}, -N(R^{#})-N(R^{#})₂, -C(=NR^{#})-N(R^{#})-OR^{#}, -C(R°)=N-OR^{#}, -P(O)(R^{#})₂, -P(O)(OR^{#})₂, -O-P(O)-OR^{#}, and -P(O)(NR^{#})-N(R^{#})₂, wherein R^{#}, independently, is hydrogen or an optionally substituted aliphatic, aryl, heteroaryl, cycloaliphatic, or heterocyclyl group, or two independent occurrences of R^{#} are taken together with their intervening atom(s) to form an optionally substituted 5-7-membered aryl, heteroaryl, cycloaliphatic, or heterocyclyl. In some embodiments, R^{#}, independently, is hydrogen , C₁₋₆ aliphatic, or C₃₋₆ cycloaliphatic. Each R° is, independently, an optionally substituted aliphatic, aryl, heteroaryl, cycloaliphatic, or heterocyclyl group.

An aliphatic or heteroaliphatic group, or a non-aromatic carbocyclic or heterocyclic ring can contain one or more substituents and thus can be "optionally substituted." Unless otherwise defined above and herein, suitable substituents on the saturated carbon of an aliphatic or heteroaliphatic group, or of a non-aromatic carbocyclic or heterocyclic ring are selected from those listed above for the unsaturated carbon of an aryl or heteroaryl group and additionally include the following: =O, =S, =C(R^{∗})₂, =N-N(R^{∗})₂, =N-OR^{∗}, =N-NHC(O)R^{∗}, =N-NHCO₂R° =N-NHSO₂R° or =N-R* where R° is defined above, and each R* is independently selected from hydrogen or an optionally substituted C₁₋₆ aliphatic group.

In addition to the substituents defined above and herein, optional substituents on the nitrogen of a non-aromatic heterocyclic ring also include and are generally selected from -R^{#}, -N(R^{#})₂, -C(O)R^{#}, -C(O)OR^{#}, -C(O)C(O)R^{#}, -C(O)CH₂C(O)R^{#}, -S(O)₂R^{#}, -S(O)₂N(R^{#})₂, -C(S)N(R^{#})₂, -C(=NH)-N(R^{#})₂, or -N(R^{#})S(O)₂R^{#}; wherein each R^{#} is defined above. A ring nitrogen atom of a heteroaryl or non-aromatic heterocyclic ring also may be oxidized to form the corresponding N-hydroxy or N-oxide compound. A nonlimiting example of such a heteroaryl having an oxidized ring nitrogen atom is N-oxidopyridyl.

As detailed above, in some embodiments, two independent occurrences of R^{#} (or any other variable similarly defined in the specification and claims herein), are taken together with their intervening atom(s) to form a monocyclic or bicyclic ring selected from 3-13-membered cycloaliphatic, 3-12-membered heterocyclyl having 1-5 heteroatoms independently selected from nitrogen, oxygen, or sulfur, 6-10-membered aryl, or 5-10-membered heteroaryl having 1-5 heteroatoms independently selected from nitrogen, oxygen, or sulfur.

For purposes of clarity, all bivalent groups described herein are intended to be read from left to right, with a corresponding left-to-right reading of the formula or structure in which the variable appears.

Exemplary rings that are formed when two independent occurrences of R^{#} (or any other variable similarly defined in the specification and claims herein), are taken together with their intervening atom(s) include, but are not limited to the following: a) two independent occurrences of R^{#} (or any other variable similarly defined in the specification or claims herein) that are bound to the same atom and are taken together with that atom to form a ring, for example, N(R^{#})₂, where both occurrences of R^{#} are taken together with the nitrogen atom to form a piperidin-1-yl, piperazin-1-yl, or morpholin-4-yl group; and b) two independent occurrences of R^{#} (or any other variable similarly defined in the specification or claims herein) that are bound to different atoms and are taken together with both of those atoms to form a ring, for example where a phenyl group is substituted with two occurrences of OR^{#} 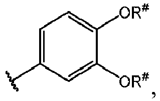 these two occurrences of R^{#} are taken together with the oxygen atoms to which they are bound to form a fused 6-membered oxygen containing ring: 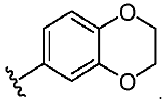 It will be appreciated that a variety of other rings (e.g., spiro and bridged rings) can be formed when two independent occurrences of R^{#} (or any other variable similarly defined in the specification and claims herein) are taken together with their intervening atom(s) and that the examples detailed above are not intended to be limiting.

Unless otherwise stated, structures depicted herein are also meant to include all stereoisomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the present disclosure. Unless otherwise stated, all tautomeric forms of the compounds disclosed herein are within the scope of the present disclosure. Additionally, unless otherwise stated, structures depicted herein are also meant to include compounds that differ only in the presence of one or more isotopically enriched atoms. For example, compounds having the present structures where there is a replacement of hydrogen by deuterium or tritium, or a replacement of a carbon by a ¹³C- or ¹⁴C-enriched carbon are within the scope of this disclosure. Such compounds are useful, as a nonlimiting example, as analytical tools or probes in biological assays.

It is to be understood that, when a disclosed compound has at least one chiral center, the present disclosure encompasses one enantiomer free or substantially free from the corresponding optical isomer, a racemic mixture of the compound, and mixtures enriched in one enantiomer relative to its corresponding optical isomer. When a mixture is enriched in one enantiomer relative to its optical isomers, the mixture contains, for example, an enantiomeric excess of at least 50%, 75%, 80%, 85%, 90%, 95%, 99%, or 99.5%.

The enantiomers of the present disclosure may be resolved by methods known to those skilled in the art, for example by formation of diastereoisomeric salts which may be separated, for example, by crystallization; formation of diastereoisomeric derivatives or complexes which may be separated, for example, by crystallization, gas-liquid or liquid chromatography; selective reaction of one enantiomer with an enantiomer-specific reagent, for example enzymatic esterification; or gas-liquid or liquid chromatography in a chiral environment, for example on a chiral support for example silica with a bound chiral ligand or in the presence of a chiral solvent. Where the desired enantiomer is converted into another compound by one of the separation procedures described above, a further step is required to liberate the desired enantiomeric form. Alternatively, specific enantiomers may be synthesized by asymmetric synthesis using optically active reagents, substrates, catalysts or solvents, or by converting one enantiomer into the other by asymmetric transformation.

When a disclosed compound has at least two chiral centers, the present disclosure encompasses a diastereomer free or substantially free of other diastereomers, a pair of diastereomers free or substantially free from other diasteromeric pairs, mixtures of diasteromers, mixtures of diasteromeric pairs, mixtures of diasteromers in which one diastereomer is enriched relative to the other diastereomer(s) and mixtures of diasteromeric pairs in which one diastereomeric pair is enriched relative to the other diastereomeric pair(s). When a mixture is enriched in one diastereomer or diastereomeric pair(s) relative to the other diastereomers or diastereomeric pair(s), the mixture is enriched with the depicted or referenced diastereomer or diastereomeric pair(s) relative to other diastereomers or diastereomeric pair(s) for the compound, for example, by a molar excess of at least 50%, 75%, 80%, 85%, 90%, 95%, 99% or 99.5%.

The diastereoisomeric pairs may be separated by methods known to those skilled in the art, for example chromatography or crystallization and the individual enantiomers within each pair may be separated as described above. Specific procedures for chromatographically separating diastereomeric pairs of precursors used in the preparation of compounds disclosed herein are provided the examples herein.

### Compounds of Formula (I)

The present disclosure provides a compound of Formula (I): 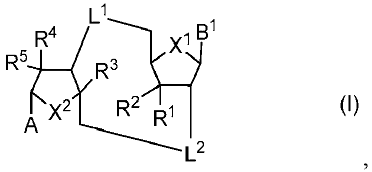 or a pharmaceutically acceptable salt thereof, wherein:
A is B² or -Y-B³, wherein Y is -O-, -S-, -NH-, or -NR^{a}-;
X¹ and X² are independently -O-, -S-, or -CH₂-;
at least one of L¹ and L² is -OS(O₂)NH-, or -NHS(O₂)-O-; and the other is -O-P(O)(R^{c})-O-, -OS(O₂)NH-, or -NHS(O₂)-O-;
R¹ is hydrogen, fluoro, -OH, -NH₂, -OR^{b}, or -NHR^{b};
R² is hydrogen or fluoro;
R³ is hydrogen or fluoro; R⁴ is hydrogen, fluoro, -OH, -NH₂, -OR^{b}, or -NHR^{b}; or R³ and R⁴ are taken together to form -CH₂O-;
R⁵ is hydrogen or fluoro;
R^{a} is C₁-C₄alkyl;
R^{b} is C₁-C₆alkyl, halo(C₁-C₆)alkyl, or C₃-C₆cycloalkyl;
R^{c} is -OH or -SH;
B¹ and B² are independently an optionally substituted 9- or 10-membered bicyclic heteroaryl ring containing 2-5 heteroatoms selected from N, O, or S; wherein B¹ and B² comprise at least two N atoms in the ring; and
B³ is an optionally substituted 5- or 6-membered monocyclic heteroaryl ring containing 1-4 heteroatoms selected from N, O, or S, or an optionally substituted 9- or 10-membered bicyclic heteroaryl ring containing 1-5 heteroatoms selected from N, O, or S; wherein B³ comprises at least one N atom in the ring, and wherein Y is attached to a carbon atom of B³.

In some embodiments, the compound of Formula (I) is represented by Formula (I-A), (I-B), (I-C), or (I-D): 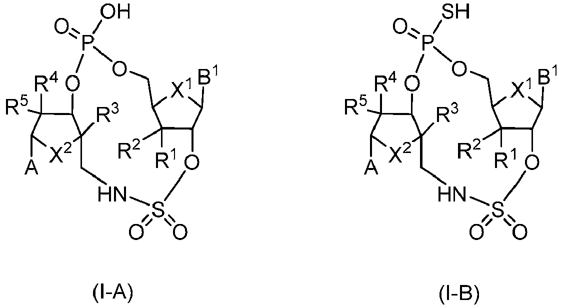 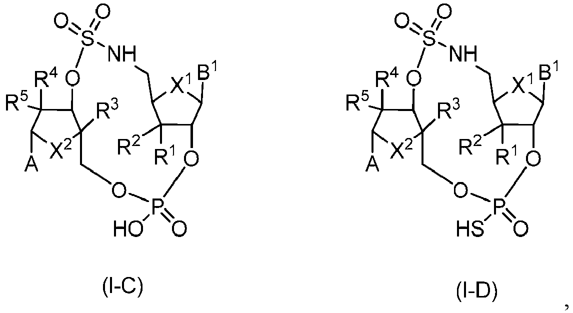 wherein X¹, X², A, B¹, R¹, R², R³, R⁴, and R⁵ have the values described herein.

In some embodiments, the compound of Formula (I) is represented by Formula (I-E), (I-F), (I-G), or (I-H): 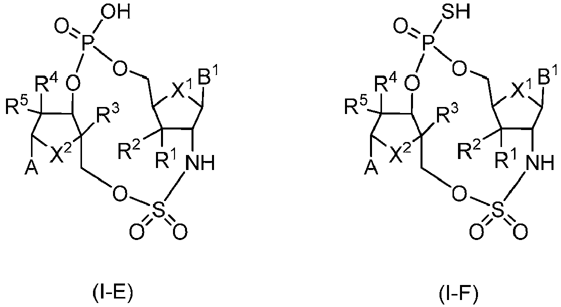 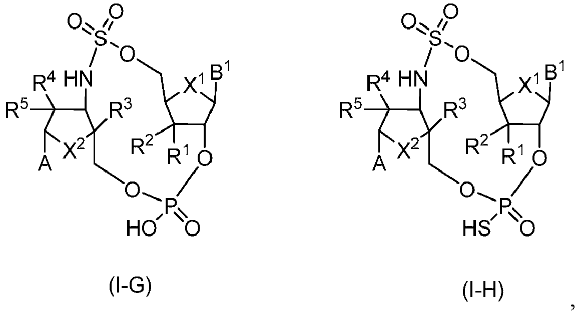 wherein X¹, X², A, B¹, R¹, R², R³, R⁴, and R⁵ have the values described herein.

In some embodiments, the compound of Formula (I) is represented by Formula (II): 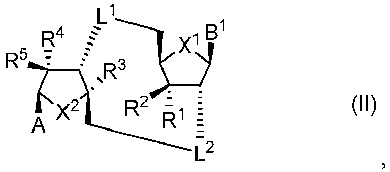 or a pharmaceutically acceptable salt thereof, wherein
A is B² or -Y-B³, wherein Y is -O-, -S-, -NH-, or -NR^{a}-;
X¹ and X² are independently -O-, -S-, or -CH₂-;
at least one of L¹ and L² is -OS(O₂)NH-, or -NHS(O₂)-O-; and the other is -O-P(O)(R^{c})-O-, -OS(O₂)NH-, or -NHS(O₂)-O-;
R¹ is hydrogen, fluoro, -OH, -NH₂, -OR^{b}, or -NHR^{b};
R² is hydrogen or fluoro;
R³ is hydrogen or fluoro; R⁴ is hydrogen, fluoro, -OH, -NH₂, -OR^{b}, or -NHR^{b}; or R³ and R⁴ are taken together to form -CH₂O-;
R⁵ is hydrogen or fluoro;
R^{a} is C₁-C₄alkyl;
R^{b} is C₁-C₆alkyl, halo(C₁-C₆)alkyl, or C₃-C₆cycloalkyl;
R^{c} is -OH or -SH;
B¹ and B² are independently an optionally substituted 9- or 10-membered bicyclic heteroaryl ring containing 2-5 heteroatoms selected from N, O, or S; wherein B¹ and B² comprise at least two N atoms in the ring; and
B³ is an optionally substituted 5- or 6-membered monocyclic heteroaryl ring containing 1-4 heteroatoms selected from N, O, or S, or an optionally substituted 9- or 10-membered bicyclic heteroaryl ring containing 1-5 heteroatoms selected from N, O, or S; wherein B³ comprises at least one N atom in the ring, and wherein Y is attached to a carbon atom of B³.

In some embodiments, the compound of Formula (I) is represented by Formula (II-A), (II-B), (II-C), or (II-D): 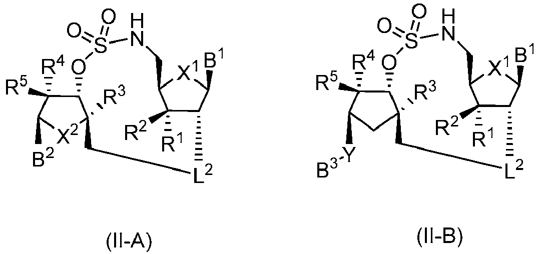 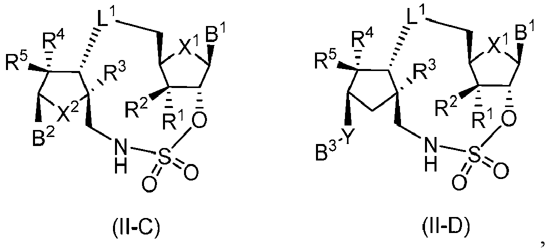 wherein X¹, X², Y, B¹, B², B³, L¹, L², R¹, R², R³, R⁴, and R⁵ have the values described herein. In some embodiments, L¹ or L² is -O-P(O)(SH)-O- or -O-P(O)(OH)-O-. In some embodiments, L¹ or L² is -O-P(O)(SH)-O-. In some embodiments, L¹ or L² is -O-P(O)(OH)-O-. In some embodiments, Y is -O- or -NH-.

In some embodiments, the compound of Formula (I) is represented by Formula (II-E), (II-F), (II-G), or (II-H): 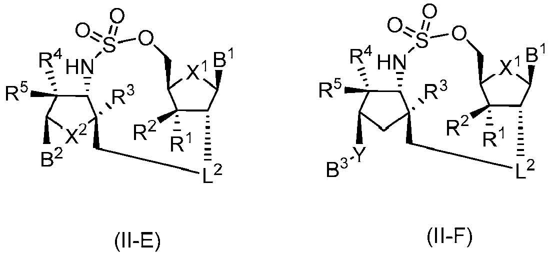 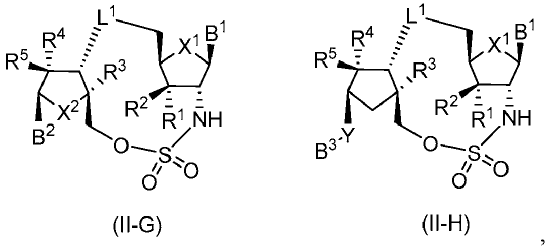 wherein X¹, X², Y, B¹, B², B³, L¹, L², R¹, R², R³, R⁴, and R⁵ have the values described herein. In some embodiments, L¹ or L² is -O-P(O)(SH)-O- or -O-P(O)(OH)-O-. In some embodiments, L¹ or L² is -O-P(O)(SH)-O-. In some embodiments, L¹ or L² is -O-P(O)(OH)-O-. In some embodiments, Y is -O- or -NH-.

In some embodiments, the compound of Formula (I) is represented by Formula (III): 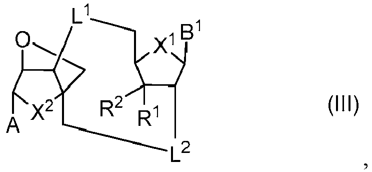 or a pharmaceutically acceptable salt thereof, wherein
A is B² or -Y-B³, wherein Y is -O-, -S-, -NH-, or -NR^{a}-;
X¹ and X² are independently -O-, -S-, or -CH₂-;
at least one of L¹ and L² is -OS(O₂)NH-, or -NHS(O₂)-O-; and the other is -O-P(O)(R^{c})-O-, -OS(O₂)NH-, or -NHS(O₂)-O-;
R¹ is hydrogen, fluoro, -OH, -NH₂, -OR^{b}, or -NHR^{b};
R² is hydrogen or fluoro;
R^{a} is C₁-C₄alkyl;
R^{b} is C₁-C₆alkyl, halo(C₁-C₆)alkyl, or C₃-C₆cycloalkyl;
R^{c} is -OH or -SH;
B¹ and B² are independently an optionally substituted 9- or 10-membered bicyclic heteroaryl ring containing 2-5 heteroatoms selected from N, O, or S; wherein B¹ and B² comprise at least two N atoms in the ring; and
B³ is an optionally substituted 5- or 6-membered monocyclic heteroaryl ring containing 1-4 heteroatoms selected from N, O, or S, or an optionally substituted 9- or 10-membered bicyclic heteroaryl ring containing 1-5 heteroatoms selected from N, O, or S; wherein B³ comprises at least one N atom in the ring, and wherein Y is attached to a carbon atom of B³.

In some embodiments, the compound of Formula (I) is represented by Formula (III-A): 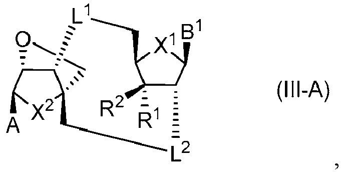 wherein X¹, X², A, B¹, L¹, L², R¹, and R² have the values described herein.

In some embodiments, the compound of Formula (I) is represented by Formula (IV-A), (IV-B), (IV-C), or (IV-D): 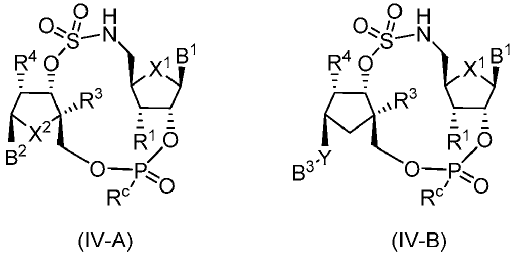 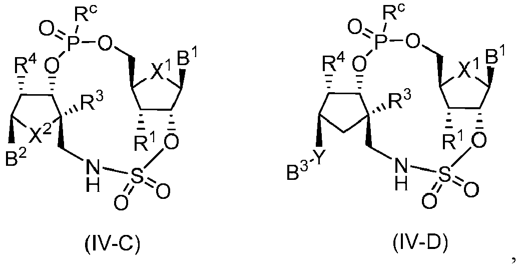 wherein X¹, X², Y, B¹, B², B³, R¹, R³, R⁴, and R^{c} have the values described herein. In some embodiments, X¹ is -O- or -CH₂-. In some embodiments, X² is -O- or -CH₂-. In some embodiments, Y is -O- or -NH-. In some embodiments, R¹ and R⁴ are each independently hydrogen, fluoro, or -OH. In some embodiments, R¹ and R⁴ are each independently hydrogen, fluoro, -OH, or -OCH₂CF₃. In some embodiments, R^{c} is -SH or -OH. In some embodiments, R^{c} is -SH. In some embodiments, R^{c} is -OH.

In some embodiments, the compound of Formula (I) is represented by Formula (IV-C) or (IV-D): 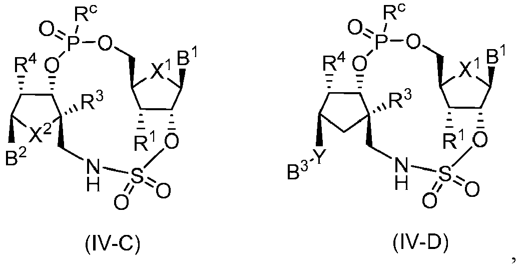 wherein X¹, X², Y, B¹, B², B³, R¹, R³, R⁴, and R^{c} have the values described herein. In some embodiments, X¹ is -O- or -CH₂-. In some embodiments, X² is -O- or -CH₂-. In some embodiments, Y is -O- or -NH-. In some embodiments, R¹ and R⁴ are each independently hydrogen, fluoro, or -OH. In some embodiments, R¹ and R⁴ are each independently hydrogen, fluoro, -OH, or -OCH₂CF₃. In some embodiments, R^{c} is -SH or -OH. In some embodiments, R^{c} is -SH. In some embodiments, R^{c} is -OH.

In some embodiments, the compound of Formula (I) is represented by Formula (IV-E) or (IV-F): 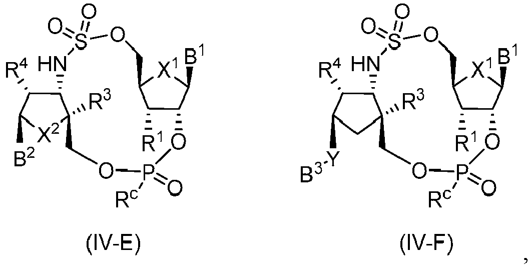 wherein X¹, X², Y, B¹, B², B³, R¹, R³, R⁴, and R^{c} have the values described herein. In some embodiments, X¹ is -O- or -CH₂-. In some embodiments, X² is -O- or -CH₂-. In some embodiments, Y is -O- or -NH-. In some embodiments, R¹ and R⁴ are each independently hydrogen, fluoro, or -OH. In some embodiments, R¹ and R⁴ are each independently hydrogen, fluoro, -OH, or -OCH₂CF₃. In some embodiments, R^{c} is -SH or -OH. In some embodiments, R^{c} is -SH. In some embodiments, R^{c} is -OH.

In some embodiments, the compound of Formula (I) is represented by Formula (V-A), (V-B), (V-C), or (V-D): 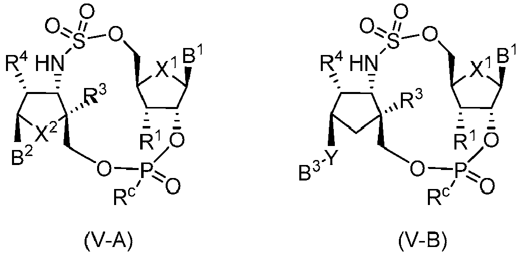 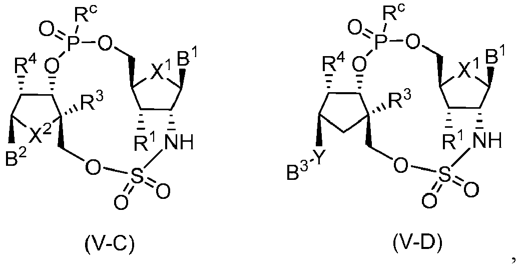 wherein X¹, X², Y, B¹, B², B³, R¹, R³, R⁴, and R^{c} have the values described herein. In some embodiments, X¹ is -O- or -CH₂-. In some embodiments, X² is -O- or -CH₂-. In some embodiments, Y is -O- or -NH-. In some embodiments, R¹ and R⁴ are each independently hydrogen, fluoro, or -OH. In some embodiments, R¹ and R⁴ are each independently hydrogen, fluoro, -OH, or -OCH₂CF₃. In some embodiments, R^{c} is -SH or -OH. In some embodiments, R^{c} is -SH. In some embodiments, R^{c} is -OH.

In some embodiments, the compound of Formula (I) is represented by Formula (VI): 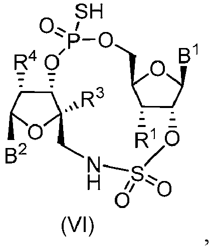 wherein B¹, B², R¹, R³, and R⁴ have the values described herein. In some embodiments, R⁴ and R³ are hydrogen, and R⁴ is fluoro. In some embodiments, R³ is hydrogen, and R¹ and R⁴ are fluoro.

In some embodiments, the compound of Formula (I) is represented by Formula (VII-A), (VII-B), (VII-C), or (VII-D): 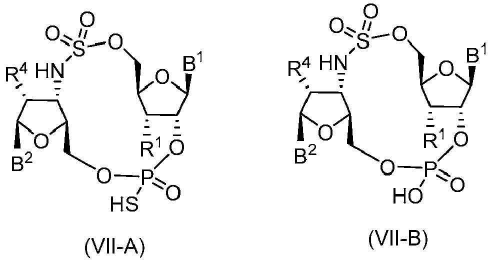 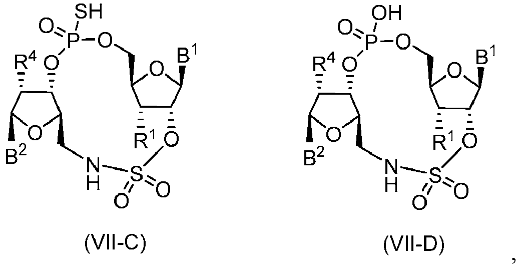 wherein B¹, B², R¹, R³, and R⁴ have the values described herein. In some embodiments, R¹ is hydrogen, fluoro, or -OR^{b}. In some embodiments, R¹ is hydrogen, fluoro, or -OCH₂CF₃. In some embodiments, R⁴ is hydrogen or fluoro. In some embodiments, R⁴ is fluoro.

The following embodiments apply to any of formulas (I), (I-A), (I-B), (I-C), (I-D), (I-E), (I-F), (I-G), (I-H), (II), (II-A), (II-B), (II-C), (II-D), (II-E), (II-F), (II-G), (II-H), (III), (III-A), (IV-A), (IV-B), (IV-C), (IV-D), (IV-E), (IV-F), (V-A), (V-B), (V-C), (V-D), (VI), (VII-A), (VII-B), (VII-C), and (VII-D).

In some embodiments, at least one of L¹ and L² is -OS(O₂)NH-, or -NHS(O₂)-O-; and the other is -O-P(O)(OH)-O- or -O-P(O)(SH)-O-. In some embodiments, when L¹ or L² is -O-P(O)(SH)-O-, the phosphorus atom of each phosphorothioate linkage is chiral and is independently *R*ₚ or *S*ₚ. In some embodiments, the phosphorus atom of each phosphorothioate linkage is *R*ₚ.

In some embodiments, X¹ and X² are independently -O-, -S-, or -CH₂-. In some embodiments, X¹ and X² are independently -O- or -CH₂-. In some embodiments, X¹ and X² are both -O-. In some embodiments, X¹ is -O- and X² is -CH₂-.

In some embodiments, Y is -O-, -S-, -NH-, or -NR^{a}-, wherein R^{a} is C₁-C₄alkyl. In some embodiments, Y is -O-. In some embodiments, Y is -NH-. In some embodiments, Y is -NMe-.

In some embodiments, R¹ is hydrogen, fluoro, -OH, -NH₂, -OR^{b}, or -NHR^{b}, wherein R^{b} is C₁-C₆alkyl, halo(C₁-C₆)alkyl, or C₃-C₆cycloalkyl. In some embodiments, R¹ is hydrogen, fluoro, -OH, or -OR^{b}. In some embodiments, R¹ is hydrogen. In some embodiments, R¹ is fluoro. In some embodiments, R¹ is -OH. In some embodiments, R¹ is -OR^{b}, wherein R^{b} is C₁-C₆alkyl or halo(C₁-C₆)alkyl. In some embodiments, R¹ is -OCH₃, -OCF₃, -OCH₂CH₃, or OCH₂CF₃.

In some embodiments, R² is hydrogen, or fluoro. In some embodiments, R² is hydrogen. In some embodiments, R² is fluoro.

In some embodiments, R¹ and R² are both hydrogen. In some embodiments, R¹ and R² are both fluoro. In some embodiments, R¹ is -OH and R² is hydrogen. In some embodiments, R¹ is fluoro and R² is hydrogen.

In some embodiments, R³ is hydrogen or fluoro; R⁴ is hydrogen, fluoro, -OH, -NH₂, -OR^{b}, or -NHR^{b}, wherein R^{b} is C₁-C₆alkyl, halo(C₁-C₆)alkyl, or C₃-C₆cycloalkyl. In some embodiments, R³ and R⁴ are taken together to form -CH₂O-. In some embodiments, R³ and R⁴ are taken together to form -CH₂O-, wherein the carbon atom of -CH₂O- is directly connected to the carbon atom to which R³ is directly connected.

In some embodiments, R³ and R⁴ are both hydrogen. In some embodiments, R³ is hydrogen and R⁴ is fluoro. In some embodiments, R³ is hydrogen and R⁴ is -OH. In some embodiments, R³ is fluoro and R⁴ is hydrogen. In some embodiments, R³ and R⁴ are both fluoro. In some embodiments, R³ is fluoro and R⁴ is -OH.

In some embodiments, R⁵ is hydrogen or fluoro. In some embodiments, R⁵ is hydrogen. In some embodiments, R⁵ is fluoro.

In some embodiments, R³, R⁴, R⁵ are all hydrogen. In some embodiments, R³ is hydrogen, R⁴ is fluoro, and R⁵ is hydrogen. In some embodiments, R³ is hydrogen, R⁴ is -OH, and R⁵ is hydrogen. In some embodiments, R³ is hydrogen, R⁴ is hydrogen, and R⁵ is fluoro. In some embodiments, R³ is hydrogen, and both R⁴ and R⁵ are fluoro. In some embodiments, R³ is fluoro, and both R⁴ and R⁵ are hydrogen. In some embodiments, both R³ and R⁴ are fluoro, and R⁵ is hydrogen. In some embodiments, R³ is fluoro, R⁴ is -OH, and R⁵ is hydrogen. In some embodiments, R⁴ is hydrogen, and both R³ and R⁵ are fluoro. In some embodiments, R³, R⁴, and R⁵ are all fluoro.

In some embodiments, R¹, R², R³, and R⁵ are hydrogen, and R⁴ is fluoro. In some embodiments, R², R³, and R⁵ are hydrogen, and R¹ and R⁴ are fluoro.

In some embodiments, A is B² or -Y-B³, wherein Y is -O-, -S-, -NH-, or -NR^{a}-, and R^{a} is C₁-C₄alkyl. B² and B³ have the values described herein. In some embodiments, A is - Y-B³, and B³ is an optionally substituted 5- or 6-membered monocyclic heteroaryl ring containing 1-4 heteroatoms selected from N, O, or S, or an optionally substituted 9- or 10-membered bicyclic heteroaryl ring containing 1-5 heteroatoms selected from N, O, or S; wherein B³ comprises at least one N atom in the ring, and wherein Y is attached to a carbon atom of B³.

In some embodiments, B³ is an optionally substituted 6-membered monocyclic heteroaryl ring containing 1-3 N atoms. In some embodiments, B³ is an optionally substituted 6-membered monocyclic heteroaryl ring containing one N atom, such pyridinyl. In some embodiments, B³ is an optionally substituted 6-membered monocyclic heteroaryl ring containing two N atoms, such as pyridazinyl, pyrimidinyl, and pyrazinyl. In some embodiments, B³ is an optionally substituted 6-membered monocyclic heteroaryl ring containing three N atoms, such as 1,2,4-triazinyl, 1,3,5-triazinyl, thyminyl, and uracilyl.

In some embodiments, B³ is 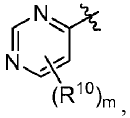 wherein:
each R¹⁰ is independently hydrogen, halogen, -OH, -NH₂, C₁-C₆alkyl, halo(C₁-C₆)alkyl, C₃-C₆cycloalkyl, -OR¹¹, -NHR¹¹, -CN, -NO₂, or -C(O)NHR¹²;
R¹¹ is C₁-C₆alkyl, halo(C₁-C₆)alkyl, or C₃-C₆cycloalkyl;
R¹² is hydrogen, C₁-C₆alkyl, halo(C₁-C₆)alkyl, or C₃-C₆cycloalkyl; and
m is 0, 1, or 2.

In some embodiments, each R¹⁰ is independently hydrogen, fluoro, chloro, -OH, -NH₂, -CH₃, -CF₃, -OCH₃, -CN, -NO₂, or -C(O)NH₂.

In some embodiments, each R¹⁰ is independently hydrogen, fluoro, chloro, -CN, or C₁-C₃alkyl.

In some embodiments, R¹⁰ is fluoro. In some embodiments, R¹⁰ is chloro. In some embodiments, R¹⁰ is -CN. In some embodiments, R¹⁰ is -CH₃. In some embodiments, R¹⁰ is -C(O)NH₂.

In some embodiments, B³ is 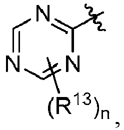 wherein:
each R¹³ is independently hydrogen, halogen, -OH, -NH₂, C₁-C₆alkyl, halo(C₁-C₆)alkyl, C₃-C₆cycloalkyl, -OR¹¹, -NHR¹¹, -CN, -NO₂, or -C(O)NHR¹²;
R¹¹ is C₁-C₆alkyl, halo(C₁-C₆)alkyl, or C₃-C₆cycloalkyl;
R¹² is hydrogen, C₁-C₆alkyl, halo(C₁-C₆)alkyl, or C₃-C₆cycloalkyl; and
n is 0, 1, or 2.

In some embodiments, each R¹³ is independently hydrogen, fluoro, chloro, -OH, -NH₂, -CH₃, -CF₃, -OCH₃, -CN, -NO₂, or -C(O)NH₂.

Examples of optionally substituted 6-membered monocyclic heteroaryl ring include, but not limited to the following:

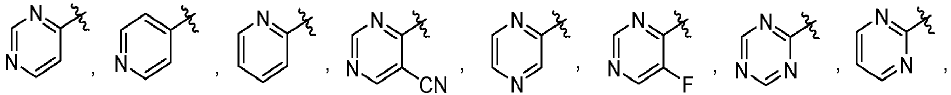

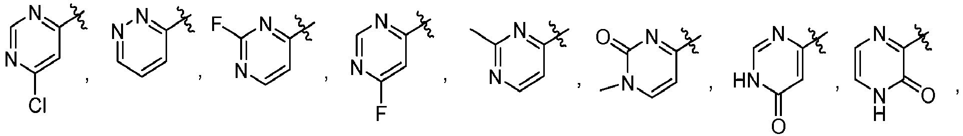

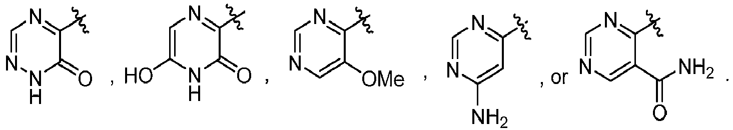

In some embodiments, B³ is an optionally substituted 5-membered monocyclic heteroaryl ring containing 1-4 heteroatoms selected from N, O, or S. Examples of 5-membered monocyclic heteroaryl rings include but not limited to pyrrolyl, furanyl, thiophenyl, oxazolyl, pyrazolyl, isoxazolyl, thiazolyl, isothiazolyl, imidazolyl, 1,2,3-triazolyl, 1,2,4-triazolyl, tetrazolyl, 1,2,5-oxadizaolyl, 1,2,3,-oxadizaolyl, 1,3,4-thiadiazolyl, and 1,2,5-thiadizaolyl.

In some embodiments, B³ is an optionally substituted 9-or 10-membered bicyclic heteroaryl ring containing 1-5 heteroatoms selected from N, O, or S. In some embodiments, B³ is an optionally substituted 9-membered bicyclic heteroaryl ring containing at least 2 N atoms. In some embodiments, B³ is an optionally substituted 9-membered bicyclic heteroaryl ring that comprises a pyrimidine ring.

Examples of optionally substituted 9-membered bicyclic heteroaryl rings include, but not limited to the following:

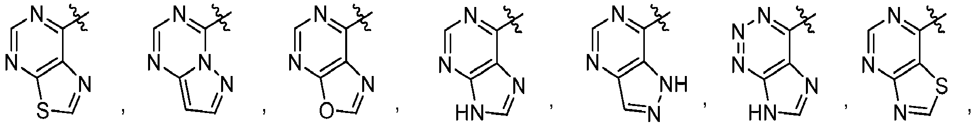

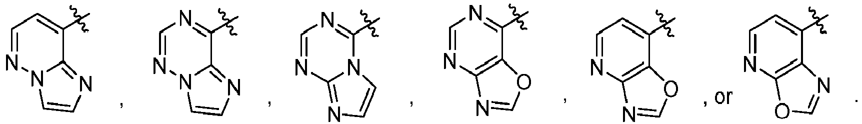

In some embodiments, B³ can comprise a 6-membered monocyclic heteroaryl ring (e.g., a pyrimidine ring) fused to a non-aromatic ring, such as:

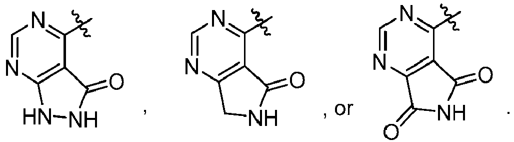

In some embodiments, B¹ and B² are independently an optionally substituted 9- or 10-membered bicyclic heteroaryl ring containing 2-5 heteroatoms selected from N, O, or S. In some embodiments, B¹ and B² comprise at least two N atoms in the ring. For example, WO2017/027645 and WO2017/027646 describe various 9-membered bicyclic heteroaryl rings. WO2017/027645 and WO2017/027646 are incorporated herein by reference in their entitirety.

In some embodiments, B¹ and B² are independently an optionally substituted 9-membered bicyclic heteroaryl ring containing 3-5 N atoms. In some embodiments, the X¹-containing 5-membered ring is attached to a nitrogen atom or a carbon atom of B¹. In some embodiments, the X²-containing 5-membered ring is attached to a nitrogen atom or a carbon atom of B².

In some embodiments, B¹ and B² are independently: 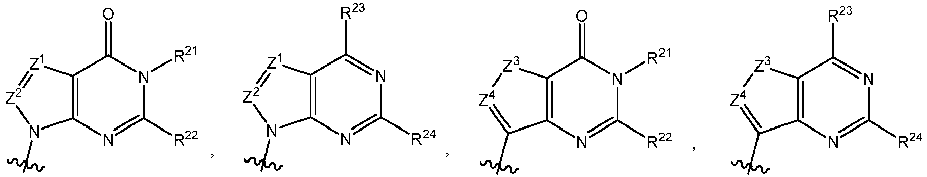 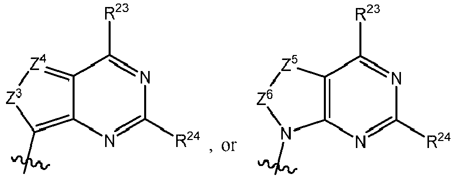 wherein:
Z¹, Z², and Z⁴ are each independently N or CR²⁰;
Z³ is NR²¹ or CHR²⁰;
Z⁵ and Z⁶ are each independently C(O) or NR²¹;
R²¹ is hydrogen or C₁-C₆alkyl, halo(C₁-C₆)alkyl, or C₃-C₆cycloalkyl;
R²³ is hydrogen or -NH₂; and
R²⁰, R²², and R²⁴ are each independently hydrogen, halogen, -OH, -NH₂, -CN, C₁-C₆alkyl, halo(C₁-C₆)alkyl, or C₃-C₆cycloalkyl.

In some embodiments, Z¹, Z², and Z⁴ are each independently N, CH, or CF. In some embodiments, Z³ is NH or NCH₃. In some embodiments, one of Z⁵ and Z⁶ is C(O) and the other is NH.

In some embodiments, R²¹ is hydrogen or -CH₃. In some embodiments, R²² and R²⁴ are each independently hydrogen, -NH₂, or -CH₃.

In some embodiments, B¹ and B² are independently a 9- membered bicyclic heteroaryl ring having the following structures: 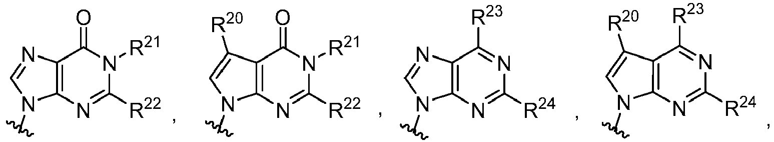 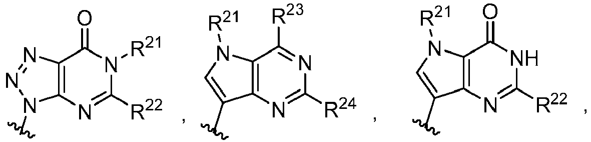 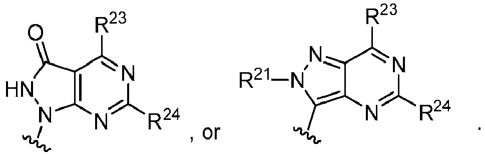 wherein R²⁰, R²¹, R²², R²³, and R²⁴ have the values described herein.

In some embodiments, R²⁰ is hydrogen or fluoro. In some embodiments, R²¹ is hydrogen or -CH₃, and R²² is hydrogen, -NH₂, or -CH₃. In some embodiments, R²³ is hydrogen or -NH₂, and R²⁴ is hydrogen, -NH₂, or -CH₃.

In some embodiments, B¹ and B² are independently selected from:

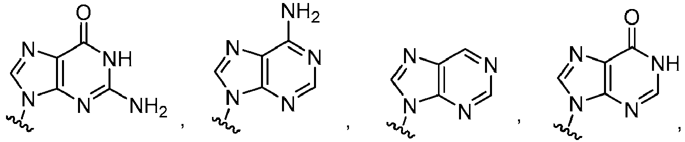

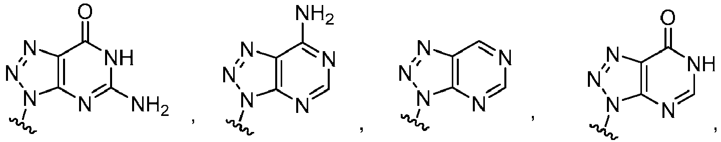

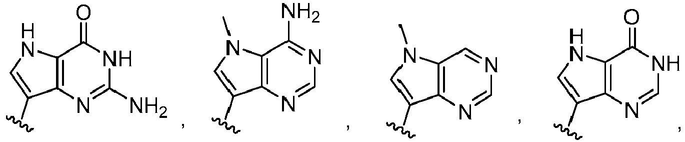

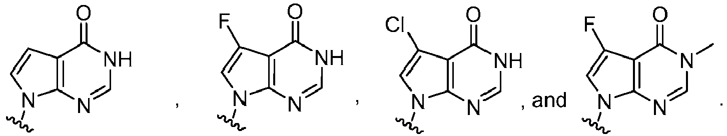

In some embodiments, B¹ is:

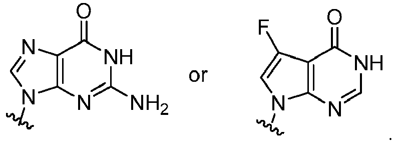

In some embodiments, B¹ is:

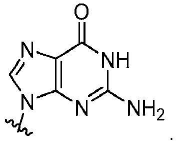

In some embodiments, B² is:

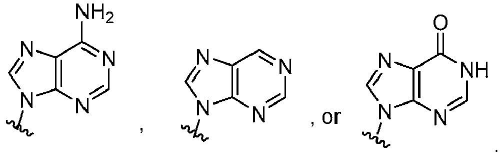

In some embodiments, B² is:

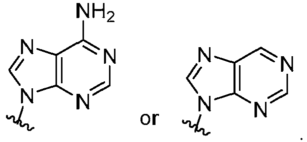

B¹, B², and B³ in the compounds disclosed herein can be optionally substituted. When B¹, B², or B³ are substituted, the substituent is one or more substitutents independently selected from the group consisting of halogen, -OH, -CN, -NO₂, C₁-C₆alkyl, halo(C₁-C₆)alkyl, C₃-C₆cycloalkyl, -OR¹¹, -SR¹², -N(R¹²)₂, -NR¹²C(O)R¹², -C(O)R¹², -C(O)OR¹², -C(O)N(R¹²)₂, and -SO₂N(R¹²)₂; or two adjacent substituents, taken together with the intervening ring atoms, form a 4- to 8-membered ring; wherein R¹¹ is C₁-C₆alkyl, halo(C₁-C₆)alkyl, or C₃-C₆cycloalkyl; and R¹² is hydrogen, C₁-C₆alkyl, halo(C₁-C₆)alkyl, or C₃-C₆cycloalkyl.

In some embodiments, B¹, B², and B³ can be optionally substituted by one or more substitutents independently selected from fluoro, chloro, -OH, -CN, -NO₂, -CH₃, -OCH₃, -NH₂, -NHMe, -NMe₂, or -C(O)NH₂.

In some embodiments, two adjacent substituents on B¹, B², or B³, taken together with the intervening ring atoms, form a 4- to 8-membered ring, where the 4- to 8-membered ring can be saturated or partially unsaturated, cycloaliphatic or heterocyclic rings.

Representative examples of compounds of Formula (I) are shown in Table 1.

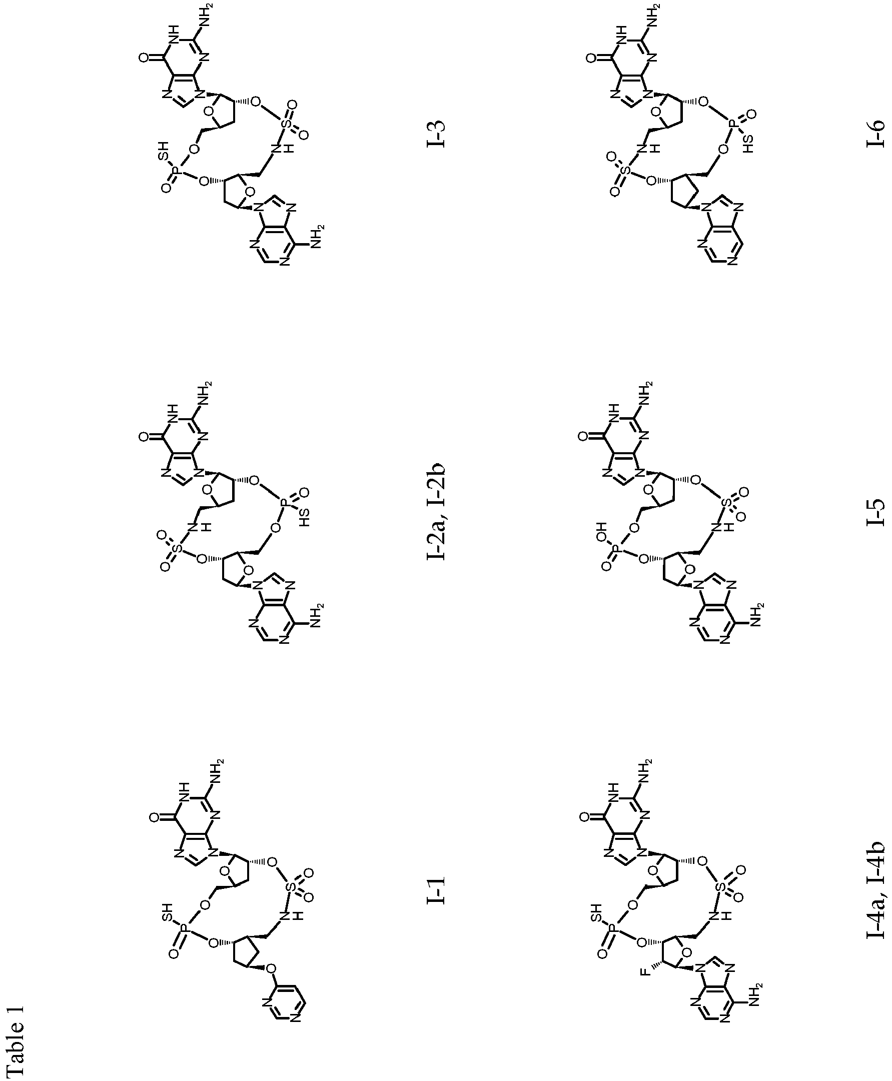

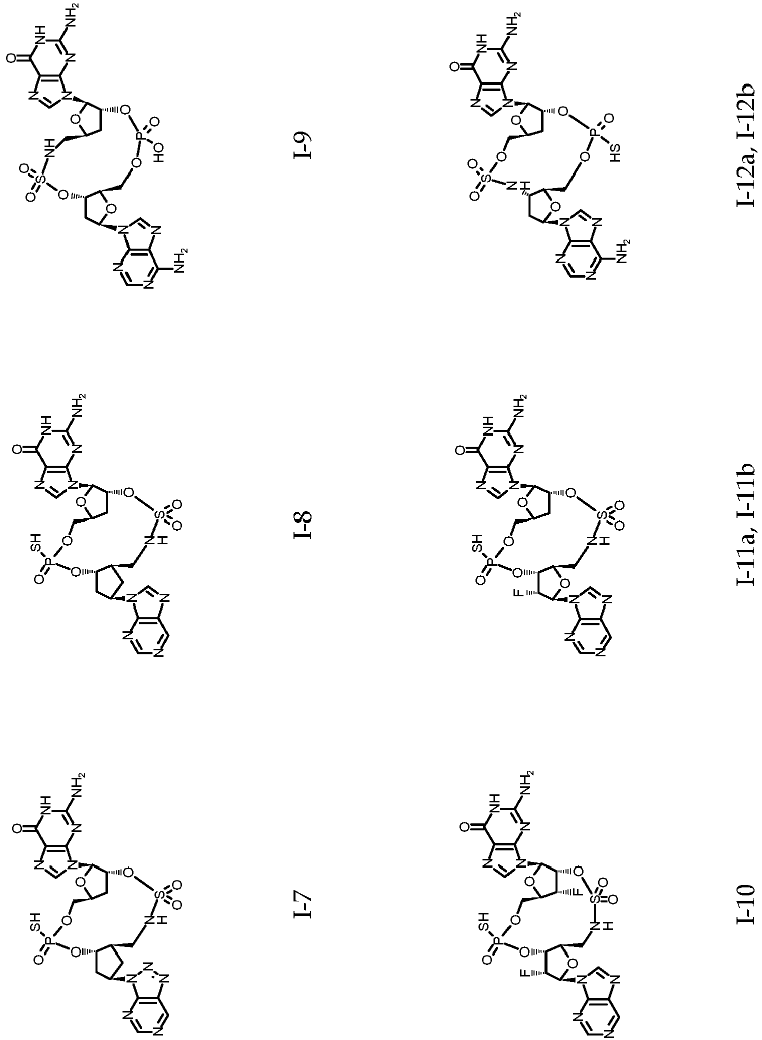

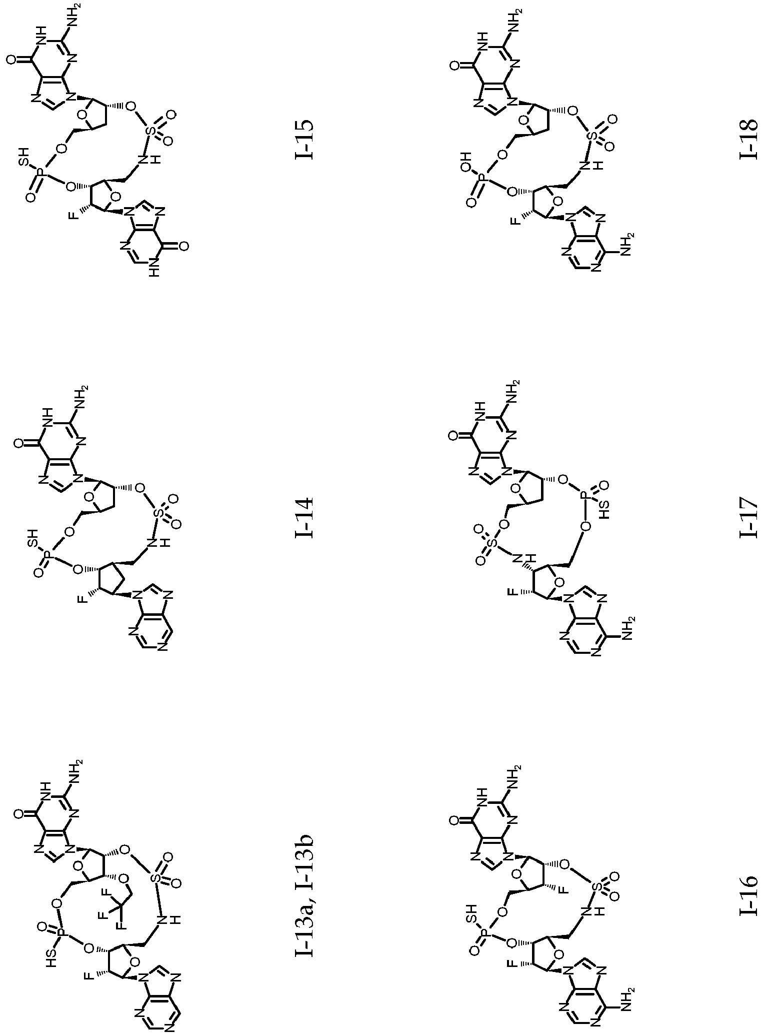

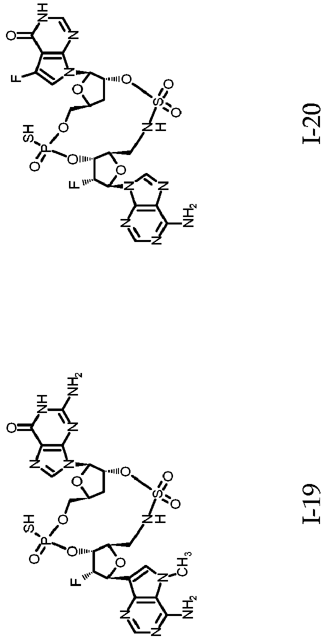

The compounds in Table 1 may also be identified by the following chemical names:

| **Compound** | **Name** |
|---|---|
| **I-1** | 2-amino-9-[(2S,5S,7R,8R,12aR,14R,15aS)-2,10,10-trioxido-14-(pyrimidin-4-yloxy)-2-sulfanyldecahydro-11H-5,8-methanocyclopenta[m] [1,3,6,9,10,11,2]tetraoxathiazaphosphacyclotetradecin-7-yl]-1,9-dihydro-6H-purin-6-one or 2-amino-9-[(2R,5S,7R,8R,12aR,14R,15aS)-2,10,10-trioxido-14-(pyrimidin-4-yloxy)-2-sulfanyldecahydro-11H-5,8-methanocyclopenta[m] [1,3,6,9,10,11,2]tetraoxathiazaphosphacyclotetradecin-7-yl]-1,9-dihydro-6H-purin-6-one |
| **I-2a** | 2-amino-9-[(5S,7R,8R,10S,12aR,14R,15aS)-14-(6-amino-9H-purin-9-yl)-2,2,10-trioxido-10-sulfanyloctahydro-3H,12H-5,8-methanofuro [3,2-l][1,3,6,11,10,9,2]tetraoxathiazaphosphacyclotetradecin-7-yl]-1,9-dihydro-6H-purin-6-one or 2-amino-9-[(5S,7R,8R,10R,12aR,14R,15aS)-14-(6-amino-9H-purin-9-yl)-2,2,10-trioxido-10-sulfanyloctahydro-3H,12H-5,8-methanofuro[3,2-l][1,3,6,11,10,9,2]tetraoxathiazaphosphacyclotetradecin-7-yl]-1,9-dihydro-6H-purin-6-one |
| **I-2b** | 2-amino-9-[(5S,7R,8R,10S,12aR,14R,15aS)-14-(6-amino-9H-purin-9-yl)-2,2,10-trioxido-10-sulfanyloctahydro-3H,12H-5,8-methanofuro [3,2-l][1,3,6,11,10,9,2]tetraoxathiazaphosphacyclotetradecin-7-yl]-1,9-dihydro-6H-purin-6-one or 2-amino-9-[(5S,7R,8R,10R,12aR,14R,15aS)-14-(6-amino-9H-purin-9-yl)-2,2,10-trioxido-10-sulfanyloctahydro-3H,12H-5,8-methanofuro[3,2-l][1,3,6,11,10,9,2]tetraoxathiazaphosphacyclotetradecin-7-yl]-1,9-dihydro-6H-purin-6-one |
| **I-3** | 2-amino-9-[(2S,5S,7R,8R,12aR,14R,15aS)-14-(6-amino-9H-purin-9-yl)-2,10,10-trioxido-2-sulfanyldecahydro-5,8-methanofuro [2,3-m][1,3,6,9,10,11,2]tetraoxathiazaphosphacyclotetradecin-7-yl]-1,9-dihydro-6H-purin-6-one or 2-amino-9-[(2R,5S,7R,8R,12aR,14R,15aS)-14-(6-amino-9H-purin-9-yl)-2,10,10-trioxido-2-sulfanyldecahydro-5,8-methanofuro[2,3-m][1,3,6,9,10,11,2]tetraoxathiazaphosphacyclotetradecin-7-yl]-1,9-dihydro-6H-purin-6-one |
| **I-4a** | 2-amino-9-[(2S,5S,7R,8R,12aR,14R,15R,15aR)-14-(6-amino-9H-purin-9-yl)-15-fluoro-2,10,10-trioxido-2-sulfanyldecahydro-5,8-methanofuro [2,3-m][1,3,6,9,10,11,2]tetraoxathiazaphosphacyclotetradecin-7-yl]-1,9-dihydro-6H-purin-6-one or 2-amino-9-[(2R,5S,7R,8R,12aR,14R,15R,15aR)-14-(6-amino-9H-purin-9-yl)-15-fluoro-2,10,10-trioxido-2-sulfanyldecahydro-5,8-methanofuro[2,3-m] [1,3,6,9,10,11,2]tetraoxathiazaphosphacyclotetradecin-7-yl]-1,9-dihydro-6H-purin-6-one |
| **I-4b** | 2-amino-9-[(2S,5S,7R,8R,12aR,14R,15R,15aR)-14-(6-amino-9H-purin-9-yl)-15-fluoro-2,10,10-trioxido-2-sulfanyldecahydro-5,8-methanofuro [2,3-m][1,3,6,9,10,11,2]tetraoxathiazaphosphacyclotetradecin-7-yl]-1,9-dihydro-6H-purin-6-one or 2-amino-9-[(2R,5S,7R,8R,12aR,14R,15R,15aR)-14-(6-amino-9H-purin-9-yl)-15-fluoro-2,10,10-trioxido-2-sulfanyldecahydro-5,8-methanofuro[2,3-m] [1,3,6,9,10,11,2]tetraoxathiazaphosphacyclotetradecin-7-yl]-1,9-dihydro-6H-purin-6-one |
| **I-5** | 2-amino-9-[(5S,7R,8R,12aR,14R,15aS)-14-(6-amino-9H-purin-9-yl)-2-hydroxy-2,10,10-trioxidodecahydro-5,8-methanofuro [2,3-m][1,3,6,9,10,11,2]tetraoxathiazaphosphacyclotetradecin-7-yl]-1,9-dihydro-6H-purin-6-one |
| **I-6** | 2-amino-9-[(5S,7R,8R,10S,12aR,14R,15aS)-2,2,10-trioxido-14-(9H-purin-9-yl)-10-sulfanyldecahydro-3H-5,8-methanocyclopenta[l][1,3,6,11,10,9,2]tetraoxathiazaphosphacyclotetradecin-7-yl]-1,9-dihydro-6H-purin-6-one or 2-amino-9-[(5S,7R,8R,10R,12aR,14R,15aS)-2,2,10-trioxido-14-(9H-purin-9-yl)-10-sulfanyldecahydro-3H-5,8-methanocyclopenta[l][1,3,6,11,10,9,2]tetraoxathiazaphosphacyclotetradecin-7-yl]-1,9-dihydro-6H-purin-6-one |
| **I-7** | 2-amino-9-[(2S,5S,7R,8R,12aR,14R,15aS)-2,10,10-trioxido-2-sulfanyl-14-(3H-[1,2,3]triazolo[4,5-d]pyrimidin-3-yl)decahydro-11H-5,8-methanocyclopenta[m] [1,3,6,9,10,11,2]tetraoxathiazaphosphacyclotetradecin-7-yl]-1,9-dihydro-6H-purin-6-one or 2-amino-9-[(2R,5S,7R,8R,12aR,14R,15aS)-2,10,10-trioxido-2-sulfanyl-14-(3H-[1,2,3]triazolo[4,5-d]pyrimidin-3-yl)decahydro-11H-5,8-methanocyclopenta[m] [1,3,6,9,10,11,2]tetraoxathiazaphosphacyclotetradecin-7-yl]-1,9-dihydro-6H-purin-6-one |
| **I-8** | 2-amino-9-[(2S,5S,7R,8R,12aR,14R,15aS)-2,10,10-trioxido-14-(9H-purin-9-yl)-2-sulfanyldecahydro-11H-5,8-methanocyclopenta[m] [1,3,6,9,10,11,2]tetraoxathiazaphosphacyclotetradecin-7-yl]-1,9-dihydro-6H-purin-6-one or 2-amino-9-[(2R,5S,7R,8R,12aR,14R,15aS)-2,10,10-trioxido-14-(9H-purin-9-yl)-2-sulfanyldecahydro-11H-5,8-methanocyclopenta[m] [1,3,6,9,10,11,2]tetraoxathiazaphosphacyclotetradecin-7-yl]-1,9-dihydro-6H-purin-6-one |
| **I-9** | 2-amino-9-[(5S,7R,8R,12aR,14R,15aS)-14-(6-amino-9H-purin-9-yl)-10-hydroxy-2,2,10-trioxidooctahydro-3H,12H-5,8-methanofuro [3,2-l][1,3,6,11,10,9,2]tetraoxathiazaphosphacyclotetradecin-7-yl]-1,9-dihydro-6H-purin-6-one |
| **I-10** | 2-amino-9-[(2S,5R,7R,8S,12aR,14R,15R,15aR,16R)-15,16-difluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-1,9-dihydro-6H-purin-6-one or 2-amino-9-[(2S,5R,7R,8S,12aR,14R,15R,15aR,16R)-15,16-difluoro-2,10,10-trioxido-14-(9H-purin-9-yl)-2-sulfanyldecahydro-5,8-methanofuro[2,3-m] [1,3,6,9,10,11,2]tetraoxathiazaphosphacyclotetradecin-7-yl]-1,9-dihydro-6H-purin-6-one |
| **I-11a** | 2-amino-9-[(2S,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-1,9-dihydro-6H-purin-6-one or 2-amino-9-[(2S,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-1,9-dihydro-6H-purin-6-one |
| **I-11b** | 2-amino-9-[(2R,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-1,9-dihydro-6H-purin-6-one or 2-amino-9-[(2S,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-1,9-dihydro-6H-purin-6-one |
| **I-12a** | 2-amino-9-[(5S,7R,8R,10S,12aS,14R,15aS)-14-(6-amino-9H-purin-9-yl)-2,2,10-trioxido-10-sulfanyldecahydro-5,8-methanofuro [3,2-l][1,3,6,9,10,11,2]tetraoxathiazaphosphacyclotetradecin-7-yl]-1,9-dihydro-6H-purin-6-one or 2-amino-9-[(5S,7R,8R,10R,12aS,14R,15aS)-14-(6-amino-9H-purin-9-yl)-2,2,10-trioxido-10-sulfanyldecahydro-5,8-methanofuro[3,2-l][1,3,6,9,10,11,2]tetraoxathiazaphosphacyclotetradecin-7-yl]-1,9-dihydro-6H-purin-6-one |
| **I-12b** | 2-amino-9-[(5S,7R,8R,10S,12aS,14R,15aS)-14-(6-amino-9H-purin-9-yl)-2,2,10-trioxido-10-sulfanyldecahydro-5,8-methanofuro [3,2-l][1,3,6,9,10,11,2]tetraoxathiazaphosphacyclotetradecin-7-yl]-1,9-dihydro-6H-purin-6-one or 2-amino-9-[(5S,7R,8R,10R,12aS,14R,15aS)-14-(6-amino-9H-purin-9-yl)-2,2,10-trioxido-10-sulfanyldecahydro-5,8-methanofuro[3,2-l][1,3,6,9,10,11,2]tetraoxathiazaphosphacyclotetradecin-7-yl]-1,9-dihydro-6H-purin-6-one |
| **I-13a** | 2-amino-9-[(2S,5R,7R,8R,12aR,14R,15R,15aR,16R)-15-fluoro-2,10,10-trioxido-14-(9H-purin-9-yl)-2-sulfanyl-16-(2,2,2-trifluoroethoxy)decahydro-5,8-methanofuro[2,3-m] [1,3,6,9,10,11,2] tetraoxathiazaphosphacyclotetradecin-7-yl]-1,9-dihydro-6H-purin-6-one or 2-amino-9-[(2R,5R,7R,8R,12aR,14R,15R,15aR,16R)-15-fluoro-2,10,10-trioxido-14-(9H-purin-9-yl)-2-sulfanyl-16-(2,2,2-trifluoroethoxy)decahydro-5,8-methanofuro[2,3-m] [1,3,6,9,10,11,2]tetraoxathiazaphosphacyclotetradecin-7-yl]-1,9-dihydro-6H-purin-6-one |
| **I-13b** | 2-amino-9-[(2S,5R,7R,8R,12aR,14R,15R,15aR,16R)-15-fluoro-2,10,10-trioxido-14-(9H-purin-9-yl)-2-sulfanyl-16-(2,2,2-trifluoroethoxy)decahydro-5,8-methanofuro[2,3-m] [1,3,6,9,10,11,2] tetraoxathiazaphosphacyclotetradecin-7-yl]-1,9-dihydro-6H-purin-6-one or 2-amino-9-[(2R,5R,7R,8R,12aR,14R,15R,15aR,16R)-15-fluoro-2,10,10-trioxido-14-(9H-purin-9-yl)-2-sulfanyl-16-(2,2,2-trifluoroethoxy)decahydro-5,8-methanofuro[2,3-m] [1,3,6,9,10,11,2]tetraoxathiazaphosphacyclotetradecin-7-yl]-1,9-dihydro-6H-purin-6-one |
| **I-14** | 2-amino-9-[(2S,5S,7R,8R,12aR,14R,15S,15aR)-15-fluoro-2,10,10-trioxido-14-(9H-purin-9-yl)-2-sulfanyldecahydro-11H-5,8-methanocyclopenta[m] [1,3,6,9,10,11,2]tetraoxathiazaphosphacyclotetradecin-7-yl]-1,9-dihydro-6H-purin-6-one or 2-amino-9-[(2R,5S,7R,8R,12aR,14R,15S,15aR)-15-fluoro-2,10,10-trioxido-14-(9H-purin-9-yl)-2-sulfanyldecahydro-11H-5,8-methanocyclopenta[m] [1,3,6,9,10,11,2]tetraoxathiazaphosphacyclotetradecin-7-yl]-1,9-dihydro-6H-purin-6-one |
| **I-15** | 2-amino-9-[(2S,5S,7R,8R,12aR,14R,15R,15aR)-15-fluoro-2,10,10-trioxido-14-(6-oxo-1,6-dihydro-9H-purin-9-yl)-2-sulfanyldecahydro-5,8-methanofuro[2,3-m][1,3,6,9,10,11,2]tetraoxathiazaphosphacyclotetradecin-7-yl]-1,9-dihydro-6H-purin-6-one or 2-amino-9-[(2R,5S,7R,8R,12aR,14R,15R,15aR)-15-fluoro-2,10,10-trioxido-14-(6-oxo-1,6-dihydro-9H-purin-9-yl)-2-sulfanyldecahydro-5,8-methanofuro[2,3-m][1,3,6,9,10,11,2]tetraoxathiazaphosphacyclotetradecin-7-yl]-1,9-dihydro-6H-purin-6-one |
| **I-16** | 2-amino-9-[(2S,5R,7R,8S,12aR,14R,15R,15aR,16R)-14-(6-amino-9H-purin-9-yl)-15,16-difluoro-2,10,10-trioxido-2-sulfanyldecahydro-5,8-methanofuro [2,3-m][1,3,6,9,10,11,2]tetraoxathiazaphosphacyclotetradecin-7-yl]-1,9-dihydro-6H-purin-6-one or 2-amino-9-[(2R,5R,7R,8S,12aR,14R,15R,15aR,16R)-14-(6-amino-9H-purin-9-yl)-15,16-difluoro-2,10,10-trioxido-2-sulfanyldecahydro-5,8-methanofuro[2,3-m] [1,3,6,9,10,11,2]tetraoxathiazaphosphacyclotetradecin-7-yl]-1,9-dihydro-6H-purin-6-one |
| **I-17** | 2-amino-9-[(5S,7R,8R,10S,12aS,14R,15R,15aR)-14-(6-amino-9H-purin-9-yl)-15-fluoro-2,2,10-trioxido-10-sulfanyldecahydro-5,8-methanofuro [3,2-l][1,3,6,9,10,11,2]tetraoxathiazaphosphacyclotetradecin-7-yl]-1,9-dihydro-6H-purin-6-one or 2-amino-9-[(5S,7R,8R,10R,12aS,14R,15R,15aR)-14-(6-amino-9H-purin-9-yl)-15-fluoro-2,2,10-trioxido-10-sulfanyldecahydro-5,8-methanofuro[3,2-l][1,3,6,9,10,11,2]tetraoxathiazaphosphacyclotetradecin-7-yl]-1,9-dihydro-6H-purin-6-one |
| **I-18** | 2-amino-9-[(5S,7R,8R,12aR,14R,15R,15aR)-14-(6-amino-9H-purin-9-yl)-15-fluoro-2-hydroxy-2,10,10-trioxidodecahydro-5,8-methanofuro [2,3-m][1,3,6,9,10,11,2]tetraoxathiazaphosphacyclotetradecin-7-yl]-1,9-dihydro-6H-purin-6-one |
| **I-19** | 2-amino-9-[(2S,5S,7R,8R,12aR,14S,15S,15aR)-14-(4-amino-5-methyl-5H-pyrrolo[3,2-d]pyrimidin-7-yl)-15-fluoro-2,10,10-trioxido-2-sulfanyldecahydro-5,8-methanofuro[2,3-m] [1,3,6,9,10,11,2]tetraoxathiazaphosphacyclotetradecin-7-yl]-1,9-dihydro-6H-purin-6-one or 2-amino-9-[(2R,5S,7R,8R,12aR,14S,15S,15aR)-14-(4-amino-5-methyl-5H-pyrrolo[3,2-d]pyrimidin-7-yl)-15-fluoro-2,10,10-trioxido-2-sulfanyldecahydro-5,8-methanofuro[2,3-m] [1,3,6,9,10,11,2] tetraoxathiazaphosphacyclotetradecin-7-yl]-1,9-dihydro-6H-purin-6-one |
| **I-20** | 7-[(2S,5S,7R,8R,12aR,14R,15R,15aR)-14-(6-amino-9H-purin-9-yl)-15-fluoro-2,10,10-trioxido-2-sulfanyldec ahydro-5,8-methanofuro [2,3-m][1,3,6,9,10,11,2]tetraoxathiazaphosphacyclotetradecin-7-yl]-5-fluoro-3,7-dihydro-4H-pyrrolo[2,3-d]pyrimidin-4-one or 7-[(2R,5S,7R,8R,12aR,14R,15R,15aR)-14-(6-amino-9H-purin-9-yl)-15-fluoro-2,10,10-trioxido-2-sulfanyldecahydro-5,8-methanofuro[2,3-m][1,3,6,9,10,11,2]tetraoxathiazaphosphacyclotetradecin-7-yl]-5-fluoro-3,7-dihydro-4H-pyrrolo[2,3-d]pyrimidin-4-one |

In some embodiments, the compounds of Formula (I) include compounds I-1, I-2a, I-2b, I-3, I-4a, I-4b, I-5, I-6, I-7, I-8, I-9, I-10, and I-11a. In some embodiments, the compounds of Formula (I) include compounds I-11b, I-12a, I-12b, I-13a, I-13b, I-14, I-15, I-16, I-17, I-18, I-19, and I-20.

When compound (I) is in a form of a salt, the salt is typically a pharmacologically acceptable salt. Examples include salts with inorganic base, salts with organic base, salts with inorganic acid, salts with organic acid, and salts with basic or acidic amino acid.

Useful examples of the salt with inorganic base include alkali metal salts such as sodium salt, potassium salt and the like; alkaline-earth metal salts such as calcium salt, magnesium salt and the like; aluminium salt and ammonium salt.

Useful examples of the salt with organic base include salts with trimethylamine, triethylamine, pyridine, picoline, ethanolamine, diethanolamine, triethanolamine, tromethamine [tris(hydroxymethyl)methylamine], tert-butylamine, cyclohexylamine, benzylamine, dicyclohexylamine and N,N-dibenzyl ethylene diamine.

Useful examples of the salt with inorganic acid include salts with hydrochloric acid, hydrobromic acid, nitric acid, sulfuric acid and phosphoric acid.

Useful examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid and p-toluenesulfonic acid.

Useful examples of the salt with basic amino acid include salts with arginine, lysine and ornithine.

Useful examples of the salt with acidic amino acid include salts with aspartic acid and glutamic acid.

When a compound of Formula (I) is in a form of a salt, in one embodiment, the salt is a salt with triethylamine or sodium. In some embodiments, the salt is a salt with sodium. In some embodiments, the salt is a salt with triethylamine.

### General Synthetic Methods and Intermediates

The compounds of the present disclosure can be prepared by methods known to one of ordinary skill in the art and / or by reference to the schemes shown below and the synthetic examples. See e.g., Org. Lett., 2010, 12 (14), pp 3269-3271; WO 2017/075477 (A1). Starting materials and intermediates are purchased from commercial sources, prepared via published procedures or are illustrated below. Exemplary synthetic routes are set forth in Schemes below, and in the Examples.

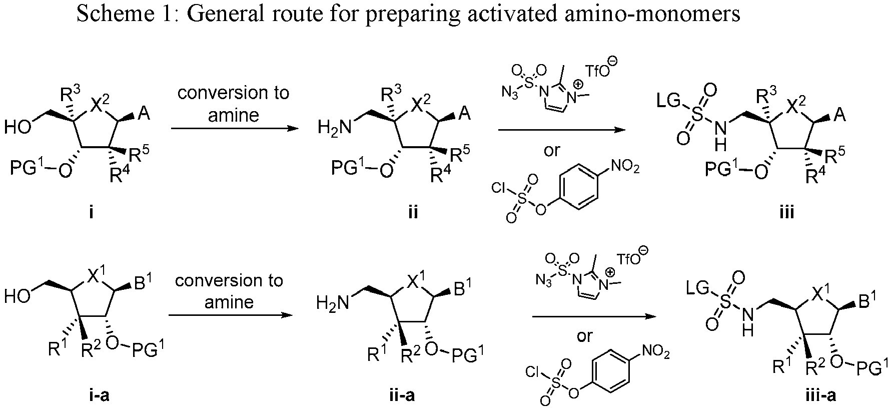

Scheme 1 shows two general routes for the preparation of compounds of the formula **iii** and **iii-a** from nucleotides or cyclopentane analogs such as **i** and **i-a** where PG¹ is a silyl protecting group such as tri-isopropylsilyl or *tert*-butyl di-methylsilyl, X² is oxygen, sulfur or CH₂, R³ is hydrogen or fluoro, R⁴ is an acyl protected hydroxyl, hydrogen, fluoro or is taken together with R³ to form OCH₂. Conversion of the primary alcohol of **i** or **i-a** to an amine can be accomplished by a variety of methods known to one skilled in the art such as reaction of the alcohol with diphenylphosphoryl azide, displacement of the resulting phosphate with sodium azide, and reduction to an amine via catalytic hydrogenation. Activation of the resulting amine **ii** or **ii-a** in preparation for sulfamate formation is effected by treatment with either 4-nitrophenyl sulfurochloridate or 1-(azidosulfonyl)-2,3-dimethyl-1H-imidazol-3-ium trifluoromethanesulfonate to provide compounds of the formula **iii** or **iii-a** where LG is either an azide or p-nitrophenyl moiety.

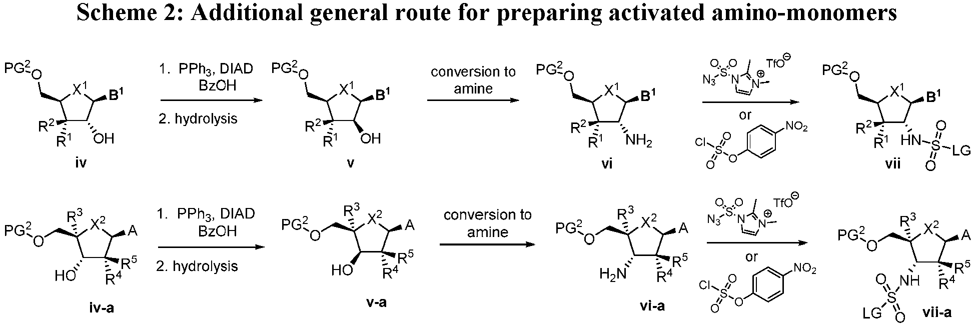

Scheme 2 shows two general routes for the preparation of compounds of the formula **vii** and **vii-a** from a protected nucleotide or cyclopentane derivative such as **iv** or **iv-a** (where PG² is for example dimethoxytrityl group, R¹ is hydrogen or fluoro, R² a silyl protected hydroxyl, hydrogen, fluoro, X¹ and X² are oxygen, sulfur or CH₂, R³ is hydrogen or fluoro, R⁴ is an acyl protected hydroxyl, hydrogen, fluoro or is taken together with R³ to form OCH₂). Inversion of the unprotected alcohol can be accomplished with a Mitsunobu reaction followed by deprotection (for example hydrolysis of a benzoate ester) to afford **v** or **v-a**. Subsequent conversion of the resulting secondary alcohol to an amine can be accomplished by a variety of methods known to one skilled in the art to provide a compound of formula **vi** or **vi-a** (for example, reaction of alcohol **v** or **v-a** with an activating reagent such as methansulfonyl chloride, displacement with sodium azide and reduction to an amine via catalytic hydrogenation). Activation of this amine in preparation for construction of the sulfamate linkage can be accomplished as described above in Scheme 1.

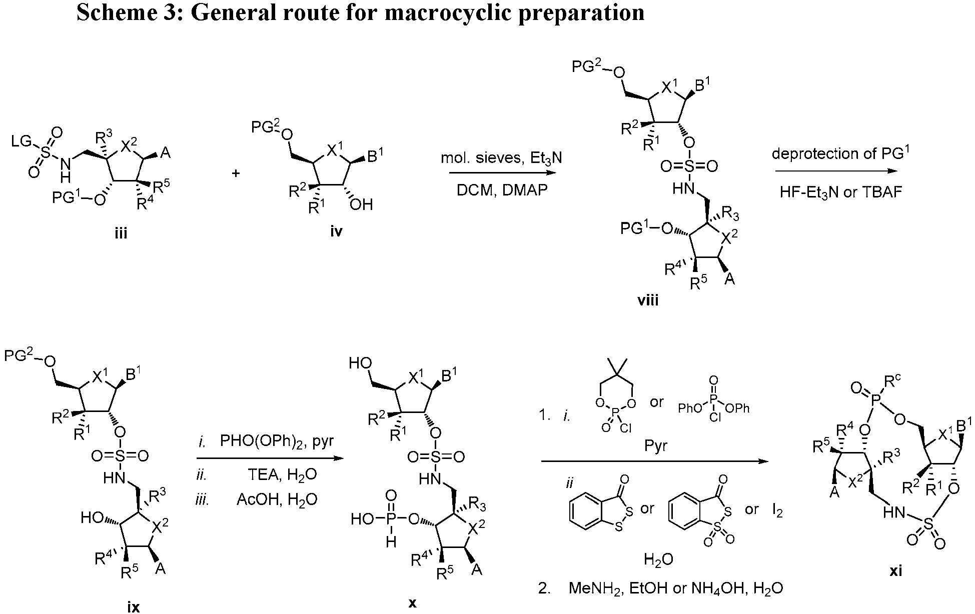

Scheme 3 shows a general method for the preparation of compounds of formula **xi** from two monomers **iii** and **iv.** Sulfamate formation is accomplished by displacement of the leaving group (LG) on **iii** with the secondary alcohol of **iv** and results in the sulfamate **viii.** Removal of protecting group PG¹ using, for example, hydrogen fluoride-triethyl amine or TBAF liberates the secondary alcohol to afford **ix.** Introduction of the H-phosphonate followed by removal of the primary ether protecting group (for example removal of a dimethoxytrityl ether by treatment with AcOH/H₂O) provides alcohol **x.** Cyclization of **x** with 2-chloro-5,5-dimethyl-1,3,2-dioxaphosphorinane 2-oxide or diphenyl chlorophosphate in the presence of pyridine followed by oxidation with iodine, when R^{c} is OH or with a sulfur transfer reagent (such as 3H-1,2-benzodithiol-3-one 1,1-dioxide or 3H-1,2-benzodithiol-3-one, when R^{c} is SH) and subsequent removal of remaining protection groups provides macrocycles of the formula xi (where R^{c} is OH or SH).

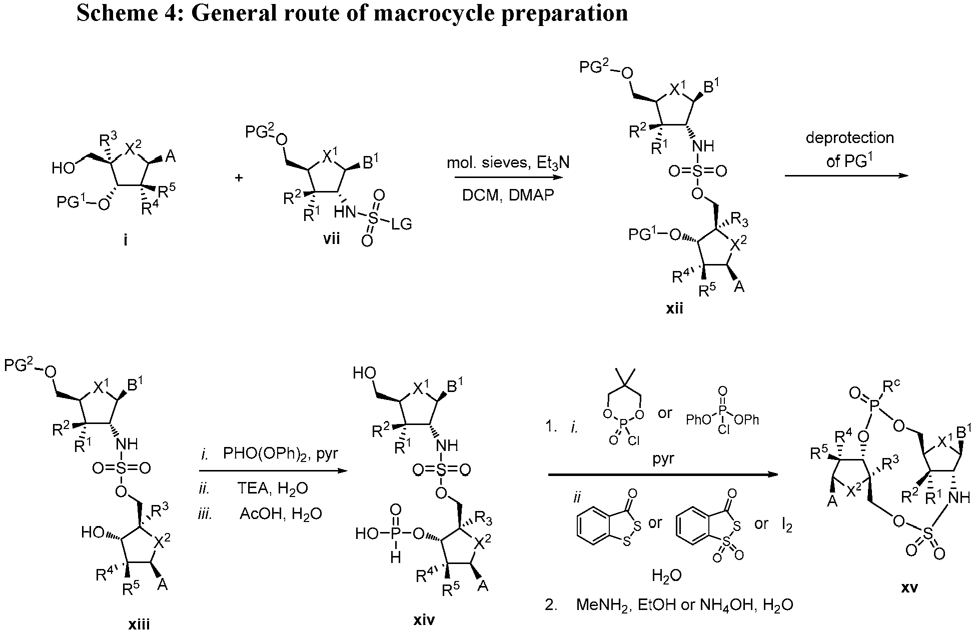

Scheme 4 shows a general method for the preparation of compounds of formula xv from two nucleotide monomers **i** and **vii** following the general methods described in Scheme 3.

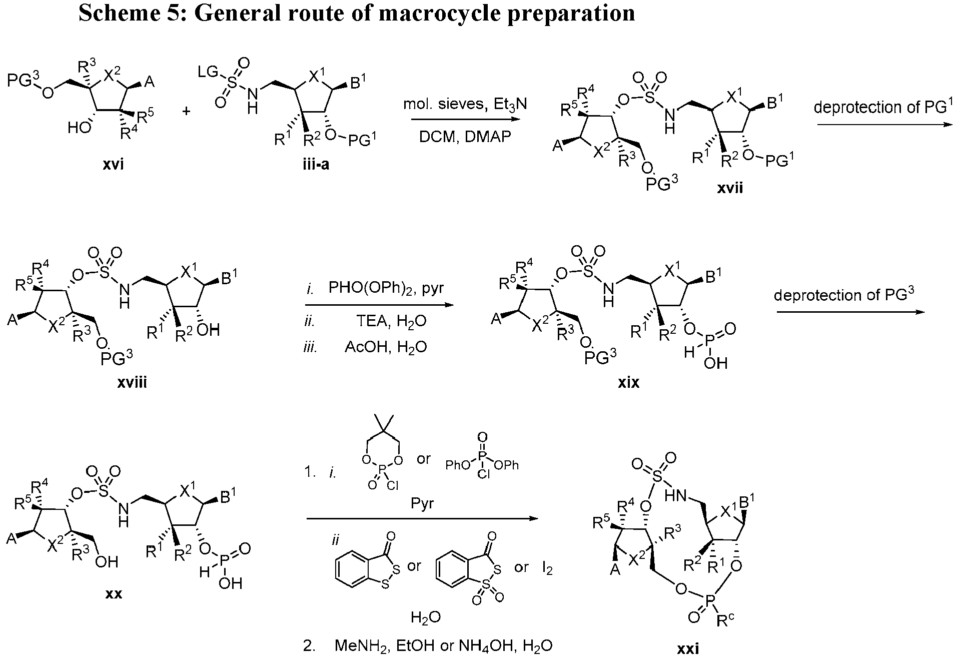

Scheme 5 shows a general method for the preparation of compounds of formula **xxi** from two nucleotide or cyclopentane-dervied monomers **xvi** and **iii-a** (where PG³ of **xvi** and PG¹ of **iii-a** are orthogonal protecting groups) following the general methods described in Scheme 3.

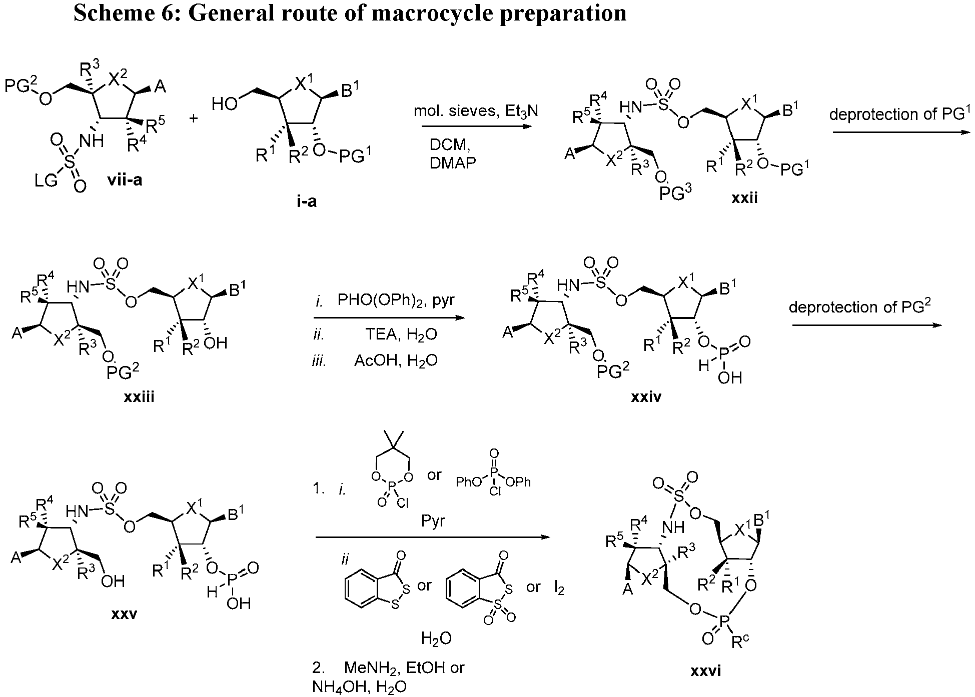

Scheme 6 shows a general method for the preparation of compounds of formula **xxvi** from two nucleotide monomers **i-a** and **vii-a** following the general methods described in Scheme 3.

### Method of Use of Compounds of Formula (I)

Compound of the present disclosure show STING modulating/agonistic activity.

A compound of the present disclosure can be used for increasing STING activity in a mammal (e.g., mouse, rat, hamster, rabbit, cat, dog, cow, sheep, monkey, and human).

A compound of the present disclosure can be used as a medicament such as an agent for the prophylaxis or treatment of diseases that can be influenced by STING (in the present specification, sometimes to be abbreviated as "STING-related diseases"), for example, cancers -e.g., colorectal cancers (e.g., colorectal cancer, rectal cancer, anus cancer, familial colorectal cancer, hereditary nonpolyposis colorectal cancer, gastrointestinal stromal tumor), lung cancers (e.g., non-small-cell lung cancer, small-cell lung cancer, malignant mesothelioma), mesothelioma, pancreatic cancers (e.g., pancreatic ductal carcinoma, pancreatic endocrine tumor), pharynx cancer, larynx cancer, esophageal cancer, stomach cancers (e.g., papillary adenocarcinoma, mucinous adenocarcinoma, adenosquamous carcinoma), duodenal cancer, small intestinal cancer, breast cancers (e.g., invasive ductal carcinoma, non-invasive ductal carcinoma, inflammatory breast cancer), ovarian cancers (e.g., ovarian epithelial cancer, extragonadal germ cell tumor, ovarian germ cell tumor, ovarian low-malignant potential tumor), testis tumor, prostate cancers (e.g., hormone-dependent prostate cancer, non-hormone dependent prostate cancer, castration-resistant prostate cancer), liver cancers (e.g., hepatocellular cancer, primary liver cancer, extrahepatic bile duct cancer), thyroid cancers (e.g., medullary thyroid carcinoma), renal cancers (e.g., renal cell cancers (e.g., clear cell renal cell cancer), transitional cell cancer of renal pelvis and ureter), uterine cancers (e.g., cervical cancer, uterine body cancer, uterus sarcoma), gestational choriocarcinoma, brain tumors (e.g., medulloblastoma, glioma, pineal astrocytic tumors, pilocytic astrocytoma, diffuse astrocytoma, anaplastic astrocytoma, pituitary adenoma), retinoblastoma, skin cancers (e.g., basalioma, malignant melanoma), sarcomas (e.g., rhabdomyosarcoma, leiomyosarcoma, soft tissue sarcoma, spindle cell sarcoma), malignant bone tumor, bladder cancer, blood cancers (e.g., multiple myeloma, leukemias (e.g., acute myelogenous leukemia), malignant lymphoma, Hodgkin's disease, chronic myeloproliferative disease), cancer of unknown primary; a cancer growth inhibitor; a cancer metastasis inhibitor; an apoptosis promoter; an agent for the treatment of precancerous lesions (e.g., myelodysplastic syndromes); and the like.

In certain embodiments, a compound of the present disclosure can be used as a medicament for colorectal cancer, breast cancer, skin cancer, malignant lymphoma or lung cancer.

A compound of the present disclosure can be administered orally or parenterally as is or in a mixture with a pharmacologically acceptable carrier as a medicament, to a mammal (typically a human).

The medicament containing a compound of the present disclosure (hereinafter sometimes to be abbreviated as "the medicament of the present disclosure") is explained in detail below. Examples of the dosage form of the medicament of the present disclosure include oral preparations such as tablet (e.g., sugar-coated tablet, film-coated tablet, sublingual tablet, buccal, orally disintegrating tablet), pill, granule, powder, capsule (e.g., soft capsule, microcapsule), syrup, emulsion, suspension, films (e.g., orally disintegrable films, oral mucosa-adhesive film) and the like. In addition, examples of the dosage form of the medicament of the present disclosure include parenteral preparations such as injection, drip infusion, transdermal absorption type preparation (e.g., iontophoresis transdermal absorption type preparation), suppository, ointment, nasal preparation, pulmonary preparation, eye drop and the like. Moreover, the medicament of the present disclosure may be a release control preparation such as an immediate-release preparation, a sustained-release preparation (e.g., a sustained-release microcapsule) and the like.

As the dosage form of the medicament of the present disclosure, a nanoparticle preparation and a preparation using a bacteria-derived membrane can also be used.

The medicament of the present disclosure may be prepared according to a method known per se (e.g., the method described in the US Pharmacopoeia etc.) generally used in the field of preparation. In addition, the medicament of the present disclosure may contain a suitable amount of an additive such as an excipient, a binder, a disintegrant, a lubricant, a sweetening agent, a surfactant, a suspending agent, an emulsifier, a colorant, a preservative, an aromatic, a corrigent, a stabilizer, a thickening agent and the like generally used in the field of preparation as necessary. Examples of the pharmacologically acceptable carrier include these additives.

For example, tablet may be prepared using an excipient, a binder, a disintegrant, a lubricant and the like, and pill or granule may be prepared using an excipient, a binder and a disintegrant. Powder or capsule may be prepared using an excipient and the like, syrup may be prepared using a sweetening agent and the like, and emulsion or suspension may be prepared using a suspending agent, a surfactant, an emulsifier and the like.

Examples of useful excipients include lactose, sucrose, glucose, starch, sucrose, crystalline cellulose, powdered glycyrrhiza, mannitol, sodium hydrogencarbonate, calcium phosphate and calcium sulfate.

Examples of useful binders include 5 to 10 wt% starch liquid paste, 10 to 20 wt% gum arabic solution or gelatin solution, 1 to 5 wt% tragacanth solution, carboxymethyl cellulose solution, sodium alginate solution and glycerin.

Examples of useful disintegrants include starch and calcium carbonate.

Examples of useful lubricants include magnesium stearate, stearic acid, calcium stearate and purified talc.

Examples of useful sweeteners include glucose, fructose, invert sugar, sorbitol, xylitol, glycerin and simple syrup.

Examples of useful surfactants include sodium lauryl sulfate, polysorbate 80, sorbitan monofatty acid ester and polyoxyl 40 stearate.

Examples of useful suspending agents include gum arabic, sodium alginate, sodium carboxymethyl cellulose, methyl cellulose and bentonite.

Examples of useful emulsifiers include gum arabic, tragacanth, gelatin and Polysorbate 80.

For example, when the medicament of present disclosure is a tablet, for example, an excipient (e.g., lactose, sucrose, starch), a disintegrant (e.g., starch, calcium carbonate), a binder (e.g., starch, gum arabic, carboxymethyl cellulose, polyvinyl pyrrolidone, hydroxypropyl cellulose) or a lubricant (e.g., talc, magnesium stearate, polyethylene glycol 6000) is added to a compound of the present disclosure, and the mixture is compression-molded, according to a method known per se, and then where necessary, the molded product is coated according to a method known per se for the purpose of masking of taste, enteric property or durability, to give a tablet. As the coating agent for the coating, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (methacrylic acid-acrylic acid copolymer, manufactured by Rohm, DE) and pigment (e.g., iron oxide red, titanium dioxide) may be used.

Examples of the injection include intravenous injection as well as subcutaneous injection, intracutaneous injection, intramuscular injection, intraperitoneal injection, drip injection, intratumoral injection and the like.

Such injections are prepared according to a method known *per se*, or by dissolving, suspending or emulsifying a compound of the present disclosure in a sterilized aqueous or oily liquid. Examples of the aqueous liquid include physiological saline, isotonic solutions containing glucose or other auxiliary drugs (e.g., D-sorbitol, D-mannitol, sodium chloride) and the like. The aqueous liquid may contain a suitable solubilizing agent such as an alcohol (e.g., ethanol), a polyalcohol (e.g., propylene glycol, polyethylene glycol), a nonionic surfactant (e.g., polysorbate 80, HCO-50) and the like. Examples of the oily liquid include sesame oil, soybean oil and the like. The oily liquid may contain a solubilizing agent. Examples of the solubilizing agent include benzyl benzoate, benzyl alcohol and the like. In addition, the injection may be blended with a buffer (e.g., phosphate buffer, sodium acetate buffer), a soothing agent (e.g., benzalkonium chloride, procaine hydrochloride), a stabilizer (e.g., human serum albumin, polyethylene glycol), a preservative (e.g., benzyl alcohol, phenol) and the like. A prepared injection may be generally filled in an ampoule.

While the content of a compound of the present disclosure in the medicament of the present disclosure varies depending on the form of the pharmaceutical preparation, it is generally about 0.01 to about 100 wt%, typically about 2 to about 85 wt%, or about 5 to about 70 wt%, relative to the entire preparation.

While the content of the additive in the medicament of the present disclosure varies depending on the form of the pharmaceutical preparation, it is generally about 1 to about 99.9 wt%, typically about 10 to about 90 wt%, relative to the entire preparation.

While the daily dose of a compound of the present disclosure varies depending on the condition and body weight of patients, the kind of compound, administration route and the like, in the case of, for example, oral administration to patients for the treatment of cancer, the daily dose to an adult (body weight about 60 kg) is about 1 to about 1000 mg, typically about 3 to about 300 mg, or about 10 to about 200 mg, as a compound of the present disclosure, which may be given in a single administration or administered in 2 or 3 portions a day.

When a compound of the present disclosure is administered parenterally, it is generally administered in the form of a liquid (e.g., injection). While the dose of a compound of the present disclosure varies depending on the subject of administration, target organ, symptom, administration method and the like, it is, for example, about 0.01 mg to about 100 mg, typically about 0.01 to about 50 mg, ory about 0.01 to about 20 mg, relative to 1 kg body weight, which is typically given by intravenous injection.

The present disclosure also includes a compound of the present disclosure for use (i) in, (ii) as a medicament for, or (iii) in the preparation of a medicament for: (a) inducing an immune response in a patient, or (b) inducing a STING-dependent cytokine production in a patient. In these uses, the compounds of the present disclosure can optionally be employed in combination with one or more second therapeutic agents.

As used herein, the term"immune response" relates to any one or more of the following: specific immune response, non-specific immune response, both specific and non-specific response, innate response, primary immune response, adaptive immune response, secondary immune response, memory immune response, immune cell activation, immune cell proliferation, immune cell differentiation, and cytokine expression.

In one embodiment, the compound of Formula (I) disclosed herein can be used for inducing a STING-dependent type I interferon production in a subject. The compound of Formula (I) or a pharmaceutically acceptable salt thereof can be administered by means that produces contact of the active agent with the agent's site of action. They can be administered by conventional means available for use in conjunction with pharmaceuticals, either as individual therapeutic agents or in a combination of therapeutic agents. They can be administered alone, but typically are administered with a pharmaceutical carrier selected on the basis of the chosen route of administration and standard pharmaceutical practice.

The compounds of the present disclosure may also be used as adjuvants to improve the immune response raised to any given antigen and/or reduce reactogenicity/toxicity in a patient, particularly a human, in need thereof. As such, a compound of the present disclosure may be used in combination with vaccine compositions to modify, especially to enhance, the immune response for example by increasing the level or duration of protection and/or allowing a reduction in the antigenic dose. The compounds of Formula (I) and pharmaceutically acceptable salts thereof may be used in combination with one or more vaccines or immunogenic antigens useful in the prevention or treatment of viral infections.

The present disclosure also provides a vaccine composition comprising an antigen and the compounds of Formula (I) or a pharmaceutically acceptable salt thereof.

A compound of the present disclosure can be used concurrently with other drugs. To be specific, a compound of the present disclosure can be used together with medicaments such as hormonal therapeutic agents, chemotherapeutic agents, immunotherapeutic agents, medicaments inhibiting the action of cell growth factors or cell growth factor receptors and the like. In the following, the drugs that can be used in combination with a compound of the present disclosure are abbreviated as concomitant drugs.

Examples of the "hormonal therapeutic agents" include fosfestrol, diethylstylbestrol, chlorotrianisene, medroxyprogesterone acetate, megestrol acetate, chlormadinone acetate, cyproterone acetate, danazol, allylestrenol, gestrinone, mepartricin, raloxifene, ormeloxifene, levormeloxifene, anti-estrogens (e.g., tamoxifen citrate, toremifene citrate), pill preparations, mepitiostane, testrolactone, aminoglutethimide, LH-RH agonists (e.g., goserelin acetate, buserelin, leuprorelin, leuprorelin acetate), droloxifene, epitiostanol, ethinylestradiol sulfonate, aromatase inhibitors (e.g., fadrozole hydrochloride, anastrozole, retrozole, exemestane, vorozole, formestane), anti-androgens (e.g., flutamide, bicartamide, nilutamide, enzalutamide), 5α-reductase inhibitors (e.g., finasteride, epristeride, dutasteride), aderenal cortex hormone drugs (e.g., dexamethasone, prednisolone, betamethasone, triamcinolone), androgen synthesis inhibitors (e.g., abiraterone), retinoid and drugs that retard retinoid metabolism (e.g., liarozole), thyroid hormone, and DDS (Drug Delivery System) preparations thereof.

Examples of the "chemotherapeutic agents" include alkylating agents, antimetabolites, anticancer antibiotics and plant-derived anticancer agents.

Examples of the "alkylating agents" include nitrogen mustard, nitrogen mustard-N-oxide hydrochloride, chlorambutyl, cyclophosphamide, ifosfamide, thiotepa, carboquone, improsulfan tosylate, busulfan, nimustine hydrochloride, mitobronitol, melphalan, dacarbazine, ranimustine, sodium estramustine phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulphan, trophosphamide, zinostatin stimalamer, adozelesin, cystemustine, bizelesin, and DDS preparations thereof.

Examples of the "antimetabolites" include mercaptopurine, 6-mercaptopurine riboside, thioinosine, methotrexate, pemetrexed, enocitabine, cytarabine, cytarabine ocfosfate, ancitabine hydrochloride, 5-FU drugs (e.g., fluorouracil, tegafur, UFT, doxifluridine, carmofur, gallocitabine, emitefur, capecitabine), aminopterine, nelzarabine, leucovorin calcium, tabloid, butocine, calcium folinate, levofolinate calcium, cladribine, emitefur, fludarabine, gemcitabine, hydroxycarbamide, pentostatin, piritrexim, idoxuridine, mitoguazone, thiazophrine, ambamustine, bendamustine, and DDS preparations thereof.

Examples of the "anticancer antibiotics" include actinomycin-D, actinomycin-C, mitomycin-C, chromomycin-A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarcomycin, carzinophilin, mitotane, zorubicin hydrochloride, mitoxantrone hydrochloride, idarubicin hydrochloride, and DDS preparations thereof (e.g., doxorubicin-including PEG liposome).

Examples of the "plant-derived anticancer agents" include etoposide, etoposide phosphate, vinblastine sulfate, vincristine sulfate, vindesine sulfate, teniposide, paclitaxel, docetaxel, cabazitaxel, vinorelbine, and DDS preparations thereof.

Examples of the "immunotherapeutic agents (BRM)" include picibanil, krestin, sizofiran, lentinan, ubenimex, interferons, interleukins, macrophage colony-stimulating factor, granulocyte colony-stimulating factor, erythropoietin, lymphotoxin, BCG vaccine, *Corynebacterium parvum*, levamisole, polysaccharide K, procodazole, anti-CTLA4 antibodies (e.g., ipilimumab, tremelimumab), anti-PD-1 antibodies (e.g., nivolumab, pembrolizumab), and anti-PD-L1 antibody.

Example of the "cell growth factors" in the "medicaments inhibiting the action of cell growth factors or cell growth factor receptors" include any substances that promote cell proliferation, which are normally peptides having not more than 20,000 molecular weight that are capable of exhibiting their activity at low concentrations by binding to a receptor, including (1) EGF (epidermal growth factor) or substances possessing substantially the same activity as EGF [e.g., TGFα], (2) insulin or substances possessing substantially the same activity as insulin [e.g., insulin, IGF (insulin-like growth factor)-1, IGF-2], (3) FGF (fibroblast growth factor) or substances possessing substantially the same activity as FGF [e.g., acidic FGF, basic FGF, KGF (keratinocyte growth factor), FGF-10], and (4) other cell growth factors [e.g., CSF (colony stimulating factor), EPO (erythropoietin), IL-2 (interleukin-2), NGF (nerve growth factor), PDGF (platelet-derived growth factor), TGFβ (transforming growth factor β), HGF (hepatocyte growth factor), VEGF (vascular endothelial growth factor), heregulin, angiopoietin].

Examples of the "cell growth factor receptors" include any receptors capable of binding to the aforementioned cell growth factors, including EGF receptor, heregulin receptor (e.g., HER3), insulin receptor, IGF receptor-1, IGF receptor-2, FGF receptor-1 or FGF receptor-2, VEGF receptor, angiopoietin receptor (e.g., Tie2), PDGF receptor and the like.

Examples of the "medicaments inhibiting the action of cell growth factors or cell growth factor receptors" include EGF inhibitor, TGFα inhibitor, heregulin inhibitor, insulin inhibitor, IGF inhibitor, FGF inhibitor, KGF inhibitor, CSF inhibitor, EPO inhibitor, IL-2 inhibitor, NGF inhibitor, PDGF inhibitor, TGFβ inhibitor, HGF inhibitor, VEGF inhibitor, angiopoietin inhibitor, EGF receptor inhibitor, HER2 inhibitor, HER4 inhibitor, insulin receptor, IGF-1 receptor inhibitor, IGF-2 receptor inhibitor, FGF receptor-1 inhibitor, FGF receptor-2 inhibitor, FGF receptor-3 inhibitor, FGF receptor-4 inhibitor, VEGF receptor inhibitor, Tie-2 inhibitor, PDGF receptor inhibitor, Abl inhibitor, Raf inhibitor, FLT3 inhibitor, c-Kit inhibitor, Src inhibitor, PKC inhibitor, Smo inhibitor, ALK inhibitor, ROR1 inhibitor, Trk inhibitor, Ret inhibitor, mTOR inhibitor, Aurora inhibitor, PLK inhibitor, MEK (MEK1/2) inhibitor, MET inhibitor, CDK inhibitor, Akt inhibitor, ERK inhibitor, PI3K inhibitor and the like. More specifically, anti-VEGF antibody (e.g., Bevacizumab, Ramucurumab), anti-HER2 antibody (e.g., Trastuzumab, Pertuzumab), anti-EGFR antibody (e.g., Cetuximab, Panitumumab, Matuzumab, Nimotuzumab), anti-HGF antibody, Imatinib, Erlotinib, Gefitinib, Sorafenib, Sunitinib, Dasatinib, Lapatinib, Vatalanib, Ibrutinib, Bosutinib, Cabozantinib, Crizotinib, Alectinib, Vismodegib, Axitinib, Motesanib, Nilotinib, 6-[4-(4-ethylpiperazin-1-ylmethyl)phenyl]-N-[1(R)-phenylethyl]-7H-pyrrolo[2,3-d]pyrimidin-4-amine (AEE-788), Vandetanib, Temsirolimus, Everolimus, Enzastaurin, Tozasertib, 2-[N-[3-[4-[5-[N-(3-fluorophenyl)carbamoylmethyl]-1H-pyrazol-3-ylamino]quinazolin-7-yloxy]propyl]-N-ethylamino]ethyl phosphate (AZD-1152), 4-[9-chloro-7-(2,6-difluorophenyl)-5H-pyrimido[5,4-d][2]benzazepin-2-ylamino]benzoic acid, N-[2-methoxy-5-[(E)-2-(2,4,6-trimethoxyphenyl)vinylsulfonylmethyl]phenyl]glycine sodium salt (ON-1910Na), Volasertib, Selumetinib, Trametinib, N-[2(R),3-dihydroxypropoxy]-3,4-difluoro-2-(2-fluoro-4-iodophenylamino)benzamide (PD-0325901), Bosutinib, Regorafenib, Afatinib, Idelalisib, Ceritinib, Dabrafenib and the like may be used.

In addition to the aforementioned drugs, L-asparaginase, L-arginase, arginine deiminase, aceglatone, procarbazine hydrochloride, protoporphyrin-cobalt complex salt, mercuric hematoporphyrin-sodium, topoisomerase I inhibitors (e.g., irinotecan, topotecan, indotecan, Indimitecan), topoisomerase II inhibitors (e.g., sobuzoxane), differentiation inducers (e.g., retinoid, vitamin D), other angiogenesis inhibitors (e.g., humagillin, shark extract, COX-2 inhibitor), α-blockers (e.g., tamsulosin hydrochloride), bisphosphonic acids (e.g., pamidronate, zoledronate), thalidomide, lenalidomide, pomalidomide, azacytidine, decitabine, proteasome inhibitors (e.g.,bortezomib, carfilzomib, ixazomib), NEDD8 inhibitors (e.g., Pevonedistat), UAE inhibitors, PARP inhibitors (e.g., Olaparib, Niraparib, Veliparib), antitumor antibodies such as anti-CD20 antibodies (e.g., Rituximab, Obinutuzumab), anti-CCR4 antibodies (e.g., Mogamulizumab) and the like, antibody-drug conjugates (e.g., trastuzumab emtansine, Brentuximab vedotin) and the like may also be used as a concomitant drug.

By combining a compound of the present disclosure and a concomitant drug, a superior effect such as (1) the dose may be reduced as compared to single administration of a compound of the present disclosure or a concomitant drug, (2) the drug to be combined with a compound of the present disclosure may be selected according to the condition of patients (mild case, severe case and the like), (3) the period of treatment may be set longer, (4) a sustained treatment effect may be designed, (5) a synergistic effect may be afforded by a combined use of a compound of the present disclosure and a concomitant drug, and the like, may be achieved.

In the present specification, a compound of the present disclosure and a concomitant drug used in combination are referred to as the "combination agent of the present disclosure".

For use of the combination agent of the present disclosure, the administration time of a compound of the present disclosure and the concomitant drug is not restricted, and a compound of the present disclosure and the concomitant drug can be administered to an administration subject simultaneously, or may be administered at different times. When administered at a time interval, the interval differs depending on the effective ingredient to be administered, dosage form and administration method, and for example, when the concomitant drug is administered first, a compound of the present disclosure may be administered within time range of from 1 min to 3 days, typically from 10 min to 1 day, or from 15 min to 1 hr after administration of the concomitant drug. When a compound of the present disclosure is administered first, the concomitant drug is administered within time range of from 1 min to 1 day, typically from 10 min to 6 hrs, or from 15 min to 1 hr after administration of a compound of the present disclosure. The dosage of the concomitant drug may be determined according to the dose clinically set, and may be appropriately selected depending on the administration subject, administration route, disease, combination and the like.

Examples of the administration mode of the combined use of a compound of the present disclosure and the concomitant drug include the following methods: (1) A compound of the present disclosure and the concomitant drug are simultaneously produced to give a single preparation, which is then administered. (2) A compound of the present disclosure and the concomitant drug are separately produced to give two kinds of preparations which are administered simultaneously by the same administration route. (3) A compound of the present disclosure and the concomitant drug are separately produced to give two kinds of preparations which are administered by the same administration route at different times. (4) A compound of the present disclosure and the concomitant drug are separately produced to give two kinds of preparations which are administered simultaneously by different administration routes. (5) A compound of the present disclosure and the concomitant drug are separately produced to give two kinds of preparations which are administered by different administration routes at different times (e.g., a compound of the present disclosure and the concomitant drug are administered in this order, or in the reverse order).

The dose of the concomitant drug may be appropriately determined in accordance with its clinical dose, and the ratio of a compound of the present disclosure and the concomitant drug may be appropriately determined depending on the administration subject, administration route, target disease, symptom, combination, and the like. For example, when the administration subject is human, the concomitant drug is used in 0.01 to 100 (parts by weight), relative to 1 part by weight of a compound of the present disclosure.

Furthermore, a compound of the present disclosure or the combination agent of the present disclosure may be used concurrently with a non-drug therapy. To be precise, a compound of the present disclosure or the combination agent of the present disclosure may be combined with a non-drug therapy such as (1) surgery, (2) hypertensive chemotherapy using angiotensin II etc., (3) gene therapy, (4) thermotherapy, (5) cryotherapy, (6) laser cauterization and (7) radiotherapy.

For example, by using a compound of the present disclosure or the combination agent of the present disclosure before or after the above-mentioned surgery and the like, or before or after a combined treatment of two or three kinds thereof, effects such as prevention of emergence of resistance, prolongation of Disease-Free Survival, suppression of cancer metastasis or recurrence, prolongation of life and the like may be afforded.

In addition, it is possible to combine a treatment with a compound of the present disclosure or the combination agent of the present disclosure with a supportive therapy: (i) administration of antibiotic (e.g., β-lactam type such as pansporin and the like, macrolide type such as clarithromycin and the like) for the complication with various infectious diseases, (ii) administration of high-calorie transfusion, amino acid preparation or general vitamin preparation for the improvement of malnutrition, (iii) administration of morphine for pain mitigation, (iv) administration of a pharmaceutical agent for ameliorating side effects such as nausea, vomiting, anorexia, diarrhea, leucopenia, thrombocytopenia, decreased hemoglobin concentration, hair loss, hepatopathy, renopathy, DIC, fever and the like and (v) administration of a pharmaceutical agent for suppressing multiple drug resistance of cancer and the like.

In one embodiment, the disclosure provides a kit comprising two or more separate pharmaceutical compositions, at least one of which contains a compound of Formula (I) or pharmaceutically acceptable salt thereof. A kit of this disclosure may be used for administration of different dosage forms, for example, oral and parenteral, for administration of the separate compositions at different dosage intervals, or for titration of the separate compositions against one another. To assist with compliance, a kit of the disclosure typically comprises directions for administration.

### EXAMPLES

### EXEMPLIFICATION

As depicted in the Examples below, in certain exemplary embodiments, compounds are prepared according to the following general procedures. It will be appreciated that, although the general methods depict the synthesis of certain compounds of the present invention, the following general methods, and other methods known to one of ordinary skill in the art, can be applied to all compounds and subclasses and species of each of these compounds, as described herein.

### Definitions

- AA: LCMS method using ammonium acetate
- Ac: acetate
- ACN: acetonitrile
- atm: atmosphere
- aq: aqueous
- BBN: borabicyclo(3.3.1)nonane
- Bn: benzyl
- Boc: *tert*-butoxycarbonyl
- (Bpin)₂: bis(pinacolato)diboron
- *t*Bu: *tert*-butyl
- Bz: benzoyl
- C: Celsius
- DBAD: di-*tert*-butyl azodicarboxylate
- DBU: 1,8-diazabicyclo[5.4.0]undec-7-ene
- DCM: dichloromethane
- DEAD: diethyl azodicarboxylate
- DIAD: diisopropyl azodicarboxylate
- DIPEA: *N*,*N-*diisopropylethylamine
- DMAP: 4-dimethylaminopyridine
- DMF: *N*,*N-*dimethylformamide
- DMSO: dimethylsulfoxide
- DMTr: 4,4'-dimethoxytrityl
- DPPA: diphenyl phosphorylazide
- Et: ethyl
- EtOH: ethanol
- EtOAc: ethyl acetate
- FA: LCMS method using formic acid
- h: hours
- HPLC: high pressure liquid chromatography
- IC₅₀: inhibitory concentration 50%
- IPC: diisopinocampheyl
- LCMS: liquid chromatography mass spectrometry
- LDA: lithium diisopropylamide
- LHMDS: lithium bis(trimethylsilyl)amide
- *m*/*z*: mass to charge
- MHz: mega hertz
- Me: methyl
- MeOH: methanol
- min: minutes
- mL: milliliters
- MS: mass spectrum
- nBu: n-butane
- NMP: 1-methyl-2-pyrrolidinone
- NMR: nuclear magnetic resonance
- PE: petroleum ether
- Ph: phenyl
- psi: pounds per square inch
- Pyr: pyridine
- rt: room temperature
- T3P: 2,4,6-tripropyl-1,3,5,2,4,6-trioxatriphosphorinane-2,4,6-trioxide
- TBAF: tetrabutylammonium fluoride
- TBS: *tert*-butyldimethylsilyl
- TBTU: *O*-(benzotriazol-1-yl)-*N*,*N*,*N'*,*N'*-tetramethyluronium tetrafluoroborate
- TEA: triethylamine
- TFA: trifluoroacetic acid
- TIDPSi: 1,1,3,3-tetraisopropyldisiloxane
- TIPS: triisopropylsilyl
- THF: tetrahydrofuran
- TsOH: p-toluene sulfonic acid
- UPLC: ultra performance liquid chromatography
- Xantphos: 4,5-bis(diphenylphosphino)-9,9-dimethylxanthene

### Analytical Methods

### NMR conditions:

¹H NMR spectra were run on a 400 MHz Bruker spectrometer unless otherwise stated. ³¹P NMR spectra were run on a 400 MHz Bruker spectrometer and acquired with ¹H decoupling unless otherwise stated.

### LCMS conditions:

LCMS spectra were recorded on a Hewlett-Packard HP1100 or Agilent 1100 Series LC system connected to a Micromass mass spectrometer using reverse phase C18 columns. Various gradients and run times were selected in order to best characterize the compounds. Mobile phases were based on ACN/water or MeOH/water gradients and contained either 0.1% formic acid (methods indicated as **FA**) or 10 mM ammonium acetate (methods indicated as **AA**). One example of a solvent gradient that was used was 100% mobile phase A (mobile phase A = 99% water + 1% ACN + 0.1% formic acid) to 100% mobile phase B (mobile phase B = 95% ACN + 5% water + 0.1% formic acid) at a flow rate of 1 mL/min for a 16.5 min run.

In some cases, LCMS spectra were recorded on an Agilent 1290 Infinity UPLC system connected to an Agilent 6130 mass spectrometer, a Waters Acquity UPLC system connected to a Waters Acquity SQ mass spectrometer, or an Agilent 1100 Series HPLC system connected to a Waters Micromass ZQ mass spectrometer using reverse phase C18 columns. Various gradients and run times were selected in order to best characterize the compounds. Mobile phases were based on ACN/water or MeOH/water gradients and contained either 0.1% formic acid (methods indicated as **FA**) or 10 mM ammonium acetate (methods indicated as **AA**). One example of a solvent gradient that was used was 95% mobile phase A (mobile phase A = 99% water + 1% ACN + 0.1% formic acid) to 100% mobile phase B (mobile phase B = 95% ACN + 5% water + 0.1% formic acid) at a flow rate of 0.5 mL/min for a 5 min run.

### Preparative HPLC:

Preparative HPLC are conducted using 18x150 mm Sunfire C-18 columns eluting with water-ACN gradients using a Gilson instrument operated by 322 pumps with the UV/visible 155 detector triggered fraction collection set to between 200 nm and 400 nm. Mass gated fraction collection is conducted on an Agilent 1100 LC/MSD instrument.

One of ordinary skill in the art will recognize that modifications of the gradient, column length, and flow rate are possible and that some conditions may be more suitable for compound characterization than others, depending on the chemical species being analyzed.

### Preparative SFC:

Preparative SFC is conducted using 10, 20 or 30mm x 250mm ChiralPak columns (typically IA, IB, IC, ID, IE and IF), 10 or 20 mm x 250 mm Phenomenex Lux Cellulose-4, or 2-ethylpyridine columns eluting with appropriate percentages of supercritical carbon dioxide and alcohol containing 0.3% diethyl amine or 0.3% TEA or 0.3% formic acid or without any acid or base additives. Isocratic conditions with flow rates in the range of 10-100 mL/min and a column temperature of 40 °C are typical. Preparative SFC is conducted on A Jasco SFC prep purification system with UV/visible triggered fraction collection set to between 200 nm and 400 nm and back pressure regulation set to 10 MPa.

One of ordinary skill in the art will recognize that modifications of the gradient, column length, and flow rate are possible and that some conditions may be more suitable for compound characterization than others, depending on the chemical species being analyzed.

### Example 1

### 2-amino-9-[(5S,7R,8R,12aR,14R,15aS)-14-(6-amino-9H-purin-9-yl)-2-hydroxy-2,10,10-trioxidodecahydro-5,8-methanofuro[2,3-m][1,3,6,9,10,11,2] tetraoxathiazaphosphacyclotetradecin-7-yl]-1,9-dihydro-6H-purin-6-one - N,N-diethylethanamine (compound I-5)

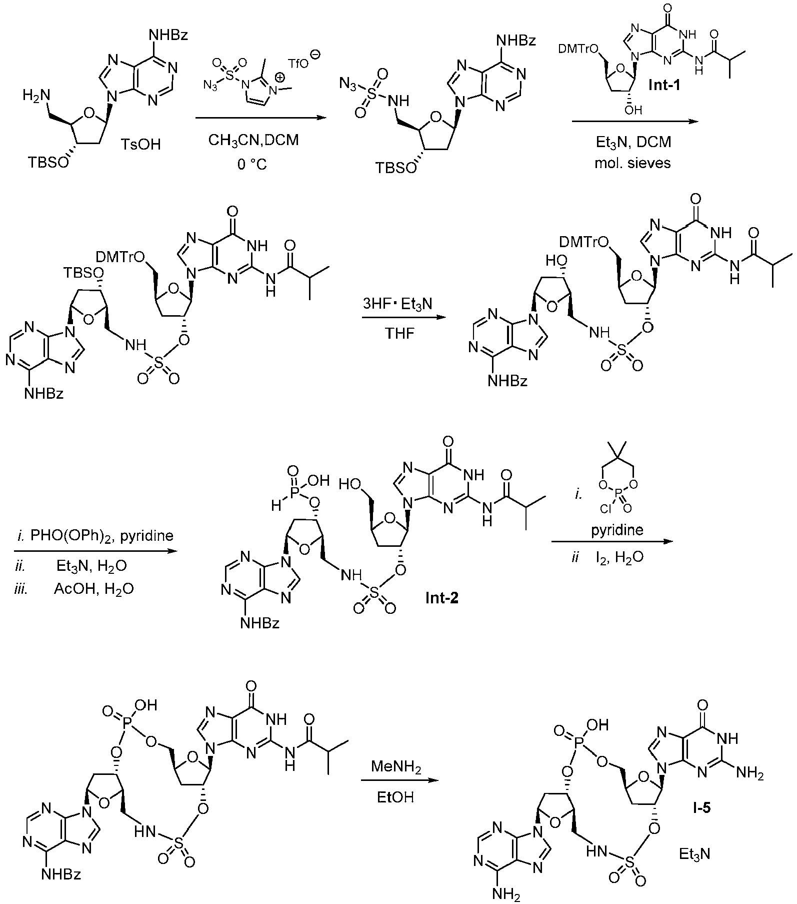

### Step 1: N-[9-[(2R,4S,SR)-5-[(azidosulfonylamino)methyl]-4-[tert-butyl(dimethyl) silyl]oxy-tetrahydrofuran-2-yl]purin-6-yl]benzamide

A mixture of N-[9-[(2R,4S,5R)-5-(aminomethyl)-4-[*tert*-butyl(dimethyl)silyl]oxy-tetrahydrofuran-2-yl]purin-6-yl]benzamide;4-methylbenzenesulfonic acid (prepared as described in Journal of Organic Chemistry, 57(17), 4569-70, 1992; 114 mg, 0.176 mmol) in anhydrous acetonitrile (0.47 mL) and anhydrous dichloromethane (0.47 mL) was cooled in an ice bath under a nitrogen atmosphere. Triethylamine (0.040 mL, 0.27 mmol) was added and the solution was stirred at 0 °C for 10 minutes. A solution of N-diazo-2,3-dimethyl-imidazol-3-ium-1-sulfonamide; trifluoromethanesulfonate (78.7 mg, 0.224 mmol) in anhydrous acetonitrile (0.12 mL) was added dropwise and the reaction mixture was allowed to warm to room temperature over 2 hours. The reaction mixture was concentrated under reduced pressure and the residue was purified using silica gel chromatography (0-5% EtOH in DCM) to provide N-[9-[(2R,4S,5R)-5-[(azidosulfonylamino)methyl]-4-[*tert*-butyl(dimethyl)silyl]oxy-tetrahydrofuran-2-yl]purin-6-yl]benzamide (0.075 g, 74%). LCMS (FA): *m*/*z* = 574.2 (M+H). ¹H NMR (400 MHz, CDCl₃) δ 0.15 (s, 6H), 0.94 (s, 9H), 2.29 (dd, *J*=13.36, 5.58 Hz, 1 H), 3.01 - 3.11 (m, 1 H), 3.58 (br d, *J*=4.39 Hz, 2 H), 4.31 (s, 1 H), 4.69 (br d, *J*=5.40 Hz, 1 H), 6.34 (dd, *J*=9.35, 5.46 Hz, 1 H), 7.53 - 7.58 (m, 2 H), 7.62 - 7.67 (m, 1 H), 8.04 (d, *J*=7.40 Hz, 2 H), 8.08 (s, 1 H), 8.86 (s, 1 H), 8.91 (s, 1 H), 9.96 (br t, *J*=5.14 Hz, 1 H).

### Step 2: [(2R,3R,5S)-5-[[bis(4-methoxyphenyl)-phenyl-methoxy]methyl]-2-[2-(2-methylpropanoylamino)-6-oxo-1H-purin-9-yl]tetrahydrofuran-3-yl] N-[[(2R,3S,5R)-5-(6-benzamidopurin-9-yl)-3-[tert-butyl(dimethyl)silyl]oxy-tetrahydrofuran-2-yl] methyl]sulfamate

A mixture of N-[9-[(2R,3R,5S)-5-[[bis(4-methoxyphenyl)-phenyl-methoxy]methyl]-3-hydroxy-tetrahydrofuran-2-yl]-6-oxo-1H-purin-2-yl]-2-methyl-propanamide (**Intermediate 1,** 80.7 mg, 0.126 mmol) in anhydrous dichloromethane (1.3 mL) was stirred with 4Å molecular sieves for 30 minutes. Solid N-[9-[(2R,4S,5R)-5-[(azidosulfonylamino)methyl]-4-[*tert-*butyl(dimethyl)silyl]oxy-tetrahydrofuran-2-yl]purin-6-yl]benzamide (72.4 mg, 0.126 mmol) was then added and the reaction mixture was stirred at room temperature for 5 hours. The solids were removed by filtration, washed with additional dichloromethane and the combined organics were concentrated. The residue obtained was purified using silica gel chromatography (0-15% EtOH in DCM) to afford [(2R,3R,5S)-5-[[bis(4-methoxyphenyl)-phenyl-methoxy]methyl]-2-[2-(2-methylpropanoylamino)-6-oxo-1H-purin-9-yl]tetrahydrofuran-3-yl] N-[[(2R,3S,5R)-5-(6-benzamidopurin-9-yl)-3-[*tert*-butyl(dimethyl)silyl]oxy-tetrahydrofuran-2-yl]methyl]sulfamate (37 mg, 25%) as a white solid. LCMS (FA): *m*/*z* = 1170.5 (M+H).

### Step 3: [(2R,3R,5S)-5-[[bis(4-methoxyphenyl)-phenyl-methoxy]methyl]-2-[2-(2-methylpropanoylamino)-6-oxo-1H-purin-9-yl]tetrahydrofuran-3-yl] N-[[(2R,3S,5R)-5-(6-benzamidopurin-9-yl)-3-hydroxy-tetrahydrofuran-2-yl]methyl]sulfamate

To a solution of [(2R,3R,5S)-5-[[bis(4-methoxyphenyl)-phenyl-methoxy]methyl]-2-[2-(2-methylpropanoylamino)-6-oxo-1H-purin-9-yl]tetrahydrofuran-3-yl] N-[[(2R,3S,5R)-5-(6-benzamidopurin-9-yl)-3-[*tert*-butyl(dimethyl)silyl]oxy-tetrahydrofuran-2-yl]methyl]sulfamate (67.0 mg, 0.057 mmol) in tetrahydrofuran (1.2 mL) in a polypropylene tube was added triethylamine trihydrofluoride (0.040 mL, 0.12 mmol) and triethylamine (0.016 mL, 0.12 mmol). The reaction solution was stirred at room temperature overnight, diluted with dichloromethane and washed successively with saturated aqueous NaHCO₃ and brine. The organic phase was dried over anhydrous MgSO₄, filtered and concentrated. The residue obtained was purified on silica gel chromatography (0-10% EtOH in DCM) to afford [(2R,3R,5S)-5-[[bis(4-methoxyphenyl)-phenyl-methoxy]methyl]-2-[2-(2-methylpropanoylamino)-6-oxo-1H-purin-9-yl]tetrahydrofuran-3-yl] N-[[(2R,3S,SR)-5-(6-benzamidopurin-9-yl)-3-hydroxy-tetrahydrofuran-2-yl]methyl]sulfamate (60.0 mg, 99%). LCMS (FA): *m*/*z* = 1056.4 (M+H).

### Step 4: [(2R,3S,5R)-5-(6-benzamidopurin-9-yl)-2-[[[(2R,3R,5S)-5-(hydroxymethyl)-2-[2-(2-methylpropanoylamino)-6-oxo-1H-purin-9-yl]tetrahydrofuran-3-yl] oxysulfonylamino]methyl]tetrahydrofuran-3-yl]oxyphosphinic acid (Intermediate 2)

Diphenyl phosphite (0.025 mL, 0.13 mmol) was added to a solution of [(2R,3R,5S)-5-[[bis(4-methoxyphenyl)-phenyl-methoxy]methyl]-2-[2-(2-methylpropanoylamino)-6-oxo-1H-purin-9-yl]tetrahydrofuran-3-yl] N-[[(2R,3S,5R)-5-(6-benzamidopurin-9-yl)-3-hydroxy-tetrahydrofuran-2-yl]methyl]sulfamate (67.0 mg, 0.063 mmol) in pyridine (0.12 mL) cooled to 0 °C. Upon complete addition, the reaction mixture was allowed to stir at room temperature for 1 hour. Triethylamine (0.063 mL, 0.44 mmol) and water (0.061 mL, 3.43mmol) were then added and stirring was continued at room temperature for 30 minutes. The reaction mixture was concentrated and co-evaporated with toluene three times. The residue was dissolved in acetic acid (0.15 mL, 2.6 mmol) and water (0.04 mL, 2.2 mmol), and allowed to stir at room temperature for 1 hour. Toluene (15 mL) was added and the reaction mixture was concentrated. The residue was dissolved in toluene and concentrated (2 x 15 mL) and then dried on high vacuum for 10 minutes. The crude compound was purified using silica gel chromatography (0-70% MeOH in DCM) to provide [(2R,3S,5R)-5-(6-benzamidopurin-9-yl)-2-[[[(2R,3R,5S)-5-(hydroxymethyl)-2-[2-(2-methylpropanoylamino)-6-oxo-1H-purin-9-yl]tetrahydrofuran-3-yl]oxysulfonylamino]methyl]tetrahydrofuran-3-yl]oxyphosphinic acid as a white solid (**Intermediate 2**, 39.6 mg, 76%). LCMS (FA): *m*/*z* = 818.3 (M+H).

### Step 5: N-(9-{(5S,7R,8R,12aR,14R,15aS)-2-hydroxy-7-[2-(isobutyrylamino)-6-oxo-1,6-dihydro-9H-purin-9-yl]-2,10,10-trioxidodecahydro-5,8-methanofuro[2,3-m][1,3,6,9,10,11,2]tetraoxathiazaphosphacyclotetradecin-14-yl}-9H-purin-6-yl)benzamide

[(2R,3S,5R)-5-(6-benzamidopurin-9-yl)-2-[[[(2R,3R,5S)-5-(hydroxymethyl)-2-[2-(2-methylpropanoylamino)-6-oxo-1H-purin-9-yl]tetrahydrofuran-3-yl]oxysulfonylamino]methyl]tetrahydrofuran-3-yl]oxyphosphinic acid (Intermediate 2, 38.3 mg, 0.047 mmol) was concentrated from anhydrous acetonitrile prior to use. The solid was dissolved in anhydrous pyridine (1.0 mL, 12.4 mmol) and solid 2-chloro-5,5-dimethyl-1,3,2-dioxaphosphorinane 2-oxide (31.5 mg, 0.164 mmol) was added. The reaction mixture was stirred at room temperature under an argon atmosphere for 45 minutes. Water (0.03 mL, 1.7mmol) and iodine (15.5 mg, 0.0609 mmol) were then added and stirring was continued for an additional 10 minutes. The reaction mixture was quenched with the addition of a solution of sodium thiosulfate (10 mg, 0.06 mmol) in water (1 mL). The solution was concentrated and the residue obtained was purified using silica gel chromatography (0-25% MeOH in DCM) to afford N-(9-{(5S,7R,8R,12aR,14R,15aS)-2-hydroxy-7-[2-(isobutyrylamino)-6-oxo-1,6-dihydro-9H-purin-9-yl]-2,10,10-trioxidodecahydro-5,8-methanofuro[2,3-m][1,3,6,9,10,11,2]tetraoxathiazaphosphacyclotetradecin-14-yl}-9H-purin-6-yl)benzamide was a white solid (30.0 mg, 78.5%). LCMS (FA): *m*/*z* = 816.5 (M+H).

### Step 6: 2-amino-9-[(5S,7R,8R,12aR,14R,15aS)-14-(6-amino-9H-purin-9-yl)-2-hydroxy-2,10,10-trioxidodecahydro-5,8-methanofuro[2,3-m][1,3,6,9,10,11,2] tetraoxathiazaphosphacyclotetradecin-7-yl]-1,9-dihydro-6H-purin-6-one - N,N-diethylethanamine (compound I-5)

N-(9-f(SS,7R,8R,12aR,14R,15aS)-2-hydroxy-7-[2-(isobutyrylamino)-6-oxo-1,6-dihydro-9H-purin-9-yl]-2,10,10-trioxidodecahydro-5,8-methanofuro[2,3-m][1,3,6,9,10,11,2]tetraoxathiazaphosphacyclotetradecin-14-yl}-9H-purin-6-yl)benzamide (27.8 mg, 0.034 mmol) was dissolved in a solution of methylamine (33% in EtOH, 1.7 mL, 13.63 mmol) and allowed to stir at room temperature for 90 minutes. The reaction mixture was concentrated and purified by reverse phase flash column chromatography (0-100% ACN in aqueous triethylammonium acetate (10 mM)) to provide 2-amino-9-[(5S,7R,8R,12aR,14R,15aS)-14-(6-amino-9H-purin-9-yl)-2-hydroxy-2,10,10-trioxidodecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine as a white solid (4.5 mg, 18 %). LCMS (FA): *m*/*z* = 642.1 (M+H), ¹H NMR (400 MHz, D₂O) δ 1.25 (br t, *J*=6.90 Hz, 9 H), 2.55 - 2.79 (m, 3 H), 2.83 (br s, 2 H), 3.17 (br d, *J*=7.15 Hz, 6 H), 3.44 (br d, *J*=13.05 Hz, 1 H), 3.54 - 3.71 (m, 1 H), 4.05 - 4.14 (m, 1 H), 4.23 (br d, *J*=9.66 Hz, 1 H), 4.28 - 4.36 (m, 1 H), 4.63 (br d, *J*=7.91 Hz, 2 H), 5.37 (br s, 1 H), 5.94 - 6.02 (m, 1 H), 6.12 (br d, *J*=6.78 Hz, 1 H), 6.45 (br s, 1 H), 7.37 (br s, 1 H), 7.93 (br s, 1 H), 8.06 (br s, 1 H). ³¹P NMR (162 MHz, D₂O) δ -0.67 (s, 1 P).

### Example 1A

The compound listed below was prepared as described in Example 1, steps 2-6, substituting the starting material shown in the table for N-[9-[(2R,4S,5R)-5-[(azidosulfonylamino)methyl]-4-[*tert*-butyl(dimethyl)silyl]oxy-tetrahydrofuran-2-yl]purin-6-yl]benzamide.

| I Number | Starting material | Product | Analytical data |
|---|---|---|---|
| I-18* | 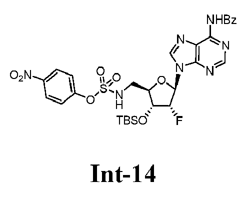 | 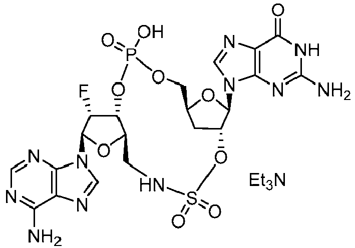 | LCMS (AA): *m*/*z* = 660.1 (M+H); ³¹P NMR (162 MHz, DMSO-d₆) δ -2.65 (s, 1P). ¹⁹F NMR (376 MHz, DMSO-d₆) δ - 197.18 (s, 1F). |

| | | | |
|---|---|---|---|
| * The conditions used in Step 2 are described in Example 8, step 2 heating at 40 °C for 16 h. | | | |

### Example 2

### 2-amino-9-[(2S,5S,7R,8R,12aR,14R,15aS)-14-(6-amino-9H-purin-9-yl)-2,10,10-trioxido-2-sulfanyldecahydro-5,8-methanofuro[2,3-m][1,3,6,9,10,11,2] tetraoxathiazaphosphacyclotetradecin-7-yl]-1,9-dihydro-6H-purin-6-one-N,N-diethylethanamine or 2-amino-9-[(2R,5S,7R,8R,12aR,14R,15aS)-14-(6-amino-9H-purin-9-yl)-2,10,10-trioxido-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine (compound I-3)

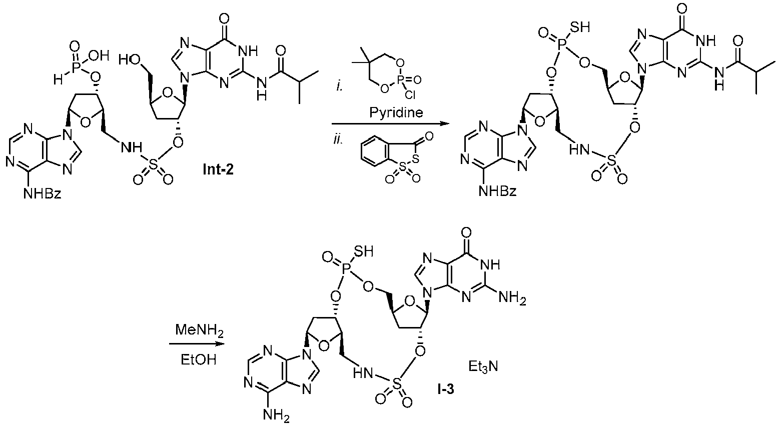

### Step 1: N-(9-{(2S,5S,7R,8R,12aR,14R,15aS)-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2]tetraoxathiazaphosphacyclotetradecin-14-yl}-9H-purin-6-yl)benzamide - pyridine or N-(9-{(2R,5S,7R,8R,12aR,14R,15aS)-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2] tetraoxathiazaphosphacyclotetradecin-14-yl}-9H-purin-6-yl)benzamide - pyridine

[(2R,3S,5R)-5-(6-benzamidopurin-9-yl)-2-[[[(2R,3R,5S)-5-(hydroxymethyl)-2-[2-(2-methylpropanoylamino)-6-oxo-1H-purin-9-yl]tetrahydrofuran-3-yl]oxysulfonylamino]methyl]tetrahydrofuran-3-yl]oxyphosphinic acid (**Intermediate 2,** 0.116 g, 0.13 mmol) was evaporated with acetonitrile prior to use. The resulting solid was dissolved in pyridine (2.5 mL, 31.0 mmol) and 2-chloro-5,5-dimethyl-1,3,2-dioxaphosphorinane 2-oxide (0.086 mg, 0.45 mmol) was added. The reaction mixture was allowed to stir at room temperature for 45 min. Water (0.080 mL, 4.5 mmol) and 3H-1,2-benzodithiol-3-one 1,1-dioxide (0.037 g, 0.18 mmol) were then added and stirring was continued at room temperature for 30 min. The reaction mixture was concentrated and co-evaporated with acetonitrile and toluene twice. The crude compound was purified using silica gel chromatography (5-60% MeOH in EtOAc) to provide N-(9-{(2S,5S,7R,8R,12aR,14R,15aS)-7-[2-(isobutyrylamino)-6-oxo-l,6-dihydro-9H-purin-9-yl]-2,10,10-trioxido-2-sulfanyldecahydro-5,8-methanofuro[2,3-m][1,3,6,9,10,11,2] tetraoxathiazaphosphacyclotetradecin-14-yl}-9H-purin-6-yl)benzamide - pyridine or N-(9-{(2R,5S,7R,8R,12aR,14R,15aS)-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2] tetraoxathiazaphosphacyclotetradecin-14-yl}-9H-purin-6-yl)benzamide (as a pyridine salt) (60.3 mg, 57%) . LCMS (AA): *m*/*z* = 832.2 (M+H), ¹H NMR (400 MHz, CD₃OD) δ 1.00 (d, *J*=6.90 Hz, 3 H), 1.15 - 1.20 (m, 3 H), 2.66 - 2.77 (m, 2 H), 2.79 - 2.89 (m, 1 H), 2.97 - 3.07 (m, 1 H), 3.11 - 3.16 (m, 1 H), 3.41 (dd, *J*=14.05, 3.51 Hz, 1 H), 3.53 - 3.69 (m, 2 H), 4.07 - 4.17 (m, 1 H), 4.31 (dt, *J*=7.78, 3.76 Hz, 1 H), 4.44 - 4.53 (m, 1 H), 4.55 - 4.62 (m, 1 H), 5.43 - 5.50 (m, 2 H), 6.02 - 6.11 (m, 2 H), 6.59 (t, *J*=6.46 Hz, 1 H), 7.44 - 7.68 (m, 6 H), 7.90 - 8.00 (m, 2 H), 8.00 (d, *J*=2.01 Hz, 1 H), 8.09 (d, *J*=7.60 Hz, 2 H), 8.16 (s, 1 H), 8.40 (s, 1 H), 8.49 (s, 1 H).

### Step 2: 2-amino-9-[(2S,5S,7R,8R,12aR,14R,15aS)-14-(6-amino-9H-purin-9-yl)-2,10,10-trioxido-2-sulfanyldecahydro-5,8-methanofuro[2,3-m][1,3,6,9,10,11,2] tetraoxathiazaphosphacyclotetradecin-7-yl]-1,9-dihydro-6H-purin-6-one - N,N-diethylethanamine or 2-amino-9-[(2R,5S,7R,8R,12aR,14R,15aS)-14-(6-amino-9H-purin-9-yl)-2,10,10-trioxido-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine

N-(9-{(2S,5S,7R,8R,12aR,14R,15aS)-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2]tetraoxathiazaphosphacyclotetradecin-14-yl}-9H-purin-6-yl)benzamide or N-(9-{(2R,5S,7R,8R,l2aR,14R,15aS)-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2] tetraoxathiazaphosphacyclotetradecin-14-yl}-9H-purin-6-yl)benzamide (55.6 mg, 0.067 mmol) was taken up in a methylamine solution (33% in EtOH, 3.3 mL, 27.0 mmol) and the reaction mixture was allowed to stir at room temperature for 90 minutes. The reaction mixture was concentrated and purified by reverse phase flash column chromatography (0-20% ACN in aqueous triethylammonium acetate (10 mM)) to provide 2-amino-9-[(2S,5S,7R,8R,12aR, 14R,15aS)-14-(6-amino-9H-purin-9-yl)-2,10,10-trioxido-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine or 2-amino-9-[(2R,5S,7R,8R,12aR,14R,15aS)-14-(6-amino-9H-purin-9-yl)-2,10,10-trioxido-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine (**compound I-3,** 36 mg, 71%). LCMS (FA): *m*/*z* = 658.2 (M+H), ¹H NMR (400 MHz, D₂O) δ 1.25 (t, *J*=7.34 Hz, 9 H), 2.58 - 2.79 (m, 2 H), 2.83 - 3.00 (m, 2 H), 3.17 (d, *J*=7.40 Hz, 6 H), 3.42 - 3.52 (m, 1 H), 3.67 (br dd, *J*=13.18, 3.26 Hz, 1 H), 4.16 (br dd, *J*=11.54, 4.02 Hz, 1 H), 4.26 - 4.41 (m, 2 H), 4.56 - 4.71 (m, 2 H), 5.52 (quin, *J*=7.59 Hz, 1 H), 5.96 - 6.03 (m, 1 H), 6.07 - 6.18 (m, 1 H), 6.49 (br dd, *J*=7.22, 2.82 Hz, 1 H), 7.32 - 7.46 (m, 2 H), 7.94 (s, 1 H), 8.09 (s, 1 H). ³¹P NMR (162 MHz, D₂O) δ 55.58 (s, 1 P).

### Example 3

### 4-({(1R,3R,4S)-3-({[tert-butyl(dimethyl)silyl]oxy}methyl)-4-[(triisopropylsilyl)oxy]cyclopentyl}oxy)pyrimidine (Intermediate 3)

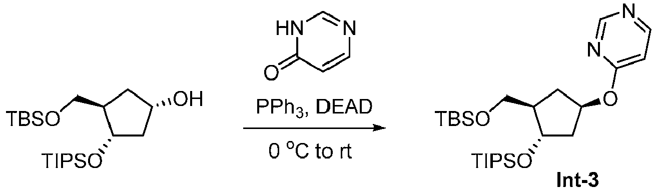

(1S,3R,4S)-3-(((*tert*-butyldimethylsilyl)oxy)methyl)-4-((triisopropylsilyl)oxy)cyclopentanol (14.6 g, 36.2 mmol), PPh₃ (10.90 g, 41.7 mmol) and 4(3H)-pyrimidone (4.26 g, 43.5 mmol) was dissolved in THF (200 mL) under an atmosphere of argon. The solution was cooled to 0 °C. DEAD (6.28 mL, 39.9 mmol) was added and the reaction mixture was allowed to stir at room temperature for 1 day. The reaction mixture was concentrated to dryness and adsorbed onto Celite. The crude compound was purified by silica gel chromatography (0-15% EtOAc in hexaness) to provide 4-({(1S,3R,4S)-3-({[*tert-*butyl(dimethyl)silyl]oxy}methyl)-4-[(triisopropylsilyl)oxy]cyclopentyl}oxy)pyrimidine (15.6 g, 90 %). LCMS (FA): m/z = 481.4 (M+H).

### Example 4

### [(1R,2S,4R)-4-purin-9-yl-2-triisopropylsilyloxy-cyclopentyl]methanamine (Intermediate 5)

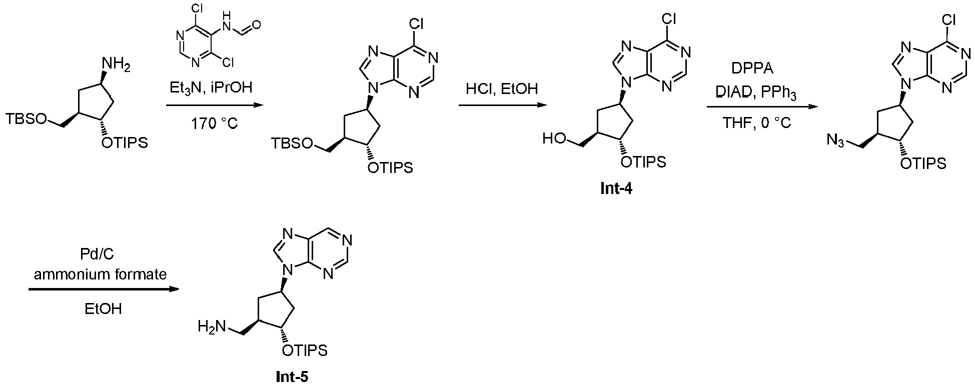

### Step 1: tert-butyl-[[(1R,2S,4R)-4-(6-chloropurin-9-yl)-2-triisopropylsilyloxy-cyclopentyl]methoxy]-dimethyl-silane

Five vials were each charged with N-(4,6-dichloropyrimidin-5-yl)formamide (2.0 g, 9.9 mmol), (1R,3R,4S)-3-[[*tert*-butyl(dimethyl)silyl]oxymethyl]-4-triisopropylsilyloxy-cyclopentanamine (prepared as reported in PCT Int. Appl., 2016004136, 2.13 g, 9.9 mmol) and triethylamine (2.76 mL, 19.8 mmol) in 2-propanol (15 mL). Each vial was sealed and then heated at 170 °C for 10 minutes in a microwave reactor. The five reaction mixtures were combined and concentrated under reduced pressure and the residue purified using silica gel chromatography (0-40% EtOAc in hexanes) to afford tert-butyl-[[(1R,2S,4R)-4-(6-chloropurin-9-yl)-2-triisopropylsilyloxy-cyclopentyl]methoxy]-dimethyl-silane (10.6 g. 39%). ¹H NMR (400 MHz, CD₃OD) δ 0.09 (d, *J*=2.89 Hz, 6 H), 0.92 (s, 9 H), 1.13 (m, 21 H), 1.93 - 2.03 (m, 1 H), 2.26 - 2.35 (m, 2 H), 2.51 - 2.60 (m, 2 H), 3.79 (d, *J*=5.40 Hz, 2 H), 4.59 - 4.67 (m, 1 H), 5.29 (quin, *J*=8.69 Hz, 1 H), 8.60 (s, 1 H), 8.70 (s, 1 H).

### Step 2: [(1R,2S,4R)-4-(6-chloropurin-9-yl)-2-triisopropylsilyloxy-cyclopentyl] methanol (Intermediate 4)

To a solution of *tert*-butyl-[[(1R,2S,4R)-4-(6-chloropurin-9-yl)-2-triisopropylsilyloxy-cyclopentyl]methoxy]-dimethyl-silane (10.6 g, 19.6 mmol) in ethanol (95 mL) was added an aqueous solution of hydrochloric acid (36%, 4.07 mL, 48.9 mmol). The reaction mixture was stirred at 35 °C for 1 hour. Upon cooling to room temperature, the reaction mixture was treated with a saturated aqueous NaHCO₃ solution and the resulting mixture was concentrated to remove ethanol and then extracted with EtOAc. The organic phase was washed with water and brine respectively, dried over anhydrous Na₂SO₄ and concentrated. The crude material was purified using silica gel chromatography (0-60% EtOAc in hexanes) to afford [(1R,2S,4R)-4-(6-chloropurin-9-yl)-2-triisopropylsilyloxy-cyclopentyl]methanol (**Intermediate 4**, 6.3 g, 76%). LCMS (FA): *m*/*z* = 425.3 (M+H).

### Step 3: [(1S,2R,4R)-2-(azidomethyl)-4-(6-chloropurin-9-yl)cyclopentoxy]-triisopropyl-silane

A solution of [(1R,2S,4R)-4-(6-chloropurin-9-yl)-2-triisopropylsilyloxy-cyclopentyl]methanol (**Intermediate 4,** 1.30 g, 3.1 mmol) and triphenylphosphine (1.13 g, 4.30 mmol) in tetrahydrofuran (12.0 mL) was cooled in an ice bath. Diisopropyl azodicarboxylate (0.85 mL, 4.2 mmol) was added dropwise by syringe over 10 minutes. Upon complete addition, a solution of diphenylphosphoryl azide (0.920 mL, 4.3 mmol) in tetrahydrofuran (3.0 mL) was added dropwise by syringe and the resulting mixture was allowed to warm to room temperature overnight. The reaction mixture was then concentrated under reduced pressure, and the crude material was purified using silica gel chromatography (0-40% EtOAc in hexanes) to afford [(1S,2R,4R)-2-(azidomethyl)-4-(6-chloropurin-9-yl)cyclopentoxy]-triisopropyl-silane (1.3 g, 94%). ¹H NMR (400 MHz, CDCl₃) δ 1.10 (m, 21 H), 1.92 - 2.00 (m, 1 H), 2.26 - 2.39 (m, 2 H), 2.48 - 2.56 (m, 1 H), 2.61 - 2.69 (m, 1 H), 3.55 - 3.65 (m, 2 H), 4.46 - 4.51 (m, 1 H), 5.19 (t, *J*=8.53 Hz, 1 H), 8.18 (s, 1 H), 8.75 (s, 1 H).

### Step 4: [(1R,2S,4R)-4-purin-9-yl-2-triisopropylsilyloxy-cyclopentyl]methanamine (Intermediate 5)

A suspension of [(1S,2R,4R)-2-(azidomethyl)-4-(6-chloropurin-9-yl)cyclopentoxy]-triisopropyl-silane (0.998 g, 2.22 mmol) and ammonium formate (2.80 g, 44.4 mmol) in ethanol (37 mL) was thoroughly degassed and palladium catalyst (10% on carbon, 0.15 g, 0.141 mmol) was added. The reaction mixture was heated at 60 °C under an argon atmosphere for 2 hours. The reaction mixture was cooled to room temperature and filtered through a pad of Celite. The filtrate was concentrated under reduced pressure, and the crude material was purified using silica gel chromatography (0-10% MeOH in DCM) to afford [(1R,2S,4R)-4-purin-9-yl-2-triisopropylsilyloxy-cyclopentyl]methanamine (**Intermediate 5,** 0.864 g, 90%). LCMS (FA): *m*/*z* = 390.3 (M+H).

### Example 5

The compound listed below (**Intermediate 6**) was prepared as described in Example 4, steps 2-4, substituting the starting material shown in the table for *tert*-butyl-[[(1R,2S,4R)-4-(6-chloropurin-9-yl)-2-triisopropylsilyloxy-cyclopentyl]methoxy]-dimethylsilane.

| Starting material | Product | Analytical data |
|---|---|---|
| 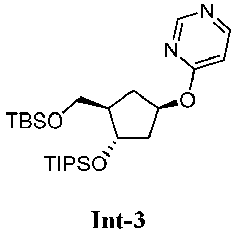 | 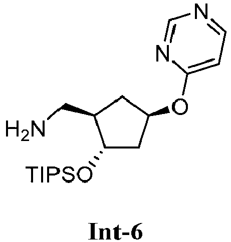 | LCMS (AA): *m*/*z* = 366.6 (M+H) |

### Example 6

### {(1R,2S,4R)-4-(3H-[1,2,3]triazolo[4,5-d]pyrimidin-3-yl)-2-[(triisopropylsilyl)oxy]cyclopentyl}methanol (Intermediate 18)

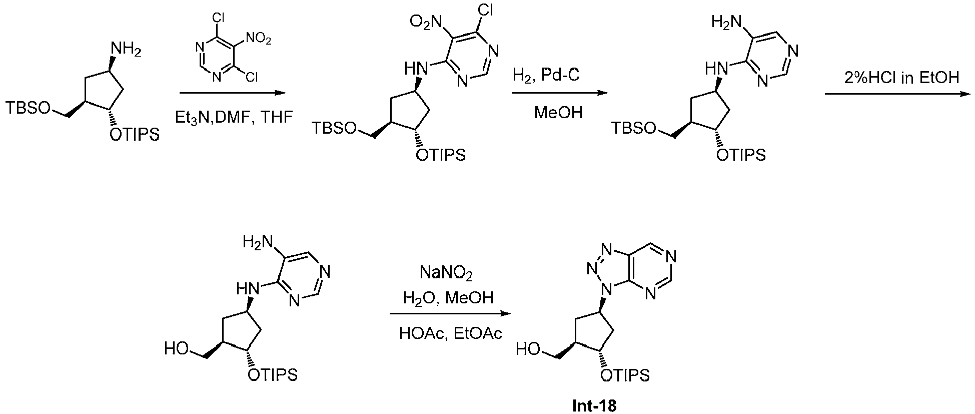

### Step 1: N-{(1R,3R,4S)-3-({[tert-butyl(dimethyl)silyl]oxy}methyl)-4-[(triisopropylsilyl)oxy]cyclopentyl}-6-chloro-5-nitropyrimidin-4-amine

A solution of (1R,3R,4S)-3-(((*tert*-butyldimethylsilyl)oxy)methyl)-4-((triisopropylsilyl)oxy)cyclopentan-1-amine (1.05 g, 2.48 mmol) and 4,6-dichloro-5-nitropyrimidine (578 mg, 2.98 mmol) in a mixture of THF (12.5 mL), DMF (6.0mL) and triethylamine (0.88 mL, 6.2 mmol) was stirred at room temperature for 1 hour. The reaction solution was partitioned between saturated aqueous NaHCO₃ (30 mL) and EtOAc (50 mL). Upon separation, the aqueous phase was extracted with additional EtOAc (2x50 mL). The combined organic phases were dried over anhydrous MgSO₄ and concentrated to afford N-{(1R,3R,4S)-3-({[*tert*-butyl(dimethyl)silyl]oxy}methyl)-4-[(triisopropylsilyl)oxy]cyclopentyl}-6-chloro-5-nitropyrimidin-4-amine (1.31 g, 94 %) which was carried on to step 2 without further purification. LCMS (FA): *m*/*z* = 559.2 (M+H).

### Step 2: N4-[(1R,3R,4S)-3-[[tert-butyl(dimethyl)silyl]oxymethyl]-4-triisopropylsilyloxy-cyclopentyl]pyrimidine-4,5-diamine

A degassed mixture of N-[(1R,3R,4S)-3-[[*tert*-butyl(dimethyl)silyl]oxymethyl]-4-triisopropylsilyloxy-cyclopentyl]-6-chloro-5-nitro-pyrimidin-4-amine (1.31 g, 2.34 mmol) and palladium (10% on carbon, 0.40 g, 0.36 mmol) in MeOH (80.0 mL) was stirred under an atmosphere of H₂ for 1 hour. The reaction mixture was filtered through a pad of Celite and the solvent was evaporated to afford crude N4-[(1R,3R,4S)-3-[[*tert*-butyl(dimethyl)silyl]oxymethyl]-4-triisopropylsilyloxy-cyclopentyl]pyrimidine-4,5-diamine (1.16g, 100%) which was carried on into step 3 without further purification. LCMS (FA): *m*/*z* = 495.3 (M+H).

### Step 3: [(1R,2S,4R)-4-[(5-aminopyrimidin-4-yl)amino]-2-triisopropylsilyloxy-cyclopentyl]methanol

To N4-[(1R,3R,4S)-3-[[*tert*-butyl(dimethyl)silyl]oxymethyl]-4-triisopropylsilyloxy-cyclopentyl]pyrimidine-4,5-diamine (1.16 g, 2.34 mmol) in ethanol (10 mL) at 0 °C was slowly added a pre-mixed solution of hydrochloric acid (0.51 mL, 12.0 M in water) and ethanol (15 mL). Upon complete addition, the reaction mixture was warmed to room temperature and stirred for one hour. Excess HCl was quenched with the addition of saturated aqueous NaHCO₃ (30 mL). The majority of ethanol was removed under reduced pressure and the aqueous solution was extracted with DCM (3x50 mL). The combined organic phases were concentrated to afford crude [(1R,2S,4R)-4-[(5-aminopyrimidin-4-yl)amino]-2-triisopropylsilyloxy-cyclopentyl]methanol (0.890 g, 90 %) which was carried on into step 4. LCMS (FA): *m*/*z* = 381.3 (M+H).

### Step 4: [(1R,2S,4R)-4-(triazolo[4,5-d]pyrimidin-3-yl)-2-triisopropylsilyloxy-cyclopentyl]methanol (Intermediate 18)

To a solution of [(1R,2S,4R)-4-[(5-aminopyrimidin-4-yl)amino]-2-triisopropylsilyloxy-cyclopentyl]methanol (0.80 g, 2.1 mmol) in EtOAc (10.0 mL), MeOH (10.0 mL) and acetic acid (0.75 mL) was added sodium nitrite (0.22 g, 3.2 mmol) and water (3.0 mL). The reaction mixture was stirred at room temperature for 16 hours. The solvent was completely evaporated and the residue co-evaporated with toluene (2x50mL) to remove acetic acid. The crude material was purified using silica gel chromatography (10-50% EtOAc in hexanes) to afford [(1R,2S,4R)-4-(triazolo[4,5-d]pyrimidin-3-yl)-2-triisopropylsilyloxy-cyclopentyl]methanol (0.41 g, 50 %). LCMS (FA): *m*/*z* = 392.3 (M+H).

### Example 7

The compound listed below (Intermediate 19) was prepared as described in Example 4 steps 3-4, substituting the starting material shown in the table for [(1R,2S,4R)-4-(6-chloropurin-9-yl)-2-triisopropylsilyloxy-cyclopentyl]methanol (Intermediate 4).

| Starting material | Product | Analytical data |
|---|---|---|
| 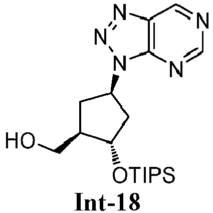 | 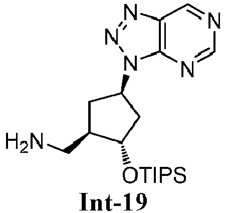 | LCMS (AA): *m*/*z* = 391.3 (M+H) |

### Example 8

### 2-amino-9-[(2S,5S,7R,8R,12aR,14R,15aS)-2,10,10-trioxido-14-(9H-purin-9-yl)-2-sulfanyldecahydro-11H-5,8-methanocyclopenta[m][1,3,6,9,10,11,2] tetraoxathiazaphosphacyclotetradecin-7-yl]-1,9-dihydro-6H-purin-6-one - N,N-diethylethanamine or 2-amino-9-[(2R,5S,7R,8R,12aR,14R,15aS)-2,10,10-trioxido-14-(9H-purin-9-yl)-2-sulfanyldecahydro-11H-5,8-methanocyclopenta[m][1,3,6,9,10,11,2] tetraoxathiazaphosphacyclotetradecin-7-yl]-1,9-dihydro-6H-purin-6-one - N,N-diethylethanamine (compound I-8)

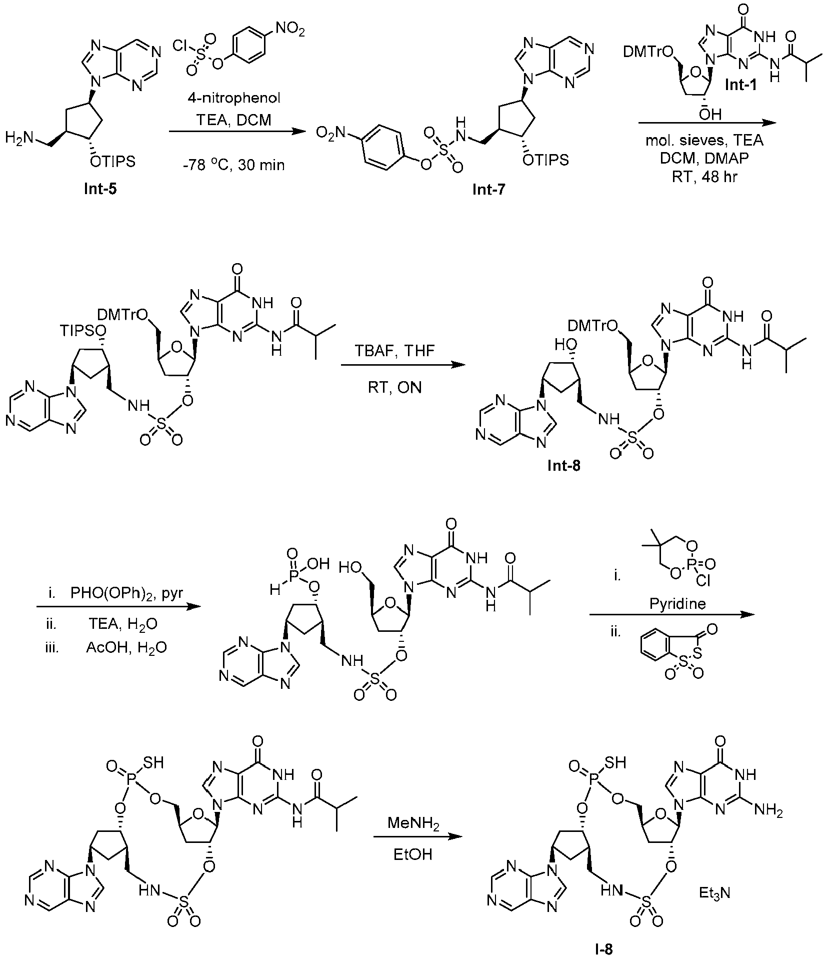

### Step 1: 4-nitrophenyl ({(1R,2S,4R)-4-(9H-purin-9-yl)-2-[(triisopropylsilyl) oxy]cyclopentyl}methyl)sulfamate (Intermediate 7)

To a solution of 1-{(1*R*,2*S*,4*R*)-4-(9H-purin-9-yl)-2-[(triisopropylsilyl)oxy]cyclopentyl}methanamine (**Intermediate 5,** 1.05 g, 2.70 mmol), 4-nitrophenol (3.79 g, 27.0 mmol) and triethylamine (4.55 mL, 32.3 mmol) in dry dichloromethane (26 mL) cooled in an acetone/dry ice bath at -78 °C under argon was added a solution of 4-nitrophenyl sulfurochloridate (1.28 mg, 5.39 mmol) in dry dichloromethane (3.5 mL) dropwise over a period of 5 minutes. Upon complete addition, the reaction mixture was maintained at -78 °C for 30 minutes, then was allowed to warm to room temperature, diluted with dichloromethane, and washed with 1 M aqueous NaH₂PO₄ three times. The aqueous phases were then re-extracted with dichloromethane twice, and the combined organic extracts were dried over anhydrous sodium sulfate and evaporated under reduced pressure. The crude material was purified using silica gel chromatography (0-20% EtOAc in DCM) to provide 4-nitrophenyl ({(1*R*,2*S*,4*R*)-4-(9H-purin-9-yl)-2-[(triisopropylsilyl)oxy]cyclopentyl}methyl)sulfamate (**Intermediate 7,** 1.21 g, 76%). LCMS (FA): *m*/*z* = 591.3 (M+H).

### Step 2: (2R,3R,5S)-5-{[bis(4-methoxyphenyl)(phenyl)methoxy]methyl}-2-[2-(isobutyrylamino)-6-oxo-1,6-dihydro-9H-purin-9-yl]tetrahydrofuran-3-yl ({(1R,2S,4R)-4-(9H-purin-9-yl)-2-[(triisopropylsilyl)oxy]cyclopentyl} methyl)sulfamate

A mixture of N-{9-[(2*R*,3*R*,5*S*)-5-{[bis(4-methoxyphenyl)(phenyl)methoxy]methyl}-3-hydroxytetrahydrofuran-2-yl]-6-oxo-6,9-dihydro-1H-purin-2-yl}-2-methylpropanamide (**Intermediate 1**, 1.10 g, 1.72 mmol), 4-nitrophenyl ({(1*R*,2*S*,4*R*)-4-(9H-purin-9-yl)-2-[(triisopropylsilyl)oxy]cyclopentyl}methyl)sulfamate (**Intermediate 7**, 1.22 g, 2.06 mmol), 4-dimethylaminopyridine (0.127 g, 1.03 mmol) and 4Å molecular sieves (3 g) in dry dichloromethane (19 mL) was sealed in a microwave vial under argon. After being stirred for 10 min, triethylamine (0.560 mL, 3.96 mmol) was added and the reaction mixture was allowed to stir at room temperature for two days. The reaction mixture was filtered and concentrated, and the resulting residue was subjected to purification by reverse phase flash column chromatography (0-100% ACN in aqueous ammonium bicarbonate (5 mM)) to provide (2*R*,3*R*,5*S*)-5-{[bis(4-methoxyphenyl)(phenyl)methoxy]methyl}-2-[2-(isobutyrylamino)-6-oxo-1,6-dihydro-9H-purin-9-yl]tetrahydrofuran-3-yl ({(1*R*,2*S*,4*R*)-4-(9H-purin-9-yl)-2-[(triisopropylsilyl)oxy]cyclopentyl}methyl)sulfamate (1.47 g, 78%). LCMS (AA): *m*/*z* = 1091.6 (M+H).

### Step 3: (2R,3R,5S)-5-{[bis(4-methoxyphenyl)(phenyl)methoxy]methyl}-2-[2-(isobutyrylamino)-6-oxo-1,6-dihydro-9H-purin-9-yl]tetrahydrofuran-3-yl {[(1R,2S,4R)-2-hydroxy-4-(9H-purin-9-yl)cyclopentyl]methyl}sulfamate (Intermediate 8).

To a solution of (2*R*,3*R*,5*S*)-5-{[bis(4-methoxyphenyl)(phenyl)methoxy] methyl}-2-[2-(isobutyrylamino)-6-oxo-1,6-dihydro-9H-purin-9-yl]tetrahydrofuran-3-yl ({(1*R*,2*S*,4*R*)-4-(9H-purin-9-yl)-2-[(triisopropylsilyl)oxy]cyclopentyl}methyl)sulfamate (1.47 g, 1.35 mmol) in tetrahydrofuran (13 mL) cooled to 0 °C was added tetrabutylammonium fluoride hydrate (0.828 g, 2.96 mmol). The reaction mixture was allowed to stir at room temperature overnight, diluted with dichloromethane and successively washed with saturated aqueous NaHCO₃ and brine. The organic extract was dried over anhydrous sodium sulfate and evaporated under reduced pressure. The crude material was purified using silica gel chromatography (0-10% MeOH in DCM with 0.5% triethylamine) to provide (2*R*,3*R*,5*S*)-5-{[bis(4-methoxyphenyl)(phenyl)methoxy]methyl}-2-[2-(isobutyrylamino)-6-oxo-1,6-dihydro-9H-purin-9-yl]tetrahydrofuran-3-yl {[(1*R*,2*S*,4*R*)-2-hydroxy-4-(9H-purin-9-yl)cyclopentyl] methyl}sulfamate (Intermediate 8, 1.38 g, 88%). LCMS (AA): *m*/*z* = 935.4 (M+H).

### Step 4: (1S,2R,4R)-2-({[({(2R,3R,5S)-5-(hydroxymethyl)-2-[2-(isobutyrylamino)-6-oxo-1,6-dihydro-9H-purin-9-yl]tetrahydrofuran-3-yl}oxy)sulfonyl]amino}methyl)-4-(9H-purin-9-yl)cyclopentyl hydrogen phosphonate.

To a solution of (2*R*,3*R*,5*S*)-5-{ [bis(4-methoxyphenyl)(phenyl)methoxy] methyl}-2-[2-(isobutyrylamino)-6-oxo-1,6-dihydro-9H-purin-9-yl]tetrahydrofuran-3-yl {[(1*R*,2*S*,4*R*)-2-hydroxy-4-(9H-purin-9-yl)cyclopentyl]methyl}sulfamate (**Intermediate 8,** 1.38 g, 1.48 mmol) in pyridine (2.9 mL) cooled in an ice bath to 0 °C was added diphenyl phosphite (0.59 mL, 3.10 mmol) The reaction mixture was allowed to stir at room temperature for 1 hour. Triethylamine (1.45 mL, 10.3 mmol) and water (1.4 mL, 79.7 mmol) were then added and stirring was continued at room temperature for 30 minutes. The reaction mixture was concentrated and co-evaporated with toluene three times. The residue was dissolved in acetic acid (3.48 mL, 60.5 mmol) and water (0.93 mL, 51.7 mmol) and allowed to stir at room temperature for 1 hour. Toluene (15 mL) was added and the reaction mixture was concentrated. The residue was co-evaporated with toluene (2 x 15 mL) and then dried under high vacuum for 10 minutes. The crude material was purified using silica gel chromatography (0-50% MeOH in DCM) to provide (1*S*,2*R*,4*R*)-2-({[({(2*R*,3*R*,5*S*)-5-(hydroxymethyl)-2-[2-(isobutyrylamino)-6-oxo-1,6-dihydro-9H-purin-9-yl]tetrahydrofuran-3-yl}oxy)sulfonyl]amino}methyl)-4-(9H-purin-9-yl)cyclopentyl hydrogen phosphonate (0.796 mg, 68%). LCMS (AA): *m*/*z* = 697.2 (M+H).

### Step 5: 2-methyl-N-{6-oxo-9-[(2S,5S,7R,8R,12aR,14R,15aS)-2,10,10-trioxido-14-(9H-purin-9-yl)-2-sulfanyldecahydro-11H-5,8-methanocyclopenta[m][1,3,6,9,10,11,2] tetraoxathiazaphosphacyclotetradecin-7-yl]-6,9-dihydro-1H-purin-2-yl} propanamide or 2-methyl-N-{6-oxo-9-[(2R,5S,7R,8R,12aR,14R,15aS)-2,10,10-trioxido-14-(9H-purin-9-yl)-2-sulfanyldecahydro-11H-5,8-methanocyclopenta[m] [1,3,6,9,10,11,2]tetraoxathiazaphosphacyclotetradecin-7-yl]-6,9-dihydro-1H-purin-2-yl}propanamide

(1*S*,2*R*,4*R*)-2-({[({(2*R*,3*R*,5*S*)-5-(hydroxymethyl)-2-[2-(isobutyrylamino)-6-oxo-1,6-dihydro-9H-purin-9-yl]tetrahydrofuran-3-yl}oxy)sulfonyl]amino}methyl)-4-(9H-purin-9-yl)cyclopentyl hydrogen phosphonate (0.25 g, 0.31 mmol) was co-evaporated with acetonitrile prior to use. The solid was dissolved in pyridine (6.08 mL, 75.2 mmol) and 2-chloro-5,5-dimethyl-1,3,2-dioxaphosphorinane 2-oxide (0.22 mg, 1.16 mmol) was added. The reaction mixture was allowed to stir at room temperature for 45 minutes. Water (0.21 mL, 11.60 mmol) and 3H-1,2-benzodithiol-3-one 1,1-dioxide (0.10 g, 0.50 mmol) were then added. The resulting mixture was stirred at room temperature for 30 minutes, concentrated and co-evaporated with acetonitrile and toluene each twice. The crude material was purified using silica gel chromatography (0-30% MeOH in DCM with 0.5% triethylamine) to provide 2-methyl-N-{6-oxo-9-[(2S,5S,7R,8R,12aR,14R,15aS)-2,10,10-trioxido-14-(9H-purin-9-yl)-2-sulfanyldecahydro-11H-5,8-methanocyclopenta[m][1,3,6,9,10,11,2]tetraoxathiazaphosphacyclotetradecin-7-yl]-6,9-dihydro-1H-purin-2-yl}propanamide - N,N-diethylethanamine or 2-methyl-N-{6-oxo-9-[(2R,5S,7R,8R,12aR,14R,15aS)-2,10,10-trioxido-14-(9H-purin-9-yl)-2-sulfanyldecahydro-11H-5,8-methanocyclopenta[m][1,3,6,9,10,11,2]tetraoxathiazaphosphacyclotetradecin-7-yl]-6,9-dihydro-1H-purin-2-yl}propanamide - N,N-diethylethanamine (157 mg, 70%). LCMS (FA): *m*/*z* = 711.2 (M+H).

### Step 6: 2-amino-9-[(2S,5S,7R,8R,12aR,14R,15aS)-2,10,10-trioxido-14-(9H-purin-9-yl)-2-sulfanyldecahydro-11H-5,8-methanocyclopenta[m][1,3,6,9,10,11,2] tetraoxathiazaphosphacyclotetradecin-7-yl]-1,9-dihydro-6H-purin-6-one - N,N-diethylethanamine or 2-amino-9-[(2R,5S,7R,8R,12aR,14R,15aS)-2,10,10-trioxido-14-(9H-purin-9-yl)-2-sulfanyldecahydro-11H-5,8-methanocyclopenta[m] [1,3,6,9,10,11,2]tetraoxathiazaphosphacyclotetradecin-7-yl]-1,9-dihydro-6H-purin-6-one - N,N-diethylethanamine (compound I-8)

2-Methyl-N-{6-oxo-9-[(2S,5S,7R,8R,12aR,14R,15aS)-2,10,10-trioxido-14-(9H-purin-9-yl)-2-sulfanyldecahydro-11H-5,8-methanocyclopenta[m][1,3,6,9,10,11,2]tetraoxathiazaphosphacyclotetradecin-7-yl]-6,9-dihydro-1H-purin-2-yl}propanamide - N,N-diethylethanamine or 2-methyl-N-{6-oxo-9-[(2R,5S,7R,8R,12aR,14R,15aS)-2,10,10-trioxido-14-(9H-purin-9-yl)-2-sulfanyldecahydro-11H-5,8-methanocyclopenta[m][1,3,6,9,10,11,2]tetraoxathiazaphosphacyclotetradecin-7-yl]-6,9-dihydro-1H-purin-2-yl}propanamide - N,N-diethylethanamine (150 mg, 0.21 mmol) was taken up in a methylamine solution (33% in EtOH, 10.5 mL, 84.4 mmol) and the resulting mixture was allowed to stir at room temperature for 90 minutes. The reaction mixture was concentrated and purified by reverse phase flash column chromatography (10-100% ACN in aqueous triethylammonium acetate (10 mM)) to provide 2-amino-9-[(2S,5S,7R,8R,12aR,14R,15aS)-2,10,10-trioxido-14-(9H-purin-9-yl)-2-sulfanyldecahydro-11H-5,8-methanocyclopenta[m] [1,3,6,9,10,11,2]tetraoxathiazaphosphacyclotetradecin-7-yl]-1,9-dihydro-6H-purin-6-one - N,N-diethylethanamine or 2-amino-9-[(2R,5S,7R,8R,12aR,14R,15aS)-2,10,10-trioxido-14-(9H-purin-9-yl)-2-sulfanyldecahydro-11H-5,8-methanocyclopenta[m][1,3,6,9,10,11,2]tetraoxathiaza phosphacyclotetradecin-7-yl]-1,9-dihydro-6H-purin-6-one - N,N-diethylethanamine (**compound I-8**) (103 mg, 66%). LCMS (FA): *m*/*z* = 741.2 (M+H), ¹H NMR (400 MHz, D₂O) δ 1.13 (t, *J*=7.3 Hz, 9H), 1.84-1.95 (m, 1H), 2.39-2.72 (m, 6H), 3.05 (q, *J*=7.3 Hz, 6H), 3.15 (dd, *J*=13.8, 8.4 Hz, 1H), 3.31 (dd, *J*=13.8, 3.9 Hz, 1H), 3.98-4.08 (m, 1H), 4.18-4.30 (m, 1H), 4.54 (dd, *J*=4.8, 2.3 Hz, 1H), 4.97-5.06 (m, 1H), 5.13-5.24 (m, 1H), 5.73-5.87 (m, 1H), 5.92 (d, *J*=7.2 Hz, 1H), 7.94 (s, 1H), 8.39 (s, 1H), 8.48 (s, 1H), 8.95 (s, 1H). ³¹P NMR (162 MHz, D₂O) δ 54.60 (s, 1 P).

### Example 9

The compounds listed below were prepared as described in Example 8 starting with Step 1, substituting the starting material shown in the table for **Intermediate 5.**

| I Number | Starting material | Product | Analytical data |
|---|---|---|---|
| I-1 | 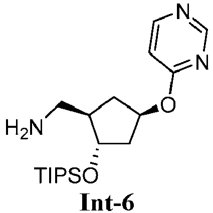 | 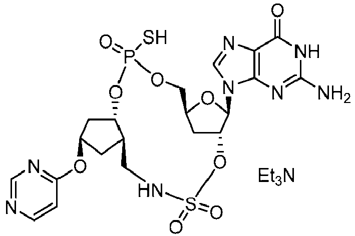 | LCMS (AA): *m*/*z* = 617.1 (M+H); ¹H NMR (400 MHz, CD₃OD) δ 1.31 - 1.25 (m, 9H), 1.51 - 1.37 (m, 1H), 2.35 (br d, *J* = 14.9 Hz, 2H), 2.60 - 2.47 (m, 1H), 2.86 - 2.61 (m, 3H), 3.00 (br dd, *J* = 12.0, 14.2 Hz, 1H), 3.18 - 3.12 (m, 6H), 3.37 - 3.32 (m, 1H), 4.10 - 3.95 (m, 1H), 4.62 - 4.41 (m, 2H), 4.92 - 4.86 (m, 1H), 5.63 (br s, 1H), 5.71 (br d, *J* = 6.2 Hz, 1H), 6.00 (br d, *J* = 5.7 Hz, 1H), 6.86 (br d, *J* = 5.9 Hz, 1H), 8.08 (s, 1H), 8.42 (br d, *J* = 5.9 Hz, 1H), 8.71 (br s, 1H),³¹P NMR (162 MHz, DMSO-d₆) δ 53.87 (br s, 1P). |
| I-7 | 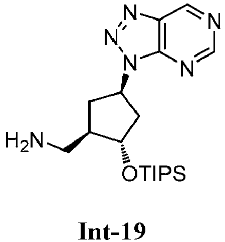 | 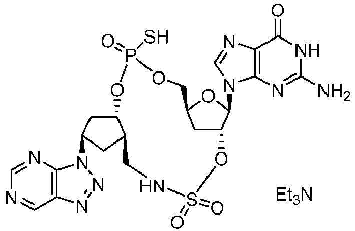 | LCMS (AA): *m*/*z* = 642.2 (M+H¹H NMR (400 MHz, D₂O) δ 1.28 (t, *J*=7.34 Hz, 9 H), 2.13 (dt, *J*=14.34, 7.08 Hz, 1 H), 2.60 - 2.72 (m, 1 H), 2.74 - 2.91 (m, 5 H), 3.20 (q, *J*=7.34 Hz, 6 H), 3.34 (dd, *J*=14.12, 9.35 Hz, 1 H), 3.41 - 3.53 (m, 1 H), 4.10 - 4.23 (m, 1 H), 4.41 (td, *J*=12.10, 1.96 Hz, 1 H), 4.70 - 4.72 (m, 1 H), 5.16 - 5.24 (m, 1 H), 5.77 - 5.85 (m, 1 H), 5.90 - 6.03 (m, 1 H), 6.08 (d, *J*=7.09 Hz, 1 H), 8.09 (s, 1 H), 9.04 (s, 1 H), 9.66 (s, 1 H). ³¹P NMR (162 MHz, D₂O) δ 54.76 (s, 1 P). |
| I-14* | 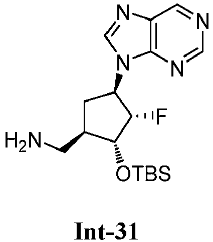 | 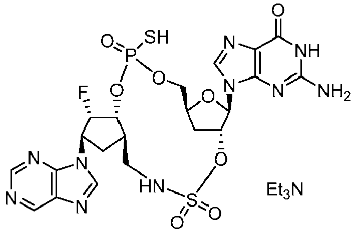 | LCMS (AA): *m*/*z* = 659.1 (M+H); ¹H NMR (400 MHz, D₂O) δ 1.27 (t, *J* = 7.3 Hz, 9H), 2.45 - 2.35 (m, 1H), 2.91 - 2.68 (m, 4H), 3.20 (q, *J* = 7.3 Hz, 6H), 3.53 - 3.42 (m, 2H), 4.14 (ddd, *J* = 1.7, 6.2, 12.1 Hz, 1H), 4.39 (dt, *J* = 2.1, 12.1 Hz, 1H), 4.70 - 4.64 (m, 1H), 5.27 - 5.16 (m, 1H), 5.47 - 5.35 (m, 1H), 5.49 (t, *J* = 3.5 Hz, 0.5H), 5.61 (t, *J* = 3.5 Hz, 0.5H), 6.12 - 6.02 (m, 2H), 8.02 (s, 1H), 8.42 (s, 1H), 8.60 (s, 1H), 9.13 (s, 1H). ³¹P NMR (162 MHz, D₂O) δ 55.41 (s, 1P). ¹⁹F NMR (376 MHz, D₂O) δ -194.91 (dt, *J* = 18.5, 49.7 Hz, 1F). |
| I-15** | 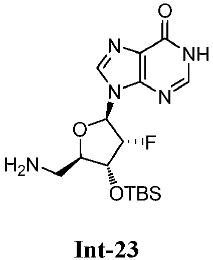 | 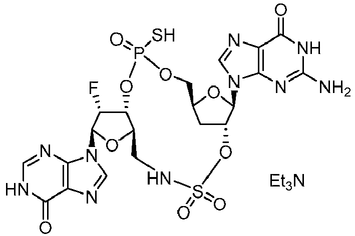 | LCMS (AA): *m*/*z* = 678.1 (M+H); ¹H NMR (400 MHz, D₂O) δ 1.13 (t, *J* = 7.3 Hz, 9H), 2.47 (dd, *J* = 7.6, 12.6 Hz, 1H), 2.69 - 2.55 (m, 1H), 3.05 (q, *J* = 7.3 Hz, 6H), 3.32 (d, *J* = 13.3 Hz, 1H), 3.61 (dd, *J* = 2.4, 13.4 Hz, 1H), 4.02 (ddd, *J* = 1.3, 5.1, 12.0 Hz, 1H), 4.26 (dt, *J* = 2.3, 11.7 Hz, 1H), 4.44 (br d, *J* = 9.3 Hz, 1H), 4.56-4.51 (m, 1H), 5.31 - 5.19 (m, 1H), 5.47 - 5.32 (m, 1H), 5.87 (d, *J* = 7.6 Hz, 1H), 6.05 (td, *J* = 7.6, 10.5 Hz, 1H), 6.44 - 6.36 (m, 1H), 7.15 (s, 1H), 7.79 (s, 1H), 8.10 (s, 1H). ³¹P NMR (162 MHz, D₂O) δ 56.19 (s, 1P). ¹⁹F NMR (376 MHz, D₂O) δ -194.89 - -195.22 (m, 1F). |
| I-19*** | 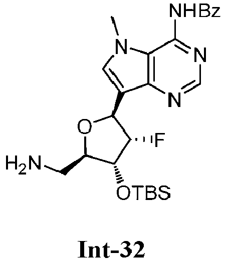 | 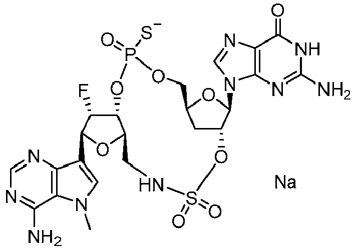 | LCMS (AA): *m*/*z* = 689.1 (M+H); ¹H NMR (400 MHz, D₂O) δ 2.53 - 2.60 (m, 1H), 2.68 - 2.77 (m, 1H), 3.22 - 3.36 (m, 2H), 3.89 (s, 3H), 4.14 - 4.25 (m, 2H), 4.38 - 4.44 (m, 1H), 4.62 - 4.66 (m, 1H), 5.19 - 5.29 (m, 1H), 5.32 - 5.48 (m, 1H), 5.53 - 5.64 (m, 2H), 5.92 (d, *J* = 7.0 Hz, 1H), 7.83 (s, 1H), 7.97 (s, 1H), 8.09 (s, 1H). ³¹P NMR (162 MHz, D₂O) δ 55.60 (s, 1P). ¹⁹F NMR (376 MHz, D₂O) δ -191.57 (s, 1F). |

* In Step 5, 3H-1,2-benzodithiol-3-one was used instead of 3H-1,2-benzodithiol-3-one 1,1-dioxide. In Step 6, deprotection was performed under the conditions described in Example 18, Step 5.** The conditions used in Step 3 are described in Example 19, Step 2. The conditions used in Step 5 are described in Example 19, Step 4.*** The conditions used in Step 3 are described in Example 19, Step 2. In Step 6, deprotection was performed under the conditions described in Example 18, Step 5 followed by Na salt formation as described in Example 29, Step 6.

### Example 10

### N-[9-[(2R,3R,5S)-5-(aminomethyl)-3-[tert-butyl(dimethyl)silyl]oxy-tetrahydrofuran-2-yl]-6-oxo-1H-purin-2-yl]-2-methyl-propanamide;4-methylbenzenesulfonic acid (Intermediate 9)

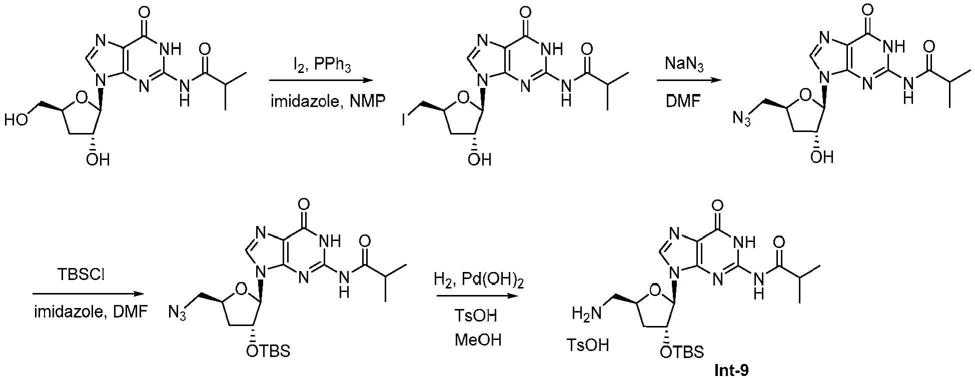

### Step 1: N-[9-[(2R,3R,5S)-3-hydroxy-5-(iodomethyl)tetrahydrofuran-2-yl]-6-oxo-1H-purin-2-yl]-2-methyl-propanamide

To a suspension of N-[9-[(2R,3R,5S)-3-hydroxy-5-(hydroxymethyl) tetrahydrofuran-2-yl]-6-oxo-1H-purin-2-yl]-2-methyl-propanamide (1.76 g, 5.22 mmol) in anhydrous acetonitrile (20.0 mL) was added imidazole (2.13 g, 31.3 mmol) and 1-methyl-2-pyrrolidinone (10.0 mL, 103 mmol) respectively. The suspension was concentrated under reduced pressure until most of the acetonitrile had been removed. Triphenylphosphine (4.38 g, 16.7 mmol) was added under a N₂ atmosphere and the resulting mixture was stirred vigorously for 3 minutes. Iodine (2.65 g, 10.4 mmol) was then added under N₂ in 4 portions. The mixture became hot and turned a clear, pale yellow. After 20 minutes, additional triphenylphosphine (3.31 g, 11.5 mmol) and iodine (2.65 g, 10.4 mmol) were added and the reaction mixture was heated at 60 °C for 1 hour. The crude reaction solution was cooled to room temperature and directly purified by reverse phase flash column chromatography (0-100% ACN in aqueous formic acid (0.1%)) to afford N-[9-[(2R,3R,5S)-3-hydroxy-5-(iodomethyl)tetrahydrofuran-2-yl]-6-oxo-1H-purin-2-yl]-2-methyl-propanamide (1.39 g, 50%) as a white solid. LCMS (FA): *m*/*z* = 448.0 (M+H).

### Step 2: N-[9-[(2R,3R,5S)-5-(azidomethyl)-3-hydroxy-tetrahydrofuran-2-yl]-6-oxo-1H-purin-2-yl]-2-methyl-propanamide

A suspension of N-[9-[(2R,3R,5S)-3-hydroxy-5-(iodomethyl) tetrahydrofuran-2-yl]-6-oxo-1H-purin-2-yl]-2-methyl-propanamide (2.39 g, 5.35 mmol) and sodium azide (1.04 g, 16.05 mmol) in anhydrous N,N-dimethylformamide (15.0 mL) was stirred at 50 °C for 8 hours under a N₂ atmosphere. Upon cooling to room temperature, water (2 mL) was added to dissolve salts and the solution was directly purified by reverse phase column chromatography (0-100% ACN in aqueous formic acid (0.1%)) to afford N-[9-[(2R,3R,5S)-5-(azidomethyl)-3-hydroxy-tetrahydrofuran-2-yl]-6-oxo-1H-purin-2-yl]-2-methyl-propanamide (1.12 g, 57 %) as a white solid. LCMS (FA): *m*/*z* = 363.2 (M+H).

### Step 3: N-[9-[(2R,3R,5S)-5-(azidomethyl)-3-[tert-butyl(dimethyl)silyl]oxy-tetrahydrofuran-2-yl]-6-oxo-1H-purin-2-yl]-2-methyl-propanamide

N-[9-[(2R,3R,5S)-5-(azidomethyl)-3-hydroxy-tetrahydrofuran-2-yl]-6-oxo-1H-purin-2-yl]-2-methyl-propanamide (1.12 g, 3.10 mmol) was co-evaporated with acetonitrile prior to use. Under an argon atmosphere, the solid was dissolved in anhydrous DMF (6.0 mL) and imidazole (1.27 g, 18.6 mmol) was added. The solution was cooled to 0 °C and *t-*butyldimethylsilyl trifluoromethanesulfonate (2.14 mL, 9.30 mmol) was added dropwise over 5 minutes. Upon complete addition, the reaction mixture was allowed to stir at room temperature for 4 hours. The reaction solution was directly purified by reverse phase column chromatography (0-100% ACN in aqueous ammonium acetate (10 mM)) to afford N-[9-[(2R,3R,5S)-5-(azidomethyl)-3-[*tert*-butyl(dimethyl)silyl]oxy-tetrahydrofuran-2-yl]-6-oxo-1H-purin-2-yl]-2-methyl-propanamide (1.37 g, 92%). LCMS (FA): *m*/*z* = 477.2 (M+H).

### Step 4: N-[9-[(2R,3R,5S)-5-(aminomethyl)-3-[tert-butyl(dimethyl)silyl]oxy-tetrahydrofuran-2-yl]-6-oxo-1H-purin-2-yl]-2-methyl-propanamide; 4-methylbenzenesulfonic acid (Intermediate 9)

To a degassed solution of N-[9-[(2R,3R,SS)-5-(azidomethyl)-3-[*tert-*butyl(dimethyl)silyl]oxy-tetrahydrofuran-2-yl]-6-oxo-1H-purin-2-yl]-2-methyl-propanamide (1.36 g, 2.86 mmol) and 4-methylbenzenesulfonic acid monohydrate (0.55 g, 2.86 mmol) in MeOH (25 mL) was added palladium hydroxide (20% on carbon, 0.201 g, 0.286 mmol), and the mixture was stirred under a balloon atmosphere of hydrogen at room temperature for 3 hours. The reaction mixture was filtered through Celite, and the filtrate was evaporated to afford N-[9-[(2R,3R,5S)-5-(aminomethyl)-3-[*tert*-butyl(dimethyl)silyl]oxy-tetrahydrofuran-2-yl]-6-oxo-1H-purin-2-yl]-2-methyl-propanamide;4-methylbenzenesulfonic acid (**Intermediate 9,** 1.613 g, 90 %) as a white solid. LCMS (FA): *m*/*z* = 451.3 (M+H). ¹H NMR (400 MHz, CD₃OD) δ -0.01 (s, 3 H) 0.05 (s, 3 H) 0.86 - 0.90 (m, 9 H) 1.21 (dd, *J*=8.16, 6.90 Hz, 6 H) 2.19 (ddd, *J*=13.33, 6.43, 3.20 Hz, 1 H) 2.37 (s, 3 H) 2.57 (ddd, *J*=13.52, 8.38, 6.21 Hz, 1 H) 2.76 (spt, *J*=6.76 Hz, 1 H) 3.35 - 3.48 (m, 2 H) 4.57 - 4.67 (m, 1 H) 4.92 (dt, *J*=5.99, 2.96 Hz, 1 H) 5.89 (d, *J*=2.64 Hz, 1 H) 7.23 (d, *J*=8.03 Hz, 2 H) 7.71 (d, *J*=8.16 Hz, 2 H) 8.16 (s, 1 H).

### Example 11

### (1S,2R,4R)-2-[[bis(4-methoxyphenyl)-phenyl-methoxy]methyl]-4-purin-9-yl-cyclopentanol (Intermediate 10)

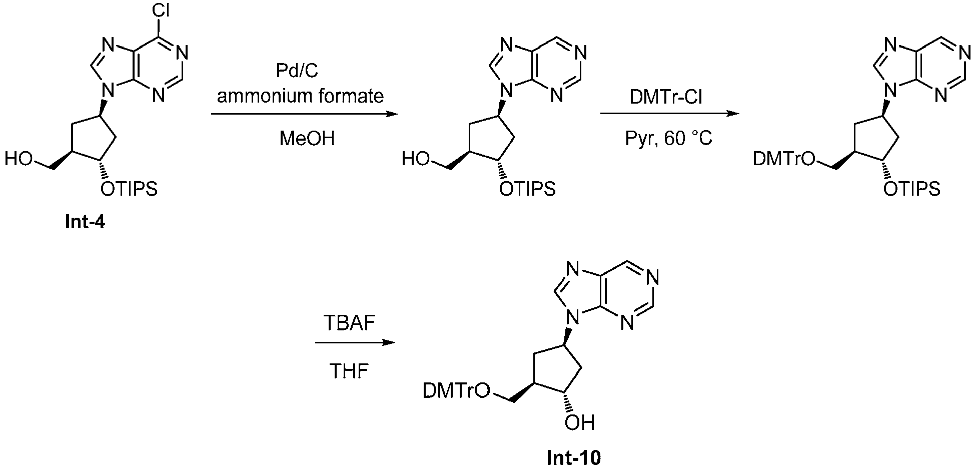

### Step 1: [(1R,2S,4R)-4-purin-9-yl-2-triisopropylsilyloxy-cyclopentyl]methanol

A suspension of [(1R,2S,4R)-4-(6-chloropurin-9-yl)-2-triisopropylsilyloxy-cyclopentyl]methanol (**Intermediate 4,** 0.823 g, 1.94 mmol) and ammonium formate (2.44 g, 38.7 mmol) in methanol (25 mL) was thoroughly degassed and palladium (10% on carbon, 0.10 g, 0.097 mmol) was added. The reaction mixture was stirred at room temperature overnight. The reaction mixture was filtered through a pad of Celite, concentrated and the residue purified by reverse phase flash column chromatography (0-100% ACN in aqueous formic acid (0.1%)) to afford [(1R,2S,4R)-4-purin-9-yl-2-triisopropylsilyloxy-cyclopentyl]methanol (0.632 g, 84%). LCMS (FA): *m*/*z* = 391.3 (M+H)

### Step 2: [(1S,2R,4R)-2-[[bis(4-methoxyphenyl)-phenyl-methoxy]methyl]-4-purin-9-yl-cyclopentoxy]-triisopropyl-silane

To a solution of [(1R,2S,4R)-4-purin-9-yl-2-triisopropylsilyloxy-cyclopentyl]methanol (0.631 g, 1.62 mmol) in pyridine (20 mL) at room temperature was added 4,4'-dimethoxytrityl chloride (1.38 g, 4.04 mmol). The reaction solution was heated at 60 °C for 1 hour, cooled to room temperature, and MeOH (5 mL) was added. The resulting mixture was stirred for 10 minutes at room temperature and then partitioned between water (30 mL) and EtOAc (30 mL). The aqueous phase was extracted with additional EtOAc (30 mL). The combined organic phases were evaporated and the residue purified using silica gel chromatography (10-100% EtOAc in hexanes) to afford [(1S,2R,4R)-2-[[bis(4-methoxyphenyl)-phenyl-methoxy]methyl]-4-purin-9-yl-cyclopentoxy]-triisopropyl-silane (0.940 g, 76 %) as a sticky yellow solid. LCMS (FA): *m*/*z* = 693.4 (M+H).

### Step 3: (1S,2R,4R)-2-[[bis(4-methoxyphenyl)-phenyl-methoxy]methyl]-4-purin-9-yl-cyclopentanol (Intermediate 10)

To a solution of [(1S,2R,4R)-2-[[bis(4-methoxyphenyl)-phenyl-methoxy]methyl]-4-purin-9-yl-cyclopentoxy]-triisopropyl-silane (0.940 g, 1.36 mmol) in THF (20 mL) was added tetrabutylammonium fluoride hydrate (0.471 g, 1.76 mmol) in a single portion. The reaction mixture was allowed to stir at room temperature for 1 hour, was then concentrated and the residue adsorbed onto silica gel and purified using silica gel chromatography (0-100 % EtOAc in hexanes) to afford (1S,2R,4R)-2-[[bis(4-methoxyphenyl)-phenyl-methoxy]methyl]-4-purin-9-yl-cyclopentanol (**Intermediate 10,** 690 mg, 95%) as a white solid. LCMS (FA): *m*/*z* = 537.3 (M+H).

### Example 12

### 2-amino-9-[(5S,7R,8R,10S,12aR,14R,15aS)-2,2,10-trioxido-14-(9H-purin-9-yl)-10-sulfanyldecahydro-3H-5,8-methanocyclopenta[l][1,3,6,11,10,9,2] tetraoxathiazaphosphacyclotetradecin-7-yl]-1,9-dihydro-6H-purin-6-one or 2-amino-9-[(5S,7R,8R,10R,12aR,14R,15aS)-2,2,10-trioxido-14-(9H-purin-9-yl)-10-sulfanyldecahydro-3H-5,8-methanocyclopenta[l][1,3,6,11,10,9,2]tetraoxathiazaphosphacyclotetradecin-7-yl]-1,9-dihydro-6H-purin-6-one - N,N-diethylethanamine (compound I-6)

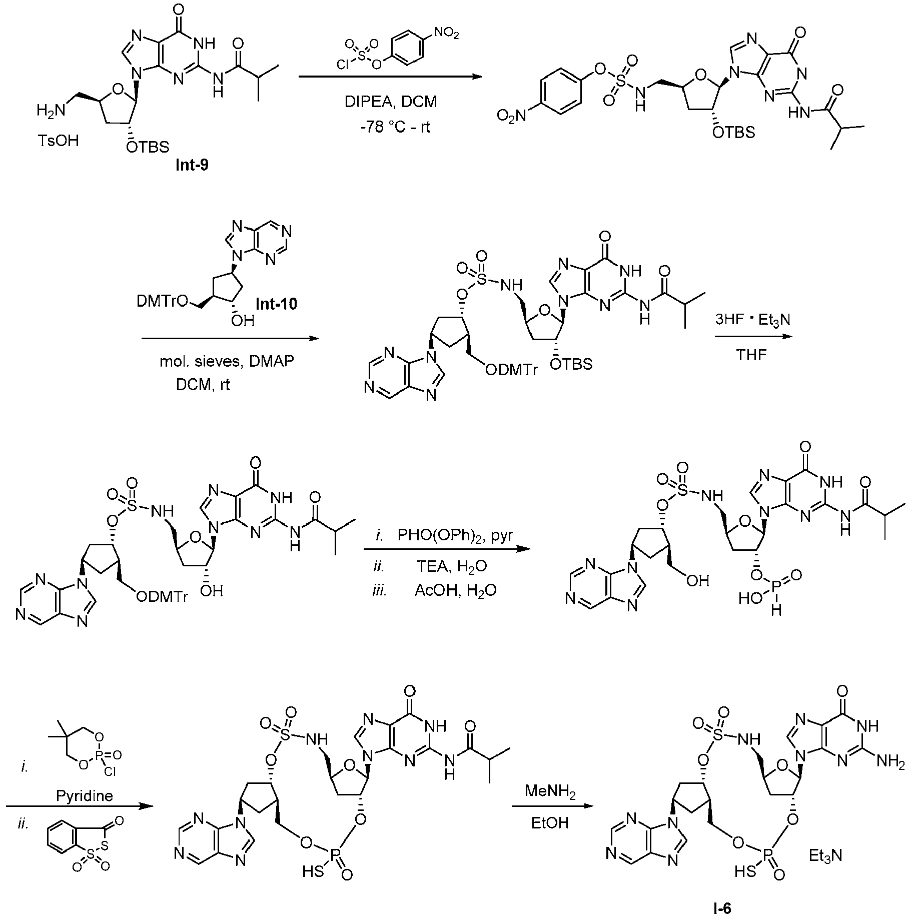

### Step 1: (4-nitrophenyl) N-[[(2S,4R,5R)-4-[tert-butyl(dimethyl)silyl]oxy-5-[2-(2-methylpropanoylamino)-6-oxo-1H-purin-9-yl]tetrahydrofuran-2-yl]methyl] sulfamate

To a mixture of N-[9-[(2R,3R,5S)-5-(aminomethyl)-3-[*tert-*butyl(dimethyl)silyl]oxy-tetrahydrofuran-2-yl]-6-oxo-1H-purin-2-yl]-2-methyl-propanamide;4-methylbenzenesulfonic acid (**Intermediate 9,** 57 mg, 0.091 mmol) and 4-nitrophenol (152 mg, 1.09 mmol) in anhydrous DCM (1.00 mL) was added DIPEA (164 mg, 1.27 mmol). The resulting solution was cooled to -78 °C and a solution of 1-chlorosulfonyloxy-4-nitro-benzene (43 mg, 0.182 mmol) in anhydrous DCM (0.12 mL) was added dropwise. Upon complete addition, the reaction mixture was allowed to stir at -78 °C for 30 minutes then warmed to room temperature for 15 minutes. The solution was diluted with DCM (15 mL) and washed with 1M aqueous NaH₂PO₄ (3 mL x 3). The aqueous phase was then re-extracted with DCM (20 mL x 2). The combined organic phases were concentrated to dryness and the residue was purified by silica gel chromatography (0-90% EtOAc in hexaness) to afford (4-nitrophenyl) N-[[(2S,4R,5R)-4-[*tert*-butyl(dimethyl)silyl]oxy-5-[2-(2-methylpropanoylamino)- 6-oxo-1H-purin-9-yl]tetrahydrofuran-2-yl]methyl]sulfamate as a white solid (41 mg, 69%). LCMS (FA): m/z = 652.2 (M+H).

### Step 2: [(1S,2R,4R)-2-[[bis(4-methoxyphenyl)-phenyl-methoxy]methyl]-4-purin-9-yl-cyclopentyl] N-[[(2S,4R,5R)-4-[tert-butyl(dimethyl)silyl]oxy-5-[2-(2-methyl propanoylamino)-6-oxo-1H-purin-9-yl]tetrahydrofuran-2-yl]methyl]sulfamate

(1S,2R,4R)-2-[[bis(4-methoxyphenyl)-phenyl-methoxy]methyl]-4-purin-9-yl-cyclopentanol (**Intermediate 10,** 519 mg, 0.97 mmol) was co-evaporated with dry THF / acetonitrile three times and then combined with (4-nitrophenyl) N-[[(2S,4R,5R)-4-[*tert*-butyl (dimethyl)silyl]oxy-5-[2-(2-methylpropanoylamino)-6-oxo-1H-purin-9-yl]tetrahydrofuran-2-yl]methyl]sulfamate (630 mg, 0.97 mmol), and 4Å molecular sieves (6.0 g) in anhydrous DCM (12.0 mL). The resulting mixture was allowed to stir under an argon atmosphere at room temperature for 45 minutes. DMAP (238 mg, 1.93 mmol) was added and the mixture was allowed to stir at room temperature for 16 hours. A mixture of MeOH and DCM (1:1, 40 mL) was added; the resulting mixture stirred at room temperature for 10 minutes and filtered through Celite. The Celite was washed with a mixture of MeOH and DCM (1: 1, 15 mL x 5). The combined filtrates were concentrated under reduced pressure and purified by reverse phase flash column chromatography (10-100% ACN in aqueous ammonium bicarbonate (5 mM)) to afford (4-nitrophenyl) N-[[(2S,4R,5R)-4-[*tert*-butyl(dimethyl)silyl]oxy-5-[2-(2-methyl propanoylamino)-6-oxo-1H-purin-9-yl]tetrahydrofuran-2-yl]methyl]sulfamate (715 mg, 69.8%). LCMS (AA): m/z = 1049.4 (M+H).

### Step 3: [(1S,2R,4R)-2-[[bis(4-methoxyphenyl)-phenyl-methoxy]methyl]-4-purin-9-yl-cyclopentyl] N-[[(2S,4R,5R)-4-hydroxy-5-[2-(2-methylpropanoylamino)-6-oxo-1H-purin-9-yl]tetrahydrofuran-2-yl]methyl]sulfamate

In a polypropylene tube, [(1S,2R,4R)-2-[[bis(4-methoxyphenyl)-phenyl-methoxy]methyl]-4-purin-9-yl-cyclopentyl] N-[[(2S,4R,5R)-4-[*tert*-butyl(dimethyl)silyl]oxy-5-[2-(2-methylpropanoylamino)-6-oxo-1H-purin-9-yl]tetrahydrofuran-2-yl]methyl]sulfamate (715 mg, 0.68 mmol) was dissolved in anhydrous THF (20.0 mL). Triethylamine (0.5 mL, 3.50 mmol) was added, followed by triethylamine trihydrofluoride (0.56 mL, 3.41 mmol). The reaction mixture was allowed to stir at room temperature overnight. Additional triethylamine (0.5 mL, 3.4mmol) was added and the reaction solution was concentrated under reduced pressure. The residue obtained was purified by reverse phase flash column chromatography (10-100% ACN in aqueous ammonium bicarbonate (5 mM)) to afford [(1S,2R,4R)-2-[[bis(4-methoxyphenyl)-phenyl-methoxy]methyl]-4-purin-9-yl-cyclopentyl]N-[[(2S,4R,5R)-4-hydroxy-5-[2-(2-methylpropanoylamino)-6-oxo-1H-purin-9-yl]tetrahydrofuran-2-yl]methyl]sulfamate (587 mg, 88%). LCMS (AA): m/z = 935.4 (M+H).

### Step 4: [(2R,3R,5S)-5-[[[(1S,2R,4R)-2-(hydroxymethyl)-4-purin-9-yl-cyclopentoxy]sulfonylamino]methyl]-2-[2-(2-methylpropanoylamino)-6-oxo-1H-purin-9-yl]tetrahydrofuran-3-yl]oxyphosphinic acid

A solution of [(1S,2R,4R)-2-[[bis(4-methoxyphenyl)-phenyl-methoxy]methyl]-4-purin-9-yl-cyclopentyl] N-[[(2S,4R,5R)-4-hydroxy-5-[2-(2-methylpropanoylamino)-6-oxo-1H-purin-9-yl]tetrahydrofuran-2-yl]methyl]sulfamate (587 mg, 0.628 mmol) in anhydrous pyridine (3.0 mL) was cooled with an ice bath to 0 °C. Diphenyl phosphite (0.25 mL, 1.31 mmol) was added and the reaction mixture was allowed to stir at room temperature for 90 minutes. The mixture was re-cooled in an ice bath and triethylamine (0.60 mL, 4.3 mmol) and water (0.60 mL) were added respectively. The mixture was allowed to stir at room temperature for 20 minutes, then concentrated under reduced pressure and subsequently co-evaporated with acetonitrile (3 x). Acetic acid (4.0 mL) and water (0.8 mL) were added to the residue and the mixture was allowed to stir at room temperature for 4 hours. Upon concentration under reduced pressure, the residue obtained was purified by reverse phase flash column chromatography (10-100% ACN in aqueous ammonium bicarbonate (5 mM)) to afford [(2R,3R,5S)-5-[[[(1S,2R,4R)-2-(hydroxymethyl)-4-purin-9-yl-cyclopentoxy] sulfonylamino]methyl]-2-[2-(2-methylpropanoylamino)-6-oxo-1H-purin-9-yl]tetrahydrofuran-3-yl]oxyphosphinic acid (277 mg, 60%) as a white powder. LCMS (AA): m/z = 697.2 (M+H).

### Step 5: 2-methyl-N-{6-oxo-9-[(5S,7R,8R,10S,12aR,14R,15aS)-2,2,10-trioxido-14-(9H-purin-9-yl)-10-sulfanyldecahydro-3H-5,8-methanocyclopenta[l][1,3,6,11,10,9,2] tetraoxathiazaphosphacyclotetradecin-7-yl]-6,9-dihydro-1H-purin-2-yl} propanamide or 2-methyl-N-{6-oxo-9-[(5S,7R,8R,10R,12aR,14R,15aS)-2,2,10-trioxido-14-(9H-purin-9-yl)-10-sulfanyldecahydro-3H-5,8-methanocyclopenta[l] [1,3,6,11,10,9,2]tetraoxathiazaphosphacyclotetradecin-7-yl]-6,9-dihydro-1H-purin-2-yl}propanamide

[(2R,3R,5S)-5-[[[(1S,2R,4R)-2-(hydroxymethyl)-4-purin-9-yl-cyclopentoxy]sulfonylamino]methyl]-2-[2-(2-methylpropanoylamino)-6-oxo-1H-purin-9-yl]tetrahydrofuran-3-yl]oxyphosphinic acid (67.5 mg, 0.097 mmol) was co-evaporated with dry acetonitrile twice and further dried under vacuum. This material was dissolved in anhydrous pyridine (1.88 mL, 23.3 mmol) under an argon atmosphere and solid 2-chloro-5,5-dimethyl-1,3,2-dioxaphosphorinane 2-oxide (69 mg, 0.36 mmol) was added. The reaction solution was allowed to stir at room temperature for 45 minutes. Water (0.087 mL, 4.84 mmol) was added followed by solid 3H-1,2-benzoldithiol-3-one 1,1-dioxide (38.8 mg, 0.194 mmol). The resulting solution was allowed to stir at room temperature for 30 minutes and then was concentrated under reduced pressure. The residue obtained was purified by reverse phase flash column chromatography (10-100% ACN in aqueous formic acid (0.1%)) to afford 2-methyl-N-{6-oxo-9-[(5S,7R,8R,10S,12aR,14R,15aS)-2,2,10-trioxido-14-(9H-purin-9-yl)-10-sulfanyldecahydro-3H-5,8-methanocyclopenta[l][1,3,6,11,10,9,2]tetraoxathiazaphosphacyclotetradecin-7-yl]-6,9-dihydro-1H-purin-2-yl}propanamide or 2-methyl-N-{6-oxo-9-[(5S,7R,8R,10R,12aR,14R,15aS)-2,2,10-trioxido-14-(9H-purin-9-yl)-10-sulfanyldecahydro-3H-5,8-methanocyclopenta[l] [1,3,6,11,10,9,2]tetraoxathiazaphosphacyclotetradecin-7-yl]-6,9-dihydro-1H-purin-2-yl} propanamide (40.5 mg, 58.8%). LCMS (FA): *m*/*z* = 711.2 (M+H).

### Step 6: 2-amino-9-[(5S,7R,8R,10S,12aR,14R,15aS)-2,2,10-trioxido-14-(9H-purin-9-yl)-10-sulfanyldecahydro-3H-5,8-methanocyclopenta[l][1,3,6,11,10,9,2] tetraoxathiazaphosphacyclotetradecin-7-yl]-1,9-dihydro-6H-purin-6-one - N,N-diethylethanamine or 2-amino-9-[(5S,7R,8R,10R,12aR,14R,15aS)-2,2,10-trioxido-14-(9H-purin-9-yl)-10-sulfanyldecahydro-3H-5,8-methanocyclopenta[l][1,3,6,11,10,9,2] tetraoxathiazaphosphacyclotetradecin-7-yl]-1,9-dihydro-6H-purin-6-one - N,N-diethylethanamine (compound I-6)

To 2-methyl-N-{6-oxo-9-[(5S,7R,8R,10S,12aR,14R,15aS)-2,2,10-trioxido-14-(9H-purin-9-yl)-10-sulfanyldecahydro-3H-5,8-methanocyclopenta[l] [1,3,6,11,10,9,2]tetraoxathiazaphosphacyclotetradecin-7-yl]-6,9-dihydro-1H-purin-2-yl}propanamide or 2-methyl-N-{6-oxo-9-[(5S,7R,8R,10R,12aR,14R,15aS)-2,2,10-trioxido-14-(9H-purin-9-yl)-10-sulfanyldecahydro-3H-5,8-methanocyclopenta[l][1,3,6,11,10,9,2] tetraoxathiazaphosphacyclotetradecin-7-yl]-6,9-dihydro-1H-purin-2-yl}propanamide (40.5 mg, 0.057 mmol) was added a methylamine solution (33% in EtOH, 2.84 mL, 22.8 mmol) and the resulting mixture was allowed to stir at room temperature for 90 minutes. The reaction was concentrated under reduced pressure and purified by reverse phase flash column chromatography (10-100% ACN in aqueous triethylammonium acetate (10 mM)) to afford 2-amino-9-[(5S,7R,8R,10S,12aR,14R,15aS)-2,2,10-trioxido-14-(9H-purin-9-yl)-10-sulfanyldecahydro-3H-5,8-methanocyclopenta[l][1,3,6,11,10,9,2]tetraoxathiazaphosphacyclotetradecin-7-yl]-1,9-dihydro-6H-purin-6-one or 2-amino-9-[(5S,7R,8R,10R,12aR,14R,15aS)-2,2,10-trioxido-14-(9H-purin-9-yl)-10-sulfanyldecahydro-3H-5,8-methanocyclopenta[l][1,3,6,11,10,9,2] tetraoxathiazaphosphacyclotetradecin-7-yl]-1,9-dihydro-6H-purin-6-one as the triethyl amine salt (24.2 mg, 57%). LCMS (FA): *m*/*z* = 641.1 (M+H). ¹H NMR (400 MHz, D₂O) δ 1.13 (t, *J*=7.28 Hz, 9 H), 1.88 - 1.99 (m, 1 H), 2.23 - 2.33 (m, 1 H), 2.45 - 2.79 (m, 5 H), 3.05 (q, *J*=7.36 Hz, 6 H), 3.35 (br dd, *J*=14.68, 5.02 Hz, 1 H), 3.49 (br d, *J*=14.56 Hz, 1 H), 3.90 - 3.97 (m, 1 H), 3.97 - 4.05 (m, 1 H),4.47 (br d, J=4.14 Hz, 1 H), 5.05 (br dd, J=10.79, 5.14 Hz, 1 H), 5.15 (br d, *J*=8.41 Hz, 1 H), 5.30 - 5.37 (m, 1 H), 5.75 (br d, *J*=5.27 Hz, 1 H), 7.65 - 7.75 (m, 1 H), 8.44 (s, 1 H), 8.71 (s, 1 H), 8.91 (s, 1 H). ³¹P NMR (162 MHz, D₂O) δ 54.01 (s, 1 P).

### Example 13

### 2-amino-9-[(5S,7R,8R,12aR,14R,15aS)-14-(6-amino-9H-purin-9-yl)-10-hydroxy-2,2,10-trioxidooctahydro-3H,12H-5,8-methanofuro[3,2-l][1,3,6,11,10,9,2] tetraoxathiazaphosphacyclotetradecin-7-yl]-1,9-dihydro-6H-purin-6-one - N,N-diethylethanamine (compound I-9)

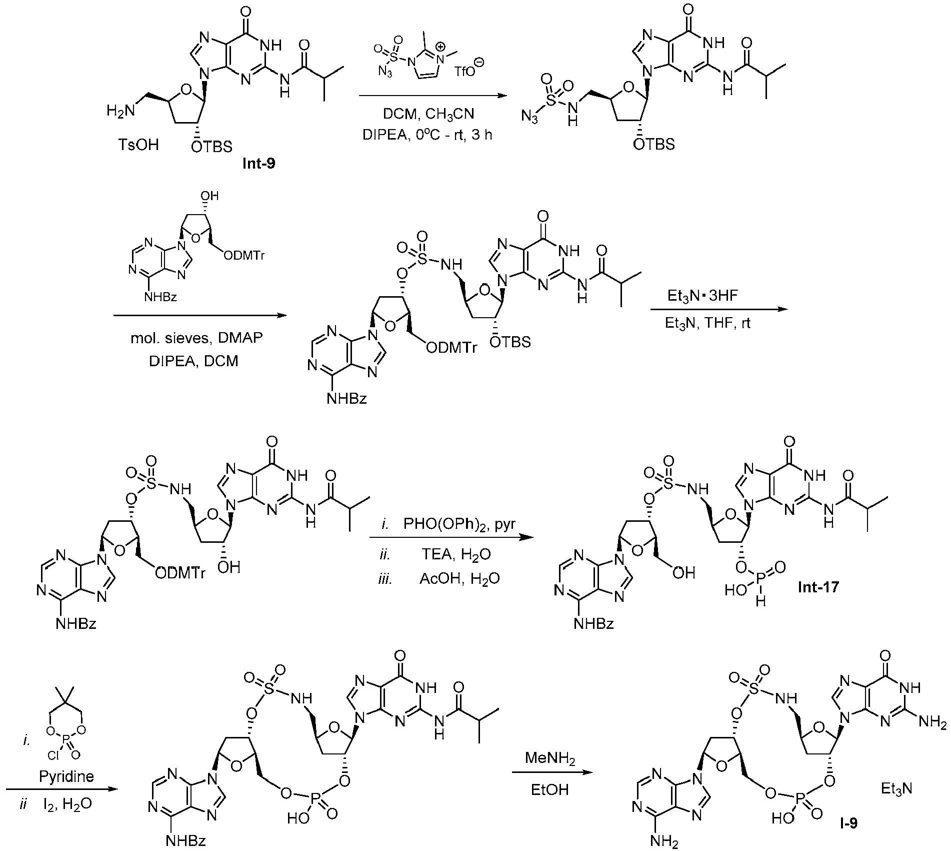

### Step 1: N-[9-[(2R,3R,5S)-5-[(azidosulfonylamino)methyl]-3-[tert-butyl(dimethyl) silyl]oxy-tetrahydrofuran-2-yl]-6-oxo-1H-purin-2-yl]-2-methyl-propanamide

A mixture of N-[9-[(2R,3R,SS)-5-(aminomethyl)-3-[tert-butyl (dimethyl)silyl]oxy-tetrahydrofuran-2-yl]-6-oxo-1H-purin-2-yl]-2-methyl-propanamide;4-methylbenzenesulfonic acid (**Intermediate 9,** 987 mg, 1.58 mmol) in anhydrous acetonitrile (5.0 mL) and dichloromethane (5.0 mL) was cooled with an ice bath to 0 °C under a N₂ atmosphere. N,N-diisopropylethylamine (0.280 mL, 1.59 mmol) was added and the mixture was allowed to stir at reduced temperature for 10 minutes. A solution of N-diazo-2,3-dimethyl-imidazol-3-ium-1-sulfonamide;trifluoromethanesulfonate (904 mg, 2.57 mmol) in anhydrous acetonitrile (1.0 mL) was then added dropwise. Upon complete addition, the mixture was allowed to warm to room temperature and stirred for 4 hours. The reaction was concentrated under reduced pressure and co-evaporated with anhydrous dichloromethane once. The residue was purified using silica gel chromatography (0-50% EtOAc / DCM)to afford N-[9-[(2R,3R,5S)-5-[(azidosulfonylamino) methyl]-3-[*tert*-butyl(dimethyl)silyl]oxy-tetrahydrofuran-2-yl]-6-oxo-1H-purin-2-yl]-2-methyl-propanamide (528 mg, 60%). LCMS (AA): *m*/*z* = 556.3 (M+H), 554.3 (M-H).

### Step 2: [(2R,3S,5R)-5-(6-benzamidopurin-9-yl)-2-[[bis(4-methoxyphenyl)-phenyl-methoxy]methyl]tetrahydrofuran-3-yl] N-[[(2S,4R,5R)-4-[tert-butyl(dimethyl)silyl]oxy-5-[2-(2-methylpropanoylamino)-6-oxo-1H-purin-9-yl]tetrahydrofuran-2-yl]methyl]sulfamate

N6-benzoyl-5'-O-(4,4'-dimethoxytrityl)-2'-deoxyadenosine (578 mg, 0.86 mmol) was co-evaporated from dry THF/acetonitrile three times. The resulting residue was combined with N-[9-[(2R,3R,5S)-5-[(azidosulfonylamino)methyl]-3-[*tert*-butyl(dimethyl) silyl]oxy-tetrahydrofuran-2-yl]-6-oxo-1H-purin-2-yl]-2-methyl-propanamide (398 mg, 0.72mmol), and 4Å molecular sieves (6.5 g) in anhydrous dichloromethane (9.0 mL) and stirred under an argon atmosphere at room temperature for 45 minutes. A solution of N, N-diisopropylethylamine (0.50 mL, 2.88 mmol) and 4-dimethylaminopyridine (25 mg, 0.20 mmol) in anhydrous dichloromethane (1.0 mL) was added and the mixture was allowed to stir at room temperature for 14 hours. A solution of 5% methanol in dichloromethane (30 mL) was added and the resulting mixture was allowed to stir for 10 minutes then filtered through a bed of Celite. The Celite was washed with 5% methanol in dichloromethane (10 mL x 10) until the filtrate showed no product by TLC. The filtrate was concentrated under reduced pressure and the residue obtained was purified using silica gel chromatography (0-2% MeOH /dichloromethane (0.5% TEA)) to give [(2R,3S,5R)-5-(6-benzamidopurin-9-yl)-2-[[bis(4-methoxyphenyl)-phenyl-methoxy]methyl]tetrahydrofuran-3-yl] N-[[(2S,4R,SR)-4-[*tert*-butyl(dimethyl)silyl]oxy-5-[2-(2-methylpropanoylamino)-6-oxo-1H-purin-9-yl]tetrahydrofuran-2-yl]methyl]sulfamate (674 mg, 78%). LCMS (AA): *m*/*z* = 1170.5(M+H).

### Step 3: [(2R,3S,5R)-5-(6-benzamidopurin-9-yl)-2-[[bis(4-methoxyphenyl)-phenyl-methoxy]methyl]tetrahydrofuran-3-yl] N-[[(2S,4R,5R)-4-hydroxy-5-[2-(2-methylpropanoylamino)-6-oxo-1H-purin-9-yl]tetrahydrofuran-2-yl]methyl] sulfamate

In a polypropylene conical tube [(2R,3S,5R)-5-(6-benzamidopurin-9-yl)-2-[[bis(4-methoxyphenyl)-phenyl-methoxy]methyl]tetrahydrofuran-3-yl] N-[[(2S,4R,5R)-4-[*tert-*butyl(dimethyl)silyl]oxy-5-[2-(2-methylpropanoylamino)-6-oxo-1H-purin-9-yl]tetrahydrofuran-2-yl]methyl]sulfamate (803 mg, 0.69 mmol) was dissolved in anhydrous tetrahydrofuran (10.0 mL). Triethylamine (0.39 mL, 2.77 mmol) was added, followed by triethylamine trihydrofluoride (0.450 mL, 2.71 mmol). The reaction mixture was allowed to stir at room temperature overnight. The mixture was diluted with dichloromethane (80 mL) and washed with saturated aqueous NaHCO₃ solution. The aqueous phase was extracted further with dichloromethane (10 mL x 5). The combined organic phases were washed with brine, dried over anhydrous Na₂SO₄, filtered and concentrated under reduced pressure. The residue was purified using silica gel chromatography (0-5% MeOH / CH₂Cl₂ (0.5 % Et₃N)) to give [(2R,3S,5R)-5-(6-benzamidopurin-9-yl)-2-[[bis(4-methoxyphenyl)-phenyl-methoxy]methyl]tetrahydrofuran-3-yl] N-[[(2S,4R,5R)-4-hydroxy-5-[2-(2-methylpropanoylamino)-6-oxo-1H-purin-9-yl]tetrahydrofuran-2-yl]methyl]sulfamate (676 mg, 89%). LCMS (AA): *m*/*z* = 1056.3(M+H), 1054.2 (M-H).

### Step 4: [(2R,3R,5S)-5-[[[(2R,3S,5R)-5-(6-benzamidopurin-9-yl)-2-(hydroxymethyl) tetrahydrofuran-3-yl]oxysulfonylamino]methyl]-2-[2-(2-methylpropanoylamino)-6-oxo-1H-purin-9-yl]tetrahydrofuran-3-yl]oxyphosphinic acid; N,N-diethylethanamine (Intermediate 17)

A solution of [(2R,3S,5R)-5-(6-benzamidopurin-9-yl)-2-[[bis(4-methoxyphenyl)-phenyl-methoxy]methyl]tetrahydrofuran-3-yl] N-[[(2S,4R,5R)-4-hydroxy-5-[2-(2-methylpropanoylamino)-6-oxo-1H-purin-9-yl]tetrahydrofuran-2-yl]methyl]sulfamate (673 mg, 0.61 mmol) in anhydrous pyridine (3.0 mL) was cooled with an ice bath to 0 °C. Diphenyl phosphite (0.25 mL, 1.31 mmol) was added and the reaction mixture was allowed to stir at room temperature for 90 minutes. The mixture was re-cooled in an ice bath and triethylamine (0.60 mL, 4.3 mmol) and water (0.60 mL) were added. The mixture was allowed to stir at room temperature for 20 minutes and then concentrated under reduced pressure, co-evaporated with acetonitrile (3x), and dried under vacuum for 1 hour to give an oily residue. Acetic acid (2.5 mL, 44.0 mmol) was added, followed by water (0.40 mL) and the mixture was allowed to stir at room temperature for 90 minutes, concentrated under reduced pressure, and co-evaporated with toluene to dryness to give a crude product. Purification using silica gel chromatography (0-30% MeOH / CH₂Cl₂ (0.5 % Et₃N)) provided [(2R,3R,5S)-5-[[[(2R,3S,5R)-5-(6-benzamidopurin-9-yl)-2-(hydroxymethyl)tetrahydrofuran-3-yl]oxysulfonylamino]methyl]-2-[2-(2-methylpropanoylamino)-6-oxo-1H-purin-9-yl]tetrahydrofuran-3-yl]oxyphosphinic acid; N,N-diethylethanamine (**Intermediate 17**, 411 mg, 70% yield) as a white powder. LCMS (AA): *m*/*z* = 818.1 (M+H), 816.1 (M-H).

### Step 5: N-(9-{(5S,7R,8R,12aR,14R,15aS)-10-hydroxy-7-[2-(isobutyrylamino)-6-oxo-1,6-dihydro-9H-purin-9-yl]-2,2,10-trioxidooctahydro-3H,12H-5,8-methanofuro[3,2-l][1,3,6,11,10,9,2]tetraoxathiazaphosphacyclotetradecin-14-yl}-9H-purin-6-yl)benzamide

[(2R,3R,5S)-5-[[[(2R,3S,5R)-5-(6-benzamidopurin-9-yl)-2-(hydroxymethyl)tetrahydrofuran-3-yl]oxysulfonylamino]methyl]-2-[2-(2-methylpropanoylamino)-6-oxo-1H-purin-9-yl]tetrahydrofuran-3-yl]oxyphosphinic acid; N,N-diethylethanamine (51.8 mg, 0.053 mmol) was co-evaporated with CH₃CN three times and dried under vacuum overnight. The material was then dissolved in anhydrous pyridine (0.80 mL). A solution of 2-chloro-5,5-dimethyl-1,3,2-dioxaphosphorinane 2-oxide (37.9 mg, 0.20mmol) in anhydrous pyridine (0.40 mL) was added and the mixture was allowed to stir at room temperature under a N₂ atmosphere for 1 hour. Additional solid 2-chloro-5, 5-dimethyl-1,3,2-dioxaphosphorinane 2-oxide (45.7 mg, 0.25 mmol) was added, and the mixture was allowed to stir at room temperature for an additional 2.5 hours. Water (36 µL) was added, followed by iodine (18.7 mg, 0.073mmol) and the brown solution was stirred at room temperature for 20 minutes. A solution of sodium thiosulfate (18.0 mg, 0.11mmol) in water (0.1 mL) was added, the resulting solution stirred for 5 minutes, and then concentrated under reduced pressure. The residue obtained was purified by reverse phase flash column chromatography (10-100% ACN in aqueous ammonium bicarbonate (5 mM)) to afford N-(9-{(5S,7R,8R,12aR,14R,15aS)-10-hydroxy-7-[2-(isobutyrylamino)-6-oxo-1,6-dihydro-9H-purin-9-yl]-2,2,10-trioxidooctahydro-3H,12H-5,8-methanofuro[3,2-1][1,3,6,11,10,9,2]tetraoxathiazaphosphacyclotetradecin-14-yl} 9H-purin-6-yl)benzamide (22 mg, 50% yield). LCMS (AA): *m*/*z* = 816.1 (M+H).

### Step 6: 2-amino-9-[(5S,7R,8R,12aR,14R,15aS)-14-(6-amino-9H-purin-9-yl)-10-hydroxy-2,2,10-trioxidooctahydro-3H,12H-5,8-methanofuro[3,2-l][1,3,6,11,10,9,2] tetraoxathiazaphosphacyclotetradecin-7-yl]-1,9-dihydro-6H-purin-6-one - N,N-diethylethanamine (compound I-9)

N-(9-f(SS,7R,8R,12aR,14R,15aS)-10-hydroxy-7-[2-(isobutyrylamino)-6-oxo-1,6-dihydro-9H-purin-9-yl]-2,2,10-trioxidooctahydro-3H,12H-5,8-methanofuro[3,2-l][1,3,6,11,10,9,2]tetraoxathiazaphosphacyclotetradecin-14-yl}-9H-purin-6-yl)benzamide (20.7 mg, 0.25 mmol) was dissolved in a solution of methylamine (33% in EtOH, 3.0 mL, 24 mmol) and the mixture was allowed to stir at room temperature for 6 hours. The mixture was concentrated with Celite under reduced pressure and dried under vacuum. Purification using C18 reverse phase chromatography (0-20% ACN in aqueous triethylammonium acetate (10 mM)) provided 2-amino-9-[(5S,7R,8R,12aR,14R,15aS)-14-(6-amino-9H-purin-9-yl)-10-hydroxy-2,2,10-trioxidooctahydro-3H,12H-5,8-methanofuro[3,2-l][1,3,6,11,10,9,2] tetraoxathiazaphosphacyclotetradecin-7-yl]-1,9-dihydro-6H-purin-6-one - N,N-diethylethanamine (**compound I-9**, 15.8 mg, 82.6%). LCMS (AA): *m*/*z* = 642.1 (M+H). H NMR (400 MHz, CD₃OD) δ 1.28 (t, *J* = 7.3 Hz, 9H), 2.40 - 2.26 (m, 1H), 2.82 - 2.66 (m, 1H), 3.05 (ddd, *J* = 2.6, 6.1, 14.8 Hz, 1H), 3.13 (q, *J* = 7.3 Hz, 6H), 3.36 - 3.32 (m, 1H), 3.50 - 3.38 (m, 1H), 3.65 - 3.50 (m, 1H), 4.12 - 3.97 (m, 1H), 4.35 - 4.21 (m, 1H), 4.47 - 4.40 (m, 1H), 4.54 - 4.48 (m, 1H), 5.41 (br d, *J* = 2.7 Hz, 2H), 5.83 (d, *J* = 5.1 Hz, 1H), 6.48 (t, *J* = 6.9 Hz, 1H), 7.78 (s, 1H), 8.20 (s, 1H), 8.32 (s, 1H). ¹P NMR (162 MHz, CD₃OD) δ -0.78 (s, 1P).

### Example 14

### 2-amino-9-[(5S,7R,8R,10S,12aR,14R,15aS)-14-(6-amino-9H-purin-9-yl)-2,2,10-trioxido-10-sulfanyloctahydro-3H,12H-5,8-methanofuro[3,2-l][1,3,6,11,10,9,2] tetraoxathiazaphosphacyclotetradecin-7-yl]-1,9-dihydro-6H-purin-6-one - N,N-diethylethanamine or 2-amino-9-[(5S,7R,8R,10R,12aR,14R,15aS)-14-(6-amino-9H-purin-9-yl)-2,2,10-trioxido-10-sulfanyloctahydro-3H,12H-5,8-methanofuro[3,2-l][1,3,6,11,10,9,2]tetraoxathiazaphosphacyclotetradecin-7-yl]-1,9-dihydro-6H-purin-6-one -N,N-diethylethanamine (compound I-2a and compound I-2b)

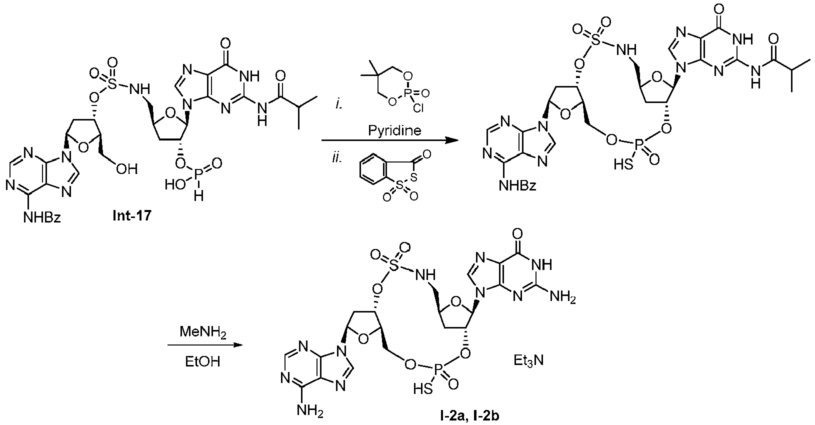

### Step 1: N-(9-{(5S,7R,8R,10S,12aR,14R,15aS)-7-[2-(isobutyrylamino)-6-oxo-1,6-dihydro-9H-purin-9-yl]-2,2,10-trioxido-10-sulfanyloctahydro-3H,12H-5,8-methanofuro[3,2-l][1,3,6,11,10,9,2]tetraoxathiazaphosphacyclotetradecin-14-yl}-9H-purin-6-yl)benzamide - N,N-diethylethanamine or N-(9-{(5S,7R,8R,10R,12aR, 14R,15aS)-7-[2-(isobutyrylamino)-6-oxo-1,6-dihydro-9H-purin-9-yl]-2,2,10-trioxido-10-sulfanyloctahydro-3H,12H-5,8-methanofuro[3,2-1][1,3,6,11,10,9,2] tetraoxathiazaphosphacyclotetradecin-14-yl}-9H-purin-6-yl)benzamide - N,N-diethylethanamine

[(2R,3R,5S)-5-[[[(2R,3S,5R)-5-(6-benzamidopurin-9-yl)-2-(hydroxymethyl)tetrahydrofuran-3-yl]oxysulfonylamino]methyl]-2-[2-(2-methylpropanoylamino)-6-oxo-1H-purin-9-yl]tetrahydrofuran-3-yl]oxyphosphinic acid;N,N-diethylethanamine **(Intermediate 17,** 202 mg, 0.22 mmol) was co-evaporated with dry CH₃CN three times, dried under vacuum overnight, co-evaporated with dry pyridine, dried under vacuum, and finally dissolved in anhydrous pyridine (5.0 mL). Solid 2-chloro-5,5-dimethyl-1,3,2-dioxaphosphorinane 2-oxide (148 mg, 0.77mmol) was added and the mixture was allowed to stir at room temperature under an argon atmosphere for 45 minutes. Water (0.14 mL) was added, followed by 3H-1, 2-benzodithiol-3-one 1,1-dioxide (66.2 mg, 0.33 mmol). The clear solution was allowed to stir at room temperature for 40 minutes, concentrated under reduced pressure, co-evaporated with acetonitrile (3x) and toluene (2x) and dried under vacuum. Purification using silica gel chromatography (0-30% MeOH / CH₂Cl₂ with 0.5% Et₃N) provided 2 impure but separated diastereomers and a set of mixed fractions. Re-purification of the early eluting peak using silica gel chromatography (0-10% MeOH / DCM with 0.5% TEA) provided N-(9-{(5S,7R,8R,10S,12aR,14R,15aS)-7-[2-(isobutyrylamino)-6-oxo-1,6-dihydro-9H-purin-9-yl]-2,2,10-trioxido-10-sulfanyloctahydro-3H,12H-5,8-methanofuro[3,2-l][1,3,6,11,10,9,2] tetraoxathiazaphosphacyclotetradecin-14-yl}-9H-purin-6-yl)benzamide - N,N-diethylethanamine or N-(9-{(5S,7R,8R,10R,12aR,14R,15aS)-7-[2-(isobutyrylamino)-6-oxo-1,6-dihydro-9H-purin-9-yl]-2,2,10-trioxido-10-sulfanyloctahydro-3H,12H-5,8-methanofuro[3,2-1][1,3,6,11,10,9,2] tetraoxathiazaphosphacyclotetradecin-14-yl}-9H-purin-6-yl)benzamide - N,N-diethylethanamine as a single isomer (175 mg, containing residual Et₃N, 38.5%). Repurification of the second eluting peak using silica gel chromatography (0-10% MeOH / DCM with 0.5% TEA) provided N-(9-{(5S,7R,8R,10S,12aR,14R,15aS)-7-[2-(isobutyrylamino)-6-oxo-1,6-dihydro-9H-purin-9-yl]-2,2,10-trioxido-10-sulfanyloctahydro-3H,12H-5,8-methanofuro[3,2-l][1,3,6,11,10,9,2] tetraoxathiazaphosphacyclotetradecin-14-yl}-9H-purin-6-yl)benzamide - N,N-diethylethanamine or N-(9-{(5S,7R,8R,10R,12aR,14R,15aS)-7-[2-(isobutyrylamino)-6-oxo-1,6-dihydro-9H-purin-9-yl]-2,2,10-trioxido-10-sulfanyloctahydro-3H,12H-5,8-methanofuro[3,2-l][1,3,6,11,10,9,2] tetraoxathiazaphosphacyclotetradecin-14-yl}-9H-purin-6-yl)benzamide - N,N-diethylethanamine as a single diastereomer (17.0 mg, containing residual Et₃N, 6.81%). For both isomers LCMS (AA): *m*/*z* = 832.1 (M+H), 830.1 (M-H).

### Step 2A: 2-amino-9-[(5S,7R,8R,10S,12aR,14R,15aS)-14-(6-amino-9H-purin-9-yl)-2,2,10-trioxido-10-sulfanyloctahydro-3H,12H-5,8-methanofuro[3,2-l][1,3,6,11,10,9,2] tetraoxathiazaphosphacyclotetradecin-7-yl]-1,9-dihydro-6H-purin-6-one - N,N-diethylethanamine (1:1) or 2-amino-9-[(5S,7R,8R,10R,12aR,14R,15aS)-14-(6-amino-9H-purin-9-yl)-2,2,10-trioxido-10-sulfanyloctahydro-3H,12H-5,8-methanofuro[3,2-l][1,3,6,11,10,9,2]tetraoxathiazaphosphacyclotetradecin-7-yl]-1,9-dihydro-6H-purin-6-one - N,N-diethylethanamine ( compoundI-2a)

N-(9-{(5S,7R,8R,10S,12aR,14R,15aS)-7-[2-(isobutyrylamino)-6-oxo-1,6-dihydro-9H-purin-9-yl]-2,2,10-trioxido-10-sulfanyloctahydro-3H,12H-5,8-methanofuro[3,2-l][1,3,6,11,10,9,2]tetraoxathiazaphosphacyclotetradecin-14-yl}-9H-purin-6-yl)benzamide-N,N-diethylethanamine or N-(9-{(5S,7R,8R,10R,12aR,14R,15aS)-7-[2-(isobutyrylamino)-6-oxo-1,6-dihydro-9H-purin-9-yl]-2,2,10-trioxido-10-sulfanyloctahydro-3H,12H-5,8-methanofuro[3,2-l][1,3,6,11,10,9,2]tetraoxathiazaphosphacyclotetradecin-14-yl}-9H-purin-6-yl)benzamide - N,N-diethylethanamine (second eluting peak isolated in Step 1 above, 15.0 mg, 82 % pure, 0.013mmol) was allowed to stir in a solution of methylamine (33% in EtOH, 1.0 mL, 8.0 mmol) at room temperature overnight. The mixture was concentrated under reduced pressure, co-evaporated with MeOH, re-dissolved in MeOH and concentrated with Celite. The crude product adsorbed on Celite was purified using reverse phase flash column chromatography (0-20% ACN in aqueous triethylammonium acetate (10 mM)) to provide 2-amino-9-[(5S,7R,8R,10S,12aR,14R,15aS)-14-(6-amino-9H-purin-9-yl)-2,2,10-trioxido-10-sulfanyloctahydro-3H,12H-5,8-methanofuro[3,2-l][1,3,6,11,10,9,2]tetraoxathiazaphosphacyclotetradecin-7-yl]-1,9-dihydro-6H-purin-6-one - N,N-diethylethanamine or 2-amino-9-[(5S,7R,8R,10R,12aR,14R,15aS)-14-(6-amino-9H-purin-9-yl)-2,2,10-trioxido-10-sulfanyloctahydro-3H,12H-5,8-methanofuro[3,2-l][1,3,6,11,10,9,2] tetraoxathiazaphosphacyclotetradecin-7-yl]-1,9-dihydro-6H-purin-6-one - N,N-diethylethanamine (compound **I-2a,** 7.6 mg, 76%). LCMS (AA): *m*/*z* = 658.1 (M+H), 656.1 (M-H). ¹H NMR (400 MHz, CD₃OD) δ 1.30 (t, *J =* 7.3 Hz, 9H), 2.39 - 2.30 (m, 1H), 2.64 (td, *J* = 9.4, 12.7 Hz, 1H), 3.15 - 3.08 (m, 1H), 3.19 (q, *J=* 7.3 Hz, 6H), 3.29 - 3.25 (m, 1H), 3.48 - 3.40 (m, 1H), 3.58 - 3.49 (m, 1H), 3.93 - 3.85 (m, 1H), 4.42 (dd, *J=* 3.5, 10.3 Hz, 1H), 4.57 - 4.50 (m, 2H), 5.69 - 5.64 (m, 1H), 5.82 - 5.69 (m, 2H), 6.49 (dd, *J=* 5.7, 8.7 Hz, 1H), 7.80 (s, 1H), 8.23 (s, 1H), 8.33 (s, 1H). ³¹P NMR (162 MHz, CD₃OD) δ 61.44 (s, 1P).

### Step 2B: 2-amino-9-[(5S,7R,8R,10S,12aR,14R,15aS)-14-(6-amino-9H-purin-9-yl)-2,2,10-trioxido-10-sulfanyloctahydro-3H,12H-5,8-methanofuro[3,2-l][1,3,6,11,10,9,2] tetraoxathiazaphosphacyclotetradecin-7-yl]-1,9-dihydro-6H-purin-6-one - N,N-diethylethanamine or 2-amino-9-[(5S,7R,8R,10R,12aR,14R,15aS)-14-(6-amino-9H-purin-9-yl)-2,2,10-trioxido-10-sulfanyloctahydro-3H,12H-5,8-methanofuro[3,2-l][1,3,6,11,10,9,2]tetraoxathiazaphosphacyclotetradecin-7-yl]-1,9-dihydro-6H-purin-6-one - N,N-diethylethanamine (compound I-2b)

N-(9-{(5S,7R,8R,10S,12aR,14R,15aS)-7-[2-(isobutyrylamino)-6-oxo-1,6-dihydro-9H-purin-9-yl]-2,2,10-trioxido-10-sulfanyloctahydro-3H,12H-5,8-methanofuro[3,2-l][1,3,6,11,10,9,2]tetraoxathiazaphosphacyclotetradecin-14-yl}-9H-purin-6-yl)benzamide - N,N-diethylethanamine or N-(9-{(5S,7R,8R,10R,12aR,14R,15aS)-7-[2-(isobutyrylamino)-6-oxo-1,6-dihydro-9H-purin-9-yl]-2,2,10-trioxido-10-sulfanyloctahydro-3H,12H-5,8-methanofuro[3,2-l][1,3,6,11,10,9,2]tetraoxathiazaphosphacyclotetradecin-14-yl}-9H-purin-6-yl)benzamide - N,N-diethylethanamine (first eluting peak in Step 1 above, 175 mg, 45% pure, 0.084 mmol) was allowed to stir in a solution of methylamine (33% in EtOH, 1.0 mL, 40.0 mmol) at room temperature overnight. The mixture was concentrated under reduced pressure, co-evaporated with MeOH, re-dissolved in MeOH and concentrated with Celite. The crude product adsorbed on Celite was purified using reverse phase flash column chromatography (0-20% ACN in aqueous triethylammonium acetate (10 mM)) to provide 2-amino-9-[(5S,7R,8R,10S,12aR,14R,15aS)-14-(6-amino-9H-purin-9-yl)-2,2,10-trioxido-10-sulfanyloctahydro-3H,12H-5,8-methanofuro[3,2-l][1,3,6,11,10,9,2] tetraoxathiazaphosphacyclotetradecin-7-yl]-1,9-dihydro-6H-purin-6-one - N,N-diethylethanamine or 2-amino-9-[(5S,7R,8R,10R, 12aR,14R,15aS)-14-(6-amino-9H-purin-9-yl)-2,2,10-trioxido-10-sulfanyloctahydro-3H,12H-5,8-methanofuro[3,2-l][1,3,6,11,10,9,2] tetraoxathiazaphosphacyclotetradecin-7-yl]-1,9-dihydro-6H-purin-6-one - N,N-diethylethanamine (compound **I-2b,** 49.3 mg, 94.9%). LCMS (AA): *m*/*z* = 658.1 (M+H), 656.1 (M-H). ¹H NMR(400 MHz, D₂O) δ 1.22 (t, *J=* 7.3 Hz, 9H), 2.34 - 2.16 (m, 1H), 2.84 - 2.66 (m, 1H), 3.09 - 2.97 (m, 2H), 3.13 (q, *J=* 7.3 Hz, 6H), 3.48 - 3.37 (m, 1H), 3.58 (br d, *J=* 13.8 Hz, 1H), 4.14 - 4.05 (m, 1H), 4.26 - 4.16 (m, 1H), 4.43 (br s, 1H), 4.51 (br s, 1H), 5.25 (br d, *J=* 4.8 Hz, 1H), 5.37 - 5.29 (m, 1H), 5.83 (br d, *J* = 4.5 Hz, 1H), 6.39 (s, 1H), 7.77 (s, 1H), 8.11 (s, 1H), 8.24 (s, 1H). ³¹P NMR (162 MHz, D₂O) δ 53.99 (s, 1P).

### Example 15

### 4-nitrophenyl {[(2R,3R,4R,5R)-3-{[tert-butyl(dimethyl)silyl]oxy}-4-fluoro-5-(9H-purin-9-yl)tetrahydrofuran-2-yl]methyl}sulfamate (Intermediate 12)

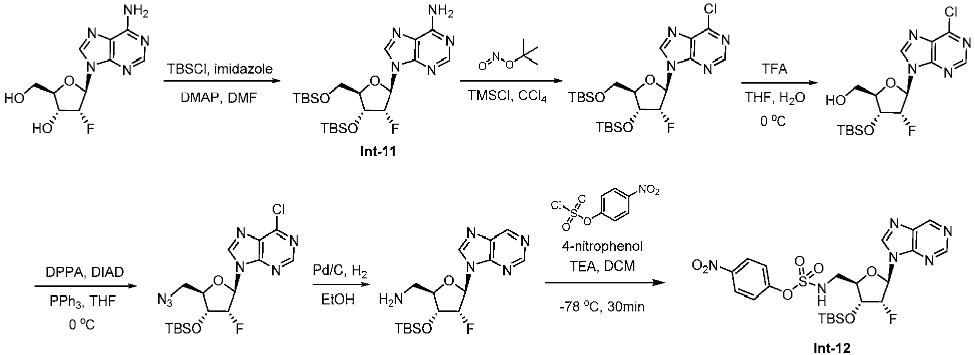

### Step 1: 9-[(2R,3R,4R,5R)-4-{[tert-butyl(dimethyl)silyl]oxy}-5-({[tert-butyl(dimethyl) silyl]oxy}methyl)-3-fluorotetrahydrofuran-2-yl]-9H-purin-6-amine (Intermediate 11)

2'-Fluoro-2'-deoxyadenosine (15.20 g, 56.5 mmol) was concentrated under vacuum twice from toluene, dried under vacuum, and dissolved in anhydrous DMF (150 mL). To the solution was added imidazole (23.30 g, 339.0 mmol), DMAP (1.04 g, 8.47 mmol) and tert-butyldimethylsilyl chloride (21.50 g, 141.0 mmol), and the reaction mixture was stirred overnight at room temperature. Saturated aqueous NaHCO₃ was added, and the resulting mixture was diluted with water and extracted with EtOAc. The organic phase was washed with water and brine respectively, dried over anhydrous Na₂SO₄, filtered and concentrated. The residue was purified using silica gel chromatography (0-100% EtOAc/hexaness) to afford 9-[(2R,3R,4R,5R)-4-{[*tert*-butyl(dimethyl)silyl]oxy}-5-({[*tert*-butyl(dimethyl)silyl]oxy}methyl)-3-fluorotetrahydrofuran-2-yl]-9H-purin-6-amine as a white solid (24.40 g, 87 %). LCMS (AA): *m*/*z* = 498.3 (M+H).

### Step 2: 9-[(2R,3R,4R,5R)-4-{[tert-butyl(dimethyl)silyl]oxyl-5-({[tert-butyl(dimethyl) silyl]oxy}methyl)-3-fluorotetrahydrofuran-2-yl]-6-chloro-9H-purine

To a degassed solution of 9-[(2R,3R,4R,5R)-4-[*tert*-butyl(dimethyl) silyl]oxy-5-[[*tert*-butyl(dimethyl)silyl]oxymethyl]-3-fluoro-tetrahydrofuran-2-yl]purin-6-amine (22.7 g, 45.6 mmol, freshly azeotroped from toluene) in carbon tetrachloride (700 mL) was added isoamyl nitrite (16.0 mL, 137.0 mmol) and the resulting solution was stirred at 50 °C overnight. Upon cooling to room temperature, the solution was concentrated under reduced pressure. The residue was dissolved in a minimum of DCM, filtered and purified by silica gel chromatography (0-50% EtOAc/hexaness) to afford 9-[(2R,3R,4R,5R)-4-{[*tert*-butyl(dimethyl)silyl]oxy}-5-({ [tert-butyl(dimethyl)silyl]oxy}methyl)-3-fluorotetrahydrofuran-2-yl]-6-chloro-9H-purine (12.8 g, 54%). LCMS (AA): *m*/*z* = 517.2 (M+H).

### Step 3: [(2R,3R,4R,5R)-3-1[tert-butyl(dimethyl)silyl]oxyl-5-(6-chloro-9H-purin-9-yl)-4-fluorotetrahydrofuran-2-yl] methanol

A solution of *tert*-butyl-[[(2R,3R,4R,5R)-3-[*tert*-butyl(dimethyl)silyl]oxy-5-(6-chloropurin-9-yl)-4-fluoro-tetrahydrofuran-2-yl]methoxy]-dimethyl-silane (692 mg, 1.34 mmol) in a mixture of THF (16 mL) and water (4 mL) was cooled in an ice bath to 0 °C and trifluoroacetic acid (4.1 mL, 53.5 mmol) was added. The reaction mixture was stirred at 0 °C for 3 hours and then quenched with the dropwise addition of saturated aqueous NaHCO₃ until basic by pH paper. The solution was extracted twice with ethyl acetate and the combined organic phases were washed with water and brine respectively, dried over anhydrous Na₂SO₄ and concentrated. The residue was purified on silica gel chromatography (0-100% EtOAc/hexanes) to afford [(2R,3R,4R,5R)-3-{[tert-butyl(dimethyl)silyl]oxy}-5-(6-chloro-9H-purin-9-yl)-4-fluorotetrahydrofuran-2-yl]methanol (372.0 mg, 69 %). LCMS (AA): *m*/*z* = 403.1 (M+H).

### Step 4: 9-[(2R,3R,4R,5R)-5-(azidomethyl)-4-{[tert-butyl(dimethyl)silyl]oxy}-3-fluorotetrahydrofuran-2-yl]-6-chloro-9H-purine

To a solution [(2R,3R,4R,SR)-3-[*tert*-butyl(dimethyl)silyl]oxy-5-(6-chloropurin-9-yl)-4-fluoro-tetrahydrofuran-2-yl]methanol (5.20 g, 12.9 mmol) and triphenylphosphine (4.47 g, 10.6 mmol) in THF (52 mL) was cooled to 0 °C. Diisopropyl azodicarboxylate (3.63 mL, 18.1 mmol) was added dropwise via a syringe. The reaction mixture was maintained at 0 °C for 10 minutes whereupon diphenylphosphoryl azide (3.89 mL, 18.1 mmol) in THF (12 mL) was added dropwise by addition funnel. Upon complete addition, the reaction solution was allowed to gradually warm to room temperature over 3 hours, then quenched with the addition of saturated aqueous NaHCO₃ and diluted with water and EtOAc. Upon separation, the aqueous phase was extracted with EtOAc and the combined organic phases were washed with water and brine respectively, dried over anhydrous Na₂SO₄ and concentrated. The residue obtained was purified by silica gel chromatography (0-40% EtOAc/hexanes) to afford 9-[(2R,3R,4R,5R)-5-(azidomethyl)-4-{[*tert*-butyl(dimethyl)silyl]oxy}-3-fluorotetrahydrofuran-2-yl]-6-chloro-9H-purine as a clear colorless oil. LCMS (AA): *m*/*z* = 428.2 (M+H).

### Step 5: 1[(2R,3R,4R,5R)-3-{[tert-butyl(dimethyl)silyl]oxy}-4-fluoro-5-(9H-purin-9-yl)tetrahydrofuran-2-yl]methanamine

A degassed mixture of [(2R,3R,4R,5R)-2-(azidomethyl)-5-(6-chloropurin-9-yl)-4-fluoro-tetrahydrofuran-3-yl]oxy-*tert*-butyl-dimethyl-silane (4.54 g, 10.6 mmol) and palladium (10% on carbon, 677 mg, 0.63 mmol) in ethanol (124 mL) was stirred under a balloon atmosphere of H₂ overnight at room temperature. The reaction mixture was filtered through Celite and the filtrate was evaporated under reduced pressure to afford 1-[(2R,3R,4R,5R)-3-{[*tert*-butyl(dimethyl)silyl]oxy}-4-fluoro-5-(9H-purin-9-yl)tetrahydrofuran-2-yl]methanamine (3.90 g, 100 %) without the need of further purification. LCMS (AA): *m*/*z* = 368.2 (M+H). Step 6: **4-nitrophenyl {[(2R,3R,4R,5R)-3-{[*tert*-butyl(dimethyl)silyl]oxy}-4-fluoro-5-(9H-purin-9-yl)tetrahydrofuran-2-yl]methyl}sulfamate (Intermediate 12)**

To a mixture of N-[9-[(2R,3S,4S,5R)-5-[[bis(4-methoxyphenyl)-phenyl-methoxy]methyl]-4-fluoro-3-hydroxy-tetrahydrofuran-2-yl]-6-oxo-1H-purin-2-yl]-2-methyl-propanamide (1.60 g, 2.43 mmol) and 4-nitrophenol (6.12 g, 43.5 mmol) in DCM (42 mL) was added triethylamine and the solution was cooled to -78 °C. A solution of 1-chlorosulfonyl-4-nitrobenzene (2.07 g, 8.70 mmol) in DCM (5.5 mL) was added dropwise. Upon complete addition, the reaction mixture was maintained at -78 °C for 30 minutes then warmed to room temperature. The solution was diluted with DCM and was washed with 1M aqueous NaH₂PO₄ (3x). The combined aqueous phases were extracted with DCM and the combined organic phases dried over anhydrous Na₂SO₄ and concentrated under reduced pressure. The residue obtained was purified on silica gel chromatography (0-30% EtOAc/hexanes) to afford 4-nitrophenyl {[(2R,3R,4R,5R)-3-{[*tert*-butyl(dimethyl)silyl]oxy}-4-fluoro-5-(9H-purin-9-yl)tetrahydrofuran-2-yl]methyl}sulfamate **(Intermediate 12,** 2.48 g, 69 %) LCMS (FA): *m*/*z* = 569.2 (M+H). ¹H NMR (400 MHz, CDCl₃) δ 0.17 (s, 3H), 0.18 (s, 3 H), 0.96 (s, 9 H), 3.69 (dd, *J*=5.50, 2.08 Hz, 2 H), 4.42 - 4.47 (m, 1 H), 4.67 - 4.72 (m, 1 H), 5.46 - 5.67 (m, 1 H), 6.16 (dd, *J*=12.59, 5.99 Hz, 1 H), 7.44 (d, *J*=8.27 Hz, 2 H), 8.14 - 8.29 (m, 3 H), 8.79 (s, 1 H), 9.15 - 9.25 (m, 2 H).

### Example 15A

The compound listed below (Intermediate 31) was prepared as described in Example 15 steps 4-5, substituting the starting material shown in the table for [(2R,3R,4R,5R)-3-{[*tert*-butyl(dimethyl)silyl]oxy}-5-(6-chloro-9H-purin-9-yl)-4-fluorotetrahydrofuran-2-yl]methanol.

| Starting material | Product | Analytical data |
|---|---|---|
| 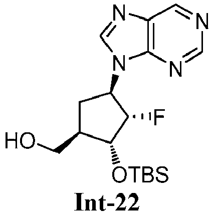 | 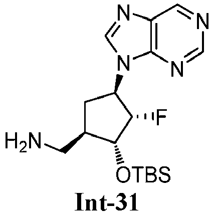 | LCMS (AA): *m*/*z* = 366.2 (M+H) |

### Example 16

### N-{9-[(2R,3R,4R,5R)-4-{[tert-butyl(dimethyl)silyl]oxy}-5-({[tert-butyl(dimethyl)silyl]oxy}methyl)-3-fluorotetrahydrofuran-2-yl]-9H-purin-6-yl}benzamide (Intermediate 13)

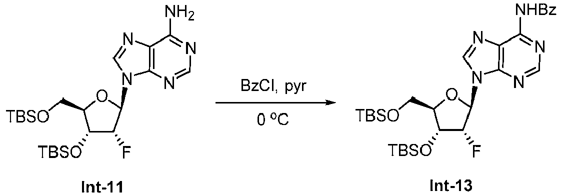

A solution of N-[9-[(2R,3R,4R,5R)-4-[*tert*-butyl(dimethyl)silyl]oxy-5-[[*tert-*butyl(dimethyl)silyl]oxymethyl]-3-fluoro-tetrahydrofuran-2-yl]purin-6-yl]benzamide **(Intermediate 11,** 5.35 g, 10.2 mmol) in pyridine (30 mL) was cooled to 0 °C. Benzoyl chloride (1.25 mL, 10.8 mmol) was added dropwise over 10 minutes. Upon complete addition, the reaction mixture was maintained at 0 °C for 3 hours and then at room temperature overnight. The reaction mixture was quenched with the addition of aqueous NH₄OH (28-30% NH₃ basis, 2 mL, 13 mmol) and diluted with water (8 mL) and MeOH (10 mL), stirred at room temperature for 30 minutes and finally concentrated under reduced pressure. The residue obtained was dissolved in EtOAc, washed with water and brine respectively, dried over anhydrous Na₂SO₄ and concentrated. The crude material was purified by silica gel chromatography (0-50 % EtOAc/ hexanes) to afford N-{9-[(2R,3R,4R,5R)-4-{[*tert*-butyl(dimethyl)silyl]oxy}-5-({[*tert-*butyl(dimethyl)silyl]oxy}methyl)-3-fluorotetrahydrofuran-2-yl]-9H-purin-6-yl}benzamide **(Intermediate 13,** 4.43 g, 72 %). LCMS (AA): *m*/*z* = 602.5 (M+H).

### Example 17

The compound listed below **(Intermediate 14)** was prepared as described in Example 15 starting with step 3, substituting the starting material shown in the table for [(2R,3R,4R,5R)-3-{[*tert*-butyl(dimethyl)silyl]oxy}-5-(6-chloro-9H-purin-9-yl)-4-fluorotetrahydrofuran-2-yl]methanol.

| Starting material | Product | Analytical data |
|---|---|---|
| 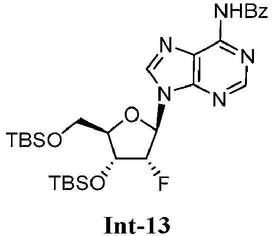 | 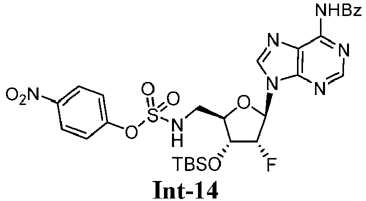 | LCMS (FA): *m*/*z* = 688.2 (M+H) |

### Example 18

### 2-amino-9-[(2S,5R,7R,8S,12aR,14R,15R,15aR,16R)-15,16-difluoro-2,10,10-trioxido-14-(9H-purin-9-yl)-2-sulfanyldecahydro-5,8-methanofuro[2,3-m][1,3,6,9,10,11,2] tetraoxathiazaphosphacyclotetradecin-7-yl]-1,9-dihydro-6H-purin-6-one - N,N-diethylethanamine or 2-amino-9-[(2R,5R,7R,8S,12aR,14R,15R,15aR,16R)-15,16-difluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine (compound I-10)

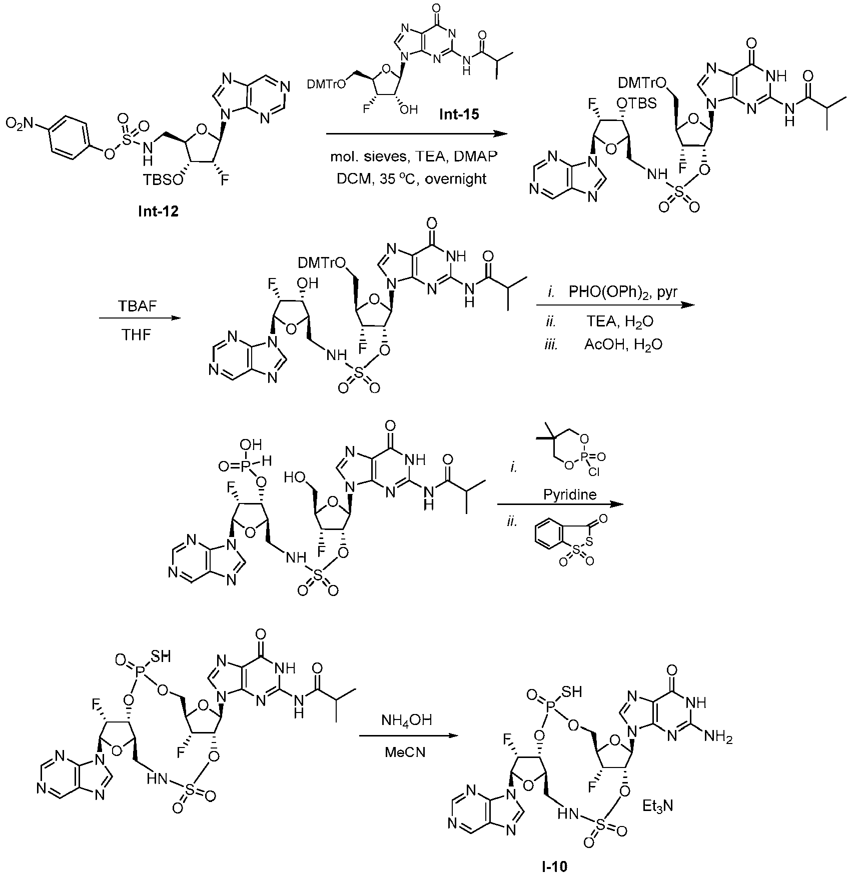

### Step 1: (2R,3S,4R,5R)-5-{[bis(4-methoxyphenyl)(phenyl)methoxy]methyl}-4-fluoro-2-[2-(isobutyrylamino)-6-oxo-1,6-dihydro-9H-purin-9-yl]tetrahydrofuran-3-yl{[(2R,3R,4R,5R)-3-{[tert-butyl(dimethyl)silyl]oxy}-4-fluoro-5-(9H-purin-9-yl)tetrahydrofuran-2-yl]methyl}sulfamate

A mixture of N-[9-[(2R,3R,5S)-5-[[bis(4-methoxyphenyl)-phenyl-methoxy]methyl]-3-hydroxy-tetrahydrofuran-2-yl]-6-oxo-1H-purin-2-yl]-2-methyl-propanamide **(Intermediate 15** prepared as described in PCT Int. Appl., 2017075477, 2.514 g, 3.930 mmol), (4-nitrophenyl) N-[[(2R,3R,4R,5R)-3-[*tert*-butyl(dimethyl)silyl]oxy-4-fluoro-5-purin-9-yl-tetrahydrofuran-2-yl]methyl]sulfamate **(Intermediate 12,** 1.66 g, 2.92 mmol), 4-dimethylaminopyridine (0.240 g, 1.95 mmol) and 4Å molecular sieves (5.0 g) in dry DCM (27 mL) was sealed in a vial under argon. After being stirred at room temperature for 10 minutes, triethylamine (0.79 mL, 5.60 mmol) was then added and the reaction mixture heated at 40 °C overnight. The reaction mixture was filtered, the solids washed with additional DCM, the combined filtrates concentrated, and the resulting residue purified by reverse phase flash column chromatography (0-100% ACN in aqueous NH₄HCO₃ (5 mM)) to provide (2R,3S,4R,5R)-5-{[bis(4-methoxyphenyl)(phenyl)methoxy]methyl}-4-fluoro-2-[2-(isobutyrylamino)-6-oxo-1,6-dihydro-9H-purin-9-yl]tetrahydrofuran-3-yl {[(2R,3R,4R,5R)-3-{[*tert*-butyl(dimethyl)silyl]oxy}-4-fluoro-5-(9H-purin-9-yl)tetrahydrofuran-2-yl]methyl}sulfamate (2.02 g, 76%). LCMS (AA): *m*/*z* = 1087.2 (M+H).

### Step 2: (2R,3S,4R,5R)-5-{[bis(4-methoxyphenyl)(phenyl)methoxy]methyl}-4-fluoro-2-[2-(isobutyrylamino)-6-oxo-1,6-dihydro-9H-purin-9-yl]tetrahydrofuran-3-yl {[(2R,3R,4R,5R)-4-fluoro-3-hydroxy-5-(9H-purin-9-yl)tetrahydrofuran-2-yl]methyl}sulfamate

To a solution of [(2R,3S,4R,5R)-5-[[bis(4-methoxyphenyl)-phenyl-methoxy]methyl]-4-fluoro-2-[2-(2-methylpropanoylamino)-6-oxo-1H-purin-9-yl]tetrahydrofuran-3-yl]N-[[(2R,3R,4R,5R)-3-[*tert*-butyl(dimethyl)silyl]oxy-4-fluoro-5-purin-9-yl-tetrahydrofuran-2-yl]methyl]sulfamate (1.60 g, 1.47 mmol) in THF (14 mL) cooled to 0 °C was added tetrabutylammonium fluoride hydrate (0.90 g, 3.24 mmol). The reaction mixture was then stirred at room temperature for 3 hours. The solution was diluted with DCM and successively washed with saturated aqueous NaHCO₃ and brine. The organic extract was dried over anhydrous MgSO₄ and evaporated under reduced pressure. The crude material was purified by reverse phase flash column chromatography (10-100% ACN in aqueous ammonium bicarbonate (5 mM)) to provide (2R,3S,4R,5R)-5-{[bis(4-methoxyphenyl)(phenyl)methoxy]methyl 1-4-fluoro-2-[2-(isobutyrylamino)-6-oxo-1,6-dihydro-9H-purin-9-yl]tetrahydrofuran-3-yl {[(2R,3R,4R,5R)-4-fluoro-3-hydroxy-5-(9H-purin-9-yl)tetrahydrofuran-2-yl]methyl}sulfamate (1.17 g, 82%). LCMS (AA): *m*/*z* = 973.1 (M+H).

### Step 3: (2R,3R,4R,5R)-4-fluoro-2-({[({(2R,3S,4R,5R)-4-fluoro-5-(hydroxymethyl)-2-[2-(isobutyrylamino)-6-oxo-1,6-dihydro-9H-purin-9-yl]tetrahydrofuran-3-yl}oxy)sulfonyl]amino}methyl)-5-(9H-purin-9-yl)tetrahydrofuran-3-yl hydrogen phosphonate - N,N-diethylethanamine

To a solution of [(2R,3S,4R,5R)-5-[[bis(4-methoxyphenyl)-phenyl-methoxy]methyl]-4-fluoro-2-[2-(2-methylpropanoylamino)-6-oxo-1H-purin-9-yl] tetrahydrofuran-3-yl] N-[[(2R,3R,4R,5R)-4-fluoro-3-hydroxy-5-purin-9-yl-tetrahydrofuran-2-yl]methyl]sulfamate (1.50 g, 1.54 mmol) in pyridine (3 mL) cooled to 0 °C was added diphenyl phosphite (0.62 mL, 3.24 mmol). Upon addition, the reaction mixture was allowed to stir at room temperature for 1 hour. Triethylamine (1.52 mL, 10.8 mmol) and water (1.5 mL, 83.3 mmol) were then added and stirring was continued at room temperature for an additional 30 minutes. The reaction mixture was concentrated and co-evaporated with toluene two times. The residue was dissolved in acetic acid (3.63 mL, 63.2 mmol) and water (0.97 mL, 54.0 mmol), and allowed to stir at room temperature for 1 hour. Toluene (15 mL) was added and the reaction mixture was concentrated. The residue was co-evaporated with toluene (2 x 15 mL) and then dried on high vacuum for 10 minutes. The crude material was purified using silica gel chromatography (0-30% MeOH in DCM with 0.5% Et₃N) to provide (2R,3R,4R,5R)-4-fluoro-2-({[({(2R,3S,4R,5R)-4-fluoro-5-(hydroxymethyl)-2-[2-(isobutyrylamino)-6-oxo-1,6-dihydro-9H-purin-9-yl]tetrahydrofuran-3-yl}oxy)sulfonyl]amino}methyl)-5-(9H-purin-9-yl)tetrahydrofuran-3-yl hydrogen phosphonate (1.00 g, 78%). LCMS (FA): *m*/*z* = 735.1 (M+H).

### Step 4: N-19-[(2S,5R,7R,8S,12aR,14R,15R,15aR,16R)-15,16-difluoro-2,10,10-trioxido-14-(9H-purin-9-yl)-2-sulfanyldecahydro-5,8-methanofuro[2,3-m] [1,3,6,9,10,11,2]tetraoxathiazaphosphacyclotetradecin-7-yl]-6-oxo-6,9-dihydro-1H-purin-2-yl}-2-methylpropanamide or N-{9-[(2R,5R,7R,8S,12aR,14R,15R,15aR,16R)-15,16-difluoro-2,10,10-trioxido-14-(9H-purin-9-yl)-2-sulfanyldecahydro-5,8-methanofuro[2,3-m] [1,3,6,9,10,11,2]tetraoxathiazaphosphacyclotetradecin-7-yl]-6-oxo-6,9-dihydro-1H-purin-2-yl}-2-methylpropanamide

(2R,3R,4R,5R)-4-fluoro-2-({ [({ (2R,3 S,4R,5R)-4-fluoro-5-(hydroxymethyl)-2-[2-(isobutyrylamino)-6-oxo-1,6-dihydro-9H-purin-9-yl]tetrahydrofuran-3-yl}oxy)sulfonyl]amino}methyl)-5-(9H-purin-9-yl)tetrahydrofuran-3-yl hydrogen phosphonate - N,N-diethylethanamine (1.0 g, 1.20 mmol) was co-evaporated with acetonitrile prior to use. The solid was dissolved in pyridine (23.2 mL) and 2-chloro-5,5-dimethyl-1,3,2-dioxaphosphorinane 2-oxide (0.851 g, 4.43 mmol) was added. The reaction mixture was allowed to stir at room temperature for 45 min. Water (0.80 mL, 44.3 mmol) and 3H-1,2-benzodithiol-3-one 1,1-dioxide (0.38 g, 1.91 mmol) were then added. The resulting mixture was stirred at room temperature for 30 min, concentrated and co-evaporated with acetonitrile and toluene each twice. The crude material was purified using silica gel chromatography (0-30% MeOH in DCM with 0.5% triethylamine). The material obtained was purified further by reverse phase flash chromatography (0- 50% ACN in aqueous ammonium bicarbonate (5mM)) to provide N-{9-[(2S,5R,7R,8S, 12aR, 14R, 15R, 15aR, 16R)-15, 16-difluoro-2, 10, 10-trioxido-14-(9H-purin-9-yl)-2-sulfanyldecahydro-5,8-methanofuro[2,3-m][1,3,6,9,10,11,2]tetraoxathiazaphosphacyclo tetradecin-7-yl]-6-oxo-6,9-dihydro-1H-purin-2-yl}-2-methylpropanamide - N,N-diethylethanamine or N-{9-[(2R,5R,7R,8S, 12aR, 14R, 15R, 15aR, 16R)-15, 16-difluoro-2, 10, 10-trioxido-14-(9H-purin-9-yl)-2-sulfanyldecahydro-5,8-methanofuro[2,3-m][1,3,6,9,10,11,2] tetraoxathiazaphosphacyclotetradecin-7-yl]-6-oxo-6,9-dihydro-1H-purin-2-yl}-2-methylpropanamide - N,N-diethylethanamine as a single diastereomer (167 mg, 18%). LCMS (FA): *m*/*z* = 749.2 (M+H).

### Step 5: 2-amino-9-[(2S,5R,7R,8S,12aR,14R,15R,15aR,16R)-15,16-difluoro-2,10,10-trioxido-14-(9H-purin-9-yl)-2-sulfanyldecahydro-5,8-methanofuro[2,3-m] [1,3,6,9,10,11,2]tetraoxathiazaphosphacyclotetradecin-7-yl]-1,9-dihydro-6H-purin-6-one - N,N-diethylethanamine or 2-amino-9-[(2R,5R,7R,8S,12aR,14R,15R,15aR,16R)-15,16-difluoro-2,10,10-trioxido-14-(9H-purin-9-yl)-2-sulfanyldecahydro-5,8-methanofuro[2,3-m] [1,3,6,9,10,11,2]tetraoxathiazaphosphacyclotetradecin-7-yl]-1,9-dihydro-6H-purin-6-one - N,N-diethylethanamine

To a solution of N-{9-[(2S,5R,7R,8S,12aR,14R,15R,15aR,16R)-15,16-difluoro-2,10,10-trioxido-14-(9H-purin-9-yl)-2-sulfanyldecahydro-5,8-methanofuro[2,3-m] [1,3,6,9,10,11,2]tetraoxathiazaphosphacyclotetradecin-7-yl]-6-oxo-6,9-dihydro-1H-purin-2-yl}-2-methylpropanamide - N,N-diethylethanamine or N-{9-[(2R,5R,7R,8S,12aR,14R,15R,15aR, 16R)-15,16-difluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-6-oxo-6,9-dihydro-1H-purin-2-yl}-2-methylpropanamide - N,N-diethylethanamine (100 mg, 0.13 mmol) in acetonitrile (4.0 mL) was added aqueous NH₄OH (28-30% NH₃ basis, 8 mL, 60 mmol). The reaction mixture was stirred in a sealed vial at 35 °C overnight. The solvents were removed under reduced pressure and the residue was purified by reverse phase flash column chromatography (0-35% ACN in aqueous triethylammonium acetate (10 mM) to provide 2-amino-9-[(2S,5R,7R,8S, 12aR, 14R, 15R, 15aR, 16R)-15, 16-difluoro-2, 10, 10-trioxido-14-(9H-purin-9-yl)-2-sulfanyldecahydro-5,8-methanofuro[2,3-m][1,3,6,9,10,11,2]tetraoxathiazaphosphacyclo tetradecin-7-yl]-1,9-dihydro-6H-purin-6-one - N,N-diethylethanamine or 2-amino-9-[(2R,5R,7R,8S,12aR,14R,15R,15,aR,16R)-15, 16-difluoro-2, 10,10-trioxido-14-(9H-purin-9-yl)-2-sulfanyldecahydro-5,8-methanofuro[2,3-m][1,3,6,9,10,11,2]tetraoxathiazaphosphacyclo tetradecin-7-yl]-1,9-dihydro-6H-purin-6-one - N,N-diethylethanamine **(compound I-10**, 68.0 mg, 65 %). LCMS (AA): *m*/*z* = 679.0 (M+H), ¹H NMR (D₂O) δ 1.13 (t, *J*=7.3 Hz, 9H), 3.05 (d, *J*=7.4 Hz, 6H), 3.44 (dd, *J*=13.4, 1.5 Hz, 1H), 3.72 (dd, *J*=13.4, 2.6 Hz, 1H), 4.00 (br d, *J*=11.4 Hz, 1H), 4.43-4.55 (m, 2H), 4.67-4.70 (m, 1H), 5.21-5.38 (m, 2H), 5.41-5.51 (m, 1H), 6.18 (s, 2H), 6.51-6.61 (m, 1H), 7.83 (s, 1H), 7.89 (s, 1H), 8.52 (s, 1H), 9.03 (s, 1H). ³¹P NMR (162 MHz, D₂O) δ 55.97 (s, 1 P).

### Example 18A

The compound listed below was prepared as described in Example 18 starting with Step 1, substituting the starting material shown in the table for **Intermediate 12.**

| I Number | Starting material | Product | Analytical data |
|---|---|---|---|
| I-16* | 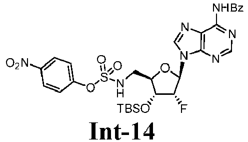 | 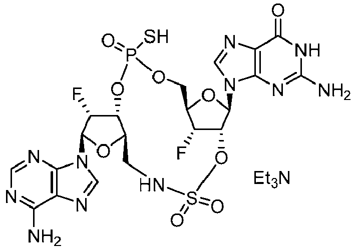 | LCMS (AA): *m*/*z* = 694.1 (M+H); ¹H NMR (400 MHz, D₂O) δ 1.28 t, *J* = 8.0 Hz, 9H), 3.20 (q, *J* = 8.0 Hz, 6H), 3.54 (br d, *J =* 12.0 Hz, 1H), 3.85 (dd, *J* = 4.0, 12.0 Hz, 1H), 4.15 (br d, *J* = 12.0 Hz, 1H), 4.66 - 4.58 (m, 2H), 4.84 - 4.80 (m, 1H), 5.68 - 5.31 (m, 3H), 6.38 - 6.25 (m, 2H), 6.57 (d, J = 20.0 Hz, 1H), 7.31 (s, 1H), 7.95 (s, 1H), 8.25 (s, 1H). ³¹P NMR (162 MHz, D₂O) δ 55.84 (s, 1P). ¹⁹F NMR (376 MHz, D₂O) δ -195.3 (s, 1F), -197.9 (s, 1F). |

| | | | |
|---|---|---|---|
| * The conditions used in Step 2 were modified as described in Example 19, Step 2. The conditions used in Step 4 were modified as described in Example 19, Step 4. | | | |

### Example 18B

The compounds listed below were prepared as described in Example 18 starting with Step 1, substituting the starting material **(Intermediate 20**) shown in the table for **Intermediate 15.**

| I Number | Starting material | Product | Analytical data | Note |
|---|---|---|---|---|
| I-13a | 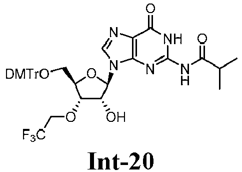 Prepared as described in WO 2017/161349 (A1) | 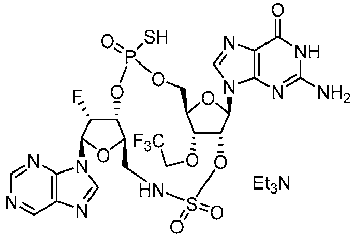 | LCMS (AA): *m*/*z* = 759.1 (M+H); ¹H NMR (400 MHz, D₂O) δ 1.13 (t, *J* = 7.3 Hz, 9H), 3.05 (q, *J* = 7.3 Hz, 6H), 3.36 - 3.31 (m, 1H), 3.73 - 3.67 (m, 1H), 4.03 - 4.00 (m, 1H), 4.32 - 4.21 (m, 2H), 4.42 - 4.37 (m, 1H), 4.52 - 4.46 (m, 3H), 5.42 - 5.24 (m, 2H), 6.13 - 6.06 (m, 2H), 6.53 (d, *J* = 19.6 Hz, 1H), 7.80 (s, 1H), 7.85 (br s, 1H), 8.52 (br s, 1H), 9.01 (s, 1H). ³¹P NMR (162 MHz, D₂O) δ 55.95 (s, 1P). ¹⁹F NMR (376 MHz, D₂O) δ -74.63 (s, 3F), -195.70 (s, 1F). | Major isomer isolated from Step 4 used as starting material in Step 5 |
| I-13b | 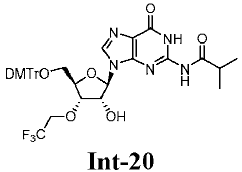 Prepared as described in WO 2017/161349 (A1) | 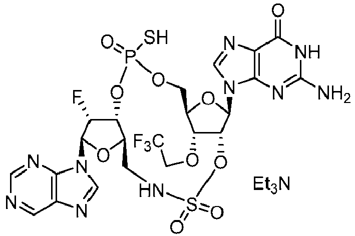 | LCMS (AA): *m*/*z* = 759.0 (M+H); ¹H NMR (400 MHz, D₂O) δ 1.13 (t, *J* = 7.4 Hz, 9H), 3.05 (q, *J* = 7.4 Hz, 6H), 3.47 - 3.43 (m, 1H), 3.75 (dd, *J* = 13.4, 3.2 Hz, 1H), 4.32 - 4.10 (m, 4H), 4.61 - 4.49 (m, 3H), 5.13 - 5.02 (m, 1H), 5.74 (dd, *J* = 50.5, 4.5 Hz, 1H), 5.90 (dd, *J =* 8.6, 4.0 Hz, 1H), 6.11 (d, *J =* 8.5 Hz, 1H), 6.53 (d, *J* = 20.5 Hz, 1H), 7.91 (s, 1H), 8.02 (s, 1H), 8.51 (s, 1H), 8.99 (s, 1H). ³¹P NMR (162 MHz, D₂O) δ 52.89 (s, 1P). ¹⁹F NMR (376 MHz, D₂O) δ -74.60 (s, 3F), -196.27 (s, 1F). | Minor isomer isolated from Step 4 used as starting material in Step 5 |

### Example 19

### 2-amino-9-[(2S,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2] tetraoxathiazaphosphacyclotetradecin-7-yl]-1,9-dihydro-6H-purin-6-one - N,N-diethylethanamine or 2-amino-9-[(2R,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine (compound I-11a)

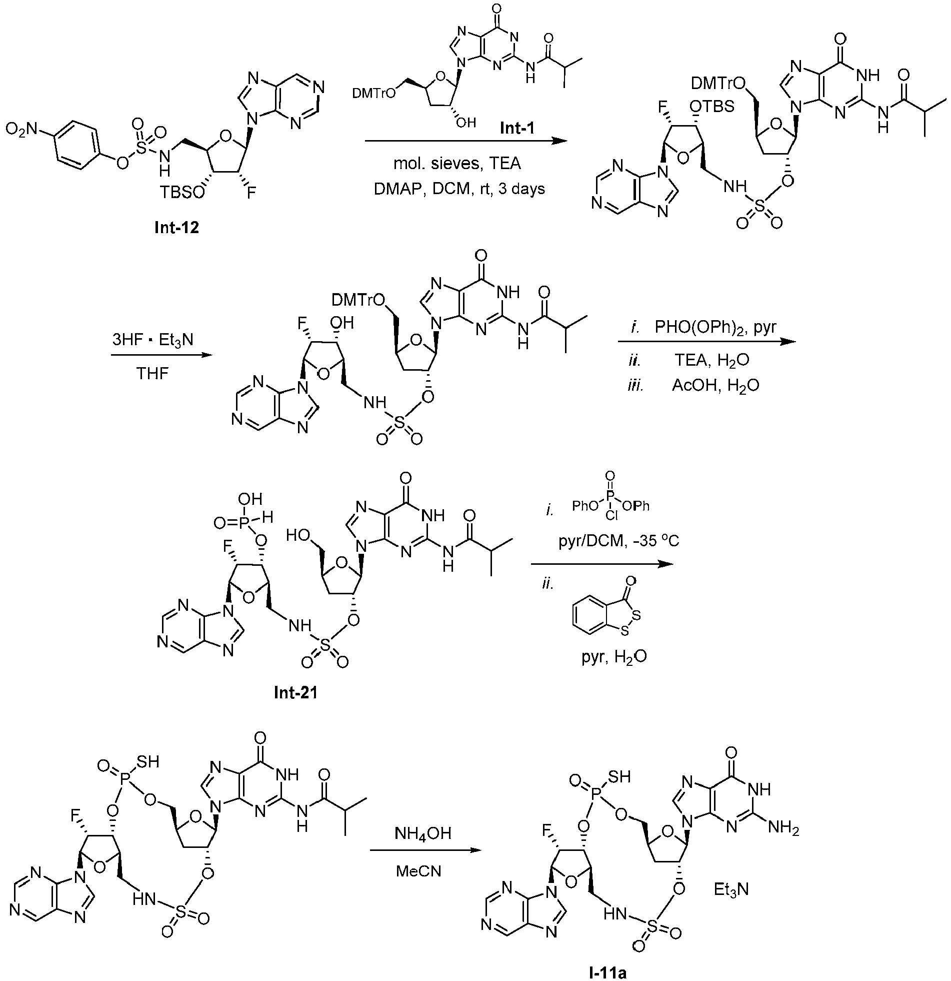

### Step 1: (2R,3R,5S)-5-{[bis(4-methoxyphenyl)(phenyl)methoxy]methyl}-2-[2-(isobutyrylamino)-6-oxo-1,6-dihydro-9H-purin-9-yl]tetrahydrofuran-3-yl {[(2R,3R,4R,5R)-3-{[tert-butyl(dimethyl)silyl]oxy}-4-fluoro-5-(9H-purin-9-yl)tetrahydrofuran-2-yl]methyl}sulfamate

A mixture of N-[9-[(2R,3R,5S)-5-[[bis(4-methoxyphenyl)-phenyl-methoxy]methyl]-3-hydroxy-tetrahydrofuran-2-yl]-6-oxo-1H-purin-2-yl]-2-methyl-propanamide (**Intermediate 1,** 2.51 g, 3.93 mmol), (4-nitrophenyl) N-[[(2R,3R,4R,SR)-3-[*tert*-butyl(dimethyl) silyl]oxy-4-fluoro-5-purin-9-yl-tetrahydrofuran-2-yl]methyl]sulfamate (**Intermediate 12,** 1.950 g, 3.43 mmol), 4-dimethylaminopyridine (0.390 g, 3.16 mmol) and 4Å molecular sieves (6.2 g) in dry DCM (39 mL) was stirred under argon at room temperature for 10 minutes. Triethylamine (1.37 mL, 9.72 mmol) was then added and the reaction mixture was allowed to stir at room temperature for three days. The reaction mixture was filtered and concentrated, and the resulting residue was subjected to purification by reverse phase column chromatography (30-100% ACN in aqueous ammonium bicarbonate (5 mM)) to provide (2R,3R,5S)-5-{[bis(4-methoxyphenyl) (phenyl)methoxy]methyl}-2-[2-(isobutyrylamino)-6-oxo-1,6-dihydro-9H-purin-9-yl] tetrahydrofuran-3-yl {[(2R,3R,4R,5R)-3-{[*tert*-butyl(dimethyl)silyl]oxy}-4-fluoro-5-(9H-purin-9-yl)tetrahydrofuran-2-yl]methyl}sulfamate (1.88 g, 51%). LCMS (AA): *m*/*z* = 1069.3 (M+H).

### Step 2: [(2R,3R,5S)-5-[[bis(4-methoxyphenyl)-phenyl-methoxy]methyl]-2-[2-(2-methylpropanoylamino)-6-oxo-1H-purin-9-yl]tetrahydrofuran-3-yl] N-[[(2R,3R,4R,5R)-4-fluoro-3-hydroxy-5-purin-9-yl-tetrahydrofuran-2-yl] methyl]sulfamate

To a solution of [(2R,3R,5S)-5-[[bis(4-methoxyphenyl)-phenyl-methoxy]methyl]-2-[2-(2-methylpropanoylamino)-6-oxo-1H-purin-9-yl]tetrahydrofuran-3-yl] N-[[(2R,3R,4R,SR)-3-[*tert*-butyl(dimethyl)silyl]oxy-4-fluoro-5-purin-9-yl-tetrahydrofuran-2-yl]methyl]sulfamate (2.07 g, 1.94 mmol) in THF (40 mL) in a polypropylene tube was added triethylamine trihydrofluoride (1.28 mL, 7.72 mmol) and triethylamine (0.55 mL, 3.90 mmol). The reaction solution was stirred at room temperature overnight and then diluted with dichloromethane and washed successively with saturated aqueous NaHCO₃ and brine. The organic phase was dried over anhydrous MgSO₄, filtered and concentrated. The residue obtained was purified on silica gel chromatography (0-10% EtOH in DCM) to afford [(2R,3R,5S)-5-[[bis(4-methoxyphenyl)-phenyl-methoxy]methyl]-2-[2-(2-methylpropanoylamino)-6-oxo-1H-purin-9-yl]tetrahydrofuran-3-yl] N-[[(2R,3R,4R,5R)-4-fluoro-3-hydroxy-5-purin-9-yl-tetrahydrofuran-2-yl]methyl]sulfamate (1.83g, 99 %). LCMS (AA): *m*/*z* = 953.3 (M-H).

### Step 3: (2R,3R,4R,5R)-4-fluoro-2-({[({(2R,3R,5S)-5-(hydroxymethyl)-2-[2-(isobutyrylamino)-6-oxo-1,6-dihydro-9H-purin-9-yl]tetrahydrofuran-3-yl}oxy) sulfonyl]amino}methyl)-5-(9H-purin-9-yl)tetrahydrofuran-3-yl hydrogen phosphonate - N,N-diethylethanamine (Intermediate 21)

Diphenyl phosphite (0.77 mL, 4.03 mmol) was added to a 0 °C solution of [(2R,3R,5S)-5-[[bis(4-methoxyphenyl)-phenyl-methoxy]methyl]-2-[2-(2-methylpropanoylamino)-6-oxo-1H-purin-9-yl]tetrahydrofuran-3-yl] N-[[(2R,3R,4R,5R)-4-fluoro-3-hydroxy-5-purin-9-yl-tetrahydrofuran-2-yl]methyl]sulfamate (1.83 g, 1.92 mmol) in pyridine (5.0 mL). Upon complete addition, the reaction mixture was allowed to stir at room temperature for 1 hour. Triethylamine (2 mL, 10 mmol) and water (2 mL, 100 mmol) were then added and the reaction mixture was stirred an additional 30 minutes at room temperature and concentrated to dryness. The residue dissolved in acetic acid (3.1 mL, 53 mmol) and water (0.82 mL, 45 mmol) was allowed to stir at room temperature for 1 hour. The reaction mixture was concentrated to dryness and the residue purified using silica gel chromatography (0-70% MeOH in DCM) to provide (2R,3R,4R,5R)-4-fluoro-2-({[({(2R,3R,5S)-5-(hydroxymethyl)-2-[2-(isobutyrylamino)-6-oxo-1,6-dihydro-9H-purin-9-yl]tetrahydrofuran-3-yl}oxy)sulfonyl]amino}methyl)-5-(9H-purin-9-yl)tetrahydrofuran-3-yl hydrogen phosphonate - N,N-diethylethanamine (0.942 g, 60 %). LCMS (AA): *m*/*z* = 717.3 (M-H).

### Step 4: N-{9-[(2S,SS,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2] tetraoxathiazaphosphacyclotetradecin-7-yl]-6-oxo-6,9-dihydro-1H-purin-2-yl}-2-methylpropanamide or N-19-[(2R,5S,7R,8R,12aR,14R,15R,15aR)-15-fluoro-2,10,10-trioxido-14-(9H-purin-9-yl)-2-sulfanyldecahydro-5,8-methanofuro[2,3-m] [1,3,6,9,10,11,2]tetraoxathiazaphosphacyclotetradecin-7-yl]-6-oxo-6,9-dihydro-1H-purin-2-yl}-2-methylpropanamide

Diphenyl phosphorochloridate (0.18 mL, 0.87 mmol) was added to pyridine (7.3 mL) and this solution was cooled to -35 °C. A solution of [(2R,3R,4R,SR)-4-fluoro-2-[[[(2R,3R,5 S)-5-(hydroxymethyl)-2-[2-(2-methylpropanoylamino)-6-oxo-1H-purin-9-yl]tetrahydrofuran-3-yl]oxysulfonylamino]methyl]-5-purin-9-yl-tetrahydrofuran-3-yl]oxyphosphinic acid- N,N-diethylethanamine (73.1 mg, 0.089 mmol) in DCM (8 mL) and pyridine (1.0 mL) was added dropwise to the cooled reaction mixture over 15 minutes. The resulting solution was stirred at -35 °C for 40 minutes. Solid 3H-1,2-benzodithiol-3-one (30.1 mg, 0.245 mmol) and water (0.064 mL) were added respectively to the reaction at reduced temperature and this mixture was further stirred at room temperature for 1 hour. The reaction solution was then cooled in an ice bath to 0 °C and was quenched with the addition of a solution of aqueous sodium thiosulfate (0.3M, 3.0 mL, 0.9 mmol). The mixture was stirred at room temperature for 5 minutes and concentrated under reduced pressure. The resulting residue was purified by reverse phase flash column chromatography (0-40% ACN in aqueous ammonium acetate (10 mM)) to afford N-{9-[(2S,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2] tetraoxathiazaphosphacyclotetradecin-7-yl]-6-oxo-6,9-dihydro-1H-purin-2-yl}-2-methylpropanamide or N-{9-[(2R,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2] tetraoxathiazaphosphacyclotetradecin-7-yl]-6-oxo-6,9-dihydro-1H-purin-2-yl}-2-methylpropanamide as a white solid (35 mg, 53 %). LCMS (AA): *m*/*z* = 729.2 (M-H).

### Step 5: 2-amino-9-[(2S,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2] tetraoxathiazaphosphacyclotetradecin-7-yl]-1,9-dihydro-6H-purin-6-one - N,N-diethylethanamine or 2-amino-9-[(2R,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine (compound I-11a)

To a solution of N-{9-[(2S,5S,7R,8R,12aR,14R,15R,15aR)-15-fluoro-2,10,10-trioxido-14-(9H-purin-9-yl)-2-sulfanyldecahydro-5, 8-methanofuro[2,3-m][1,3,6,9,10,11,2]tetraoxathiazaphosphacyclotetradecin-7-yl]-6-oxo-6,9-dihydro-1H-purin-2-yl}-2-methylpropanamide or N-{9-[(2R,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2] tetraoxathiazaphosphacyclotetradecin-7-yl]-6-oxo-6,9-dihydro-1H-purin-2-yl}-2-methylpropanamide (32 mg, 0.044 mmol) in acetonitrile (1.5 mL) was added aqueous NH₄OH (28-30% NH₃ basis, 3 mL, 20 mmol). The reaction mixture was stirred in a sealed vial at 35 °C overnight. The solvents were removed under reduced pressure and the residue purified by reverse phase column chromatography (0-40% ACN in aqueous triethylammonium acetate (10 mM)) to provide 2-amino-9-[(2S,5S,7R,8R,12aR,14R,15R,15aR)-15-fluoro-2,10,10-trioxido-14-(9H-purin-9-yl)-2-sulfanyldecahydro-5,8-methanofuro[2,3-m][1,3,6,9,10,11,2]tetraoxathiaza phosphacyclotetradecin-7-yl]-1,9-dihydro-6H-purin-6-one - N,N-diethylethanamine or 2-amino-9- [(2R, 5 S, 7R, 8R, 12aR, 14R, 15R, 15 aR)-15 -fluoro-2,10,10-trioxido-14-(9H-purin-9-yl)-2-sulfanyldecahydro-5,8-methanofuro[2,3-m][1,3,6,9,10,11,2]tetraoxathiazaphosphacyclo tetradecin-7-yl]-1,9-dihydro-6H-purin-6-one - N,N-diethylethanamine (**compound I-11a**, 14.5 mg, 44 %). LCMS (AA): *m*/*z* = 660.9 (M+H). ¹H NMR (400 MHz, D₂O) δ 1.28 (t, *J=* 7.3 Hz, 9 H), 2.59 - 2.71 (m, 1 H), 2.74 - 2.85 (m, 1 H), 3.20 (q, *J* = 7.3 Hz, 6 H), 3.49 - 3.60 (m, 1 H), 3.84 (dd, *J* = 13.5, 2.6 Hz, 1 H), 4.12 - 4.21 (m, 1 H), 4.41 (td, *J* = 11.7, 2.0 Hz, 1 H), 4.67 (brdd, *J* = 14.6, 9.7 Hz, 2H), 5.43 - 5.68 (m, 2 H), 6.05 (d, *J* = 7.6 Hz, 1 H), 6.27 (dt, *J* = 10.7, 7.6 Hz, 1 H), 6.68 (d, *J* = 1.0 Hz, 1 H), 7.95 (s, 1 H), 8.06 (s, 1 H), 8.66 (s, 1 H), 9.18 (s, 1H). ³¹P NMR (162 MHz, D₂O) δ 56.24 (s, 1 P). ¹⁹F NMR (376 MHz, D₂O) δ -195.72 (s, 1F).

### Example 20

The compound listed below **(Intermediate 16)** was prepared as described in Example 19, steps 1-3 substituting the starting material shown in the table for **Intermediate 12.**

| Starting material | Product | Analytical data |
|---|---|---|
| 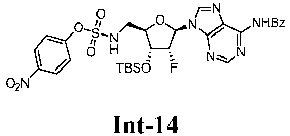 | 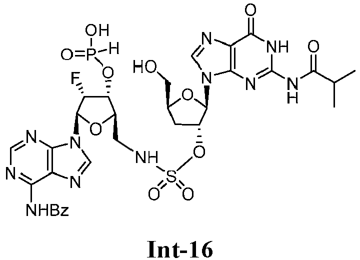 | LCMS (AA): *m*/*z* = 836.2 (M+H) |

### Example 21

### 2-amino-9-[(2S,5S,7R,8R,12aR,14R,15R,15aR)-14-(6-amino-9H-purin-9-yl)-15-fluoro-2,10,10-trioxido-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine or 2-amino-9-[(2R,5S,7R,8R,12aR,14R,15R,15aR)-14-(6-amino-9H-purin-9-yl)-15-fluoro-2,10,10-trioxido-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine (compound I-4a, compound I-4b)

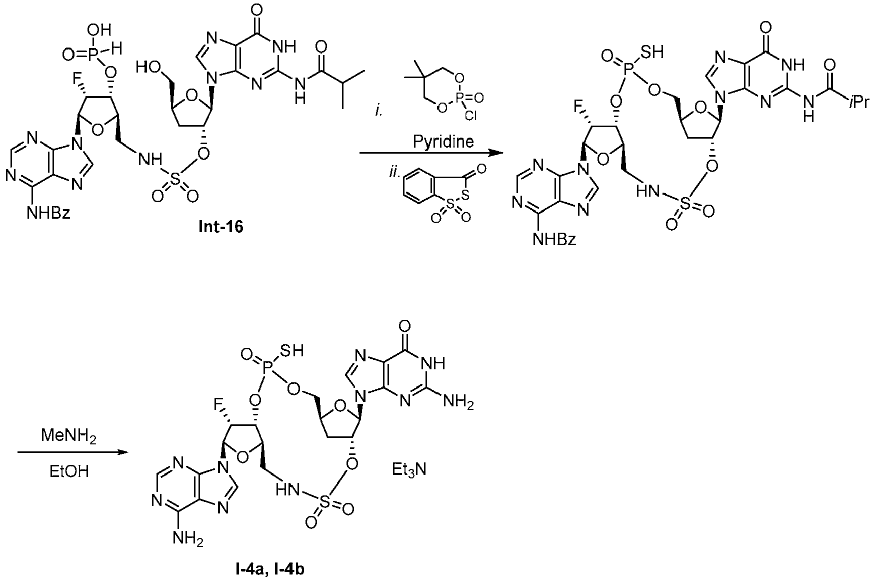

### Step 1: N-(9-{(2S,5S,7R,8R,12aR,14R,15R,15aR)-15-fluoro-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2]tetraoxathiazaphosphacyclotetradecin-14-yl}-9H-purin-6-yl)benzamide - N,N-diethylethanamine and N-(9-{(2R,5S,7R,8R,12aR, 14R,15R,15aR)-15-fluoro-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2] tetraoxathiazaphosphacyclotetradecin-14-yl}-9H-purin-6-yl)benzamide - N,N-diethylethanamine

[(2R,3R,4R,5R)-5-(6-benzamidopurin-9-yl)-4-fluoro-2-[[[(2R,3R,5S)-5-(hydroxymethyl)-2-[2-(2-methylpropanoylamino)-6-oxo-1H-purin-9-yl]tetrahydrofuran-3-yl]oxysulfonylamino]methyl]tetrahydrofuran-3-yl]oxyphosphinic acid;N,N-diethylethanamine **(Intermediate 16,** 0.282 g, 0.30 mmol) was co-evaporated with dry CH₃CN three times, dried under vacuum overnight, and finally dissolved in anhydrous pyridine (7.0 mL). 2-Chloro-5,5-dimethyl-1,3,2-dioxaphosphorinane 2-oxide (140 mg , 0.72mmol) was added and the mixture was allowed to stir at room temperature under an argon atmosphere for 50 minutes. Water (0.20 mL) was added, followed by 3H-1, 2-benzodithiol-3-one 1,1-dioxide (100 mg, 0.50 mmol). The solution was allowed to stir at room temperature for 40 minutes, concentrated under reduced pressure, co-evaporated with acetonitrile (x 3) and toluene (x 2) and dried under vacuum. Purification using silica gel chromatography (0-50% MeOH / CH₂Cl₂ with 0.5% Et₃N) provided 2 impure but separated diastereomers and a set of mixed fractions. Repurification of the early eluting peak using silica gel chromatography (0-10% MeOH / DCM with 0.5% Et₃N) provided N-(9-{(2S,5S,7R,8R,12aR,14R,15R,15aR)-15-fluoro-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2]tetraoxathiazaphosphacyclotetradecin-14-yl}-9H-purin-6-yl)benzamide - N,N-diethylethanamine or N-(9-{(2R,5S,7R,8R,12aR,14R,15R,15aR)-15-fluoro-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2]tetraoxathiazaphosphacyclotetradecin-14-yl}-9H-purin-6-yl)benzamide - N,N-diethylethanamine as a single diastereomer (0.111 g containing excess residual Et₃N). LCMS (AA): *m*/*z* = 850.2 (M+H). ³¹P NMR (162 MHz, CD₃OD) δ 57.95 (s, 1P). Repurification of the second eluting peak using silica gel chromatography (0-10% MeOH / DCM with 0.5% Et₃N) provided N-(9-{(2S,5S,7R,8R,12aR,14R,15R,15aR)-15-fluoro-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2]tetraoxathiazaphosphacyclotetradecin-14-yl}-9H-purin-6-yl)benzamide - N,N-diethylethanamine or N-(9-{(2R,5S,7R,8R,12aR,14R,15R,15aR)-15-fluoro-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2]tetraoxathiazaphosphacyclotetradecin-14-yl}-9H-purin-6-yl)benzamide - N,N-diethylethanamine as a single diastereomer (15 mg containing residual Et₃N). LCMS (AA): *m*/*z* = 850.2 (M+H). ³¹P NMR (162 MHz, CD₃OD) δ 55.30 (s, 1P).

### Step 2A: 2-amino-9-[(2S,5S,7R,8R,12aR,14R,15R,15aR)-14-(6-amino-9H-purin-9-yl)-15-fluoro-2,10,10-trioxido-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine or 2-amino-9-[(2R,5S,7R,8R,12aR,14R,15R, 15aR)-14-(6-amino-9H-purin-9-yl)-15-fluoro-2,10,10-trioxido-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine

N-(9-{(2S,SS,7R,8R,12aR,14R,15R,15aR)-15-fluoro-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2]tetraoxathiazaphosphacyclotetradecin-14-yl}-9H-purin-6-yl)benzamide - N,N-diethylethanamine or N-(9-{(2R,5S,7R,8R,12aR,14R,15R,15aR)-15-fluoro-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2]tetraoxathiazaphosphacyclotetradecin-14-yl}-9H-purin-6-yl)benzamide - N,N-diethylethanamine (the early eluting peak in Step 1 above, 114 mg, 0.031 mmol) was stirred in a solution of methylamine (33% in EtOH, 2.0 mL, 16.0 mmol) at room temperature overnight. The mixture was concentrated under reduced pressure, co-evaporated with MeOH, re-dissolved in MeOH and concentrated with Celite. The crude product adsorbed on Celite was purified by reverse phase flash column chromatography (0-25% ACN in aqueous triethylammonium acetate (10 mM)) to provide 2-amino-9-[(2S,5S,7R,8R,12aR,14R,15R,15aR)-14-(6-amino-9H-purin-9-yl)-15-fluoro-2,10,10-trioxido-2-sulfanyldecahydro-5,8-methanofuro[2,3-m][1,3,6,9, 10, 11,2]tetraoxathiaza phosphacyclotetradecin-7-yl]-1,9-dihydro-6H-purin-6-one - N,N-diethylethanamine or 2-amino-9-[(2R,5S,7R,8R,12aR,14R,15R,15aR)-14-(6-amino-9H-purin-9-yl)-15-fluoro-2,10,10-trioxido-2-sulfanyldecahydro-5,8-methanofuro[2,3-m][1,3,6,9,10,11,2]tetraoxathiazaphosphacyclo tetradecin-7-yl]-1,9-dihydro-6H-purin-6-one - N,N-diethylethanamine (**compound I-4b** 19 mg, 79%). LCMS (AA): *m*/*z* = 776.1(M+H). ¹H NMR (400 MHz, D₂O) δ1.13 (t, *J* = 7.3Hz, 9H), 2.53 - 2.42(m, 1H), 2.67 - 2.54 (m, 1H), 3.05 (d, *J* = 7.3Hz, 6H), 3.35 (dd, *J* = 1.3, 13.4 Hz, 1H), 3.67 (dd, *J =* 2.6, 13.4 Hz, 1H), 4.01 (ddd, *J=* 1.2, 5.0, 12.1 Hz, 1H), 4.24 (dt, *J* = 2.0, 11.6 Hz, 1H), 4.46 (br d, *J* = 9.4 Hz, 1H), 4.56 - 4.49 (m, 1H), 5.41 - 5.22 (m, 2H), 5.87 (d, *J* = 7.6 Hz, 1H), 6.13 - 6.04 (m, 1H), 6.45 - 6.36 (m, 1H), 7.15 (s, 1H), 7.77 (s, 1H), 8.09 (s, 1H). ³¹P NMR (162 MHz, D₂O) δ 56.15 (s, 1P).

### Step 2B: 2-amino-9-[(2S,5S,7R,8R,12aR,14R,15R,15aR)-14-(6-amino-9H-purin-9-yl)-15-fluoro-2,10,10-trioxido-2-sulfanyldecahydro-5,8-methanofuro[2,3-m] [1,3,6,9,10,11,2]tetraoxathiazaphosphacyclotetradecin-7-yl]-1,9-dihydro-6H-purin-6-one - N,N-diethylethanamine or 2-amino-9-[(2R,5S,7R,8R,12aR,14R,15R,15aR)-14-(6-amino-9H-purin-9-yl)-15-fluoro-2,10,10-trioxido-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine

N-(9-{(2S,5S,7R,8R,12aR,14R,15R,15aR)-15-fluoro-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2]tetraoxathiazaphosphacyclotetradecin-14-yl}-9H-purin-6-yl)benzamide - N,N-diethylethanamine or N-(9-{(2R,5S,7R,8R,12aR,14R,15R,15aR)-15-fluoro-7-[2-(isobutyrylamino)-6-oxo-1,6-dihydro-9H-purin-9-yl]-2,10,10-trioxido-2-sulfanyldecahydro-5,8-methanofuro[2,3-m][1,3,6,9,10,11,2]tetraoxathiazaphosphacyclotetradecin-14-yl}-9H-purin-6-yl)benzamide - N,N-diethylethanamine (the second eluting peak in Step 1 above, 14 mg, 0.011 mmol) was stirred in a solution of methylamine (33% in EtOH, 1.5 mL, 16.0 mmol) at rt for 4 hours. The mixture was concentrated under reduced pressure, co-evaporated with MeOH, acetonitrile and water respectively. The crude material was re-dissolved in MeOH and concentrated with Celite. The crude product adsorbed on Celite was purified by reverse phase flash column chromatography (0-50% ACN in aqueous triethylammonium acetate (10 mM)) to provide 2-amino-9-[(2S,5S,7R,8R,12aR,14R, 15R,15aR)-14-(6-amino-9H-purin-9-yl)-15-fluoro-2,10,10-trioxido-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine or 2-amino-9-[(2R,5S,7R,8R,12aR,14R,15R,15aR)-14-(6-amino-9H-purin-9-yl)-15-fluoro-2,10,10-trioxido-2-sulfanyldecahydro-5,8-methanofuro[2,3-m][1,3,6,9,10,11,2]tetraoxathiazaphosphacyclotetradecin-7-yl]-1,9-dihydro-6H-purin-6-one - N,N-diethylethanamine **(compound I-4a** 6.2 mg, 72 %). LCMS (AA): *m*/*z* = 776.1 (M+H). ¹H NMR (400 MHz, D₂O) δ 1.13 (t, J = 7.3 Hz, 9H), 2.65 - 2.48 (m, 2H), 3.05 (q, *J* = 7.3 Hz, 6H), 3.42 (br d, *J* = 13.3 Hz, 1H), 3.74 - 3.65 (m, 1H), 4.06 - 3.99 (m, 1H), 4.12 (br dd, *J* = 3.6, 10.9 Hz, 1H), 4.46 (br d, *J* = 8.8 Hz, 1H), 4.56 - 4.54 (m, 1H), 5.08 (td, *J* = 4.2, 8.8 Hz, 1H), 5.79 - 5.64 (m, 1H), 5.90 - 5.83 (m, 2H), 6.44 - 6.35 (m, 1H), 7.15 (s, 1H), 7.95 (s, 1H), 8.07 (s, 1H). ³¹P NMR (162 MHz, D₂O) δ 52.55 (s, 1P).

### Example 22

The compounds listed below were prepared as described in Example 21 starting with Step 1, substituting the starting material shown in the table for **Intermediate 16.**

| I Number | Starting material | Product | Analytical data | Note |
|---|---|---|---|---|
| I-11a | 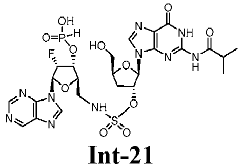 | 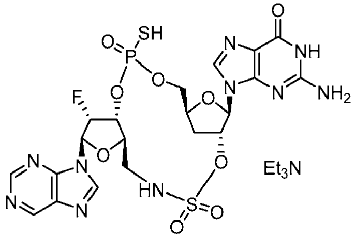 | Identical to data described in Example 19 | Major isomer isolated from Step 1 used as starting material in Step 2 |
| I-11b | 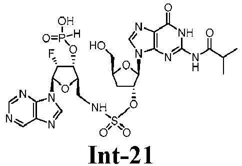 | 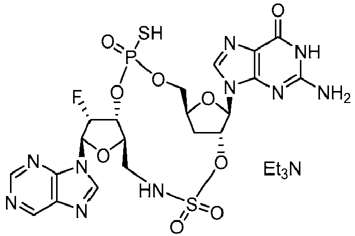 | LCMS (AA): *m*/*z* = 661.1 (M+H); ¹H NMR (400 MHz, D₂O) δ 1.25 (t, *J* = 7.3 Hz, 9H), 2.68 - 2.82 (m, 2H), 3.18 (q, *J* = 7.3 H, 6H), 3.61 (d, *J =* 11.9 Hz, 1H), 3.85 (dd, *J =* 3.5, 13.4 Hz, 1H), 4.13 - 4.19 (m, 1H), 4.23 (d, *J* = 2.9 Hz, 1H), 4.64 (d, *J =* 9.3 Hz, 2H), 5.29 - 5.40 (m, 2H), 6.02 - 6.06 (m, 2H), 6.64 - 6.71 (m, 1H), 8.14 (s, 1H), 8.17 (s, 1H), 8.64 (s, 1H), 9.14 (s, 1H). ³¹P NMR (162 MHz, D₂O) δ 52.93 (s, 1P). ¹⁹F NMR (376 MHz, D₂O) δ - 196.49 (s, 1F). | Minor isomer isolated from Step 1 used as starting material in Step 2 |

### Example 23

The compound listed below was prepared as described in Example 19 starting with Step 1, substituting the starting materials shown in the table for **Intermediate 12 and 1.**

| I Number | Starting material | Starting material | Product | Analytical data |
|---|---|---|---|---|
| I-20* | 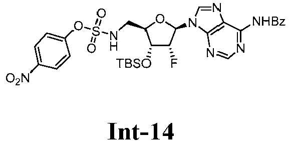 | 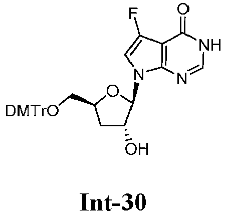 | 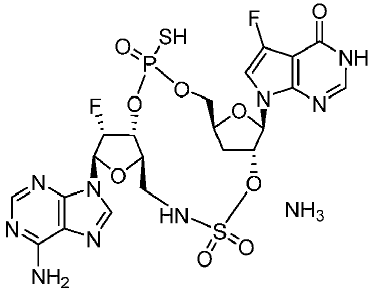 | LCMS (AA): *m*/*z* = 678.2 (M+H). ¹H NMR (400 MHz, D₂O) δ 2.59 - 2.65 (m, 1H), 2.74 - 2.83 (m, 1H), 3.54 - 3.59 (m, 1H), 3.75 - 3.80 (m, 1H), 4.08 - 4.14 (m, 1H), 4.30 - 4.37 (m, 1H), 4.47 - 4.51 (m, 1H), 4.64 - 4.69 (m, 1H), 5.14 - 5.26 (m, 1H), 5.29 - 5.49 (m, 2H), 6.36 (d, *J =* 7.2 Hz, 1H), 6.44 (d, *J* = 20.9 Hz, 1H), 7.30 (s, 1H), 7.38 (s, 1H), 7.95 (s, 1H), 8.15 (s, 1H). ³¹P NMR (162 MHz, D₂O) δ 55.76 (s, 1 P). ¹⁹F NMR (376 MHz, D₂O) δ -164.36 (s, 1F), -196.12 (s, 1F). |

| | | | | |
|---|---|---|---|---|
| * In Step 1, DIPEA was used instead of NEt₃ heating the reaction mixture at 40°C for 2 d. | | | | |

### Example 24

### [(1R,2R,3S,4R)-2-[tert-Butyl(dimethyl)silyl]oxy-3-fluoro-4-purin-9-yl-cyclopentyl]methanol (Intermediate 22)

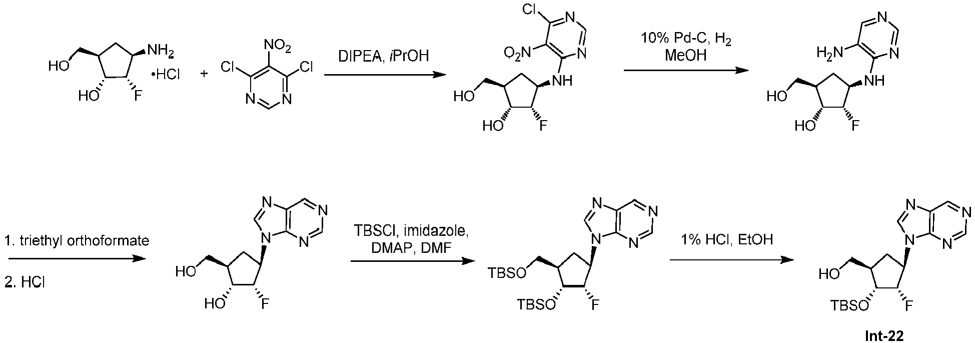

### Step 1: (1R,2S,3R,5R)-3-[(6-Chloro-5-nitro-pyrimidin-4-yl)amino]-2-fluoro-5-(hydroxymethyl)cyclopentanol

A solution of [(1*R*,2*S*,3*R*,4*R*)-2-fluoro-3-hydroxy-4-(hydroxymethyl) cyclopentyl]ammonium chloride (600 mg, 3.24 mmol) (for synthesis see Biggadike, K. et al. J. Chem. Soc. Perkin Trans. 1988, 3, 549-554.; Borthwick, A.D. et al. J. Med. Chem. 1990, 33, 179-186) and 4,6-dichloro-5-nitropyrimdine (770 mg, 3.89 mmol) in 2-propanol (9.0 mL) and DIPEA (1.7 mL, 9.73 mmol) was stirred at rt for 1 h. The reaction mixture was concentrated to dryness, then concentrated from toluene (2 x 10 mL). The crude compound was adsorbed onto Celite and purified by silica gel chromatography (0-10 % MeOH/DCM) to afford (1*R,2S,*3*R*,5*R)*-3-[(6-chloro-5-nitro-pyrimidin-4-yl)amino]-2-fluoro-5-(hydroxymethyl)cyclopentanol (778 mg, 78%). LCMS (AA): *m*/*z* = 307.0 (M+H).

### Step 2: (1R,2S,3R,5R)-3-[(5-Aminopyrimidin-4-yl)amino]-2-fluoro-5-(hydroxymethyl)cyclopentanol

(1R,2S,3R,SR)-3-[(6-Chloro-5-nitro-pyrimidin-4-yl)amino]-2-fluoro-5-(hydroxymethyl)cyclopentanol (776 mg, 2.53 mmol) was dissolved in methanol (33 mL). Palladium (10% on carbon, 388 mg, 0.36 mmol) was added and the mixture was stirred under 1 atmosphere of hydrogen for 1 day. The reaction mixture was filtered, washed with methanol and the filtrate was evaporated to dryness. The crude product was adsorbed onto Celite and purified by silica gel chromatography (0-60 % MeOH/DCM) to afford (1*R,2*S,3*R,5*R)-3-[(5-aminopyrimidin-4-yl)amino]-2-fluoro-5-(hydroxymethyl)cyclopentanol (613 mg, quantitative). LCMS (AA): *m*/*z* = 243.1 (M+H).

### Step 3: (1R,2S,3R,5R)-2-Fluoro-5-(hydroxymethyl)-3-purin-9-yl-cyclopentanol

(1*R*,2*S*,3*R*,5*R*)-3-[(5-aminopyrimidin-4-yl)amino]-2-fluoro-5-(hydroxymethyl)cyclopentanol (679 mg, 2.80 mmol) was concentrated from dry toluene (3 x 10 mL) prior to use and then placed under vacuum for 1h. The residue was then taken up in triethyl orthoformate (28 mL, 170 mmol). The mixture was heated to 100 °C and stirred under argon for 1 day. The solution was then cooled to rt and conc. HCl (12 M, 0.94 mL, 11.2 mmol) was added. The reaction mixture was stirred at rt for 2 h, diluted with water (8 mL) and treated with a saturated aqueous solution of NaHCO₃ (10 mL) to pH 8. The mixture was filtered and the filtrate was concentrated to dryness, then concentrated from toluene (2 x 10 mL). The crude compound was adsorbed onto Celite and purified by silica gel chromatography (0-40 % MeOH/DCM) to afford (1*R*,2*S*,3*R*,5*R*)-2-fluoro-5-(hydroxymethyl)-3-purin-9-yl-cyclopentanol (441 mg, 62%). LCMS (AA): *m*/*z* = 253.1 (M+H). ¹H NMR (DMSO-d₆) δ 9.18 (s, 1H), 8.95 (s, 1H), 8.77 (s, 1H), 5.40 (dd, *J* = 6.9, 5.1 Hz, 0.5H), 5.32 - 5.18 (m, 2.5H), 4.80 (t, *J* = 5.2 Hz, 1H), 4.15 - 4.07 (m, 1H), 3.61 - 3.47 (m, 2H), 2.44 - 2.34 (m, 1H), 2.19 - 2.09 (m, 1H), 1.93 - 1.83 (m, 1H). ¹⁹F NMR (DMSO- d₆) δ -200.03 (s, 1F).

### Step 4: tert-Butyl-[[(1R,2R,3S,4R)-2-[tert-butyl(dimethyl)silyl]oxy-3-fluoro-4-purin-9-yl-cyclopentyl]methoxy]-dimethyl-silane

(1*R*,2*S*,3*R*,5*R*)-2-fluoro-5-(hydroxymethyl)-3-purin-9-yl-cyclopentanol (811 mg, 3.22 mmol) was concentrated from dry toluene (3 x 10 mL) prior to use and then placed under vacuum for 1h. The residue was then taken up in dry DMF (8.1 mL) and 1*H*-imidazole (1.09 g, 16.1 mmol) and *N,N-*dimethylaminopyridine (78 mg, 0.64 mmol) were added. Then *tert-*butyldimethylsilyl chloride (2.42 g, 16.1 mmol) was added and the reaction mixture was allowed to stir at rt for 1 day. The reaction mixture was then partitioned between water and EtOAc. The organic phase was separated, washed with brine, dried (Na₂SO₄), filtered and evaporated. The crude compound was purified by silica gel chromatography (0-75% EtOAc/hexaness) to provide *tert*-butyl-[[(1*R*,2*R*,3*S*,4*R*)-2-[*tert*-butyl(dimethyl)silyl]oxy-3-fluoro-4-purin-9-yl-cyclopentyl]methoxy]-dimethyl-silane (1.39 g, 90%). LCMS (AA): *m*/*z* = 481.3.

### Step 5: [(1R,2R,3S,4R)-2-[tert-Butyl(dimethyl)silyl]oxy-3-fluoro-4-purin-9-yl-cyclopentyl]methanol, Intermediate 22

tert-Butyl-[[(1*R*,2*R*,3*R*,4*R*)-2-[*ter*-butyl(dimethyl)silyl]oxy-3-fluoro-4-purin-9-yl-cyclopentyl]methoxy]-dimethyl-silane (1.39 g, 2.89 mmol) was cooled to 0 °C and dissolved in a pre-cooled 0 °C solution of HCl (1% in EtOH, 24 mL). The reaction mixture was then placed in a refrigerator (4 °C) and let stand for 1 day. The reaction mixture was then warmed to rt and stirred for 4 h. The mixture was quenched slowly with a saturated aqueous solution of NaHCO₃ until a neutral pH was achieved. The solvent was then removed; the crude residue was partitioned between water and EtOAc. The organic phase was separated, washed with brine, dried (Na₂SO₄), filtered and concentrated. The crude compound was purified by silica gel chromatography (0-10% MeOH/DCM) to provide [(1*R*,2*R*,3*S*,4*R*)-2-[*tert*-butyl(dimethyl) silyl]oxy-3-fluoro-4-purin-9-yl-cyclopentyl]methanol (788 mg, 74%). LCMS (AA): *m*/*z* = 367.2.

### Example 25

### 9-[(2R,3R,4R,5R)-5-(Aminomethyl)-4-[tert-butyl(dimethyl)silyl]oxy-3-fluoro-tetrahydrofuran-2-yl]-1H-purin-6-one (Intermediate 23)

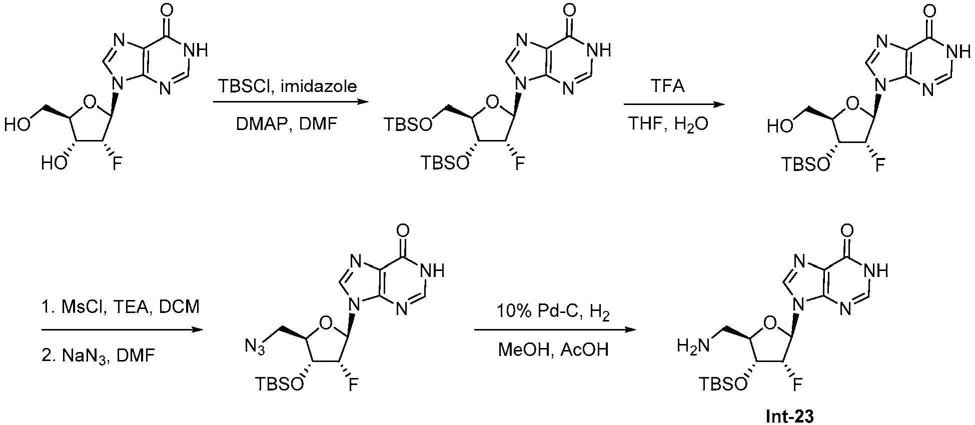

### Step 1: 9-[(2R,3R,4R,5R)-4-[tert-Butyl(dimethyl)silyl]oxy-5-[[tert-butyl (dimethyl)silyl]oxymethyl]-3-fluoro-tetrahydrofuran-2-yl]-1H-purin-6-one

The title compound was prepared starting from 2'-fluoro-2'-deoxyinosine following the procedure described in Example 15, Step 1. LCMS (AA): *m*/*z* = 499.1 (M+H).

### Step 2: 9-[(2R,3R,4R,5R)-4-[tert-Butyl(dimethyl)silyl]oxy-3-fluoro-5-(hydroxymethyl)tetrahydrofuran-2-yl]-1H-purin-6-one

The title compound was prepared from 9-[(2*R*,3*R*,4*R*,5*R*)-4-[*tert*-butyl (dimethyl)silyl]oxy-5-[[*tert*-butyl(dimethyl)silyl]oxymethyl]-3-fluoro-tetrahydrofuran-2-yl]-1H-purin-6-one following the procedure described in Example 15, Step 3. LCMS (AA): *m*/*z* = 385.2 (M+H).

### Step 3: 9-[(2R,3R,4R,5R)-5-(Azidomethyl)-4-[tert-butyl(dimethyl)silyl]oxy-3-fluoro-tetrahydrofuran-2-yl]-1H-purin-6-one

9-[(2*R*,3*R*,4*R*,5*R*)-4-[*tert*-Butyl(dimethyl)silyl]oxy-3-fluoro-5-(hydroxymethyl) tetrahydrofuran-2-yl]-1*H*-purin-6-one (856 mg, 2.23 mmol) was suspended in a mixture of DCM (10 mL), triethylamine (0.8 mL, 5.68 mmol) and DMF (22 uL) then cooled to 0 °C. To the mixture was added methanesulfonyl chloride (0.38 mL, 4.89 mmol) dropwise via syringe and the reaction mixture was allowed to stir at 0 °C for 1.5 h. The reaction mixture was quenched with water and extracted with DCM (2x). The organic phase was washed with brine, dried (Na₂SO₄), filtered and concentrated to give the crude intermediate which was then dissolved in DMF (6 mL). Sodium azide (0.955 g, 14.7 mmol) was added and the reaction mixture was heated to 70 °C for 3 h. The reaction mixture was cooled to rt and partitioned between a saturated aqueous solution of NaHCO₃ and DCM. The organic phase was separated, washed with brine, dried (Na₂SO₄), filtered and concentrated. The crude compound was purified by silica gel chromatography (0-5% MeOH/DCM) to provide 9-[(2*R*,3*R*,4*R*,5*R*)-5-(azidomethyl)-4-[*tert-*butyl(dimethyl)silyl]oxy-3-fluoro-tetrahydrofuran-2-yl]-1*H*-purin-6-one (391 mg, 43%). LCMS (AA): *m*/*z* = 410.2. ¹H NMR (DMSO-d₆) δ 12.47 (br s, 1H), 8.30 (s, 1H), 8.10 (d, *J* = 3.8 Hz, 1H), 6.27 (dd, *J* = 19.3, 2.4 Hz, 1H), 5.67 - 5.52 (m, 1H), 4.88 - 4.81 (m, 1H), 4.11 - 4.08 (m, 1H), 3.74 - 3.70 (m, 1H), 3.54 - 3.48 (m, 1H), 0.90 (s, 9H), 0.16 (s, 3H), 0.14 (s, 3H). ¹⁹F NMR (DMSO- d₆) δ -202.75 (s, 1F).

### Step 4: 9-[(2R,3R,4R,5R)-5-(Aminomethyl)-4-[tert-butyl(dimethyl)silyl]oxy-3-fluoro-tetrahydrofuran-2-yl]-1H-purin-6-one, Intermediate 23

9-[(2*R*,3*R*,4*R*,5*R*)-5-(azidomethyl)-4-[*tert*-butyl(dimethyl)silyl]oxy-3-fluoro-tetrahydrofuran-2-yl]-1*H*-purin-6-one (1.18 g, 2.88 mol) was dissolved in methanol (40 mL) and acetic acid (0.50 mL, 8.7 mmol). Palladium (10% on carbon, 250 mg, 0.24 mmol) was added and the mixture was stirred under 1 atm of hydrogen for 5 h. The reaction mixture was filtered and to the filtrate was added p-toluenesulfonic acid monohydrate (550 mg, 2.88 mmol). The reaction mixture was sonicated to dissolve the solids and the solution was evaporated to dryness to provide 9-[(2*R*,3*R*,4*R*,5*R*)-5-(aminomethyl)-4-[*tert*-butyl(dimethyl)silyl]oxy-3-fluoro-tetrahydrofuran-2-yl]-1H-purin-6-one as the 4-methylbenzenesulfonate salt (1.54 g, 91%). LCMS (AA): *m*/*z* = 384.1

### Example 25A

The compound *listed* below **(Intermediate 32**) was prepared as described in Example 25 steps 3-4, substituting the starting material shown in the table for 9-[(2*R*,3*R*,4*R*,5*R*)-4-[*tert*-butyl(dimethyl)silyl]oxy-3-fluoro-5-(hydroxymethyl)tetrahydrofuran-2-yl]-1*H*-purin-6-one.

| Starting material | Product | Analytical data |
|---|---|---|
| 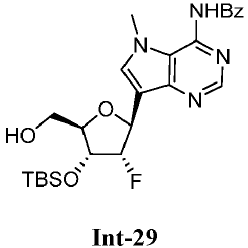 | 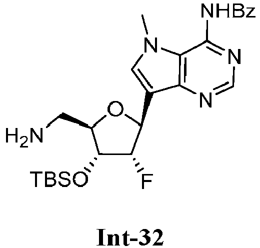 | LCMS (AA): *m*/*z* = 500.2 (M+H) |

### Example 26

### N-[9-[(2R,4S,5S)-4-Amino-5-[[bis(4-methoxyphenyl)-phenyl-methoxy]methyl] tetrahydrofuran-2-yl]-9H-purin-6-yl]benzamide (Intermediate 24)

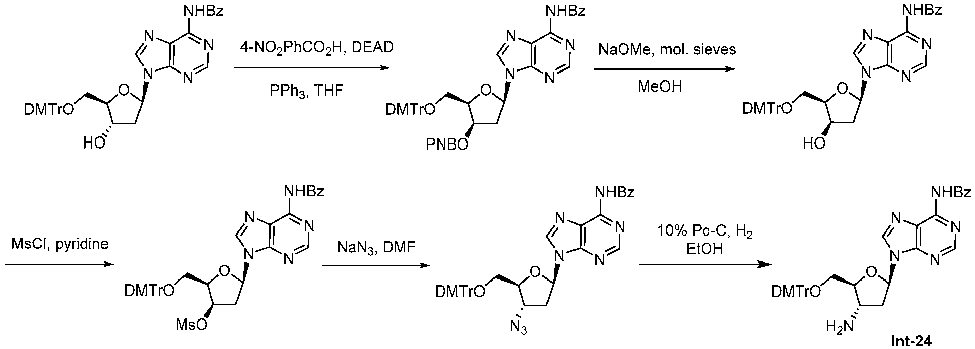

### Step 1: [(2R,3S,5R)-5-(6-Benzamidopurin-9-yl)-2-[[bis(4-methoxyphenyl)-phenyl-methoxy]methyl]tetrahydrofuran-3-yl] 4-nitrobenzoate

4-Nitrobenzoic acid (6.58 g, 39.4 mmol) was added to a solution of N-[9-[(2*R*,4*S*,5*R*)-5-[[bis(4-methoxyphenyl)-phenyl-methoxy]methyl]-4-hydroxy-tetrahydrofuran-2-yl]purin-6-yl]benzamide (25.0 g, 38.0 mmol) in dry THF (300 mL) under an argon atmosphere. Triphenyl phosphine (10.3 g, 39.4 mmol) was then added followed by diethyl azodicarboxylate (6.85 g, 39.4 mmol). The reaction mixture was then allowed to stir at rt for 4 h and concentrated to dryness. The crude residue was purified by silica gel chromatography (30-100% EtOAc/pentane) to provide [(2*R*,3*S*,5*R*)-5-(6-benzamidopurin-9-yl)-2-[[bis(4-methoxyphenyl)-phenyl-methoxy]methyl]tetrahydrofuran-3-yl] 4-nitrobenzoate 19.4 g, 63%). LCMS (AA): *m*/*z =* 807.1 (M+H).

### Step 2: N-[9-[(2R,4R,5R)-5-[[Bis(4-methoxyphenyl)-phenyl-methoxy]methyl]-4-hydroxy-tetrahydrofuran-2-yl] purin-6-yl] benzamide

To a solution of [(2*R*,3*R*,5*R*)-5-(6-benzamidopurin-9-yl)-2-[[bis(4-methoxyphenyl)-phenyl-methoxy]methyl]tetrahydrofuran-3-yl] 4-nitrobenzoate (18.9 g, 23.4 mmol) in MeOH (600 mL) was added 4Å molecular sieves (5 g) and sodium methoxide (30% solution in MeOH, 3.36 mL, 18.7 mmol) at 0 °C under an atmosphere of argon. The reaction mixture was stirred at 0 °C for 2 h. The reaction solution was then adjusted to pH 7 with hydrogen ion resin (Dowex^{®} 50WX2 hydrogen form, 100-200 mesh) and the solvents were removed. The crude residue was purified by silica gel chromatography (0-5% MeOH/EtOAc) to provide N-[9-[(2*R*,4*R*,5*R*)-5-[[bis(4-methoxyphenyl)-phenyl-methoxy]methyl]-4-hydroxy-tetrahydrofuran-2-yl]purin-6-yl]benzamide (12.6 g, 82%). LCMS (AA): *m*/*z* = 658.1 (M+H).

### Step 3: [(2R,3R,5R)-5-(6-Benzamidopurin-9-yl)-2-[[bis(4-methoxyphenyl)-phenyl-methoxy]methyl]tetrahydrofuran-3-yl]methanesulfonate

To a solution of N-[9-[(2*R*,4*R*,5*R*)-5-[[bis(4-methoxyphenyl)-phenyl-methoxy]methyl]-4-hydroxy-tetrahydrofuran-2-yl]purin-6-yl]benzamide (16.1 g, 24.4 mmol) in pyridine (200 mL) was added methanesulfonyl chloride (11.3 mL, 146 mmol) under an atmosphere of argon. The reaction mixture was stirred at rt for 16 h. The solvent was then removed by vacuum and the residue was partitioned between water and EtOAc. The organic phase was separated, washed with brine, dried (Na₂SO₄), filtered and concentrated to provide [(2*R*,3*R*,5*R*)-5-(6-benzamidopurin-9-yl)-2-[[bis(4-methoxyphenyl)-phenyl-methoxy]methyl]tetrahydrofuran-3-yl] methanesulfonate (16.8 g, 94%). LCMS (AA): *m*/*z* = 736.1 (M+H).

### Step 4: N-[9-[(2R,4S,5S)-4-Azido-5-[[bis(4-methoxyphenyl)-phenyl-methoxy]methyl] tetrahydrofuran-2-yl]-9H-purin-6-yl]benzamide

To a solution of **[(2*R*,3*R*,5*R*)-5-(6-benzamidopurin-9-yl)-2-[[bis(4-**methoxyphenyl)-phenyl-methoxy]methyl]tetrahydrofuran-3-yl] methanesulfonate (16.8 g, 22.8 mmol) in DMF (200 mL) was added 15-crown-5 (1.5 g, 6.84 mmol) followed by sodium azide (4.44 g, 68.4 mmol) under argon. The reaction mixture was heated to 90 °C and stirred for 16 h. The reaction mixture was then cooled to rt and partitioned between water and EtOAc. The organic phase was separated, washed with brine, dried (Na₂SO₄), filtered and concentrated. The crude compound was purified by silica gel chromatography (0-100% EtOAc/pentane) to provide N-[9-[(2*R*,4*S*,5*S*)-4-azido-5-[[bis(4-methoxyphenyl)-phenyl-methoxy]methyl]tetrahydrofuran-2-yl]-9*H*-purin-6-yl]benzamide (9.8 g, 63%). LCMS (AA): *m*/*z* = 683.1 (M+H).

### Step 5: N-[9-[(2R,4S,5S)-4-Amino-5-[[bis(4-methoxyphenyl)-phenyl-methoxy] methyl]tetrahydrofuran-2-yl]-9H-purin-6-yl]benzamide, Intermediate 24

N-[9-[(2*R*,4*S*,5*S*)-4-Azido-5-[[bis(4-methoxyphenyl)-phenyl-methoxy]methyl]tetrahydrofuran-2-yl]-9*H*-purin-6-yl]benzamide (10 g, 14.6 mmol) was dissolved in ethanol (100 mL). Palladium (10% on carbon, 3.09 g, 2.92 mmol) was added and the mixture was stirred under 40 psi of hydrogen for 2 days. The reaction mixture was filtered, washed with methanol and the filtrate was evaporated to dryness. The crude product was purified by silica gel chromatography (0-10 % MeOH/DCM) to afford N-[9-[(2*R*,4*S*,5*S*)-4-amino-5-[[bis(4-methoxyphenyl)-phenyl-methoxy]methyl]tetrahydrofuran-2-yl]-9*H*-purin-6-yl]benzamide (7.32 g, 76%). ¹H NMR (CDCl₃) δ 9.00(br s, 1H), 8.74 (s, 1H), 8.19 (s, 1H), 8.00 (d, *J* = 7.3 Hz, 2H), 7.63 - 7.56 (m, 1H), 7.55 - 7.47 (m, 2H), 7.39 (d, *J* = 7.3 Hz, 2H), 7.35 - 7.14 (m, 7H), 6.79 (d, *J* = 8.1 Hz, 4H), 6.43 (dd, *J* = 7.0, 3.5 Hz, 1H), 3.95 - 3.81 (m, 2H), 3.76 (s, 6H), 3.41 (d, *J* = 4.2 Hz, 2H), 2.86 - 2.81 (m, 1H), 2.40 - 2.35 (m, 1H).

### Example 27

### N-[9-[(2R,3R,4R,5S)-4-Amino-5-[[bis(4-methoxyphenyl)-phenyl-methoxy]methyl]-3-fluoro-tetrahydrofuran-2-yl]purin-6-yl]benzamide (Intermediate 25)

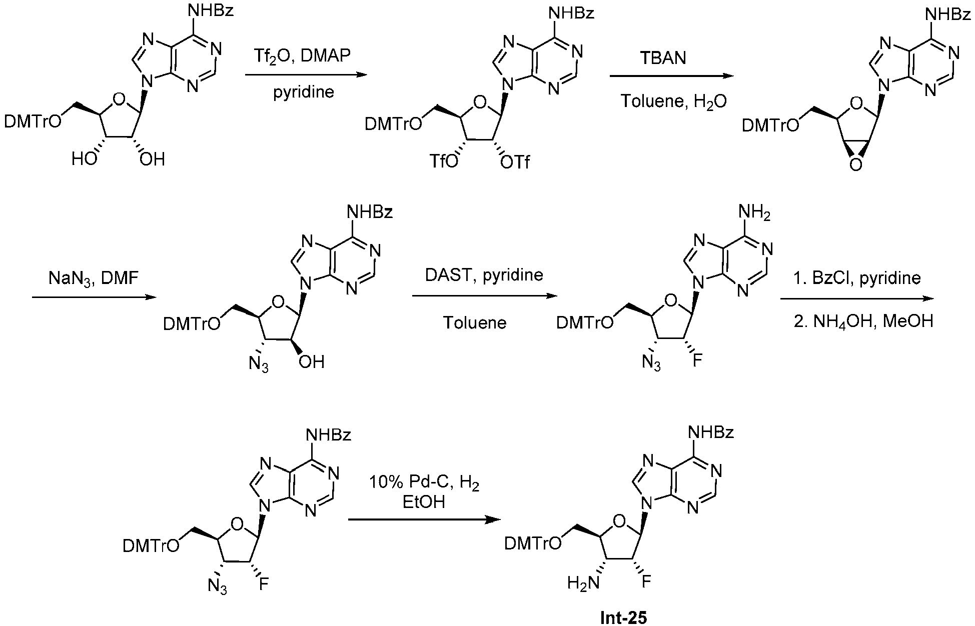

### Step 1: [(2R,3R,4R,5R)-5-(6-Benzamidopurin-9-yl)-2-[[bis(4-methoxyphenyl)-phenyl-methoxy]methyl]-4-(trifluoromethylsulfonyloxy)tetrahydrofuran-3-yl] trifluoromethanesulfonate

N-[9-[(2*R*,3*R*,4*S*,5*R*)-5-[[Bis(4-methoxyphenyl)-phenyl-methoxy]methyl] -3,4-dihydroxy-tetrahydrofuran-2-yl]purin-6-yl]benzamide (13.9 g, 20.6 mmol) and *N,N-*dimethylaminopyridine (6.37 g, 51.6 mmol) were dissolved in pyridine (170 mL) and the reaction mixture was cooled to 0 °C. Trifluoromethane sulfonic anhydride (23 mL, 134 mmol) was added dropwise and the reaction mixture was allowed to stir at 0 °C for 30 min. The reaction mixture was then warmed to rt and stirred for an additional 90 min. The reaction mixture was recooled to 0 °C and partitioned between DCM and water. The organic phase was separated, washed with brine, dried (Na₂SO₄), filtered and concentrated. The crude compound was purified by silica gel chromatography (0-75% EtOAc/hexaness) to provide [(2*R*,3*R*,4*R*,5*R*)-5-(6-benzamidopurin-9-yl)-2-[[bis(4-methoxyphenyl)-phenyl-methoxy]methyl]-4-(trifluoromethylsulfonyloxy)tetrahydrofuran-3-yl] trifluoromethanesulfonate (16.87, 87%). LCMS (AA): *m*/*z =* 938.1 (M+H).

### Step 2: N-[9-[(1S,2R,4R,5S)-4-[[Bis(4-methoxyphenyl)-phenyl-methoxy]methyl]-3,6-dioxabicyclo[3.1.0]hexan-2-yl]purin-6-yl]benzamide

A solution of [(2*R*,3*R*,4*R*,5*R*)-5-(6-benzamidopurin-9-yl)-2-[[bis(4-methoxyphenyl)-phenyl-methoxy]methyl]-4-(trifluoromethylsulfonyloxy)tetrahydrofuran-3-yl] trifluoromethanesulfonate (11.57 g, 12.3 mmol) and tetrabutylammonium nitrite (14.7 g, 49.3 mmol) in toluene (200 mL) and water (9 mL) was stirred vigorously at rt for 2 h. The reaction mixture was diluted with water (20 mL) and the organic phase was separated. The water layer was then extracted with EtOAc (2x). The combined organic phases were washed with brine, dried (Na₂SO₄), filtered and concentrated. The crude compound was purified by reverse phase flash column chromatography (0-100% ACN in aqueous ammonium acetate (10 mM)) to provide N-[9-[(1*S*,2*R*,4*R*,5*S*)-4-[[bis(4-methoxyphenyl)-phenyl-methoxy]methyl]-3,6-dioxabicyclo[3.1.0]hexan-2-yl]purin-6-yl]benzamide (4.38 g, 54%). LCMS (AA): *m*/*z* = 656.3 (M+H).

### Step 3: N-[9-[(2R,3S,4S,5S)-4-Azido-5-[[bis(4-methoxyphenyl)-phenyl-methoxy]methyl]-3-hydroxy-tetrahydrofuran-2-yl]purin-6-yl]benzamide

N-[9-[(1*S*,2*R*,4*R*,5*S*)-4-[[Bis(4-methoxyphenyl)-phenyl-methoxy]methyl]-3,6-dioxabicyclo[3.1.0]hexan-2-yl]purin-6-yl]benzamide (3.76 g, 5.74 mmol), sodium azide (2.26 g, 34.4 mmol) and ammonium chloride (620 mg, 11.5 mmol) were dissolved in DMF (37 mL) and water (5.5 mL). The reaction mixture was heated to 120 °C and allowed to stir for 20 min. The reaction mixture was cooled to rt and partitioned between EtOAc and water. The organic phase was separated, washed with brine, dried (Na₂SO₄), filtered and concentrated. The crude compound was purified by silica gel chromatography (0-80% EtOAc/hexaness) to provide N-[9-[(2*R*,3*S*,4*S*,5*S*)-4-azido-5-[[bis(4-methoxyphenyl)-phenyl-methoxy]methyl]-3-hydroxy-tetrahydrofuran-2-yl]purin-6-yl]benzamide (2.49 g, 62%). LCMS (AA): *m*/*z* = 699.3 (M+H). ¹H NMR (DMSO-d₆) δ 11.19 (s, 1H), 8.69 (s, 1H), 8.49 (s, 1H), 8.05 (d, *J=* 7.5 Hz, 2H), 7.67 - 7.62 (m, 1H), 7.58 - 7.53 (m, 2H), 7.38 (d, *J* = 7.4 Hz, 2H), 7.29 - 7.19 (m, 7H), 6.88 - 6.81 (m, 4H), 6.45 (d, *J* = 6.3 Hz, 1H), 6.23 (d, *J* = 5.3 Hz, 1H), 4.67 - 4.61 (m, 1H), 4.58 - 4.52 (m, 1H), 4.02 - 3.95 (m, 1H), 3.72 (s, 6H), 3.42 - 3.37 (m, 1H), 3.33 - 3.27 (m, 1H).

### Step 4: 9-[(2R,3R,4R,5S)-4-Azido-5-[[bis(4-methoxyphenyl)-phenyl-methoxy] methyl]-3-fluoro-tetrahydrofuran-2-yl] purin-6-amine

To a solution of N-[9-[(2*R*,3*S*,4*S*,5*S*)-4-azido-5-[[bis(4-methoxyphenyl)-phenyl-methoxy]methyl]-3-hydroxy-tetrahydrofuran-2-yl]purin-6-yl]benzamide (2.88 g, 4.12 mmol) and pyridine (10 mL, 124 mmol) in toluene (53 mL) was added *N*,*N-*diethylaminosulfur trifluoride (3.69 mL, 26.4 mmol) dropwise at rt. The reaction mixture was allowed to stir for 30 min. followed by heating to 80°C and stirring for an additional 60 min. The reaction mixture was partitioned between EtOAc and water. The organic phase was separated, washed with brine, dried (Na₂SO₄), filtered and concentrated. The crude compound was purified by silica gel chromatography (0-100% EtOAc/DCM) to provide 9-[(2*R*,3*R*,4*R*,5*S*)-4-azido-5-[[bis(4-methoxyphenyl)-phenyl-methoxy]methyl]-3-fluoro-tetrahydrofuran-2-yl]purin-6-amine (1.76 g, 72%). LCMS (AA): *m*/*z =* 597.3 (M+H).

### Step 5: N-[9-[(2R,3R,4R,5S)-4-Azido-5-[[bis(4-methoxyphenyl)-phenyl-methoxy] methyl]-3-fluoro-tetrahydrofuran-2-yl]purin-6-yl] benzamide

To a solution of 9-[(2*R*,3*R,*4*R*,5*S*)-4-azido-5-[[bis(4-methoxyphenyl)-phenyl-methoxy]methyl]-3-fluoro-tetrahydrofuran-2-yl]purin-6-amine (2.58 g, 4.3 mmol) in pyridine (32 mL) cooled to 0 °C was added benzoyl chloride (0.90 mL, 7.8 mmol) dropwise. The reaction mixture was then stirred at 0 °C for 2 h., warmed to rt and stirred for an additional 16 h. A solution of ammonium hydroxide (30% solution in water, 2 mL, 17.3 mmol) diluted in water (3.35 mL) was added dropwise at 0 °C. Then methanol (4.2 mL) was added and the reaction mixture was warmed to rt and stirred for 2 h. The reaction mixture was partitioned between EtOAc and water. The organic phase was separated, washed with brine, dried (Na₂SO₄), filtered and concentrated. The crude compound was purified by silica gel chromatography (40-100% EtOAc/hexaness) to provide N-[9-[(2*R*,3*R*,4*R*,5*S*)-4-azido-5-[[bis(4-methoxyphenyl)-phenyl-methoxy]methyl]-3-fluoro-tetrahydrofuran-2-yl]purin-6-yl]benzamide (2.63 g, 87%). LCMS (AA): *m*/*z* = 701.3 (M+H).

### Step 6: N-[9-[(2R,3R,4R,5S)-4-Amino-5-[[bis(4-methoxyphenyl)-phenyl-methoxy] methyl]-3-fluoro-tetrahydrofuran-2-yl]purin-6-yl]benzamide, Intermediate 25

N-[9-[(2*R*,3*R*,4*R*,5*S*)-4-Azido-5-[[bis(4-methoxyphenyl)-phenyl-methoxy]methyl]-3-fluoro-tetrahydrofuran-2-yl]purin-6-yl]benzamide (2.62 g, 3.74 mmol) was dissolved in ethanol (37 mL). Palladium (10% on carbon, 800 mg, 0.75 mmol) was added and the mixture was stirred under an atmosphere of hydrogen for 1 day. The reaction mixture was filtered, washed with methanol and the filtrate was evaporated to dryness to afford N-[9-[(2*R*,3*R*,4*R*,5*S*)-4-amino-5-[[bis(4-methoxyphenyl)-phenyl-methoxy]methyl]-3-fluoro-tetrahydrofuran-2-yl]purin-6-yl]benzamide (2.48 g, 98%). LCMS (AA): *m*/*z* = 675.3 (M+H). ¹H NMR (DMSO-d₆) δ 11.05 (br s, 1H), 8.74 (s, 1H), 8.58 (s, 1H), 8.04 (d, *J=* 7.3 Hz, 2H), 7.67 - 7.63 (m, 1H), 7.58 - 7.53 (m, 2H), 7.32 (d, *J=* 6.9 Hz, 2H), 7.24 - 7.15 (m, 7H), 6.83 - 6.77 (m, 4H), 6.43 (d, *J* = 20.2 Hz, 1H), 5.49 (dd, *J* = 52.6, 4.1 Hz, 1H), 4.12 - 3.93 (m, 2H), 3.71 (s, 3H), 3.70 (s, 3H), 3.34 - 3.27 (m, 2H). ¹⁹F NMR (DMSO- d₆) δ -198.21 (s, 1F).

### Example 28

### N-[9-[(2R,3R,5S)-3-[tert-Butyl(dimethyl)silyl]oxy-5-(hydroxymethyl)tetrahydrofuran-2-yl]-6-oxo-1H-purin-2-yl]-2-methyl-propanamide (Intermediate 26)

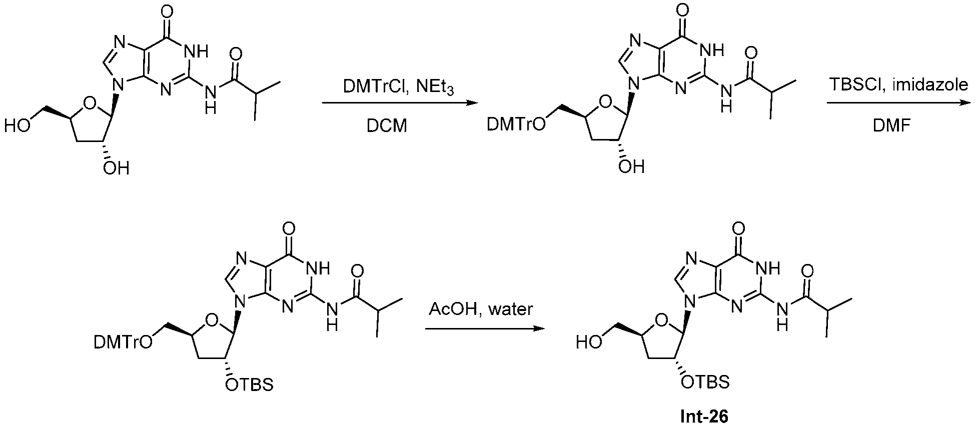

### Step 1: N-[9-[(2R,3R,5S)-5-[[Bis(4-methoxyphenyl)-phenyl-methoxy]methyl]-3-hydroxy-tetrahydrofuran-2-yl]-6-oxo-1H-purin-2-yl]-2-methyl-propanamide

To a solution of N-[9-[(2*R*,3*R*,5*S*)-3-hydroxy-5-(hydroxymethyl)tetrahydrofuran-2-yl]-6-oxo-1*H*-purin-2-yl]-2-methyl-propanamide (7.0 g, 20.7 mmol) and triethylamine (6.4 mL, 45.5 mmol) dissolved in a DCM/DMF mixture (5:1 ratio, 180 mL) was added 4,4'-dimethoxytrityl chloride (14.0 g, 41.4 mmol) at 0 °C. The reaction mixture was stirred at 0 °C for 1 h, then warmed to 5 °C and stirred for 16 h. The reaction mixture was diluted with water (100 mL), and the organic phase was separated. The organic phase was then washed with brine, dried (Na₂SO₄), filtered and concentrated. The crude compound was purified by silica gel chromatography (0-10% MeOH/DCM) to provide N-[9-[(2*R*,3*R*,5*S*)-5-[[bis(4-methoxyphenyl)-phenyl-methoxy]methyl]-3-hydroxy-tetrahydrofuran-2-yl]-6-oxo-1*H*-purin-2-yl]-2-methyl-propanamide (7.65 g, 58%). ¹HNMR (DMSO-d₆) δ12.10 (br s, 1H), 11.69 (br s, 1H), 8.08 (s, 1H), 7.33 - 7.29 (m, 2H), 7.26 - 7.16 (m, 7H), 6.83 - 6.77 (m, 4H), 5.86 - 5.84 (m, 1H), 5.68 (d, *J* = 4.0 Hz, 1H), 4.69 - 4.65 (m, 1H), 4.52 - 4.46 (m, 1H), 3.71 (s, 6H), 3.20 - 3.11 (m, 2H), 2.83 - 2.75 (m, 1H), 2.36 - 2.29 (m, 1H), 1.99 - 1.92 (m, 1H), 1.12 (d, *J* = 6.6 Hz, 6H).

### Step 2: N-[9-[(2R,3R,5S)-5-[[Bis(4-methoxyphenyl)-phenyl-methoxy]methyl]-3-[tert-butyl(dimethyl)silyl]oxy-tetrahydrofuran-2-yl]-6-oxo-1H-purin-2-yl]-2-methyl-propanamide

To a solution of N-[9-[(2*R*,3*R*,5*S*)-5-[[bis(4-methoxyphenyl)-phenyl-methoxy]methyl]-3-hydroxy-tetrahydrofuran-2-yl]-6-oxo-1*H*-purin-2-yl]-2-methyl-propanamide (19.0 g, 29.7 mmol) and 1*H*-imidazole (5.65 g, 83.1 mmol) dissolved in DMF (200 mL) was added *tert*-butyldimethylsilyl chloride (11.1 g, 74.2 mmol). The reaction mixture was allowed to stir at 5 °C for 16 h. The reaction mixture was then partitioned between water and EtOAc. The organic phase was separated, washed with brine, dried (Na₂SO₄), filtered and evaporated. The crude compound was purified by silica gel chromatography (50- 75% EtOAc/pentane) to provide N-[9-[(2*R*,3*R*,5*S*)-5-[[bis(4-methoxyphenyl)-phenyl-methoxy]methyl]-3-[*tert-*butyl(dimethyl)silyl]oxy-tetrahydrofuran-2-yl]-6-oxo-1*H*-purin-2-yl]-2-methyl-propanamide (18.5 g, 83%).

### Step 3: N-[9-[(2R,3R,5S)-3-[tert-Butyl(dimethyl)silyl]oxy-5-(hydroxymethyl) tetrahydrofuran-2-yl]-6-oxo-1H-purin-2-yl]-2-methyl-propanamide, Intermediate 26

A mixture of N-[9-[(2*R*,3*R*,5*S*)-5-[[bis(4-methoxyphenyl)-phenyl-methoxy]methyl]-3-[*tert*-butyl(dimethyl)silyl]oxy-tetrahydrofuran-2-yl]-6-oxo-1*H*-purin-2-yl]-2-methyl-propanamide (18.2 g, 24.1 mmol) in AcOH/water (4:1 mix, 100 mL) was stirred at 10 °C for 3 h. The reaction mixture was then adjusted to pH 7 by addition of a saturated aqueous NaHCO₃ solution. EtOAc was added and the organic phase was separated. The organic phase was then washed with brine, dried (Na₂SO₄), filtered and evaporated. The crude compound was triturated with a pentane/EtOAc mixture (10:1, 200 mL) to provide N-[9-[(2*R,*3*R*,5*S*)-3-[*tert-*butyl(dimethyl)silyl]oxy-5-(hydroxymethyl)tetrahydrofuran-2-yl]-6-oxo-1*H*-purin-2-yl]-2-methyl-propanamide (Intermediate 26, 9.50 g, 88%). LCMS (AA): *m*/*z* = 452.1 (M+H). ¹H NMR (CDCl₃) δ 12.13 (br s, 1H), 8.75 (s, 1H), 7.76 (s, 1H), 5.58 (d, *J* = 4.8 Hz, 1H), 4.95 - 4.85 (m, 1H), 4.77 - 4.73 (m, 1H), 4.54 - 4.49 (m, 1H), 4.00 (dd, *J =* 12.5, 2.0 Hz, 1H), 3.62 - 3.57 (m, 1H), 2.74 - 2.66 (m, 1H), 2.51 - 2.44 (m, 1H), 2.17 - 2.08 (m, 1H), 1.28 (d, *J* = 7.0 Hz, 3H), 1.27 (d, *J=* 6.6 Hz, 3H), 0.82 (s, 9H), -0.06 (s, 3H), -0.15 (s, 3H).

### Example 29

### 2-Amino-9-[(5S,7R,8R,10S,12aS,14R,15aS)-14-(6-amino-9H-purin-9-yl)-2,2,10-trioxido-10-sulfanyldecahydro-5,8-methanofuro[3,2-l][1,3,6,9,10,11,2] tetraoxathiazaphosphacyclotetradecin-7-yl]-1,9-dihydro-6H-purin-6-one - sodiumor 2-amino-9-[(5S,7R,8R,10R,12aS,14R,15aS)-14-(6-amino-9H-purin-9-yl)-2,2,10-trioxido-10-sulfanyldecahydro-5,8-methanofuro[3,2-1] [1,3,6,9,10,11,2] tetraoxathiazaphosphacyclotetradecin-7-yl]-1,9-dihydro-6H-purin-6-one - sodium (compound I-12a)

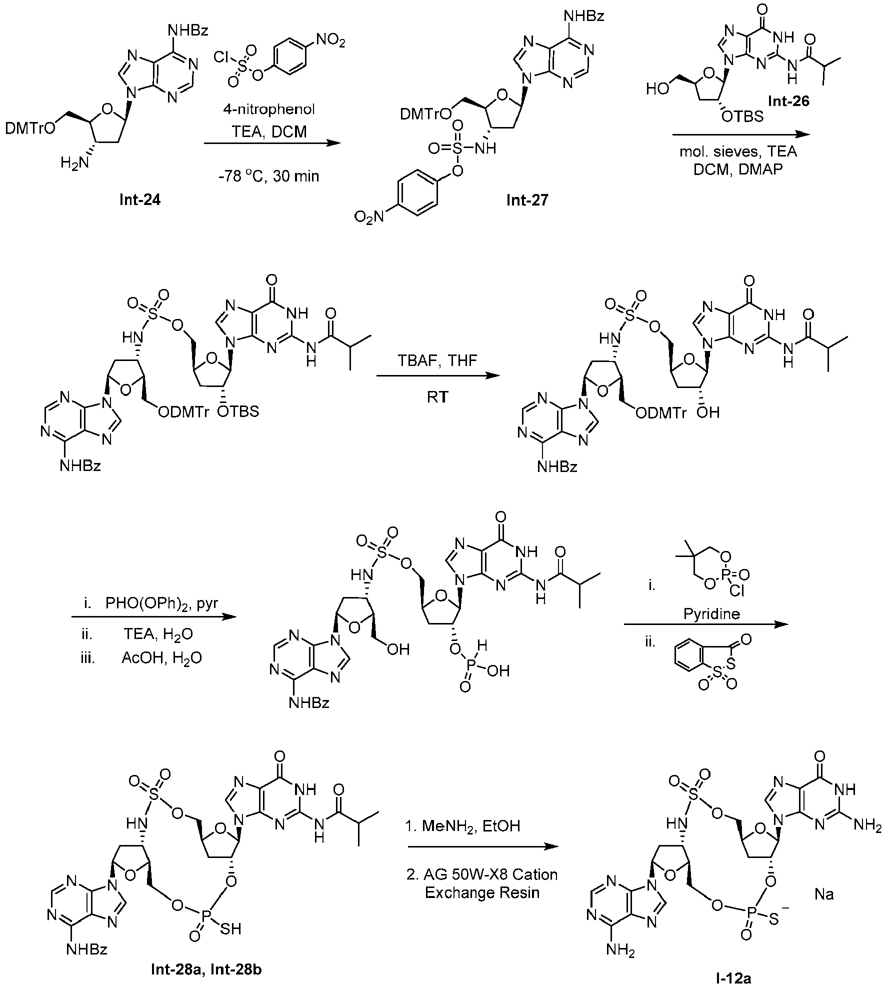

### Step 1: (4-Nitrophenyl) N-[(2S,3S,5R)-5-(6-benzamidopurin-9-yl)-2-[[bis(4-methoxyphenyl)-phenyl-methoxy] methyl] tetrahydrofuran-3-yl] sulfamate (Intermediate 27)

The title compound was prepared from N-[9-[(2*R*,4*S*,5*S*)-4-amino-5-[[bis(4-methoxyphenyl)-phenyl-methoxy]methyl]tetrahydrofuran-2-yl]-9*H*-purin-6-yl]benzamide **(Intermediate 24)** following the procedure described in Example 8, Step 1. LCMS (AA): *m*/*z* = 858.3 (M+H).

### Step 2: [(2S,4R,5R)-4-[tert-Butyl(dimethyl)silyl]oxy-5-[2-(2-methylpropanoylamino)-6-oxo-1H-purin-9-yl]tetrahydrofuran-2-yl]methyl N-[(2S,3S,5R)-5-(6-benzamidopurin-9-yl)-2-[[bis(4-methoxyphenyl)-phenyl-methoxy]methyl] tetrahydrofuran-3-yl]sulfamate

The title compound was prepared from **Intermediate 27** and **Intermediate 26** following the procedure described in Example 8, Step 2 heating at 40 °C for 16 h. LCMS (AA): *m*/*z* = 1170.5 (M+H).

### Step 3: [(2S,4R,5R)-4-Hydroxy-5-[2-(2-methylpropanoylamino)-6-oxo-1H-purin-9-yl]tetrahydrofuran-2-yl] methyl N-[(2S,3S,SR)-5-(6-benzamidopurin-9-yl)-2-[[bis(4-methoxyphenyl)-phenyl-methoxy]methyl]tetrahydrofuran-3-yl]sulfamate

The title compound was prepared from [(2*S*,4*R*,5*R*)-4-[*tert-*butyl(dimethyl)silyl]oxy-5-[2-(2-methylpropanoylamino)-6-oxo-1H-purin-9-yl]tetrahydrofuran-2-yl]methyl N-[(2*S*,3*S*,5*R*)-5-(6-benzamidopurin-9-yl)-2-[[bis(4-methoxyphenyl)-phenyl-methoxy]methyl]tetrahydrofuran-3-yl]sulfamate following the procedure described in Example 8, Step 3. LCMS (AA): *m*/*z* = 1056.4 (M+H).

### Step 4: [(2R,3R,5S)-5-[[(2S,3S,5R)-5-(6-Benzamidopurin-9-yl)-2-(hydroxymethyl) tetrahydrofuran-3-yl]sulfamoyloxymethyl]-2-[2-(2-methylpropanoylamino)-6-oxo-1H-purin-9-yl]tetrahydrofuran-3-yl]oxyphosphinic acid

The title compound was prepared from [(2*S*,4*R*,5*R*)-4-hydroxy-5-[2-(2-methylpropanoylamino)-6-oxo-1H-purin-9-yl]tetrahydrofuran-2-yl]methyl N-[(2*S*,3*S*,*R*)-5-(6-benzamidopurin-9-yl)-2-[[bis(4-methoxyphenyl)-phenyl-methoxy]methyl]tetrahydrofuran-3-yl]sulfamate following the procedure described in Example 8, Step 4. LCMS (AA): *m*/*z* = 818.2 (M+H).

### Step 5: N-(9-{(5S,7R,8R,10S,12aS,14R,15aS)-7-[2-(Isobutyrylamino)-6-oxo-1,6-dihydro-9H-purin-9-yl]-2,2,10-trioxido-10-sulfanyldecahydro-5,8-methanofuro[3,2-l][1,3,6,9,10,11,2]tetraoxathiazaphosphacyclotetradecin-14-yl}-9H-purin-6-yl) benzamide or N-(9-{(5S,7R,8R,10R,12aS,14R,15aS)-7-[2-(isobutyrylamino)-6-oxo-1,6-dihydro-9H-purin-9-yl]-2,2,10-trioxido-10-sulfanyldecahydro-5,8-methanofuro [3,2-l][1,3,6,9,10,11,2]tetraoxathiazaphosphacyclotetradecin-14-yl}-9H-purin-6-yl)benzamide, Intermediate 28a and Intermediate 28b

The title compounds were prepared from [(2*R*,3*R*,5*S*)-5-[[(2*S*,3*S*,5*R*)-5-(6-benzamidopurin-9-yl)-2-(hydroxymethyl)tetrahydrofuran-3-yl]sulfamoyloxymethyl]-2-[2-(2-methylpropanoylamino)-6-oxo-1H-purin-9-yl]tetrahydrofuran-3-yl]oxyphosphinic acid following the procedure described in Example 8, Step 5 to provide **Intermediate 28a** (major isomer), LCMS (FA): *m*/*z* = 832.1 (M+H) and **Intermediate 28b** (minor isomer), LCMS (FA): *m*/*z* = 832.1 (M+H).

### Step 6: 2-Amino-9-[(5S,7R,8R,10S,12aS,14R,15aS)-14-(6-amino-9H-purin-9-yl)-2,2,10-trioxido-10-sulfanyldecahydro-5,8-methanofuro[3,2-1] [1,3,6,9,10,11,2] tetraoxathiazaphosphacyclotetradecin-7-yl]-1,9-dihydro-6H-purin-6-one - sodium or 2-amino-9-[(5S,7R,8R,10R,12aS,14R,15aS)-14-(6-amino-9H-purin-9-yl)-2,2,10-trioxido-10-sulfanyldecahydro-5,8-methanofuro[3,2-l][1,3,6,9,10,11,2] tetraoxathiazaphosphacyclotetradecin-7-yl]-1,9-dihydro-6H-purin-6-one - sodium (compound I-12a)

The title compound was prepared from **Intermediate 28a** (250 mg, 0.268 mmol) following the procedure described in Example 8, Step 6 to provide **I-12a** as the N,N-diethylethanamine salt. Bio-Rad BT AG 50W-X8 resin, 100-200 mesh hydrogen form (2.4 g) was slurried in water (13 mL) then loaded in a glass column (0.5" x 6"), excess water was drained by gravity. An aqueous solution of NaOH (1 N, 24 mL) was passed through the column via gravity. The column was then rinsed with water (48 mL) via gravity; pH paper was used to confirm the final portion of eluted water was neutral. The N,N-diethylethanamine salt of **I-12a** (120 mg, 0.183 mmol) was dissolved in water (1 mL) and loaded onto the column, and additional water (1 mL) was used to rinse the vial onto the column. The column was eluted with water (9 mL), and collected in two fractions, the first one ~9 mL and the second fraction ~ 2 mL. By UPLC/MS only the first fraction (~9 mL) contained product, so this fraction was lyophilized overnight to give **I-12a** sodium salt (111 mg, 61%). LCMS (AA): *m*/*z* = 658.0 (M+H). ¹H NMR (D₂O) δ 1.96 - 2.12 (m, 1H), 2.12 - 2.23 (m, 1H), 2.62 - 2.71 (m, 1H), 3.00 (dd, *J* = 4.0, 13.1 Hz, 1H), 3.60 (t, *J* = 10.8 Hz, 1H), 3.75 (s, 1H), 3.80 - 3.91 (m, 1H), 3.95 (dd, *J* = 4.8, 10.6 Hz, 1H), 4.18 - 4.32 (m, 1H), 4.39 (dd, *J* = 4.2, 11.4 Hz, 1H), 4.80 (s, 1H), 4.84 - 4.97 (m, 1H), 5.92 (s, 1H), 6.27 (d, *J* = 4.8 Hz, 1H), 7.52 (s, 1H), 8.03 (s, 1H), 8.41 (s, 1H). ³¹P NMR (DMSO-d₆) δ 51.58 (s, 1P).

### Example 30

The compound below was prepared as described in Example 8 starting with Step 6, substituting the starting material shown in the table for 2-methyl-N- { 6-oxo-9-[(2S,5S,7R,8R,12aR,14R,15aS)-2,10,10-trioxido-14-(9H-purin-9-yl)-2-sulfanyldecahydro-11H-5,8-methanocyclopenta[m][1,3,6,9,10,11,2]tetraoxathiazaphosphacyclotetradecin-7-yl]-6,9-dihydro-1H-purin-2-yl}propanamide - N,N-diethylethanamine or 2-methyl-N-{6-oxo-9-[(2R,5S,7R,8R,12aR,14R,15aS)-2,10,10-trioxido-14-(9H-purin-9-yl)-2-sulfanyldecahydro-11H-5,8-methanocyclopenta[m][1,3,6,9,10,11,2]tetraoxathiazaphosphacyclotetradecin-7-yl]-6,9-dihydro-1H-purin-2-yl}propanamide - N,N-diethylethanamine.

| I Number | Starting material | Product | Analytical data |
|---|---|---|---|
| I-12b | 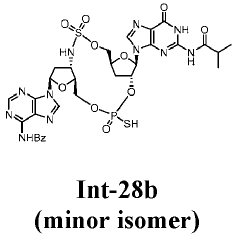 | 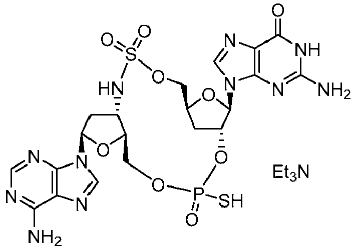 | LCMS (AA): *m*/*z* = 658.0 (M+H); ¹H NMR (400 MHz, D₂O) δ 1.13 (t, *J =* 7.3 Hz, 9H), 2.37 (ddd, *J* = 2.8, 6.9, 13.1 Hz, 1H), 2.53 - 2.73 (m, 2H), 2.87 - 2.98 (m, 1H), 3.05 (q, *J* = 7.3 Hz, 6H), 3.87 - 3.96 (m, 1H), 4.16 - 4.25 (m, 2H), 4.29 - 4.51 (m, 3H), 4.51 - 4.58 (m, 1H), 5.30 - 5.44 (m, 1H), 5.76 (d, *J* = 6.5 Hz, 1H), 6.32 (dd, *J* = 4.5, 6.2 Hz, 1H), 7.81 (s, 1H), 8.09 (s, 1H), 8.33 (s, 1H). ³¹P NMR (162 MHz, D₂O) δ 56.80 (s, 1P). |

### Example 31

The compound below was prepared as described in Example 29 starting with Step 1, substituting the starting material shown in the table for **Intermediate 24.**

| I Number | Starting material | Product | Analytical data |
|---|---|---|---|
| I-17* | 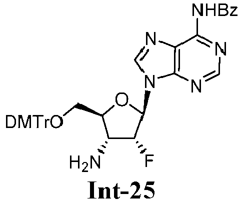 | 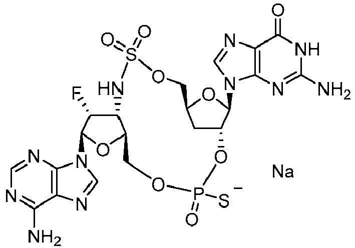 | LCMS (AA): *m*/*z* = 676.1 (M+H); ¹H NMR (400 MHz, DMSO-d₆) δ 1.86 - 2.00 (m, 1H), 2.90 (dd, *J* = 3.7, 13.2 Hz, 1H), 3.52 (s, 1H), 3.59 - 3.72 (m, 1H), 3.77 (dd, *J* = 2.3, 11.5 Hz, 1H), 3.90 (d, *J* = 10.5 Hz, 1H), 4.38 (s, 2H), 4.66 (s, 1H), 4.77 - 4.91 (m, 1H), 4.96 - 5.16 (m, 1H), 5.85 (s, 1H), 6.04 (d, *J* = 15.0 Hz, 1H), 7.41 (s, 1H), 7.70 (s, 1H), 8.17 (s, 1H); ³¹P NMR (162 MHz, DMSO-d₆) δ 51.33 (s, 1P); ¹⁹F NMR (376 MHz, DMSO-d₆) δ -198.0 - -198.20 (m, 1F). |

| | | | |
|---|---|---|---|
| * In step 5, only the major isomer was isolated upon cyclization. | | | |

### Example 32

### N-[7-[(2S,3S,4R,5R)-4-[tert-Butyl(dimethyl)silyl]oxy-3-fluoro-5-(hydroxymethyl) tetrahydrofuran-2-yl]-5-methyl-pyrrolo[3,2-d]pyrimidin-4-yl]benzamide (Intermediate 29)

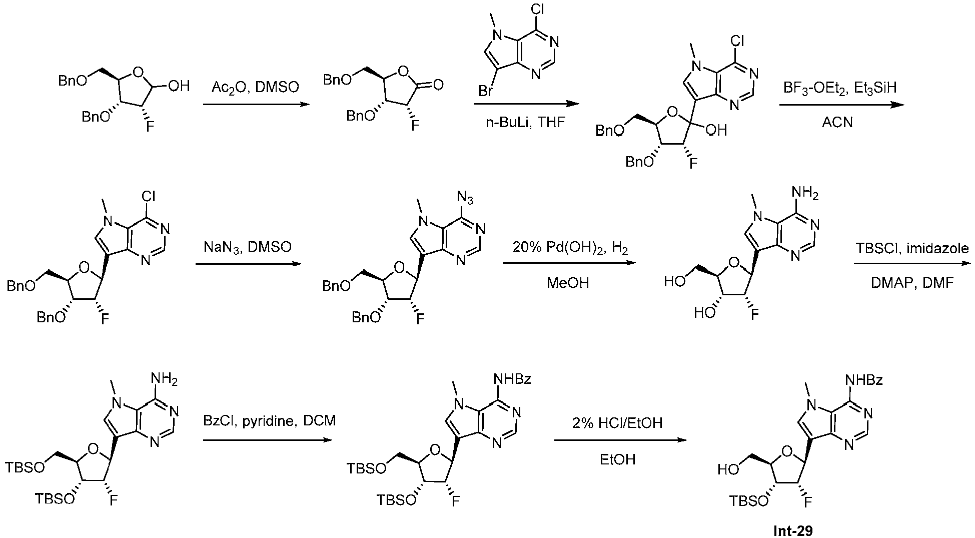

### Step 1: (3R,4R,5R)-4-Benzyloxy-5-(benzyloxymethyl)-3-fluoro-tetrahydrofuran-2-one

To a solution of (2*RS*,3*R*,4*R*,5*R*)-4-benzyloxy-5-(benzyloxymethyl)-3-fluoro-tetrahydrofuran-2-ol (120 g, 361 mmol, for synthesis see Woerpel, K.A. et al. J. Am. Chem. Soc. 2005, 127, 10879-10884) in DMSO (1.33 L) was added Ac₂O (682 mL, 7.22 mol) at room temperature. The reaction mixture was stirred at room temperature under air overnight. The reaction mixture was poured into water and extracted with EtOAc. The organic phase was separated, washed with water, a saturated aqueous solution of NaHCO₃ and brine, dried (Na₂SO₄), filtered and evaporated. The residue was purified by silica gel chromatography (5-25% EtOAc/pentane) to give a crude oil (contained residual AcOH). The oil was diluted with EtOAc and washed with a saturated aqueous solution of NaHCO₃ (2x), water, and brine, dried (Na₂SO₄), filtered and evaporated to provide (3*R*,4*R*,5*R*)-4-benzyloxy-5-(benzyloxymethyl)-3-fluoro-tetrahydrofuran-2-one (100 g, 84%). ¹H NMR (CDCl₃) δ 7.39 - 7.31 (m, 8H), 7.26 - 7.23 (m, 2H), 5.47 (dd, *J* = 49.3, 5.3 Hz, 1H), 4.84 (d, *J* = 11.8 Hz, 1H), 4.61 (s, 1H), 4.60 (d, *J* = 11.8 Hz, 1H), 4.55 - 4.46 (m, 2H), 4.30 (d, *J* = 5.3 Hz, 1H), 3.72 - 3.61 (m, 2H).

### Step 2: (2SR,3R,4R,5R)-4-Benzyloxy-5-(benzyloxymethyl)-2-(4-chloro-5-methyl-pyrrolo [3,2-d] pyrimidin-7-yl)-3-fluoro-tetrahydrofuran-2-ol

To a solution of 7-bromo-4-chloro-5-methyl-5H-pyrrolo[3,2-d]pyrimidine (22.3 g, 90.8 mmol) in anhydrous THF (220 mL) cooled to -78 °C was added n-butyllithium (2.5 M in hexaness, 36.3 mL, 90.8 mmol) dropwise. The reaction mixture was stirred at -78 °C under argon for 30 min. To this mixture was added a solution of (3*R*,4*R*,5*R*)-4-benzyloxy-5-(benzyloxymethyl)-3-fluoro-tetrahydrofuran-2-one (30 g, 90.8 mmol) in anhydrous THF (300 mL) at -78 °C. The reaction mixture was stirred at -78 °C under argon for 30 min. The reaction mixture was warmed to 0 °C, quenched with a saturated aqueous solution of NH₄Cl and extracted with EtOAc. The organic phase was separated, washed with brine, dried (Na₂SO₄), filtered and concentrated in vacuo. The crude compound was purified by silica gel chromatography (20-50% EtOAc/pentane) to provide (2*SR*,3*R*,4*R*,5*R*)-4-benzyloxy-5-(benzyloxymethyl)-2-(4-chloro-5-methyl-pyrrolo[3,2-d]pyrimidin-7-yl)-3-fluoro-tetrahydrofuran-2-ol (5 g, 11%).

### Step 3: 7-[(2S,3S,4R,5R)-4-Benzyloxy-5-(benzyloxymethyl)-3-fluoro-tetrahydrofuran-2-yl]-4-chloro-5-methyl-pyrrolo [3,2-d] pyrimidine

To an ice-bath cooled solution of (2*SR*,3*R*,4*R*,5*R*)-4-benzyloxy-5-(benzyloxymethyl)-2-(4-chloro-5-methyl-pyrrolo[3,2-d]pyrimidin-7-yl)-3-fluoro-tetrahydrofuran-2-ol (10 g, 20 mmol) in CH₃CN (100 mL) was added triethylsilane (19 mL, 120 mmol) and BF₃·Et₂O (15.1 mL, 120 mmol). The reaction mixture was stirred at 0 °C under Ar for 16 h. The cooled reaction mixture was treated with a saturated aqueous solution of NaHCO₃ and extracted with EtOAc. The organic phase was separated, washed with brine, dried (Na₂SO₄), filtered and evaporated. The crude residue was purified by silica gel chromatography (30-50% EtOAc/pentane) to provide 7-[(2*S*,3*S*,4*R*,5*R*)-4-benzyloxy-5-(benzyloxymethyl)-3-fluoro-tetrahydrofuran-2-yl]-4-chloro-5-methyl-pyrrolo[3,2-d]pyrimidine (7 g, 73%). LCMS (AA): *m*/*z* = 482.1 (M+H). ¹H NMR (CDCl₃) δ 8.55 (s, 1H), 7.42 (s, 1H), 7.29 - 7.17 (m, 10H), 5.52 (d, *J* = 23.5 Hz, 1H), 5.33 - 5.17 (m, 1H), 4.72 - 4.67 (m, 1H), 4.53 - 4.40 (m, 3H), 4.35 - 4.21 (m, 2H), 3.91 - 3.86 (m, 1H), 3.66 (s, 3H), 3.68 - 3.62 (m, 1H).

### Step 4: 4-Azido-7-[(2S,3S,4R,5R)-4-benzyloxy-5-(benzyloxymethyl)-3-fluoro-tetrahydrofuran-2-yl]-5-methyl-pyrrolo[3,2-d]pyrimidine

To a solution of 7-[(2*S*,3*S*,4*R*,5*R*)-4-benzyloxy-5-(benzyloxymethyl)-3-fluoro-tetrahydrofuran-2-yl]-4-chloro-5-methyl-pyrrolo[3,2-d]pyrimidine (14.5 g, 30 mmol) in DMSO (145 mL) was added sodium azide (3.9 g, 60 mmol). The mixture was warmed to 70 °C under Ar and stirred for 2 h. TLC analysis showed the reaction was incomplete. Additional sodium azide (0. 98 g, 15 mmol) was added and heating at 70 °C continued for additional 2 h. The reaction mixture was cooled to room temperature, treated with water and extracted with EtOAc (3x). The combined organic phases were washed with water and brine, dried (Na₂SO₄), filtered and concentrated in vacuo. The crude residue was purified by silica gel chromatography (50% EtOAc/pentane) to provide 4-azido-7-[(2*S*,3*S*,4*R*,5*R*)-4-benzyloxy-5-(benzyloxymethyl)-3-fluoro-tetrahydrofuran-2-yl]-5-methyl-pyrrolo[3,2-d]pyrimidine (14.8 g, 100%). LCMS (AA): *m*/*z* = 489.2 (M+H).

### Step 5: (2R,3R,4R,5S)-5-(4-Amino-5-methyl-pyrrolo[3,2-d]pyrimidin-7-yl)-4-fluoro-2-(hydroxymethyl)tetrahydrofuran-3-ol

A mixture of 4-azido-7-[(2*S*,3*S*,4*R*,4*R*)-4-benzyloxy-5-(benzyloxymethyl)-3-fluoro-tetrahydrofuran-2-yl]-5-methyl-pyrrolo[3,2-d]pyrimidine (14.8 g, 30.2 mmol) and Pd(OH)₂ (20%, 4.0 g, 5.73 mmol) in MeOH (604 mL) was shaken under 50 psi of H₂ gas at 65 °C for 48 h. TLC analysis showed the reaction was incomplete. The reaction mixture was filtered through Celite and to the filtrate was added Pd(OH)₂ (20%, 6.0 g, 8.60 mmol). The reaction mixture was then shaken under 50 psi of H₂ gas at 65 °C for another 48 h. The catalyst was removed by filtration through Celite and the filtrate was evaporated to dryness to provide (2*R*,3*R*,4*R*,5*S*)-5-(4-amino-5-methyl-pyrrolo[3,2-d]pyrimidin-7-yl)-4-fluoro-2-(hydroxymethyl)tetrahydrofuran-3-ol (6.9 g, 81%) which required no further purification. LCMS (AA): *m*/*z* = 283.0 (M+H).

### Step 6: 7-[(2S,3S,4R,5R)-4-[tert-Butyl(dimethyl)silyl]oxy-5-[[tert-butyl(dimethyl) silyl]oxymethyl]-3-fluoro-tetrahydrofuran-2-yl]-5-methyl-pyrrolo[3,2-d]pyrimidin-4-amine

The title compound was prepared starting from (2*R*,3*R*,4*R,*5*S*)-5-(4-amino-5-methyl-pyrrolo[3,2-d]pyrimidin-7-yl)-4-fluoro-2-(hydroxymethyl)tetrahydrofuran-3-ol (6.4 g, 22.6 mmol) following the procedure described in Example 15, Step 1 to provide 7-[(2*S*,3*S*,4*R*,5*R*)-4-[tert-butyl(dimethyl)silyl]oxy-5-[[tert-butyl(dimethyl)silyl]oxymethyl]-3-fluoro-tetrahydrofuran-2-yl]-5-methyl-pyrrolo[3,2-d]pyrimidin-4-amine (10.8 g, 94%). ¹H NMR (CDCl₃) δ 8.33 (s, 1H), 7.22 (s, 1H), 5.47 - 5.40 (m, 1H), 5.27 - 5.11 (m, 1H), 5.16 (s, 2H), 4.55 - 4.47 (m, 1H), 4.04 - 3.95 (m, 2H), 3.99 (s, 3H), 3.78 - 3.74 (m, 1H), 0.92 (s, 9H), 0.90 (s, 9H), 0.12 (s, 3H), 0.10 (s, 3H), 0.08 (s, 3H), 0.07 (s, 3H).

### Step 7: N-[7-[(2S,3S,4R,5R)-4-[tert-Butyl(dimethyl)silyl]oxy-5-[[tert-butyl(dimethyl) silyl]oxymethyl]-3-fluoro-tetrahydrofuran-2-yl]-5-methyl-pyrrolo[3,2-d]pyrimidin-4-yl]benzamide

The title compound was prepared starting from 7-[(2*S*,3*S*,4*R*,5*R*)-4-[tert-butyl(dimethyl)silyl]oxy-5-[[tert-butyl(dimethyl)silyl]oxymethyl]-3-fluoro-tetrahydrofuran-2-yl]-5-methyl-pyrrolo[3,2-d]pyrimidin-4-amine (10.8 g, 21.1 mmol) following the procedure described in Example 16, using DCM as a co-solvent to provide N-[7-[(2*S*,3*S*,4*R*,5*R*)-4-[tert-butyl(dimethyl)silyl]oxy-5-[[tert-butyl(dimethyl)silyl]oxymethyl]-3-fluoro-tetrahydrofuran-2-yl]-5-methyl-pyrrolo[3,2-d]pyrimidin-4-yl]benzamide (11 g, 85%).

### Step 8: N-[7-[(2S,3S,4R,5R)-4-[tert-Butyl(dimethyl)silyl]oxy-3-fluoro-5-(hydroxymethyl)tetrahydrofuran-2-yl]-5-methyl-pyrrolo[3,2-d]pyrimidin-4-yl]benzamide, Intermediate 29

**Intermediate 29** was prepared starting from N-[7-[(2*S*,3*S*,4*R*,5*R*)-4-[tert-butyl(dimethyl)silyl]oxy-5-[[tert-butyl(dimethyl)silyl]oxymethyl]-3-fluoro-tetrahydrofuran-2-yl]-5-methyl-pyrrolo[3,2-d]pyrimidin-4-yl]benzamide (11 g, 17.8 mmol) following the procedure described in Example 6, Step 3 stirring at 0 °C for 16 h. The crude compound was purified by silica gel chromatography (50% EtOAc/pentane) to provide N-[7-[(2*S*,3*S*,4*R*,5*R*)-4-[tert-butyl(dimethyl)silyl]oxy-3-fluoro-5-(hydroxymethyl)tetrahydrofuran-2-yl]-5-methyl-pyrrolo[3,2-d]pyrimidin-4-yl]benzamide (8 g, 90%). ¹H NMR (CDCl₃) δ 8.30 - 8.24 (m, 2H), 8.08 (br s, 1H), 7.57 - 7.46 (m, 3H), 7.38 (s, 1H), 5.65 (br s, 1H), 5.35 - 5.19 (m, 2H), 4.59 - 4.56 (m, 1H), 4.37 (br s, 3H), 4.18 - 4.15 (m, 1H), 3.97 - 3.92 (m, 1H), 3.73 - 3.66 (m, 1H), 0.95 (s, 9H), 0.15 (s, 6H).

### Example 33

### 7-[(2R,3R,5S)-5-[[bis(4-Methoxyphenyl)-phenyl-methoxy]methyl]-3-hydroxy-tetrahydrofuran-2-yl]-5-fluoro-3H-pyrrolo[2,3-d]pyrimidin-4-one (Intermediate 30)

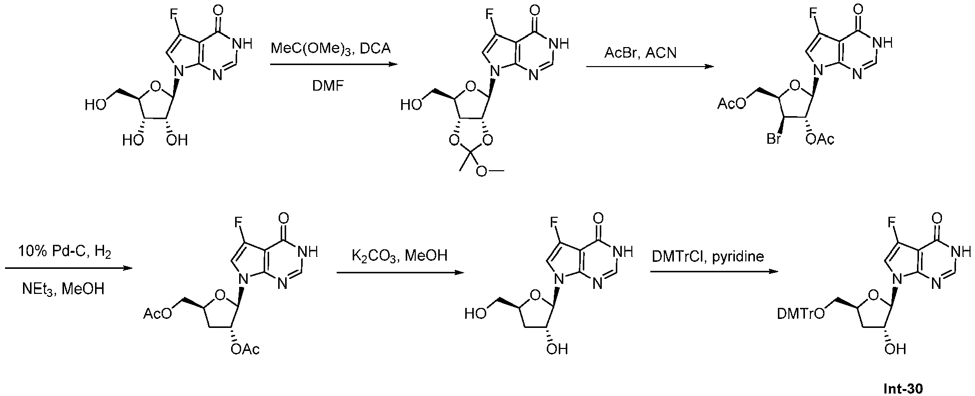

### Step 1: 7-[(3aR,4R,6R,6aR)-6-(Hydroxymethyl)-2-methoxy-2-methyl-3a,4,6,6a-tetrahydrofuro [3,4-d] [1,3] dioxol-4-yl]-5-fluoro-3H-pyrrolo [2,3-d] pyrimidin-4-one

Trimethyl orthoacetate (16.8 g, 140 mmol) and dichloroacetic acid (179 mg, 1.4 mmol) were added to a suspension of 7-[(2*R*,3*R*,4*S*,5*R*)-3,4-dihydroxy-5-(hydroxymethyl)tetrahydrofuran-2-yl]-5-fluoro-3H-pyrrolo[2,3-d]pyrimidin-4-one (8.0 g, 28 mmol, for synthesis see Seela, F. et al. Tetrahedron 2007, 63, 9850-9861) in DMF (40 mL) and the reaction mixture was heated at 60 °C for 1 h. The reaction mixture was allowed to cool to rt and DIPEA (0.2 mL, 1.15 mmol) was added. The reaction mixture was concentrated to dryness to provide crude 7-[(3a*R*,4*R*,6*R*,6a*R*)-6-(hydroxymethyl)-2-methoxy-2-methyl-3a,4,6,6a-tetrahydrofuro[3,4-d][1,3]dioxol-4-yl]-5-fluoro-3H-pyrrolo[2,3-d]pyrimidin-4-one (9.5 g, 100%).

### Step 2: [(2R,3S,4S,5R)-4-Acetoxy-3-bromo-5-(5-fluoro-4-oxo-3H-pyrrolo[2,3-d]pyrimidin-7-yl)tetrahydrofuran-2-yl]methyl acetate

A mixture of acetyl bromide (10.1 mL, 139 mmol) in ACN (10 mL) was added over 2 h to a suspension of 7-[(3a*R*,4*R*,6*R*,6a*R*)-6-(hydroxymethyl)-2-methoxy-2-methyl-3a,4,6,6a-tetrahydrofuro[3,4-d][1,3]dioxol-4-yl]-5-fluoro-3H-pyrrolo[2,3-d]pyrimidin-4-one (9.5 g, 27.8 mmol) in ACN (50 mL) cooled to 0 °C. The reaction mixture was stirred at 10 °C for 8 h and then treated with a saturated aqueous solution of NaHCO₃ until the solution reached pH of 8. The reaction mixture was extracted with EtOAc (2x) and the combined organic phases were concentrated to dryness. The crude residue was purified by silica gel chromatography (15% MeOH/DCM) to provide [(2*R*,3*S*,4*S*,5*R*)-4-acetoxy-3-bromo-5-(5-fluoro-4-oxo-3H-pyrrolo[2,3-d]pyrimidin-7-yl)tetrahydrofuran-2-yl]methyl acetate (2.0 g, 16.6%). LCMS (AA): *m*/*z* = 433.8 (M+H).

### Step 3: [(2S,4R,5R)-4-Acetoxy-5-(5-fluoro-4-oxo-3H-pyrrolo[2,3-d]pyrimidin-7-yl)tetrahydrofuran-2-yl]methyl acetate

[(2*R*,3*S*,4*S*,5*R*)-4-acetoxy-3-bromo-5-(5-fluoro-4-oxo-3H-pyrrolo[2,3-d]pyrimidin-7-yl)tetrahydrofuran-2-yl]methyl acetate (1.6 g, 3.7 mmol) was dissolved in methanol (100 mL) and triethylamine (1.53 mL, 11.1 mmol). Palladium (10% on carbon, 398 mg, 0.37 mmol) was added and the mixture was stirred under an atmosphere of hydrogen for 1 h. The reaction mixture was filtered, washed with methanol and the filtrate was evaporated to dryness to afford [(2*S*,4*R*,5*R*)-4-acetoxy-5-(5-fluoro-4-oxo-3H-pyrrolo[2,3-d]pyrimidin-7-yl)tetrahydrofuran-2-yl]methyl acetate (1.3 g, 100%). LCMS (AA): *m*/*z* = 354.1 (M+H).

### Step 4: 5-Fluoro-7-[(2R,3R,5S)-3-hydroxy-5-(hydroxymethyl)tetrahydrofuran-2-yl]-3H-pyrrolo[2,3-d]pyrimidin-4-one

To a solution of [(2*S*,4*R*,5*R*)-4-acetoxy-5-(5-fluoro-4-oxo-3H-pyrrolo[2,3-d]pyrimidin-7-yl)tetrahydrofuran-2-yl]methyl acetate (1.3 g, 3.67 mmol) in methanol (30 mL) cooled to 15 °C was added potassium carbonate (1.51 g, 11 mmol) and the reaction mixture was stirred at 15 °C for 16 h. The reaction mixture was then filtered through Celite, the filtrate was acidified to pH 7 by the addition of HCl (4 M solution in dioxane) and the filtrate was concentrated to dryness. The crude residue was purified by silica gel chromatography (10% MeOH/DCM) to provide 5-fluoro-7-[(2*R*,3*R*,5*S*)-3-hydroxy-5-(hydroxymethyl)tetrahydrofuran-2-yl]-3H-pyrrolo[2,3-d]pyrimidin-4-one (700 mg, 71%). LCMS (AA): *m*/*z* = 270.0 (M+H).

### Step 5: 7-[(2R,3R,5S)-5-[[bis(4-Methoxyphenyl)-phenyl-methoxy]methyl]-3-hydroxy-tetrahydrofuran-2-yl]-5-fluoro-3H-pyrrolo[2,3-d]pyrimidin-4-one, Intermediate 30

**Intermediate 30** was prepared starting from 5-fluoro-7-[(2*R*,3*R*,5*S*)-3-hydroxy-5-(hydroxymethyl)tetrahydrofuran-2-yl]-3H-pyrrolo[2,3-d]pyrimidin-4-one (200 mg, 0.74 mmol) following the procedure described in Example 11, Step 2 stirring at 15 °C for 2 d to provide 7-[(2*R*,3*R*,5*S*)-5-[[bis(4-methoxyphenyl)-phenyl-methoxy]methyl]-3-hydroxy-tetrahydrofuran-2-yl]-5-fluoro-3H-pyrrolo[2,3-d]pyrimidin-4-one (350 mg, 83%). LCMS (AA): *m*/*z* = 572.2 (M+H). ¹H NMR (DMSO-d₆) δ 12.15 (br s, 1H), 7.95 (s, 1H), 7.37 - 7.33 (m, 2H), 7.28 - 7.14 (m, 8H), 6.85 - 6.81 (m, 4H), 6.09 - 6.08 (m, 1H), 5.66 (d, *J* = 4.3 Hz, 1H), 4.45 - 4.39 (m, 2H), 3.72 (s, 6H), 3.16 - 3.12 (m, 2H), 2.26 - 2.18 (m, 1H), 1.93 - 1.87 (m, 1H).

### Example 34

### BIOLOGICAL PROTOCOLS AND STING BINDING ASSAY DATA

### Cloning, expression, and purification of STING

Human STING (accession number GB:BC047779) DNA encoding residues 140-379 (H232R) was cloned into pET28 as N-terminal HIS and C-terminal Avi tagged fusion protein, and a rTEV cleavage site between HIS tag and STING. The sequence was verified before recombinant protein expression using E.coli expression system.

For expression, STING expression vector was transformed into BL21(DE3) cells. The expression was induced by adding IPTG and expressing in 16 °C for 20 hours.

For purification, STING was purified by Nickel affinity resin (Thermo Scientific #25215), followed by TEV enzyme cleavage and Superdex 200 column (GE Healthcare #28-9893-35) purification. For site directed in vitro biotinylation, purified Avi-tagged STING protein was incubated with Biotin (SUPELCO #47868), ATP (SIGMA# A-7699) and BirA enzyme, followed by further purification through a Superdex 200 column (GE Healthcare #28-9893-35). Biotinylation was confirmed by Mass Spectrometry analysis.

### Human STING TR-FRET assay

40 nL of test compounds in DMSO were added to wells in a white, 384 well microtitre plate (Greiner 784075). 2 uL STING assay buffer (PBS and 0.01% BSA) containing fluorescein labeled ligand (c[G(2',5')p-2'-Fluo-AHC-A(3',5')p]- Biolog C 195, 100 nM final) and Tb labeled Streptavidin (Streptavidin-Tb cryptate- CisBio 610SATLB) were added. Then 2 uL STING assay buffer containing STING protein (100 nM final) was added and the mixture was incubated at rt for 60 minutes. The plates were then read on a BMG PheraStar Plus reader (LanthaScreen module).

For the assay method described above, test compound percent inhibition, at various concentrations, was calculated relative to untreated and DMSO only treated samples. Compound concentration versus percent inhibition curves were fitted to generate IC₅₀ values. One skilled in the art will appreciate that the values generated either as percentage inhibition at a single concentration or IC₅₀ values are subject to experimental variation.

### THP-1 Dual Lucia Reporter Gene Assay Conditions

THP1-Dual^{™} cells (InvivoGen #thpd-nfis) were derived from the human THP-1 monocyte cell line by stable integration of the Lucia luciferase gene, a secreted luciferase reporter gene, under the control of an ISG54 (interferon-stimulated gene) minimal promoter in conjunction with five interferon (IFN)-stimulated response elements. On the day of experiment, the cells were plated to a black, 384-well plate (Corning 356697) at 7500 cells/25 µl per well density in growth media (RPMI 1640, 2 mM L-glutamine, 25 mM HEPES,10% heat-inactivated fetal bovine serum, 100 µg/mL Normocin^{™}, 50 U/mL-50 µg/mL Pen-Strep, 10 µg/mL of blasticidin, and 100 µg/mL of Zeocin). The cell plates were dosed with 62.5 nL of the testing compounds, and then incubated at 37°C for 20 hours. At the end of the incubation, 15 µL/well of the QUANTI-Luc^{™} (InvivoGen # rep-qlc1) were added, and luminescence was measured immediately using the LeadSeeker.

For the assay method described above, test compound percent luminescence signal induction, at various concentrations, was calculated relative to untreated and DMSO only treated samples. Compound concentration versus percent signal induction curves were fitted to generate EC₅₀ values. One skilled in the art will appreciate that the values generated as EC₅₀ values are subject to experimental variation.

As detailed above, compounds of this disclosure modulate STING. In certain embodiments, compounds of this disclosure are STING agonists with the IC₅₀ and EC₅₀ values shown in the table below.

| Compound | TR-FRET IC₅₀ (µM) | THP1 EC₅₀ (µM) |
|---|---|---|
| I-1 | D | D |
| I-2a | D | D |
| I-2b | C | C |
| I-3 | A | A |
| I-4a | A | A |
| I-4b | A | A |
| I-5 | B | A |
| I-6 | D | D |
| I-7 | C | D |
| I-8 | B | B |
| I-9 | C | D |
| I-10 | A | A |
| I-11a | A | A |
| I-11b | A | A |
| I-12a | A | A |
| I-12b | B | D |
| I-13a | A | A |
| I-13b | A | A |
| I-14 | A | A |
| I-15 | A | A |
| I-16 | A | A |
| I-17 | A | A |
| I-18 | A | B |
| I-19 | B | D |
| I-20 | A | B |

| | | |
|---|---|---|
| TR-FRET IC₅₀: A) less than 1.0 µM; B) 1.0 µM - 10 µM; C) 10 µM - 100 µM; D) greater than 100 µM. THP1 EC₅₀: A) less than 1.0 µM; B) 1.0 µM - 10 µM; C) 10 µM - 25 µM; D) greater than 25 µM. | | |

While certain embodiments have been illustrated and described, it should be understood that changes and modifications can be made therein in accordance with ordinary skill in the art without departing from the technology in its broader aspects as defined in the following claims.

The present disclosure is not to be limited in terms of the particular embodiments described in this application. Modifications and variations can be made without departing from its spirit and scope, as will be apparent to those skilled in the art. Functionally equivalent methods and compositions within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, reagents, compounds compositions or biological systems, which can of course vary. It is also to be understood that the terminology used herein is for the purpose of describing particular embodiments only, and is not intended to be limiting.

All publications, patent applications, issued patents, and other documents referred to in this specification are herein incorporated by reference as if each individual publication, patent application, issued patent, or other document was specifically and individually indicated to be incorporated by reference in its entirety. Definitions that are contained in text incorporated by reference are excluded to the extent that they contradict definitions in this disclosure.

Aspects and features of the present disclosure are set out in the following numbered clauses which contain the subject matter of the claims of the PCT application as filed:
1. A compound of Formula I: 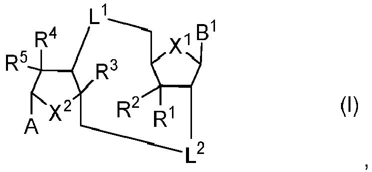 or a pharmaceutically acceptable salt thereof, wherein:
   A is B² or -Y-B³, wherein Y is -O-, -S-, -NH-, or -NR^{a}-;
   X¹ and X² are independently -O-, -S-, or -CH₂-;
   at least one of L¹ and L² is -OS(O₂)NH-, or -NHS(O₂)-O-; and the other is -O-P(O)(R^{c})-O-, -OS(O₂)NH-, or -NHS(O₂)-O-;
   R¹ is hydrogen, fluoro, -OH, -NH₂, -OR^{b}, or-NHR^{b};
   R² is hydrogen or fluoro;
   R³ is hydrogen or fluoro; R⁴ is hydrogen, fluoro, -OH, -NH₂, -OR^{b}, or -NHR^{b}; or R³ and
   R⁴ are taken together to form -CH₂O-;
   R⁵ is hydrogen or fluoro;
   R^{a} is C₁-C₄alkyl;
   R^{b} is C₁-C₆alkyl, halo(C₁-C₆)alkyl, or C₃-C₆cycloalkyl;
   R^{c} is -OH or -SH;
   B¹ and B² are independently an optionally substituted 9- or 10-membered bicyclic heteroaryl ring containing 2-5 heteroatoms selected from N, O, or S; wherein B¹ and B² comprise at least two N atoms in the ring; and
   B³ is an optionally substituted 5- or 6-membered monocyclic heteroaryl ring containing 1-4 heteroatoms selected from N, O, or S, or an optionally substituted 9- or 10-membered bicyclic heteroaryl ring containing 1-5 heteroatoms selected from N, O, or S; wherein B³ comprises at least one N atom in the ring, and wherein Y is attached to a carbon atom of B³.
2. The compound of clause 1, having Formula II: 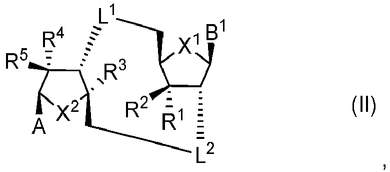 or a pharmaceutically acceptable salt thereof.
3. The compound of clause 1 or 2 or a pharmaceutically acceptable salt thereof,
   wherein:
   L¹ is -O-P(O)(SH)-O- or -O-P(O)(OH)-O-; and
   L² is -OS(O₂)NH- or -NHS(O₂)-O-.
4. The compound of clause 1 or 2 or a pharmaceutically acceptable salt thereof,
   wherein:
   L¹ is -OS(O₂)NH- or -NHS(O₂)-O-; and
   L² is -O-P(O)(SH)-O- or -O-P(O)(OH)-O-.
5. The compound of any one of clauses 1 to 4 or a pharmaceutically acceptable salt thereof, wherein X¹ and X² are independently -O- or -CH₂-.
6. The compound of any one of clauses 1 to 5 or a pharmaceutically acceptable salt thereof, wherein R¹ is hydrogen, fluoro, -OH, or -OR^{b}, and R² is hydrogen or fluoro.
7. The compound of any one of clauses 1 to 6 or a pharmaceutically acceptable salt thereof, wherein:
   R³ is hydrogen or fluoro;
   R⁴ is hydrogen, fluoro, or -OH; and
   R⁵ is hydrogen or fluoro.
8. The compound of any one of clauses 1 to 7 or a pharmaceutically acceptable salt thereof, wherein:
   R³ is hydrogen;
   R⁴ is fluoro; and
   R⁵ is hydrogen.
9. The compound of any one of clauses 1 to 6, having Formula III: 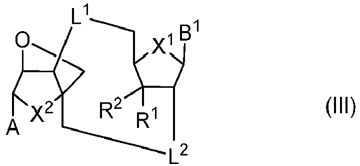 or a pharmaceutically acceptable salt thereof.
10. The compound of clause 1, having Formula (IV-A), (IV-B), (IV-C), (IV-D), (IV-E), or (IV-F): 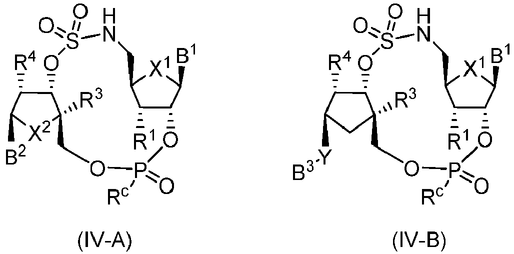 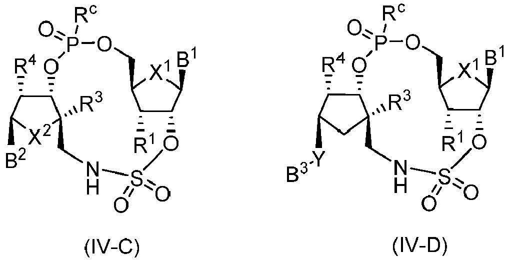 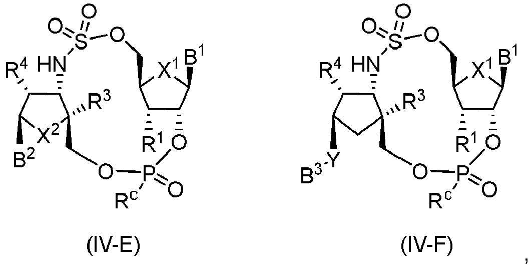 or a pharmaceutically acceptable salt thereof, wherein:
   R¹ and R⁴ are each independently hydrogen, fluoro, -OH, or -OR^{b};
   R³ is hydrogen or fluoro;
   X¹ and X² are independently -O- or -CH₂-; and
   Yis -O- or-NH-.
11. The compound of clause 10, having Formula (IV-C) or (IV-D): 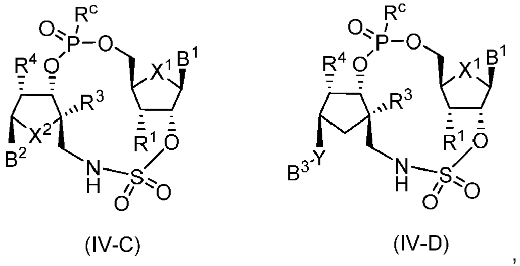 or a pharmaceutically acceptable salt thereof.
12. The compound of clause 1, having Formula (VII-A), (VII-B), (VII-D), or (VII-D): 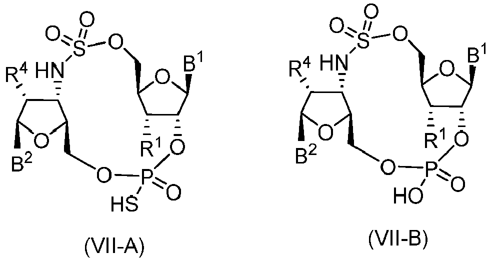 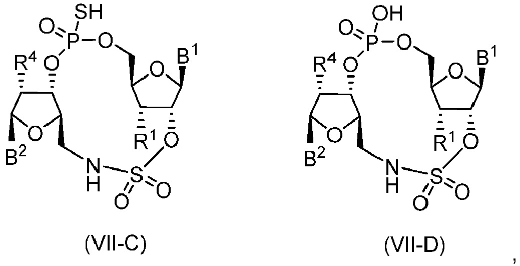 or a pharmaceutically acceptable salt thereof, wherein:
   R¹ is hydrogen, fluoro, or -OR^{b}; and
   R⁴ is hydrogen or fluoro.
13. The compound of clause 12, having Formula (VII-C) or (VII-D): 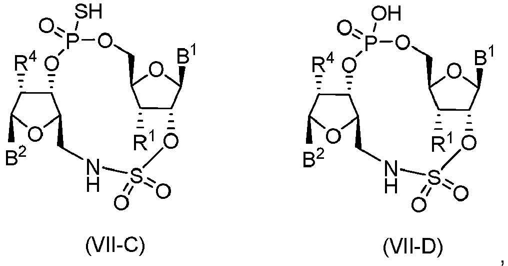 or a pharmaceutically acceptable salt thereof, wherein:
   R¹ is hydrogen, fluoro, -OCH₂CF₃; and
   R⁴ is fluoro.
14. The compound of any one of clauses 1 to 11 or a pharmaceutically acceptable salt thereof, wherein B³ is an optionally substituted 6-membered monocyclic heteroaryl ring containing 1-3 N atoms.
15. The compound of any one of clauses 1 to 9 or a pharmaceutically acceptable salt thereof, wherein A is B².
16. The compound of any one of clauses 1 to 15 or a pharmaceutically acceptable salt thereof, wherein B¹ and B² are independently an optionally substituted 9- membered bicyclic heteroaryl ring containing 3-5 N atoms.
17. The compound of clause 16 or a pharmaceutically acceptable salt thereof, wherein B¹ and B² are independently: 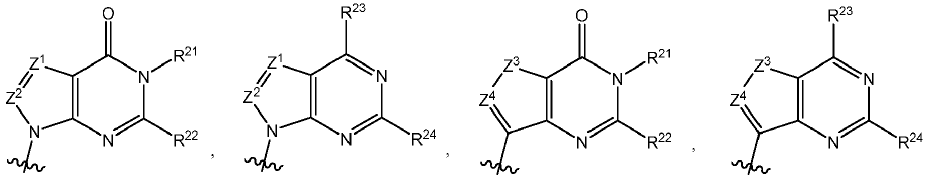 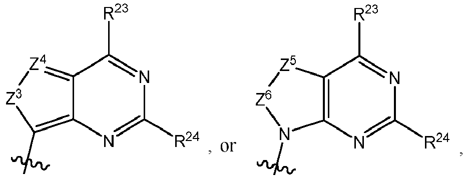
   Z¹, Z², and Z⁴ are each independently N or CR²⁰;
   Z³ is NR²¹ or CHR²⁰;
   Z⁵ and Z⁶ are each independently C(O) or NR²¹;
   R²¹ is hydrogen or C₁-C₆alkyl, halo(C₁-C₆)alkyl, or C₃-C₆cycloalkyl;
   R²³ is hydrogen or-NH₂; and
   R²⁰, R²², and R²⁴ are each independently hydrogen, halogen, -OH, -NH₂, -CN, C₁-C₆alkyl, halo(C₁-C₆)alkyl, or C₃-C₆cycloalkyl.
18. The compound of clause 17 or a pharmaceutically acceptable salt thereof, wherein:
   Z¹, Z², and Z⁴ are each independently N, CH, or CF;
   Z³ is NH or NCH₃;
   one of Z⁵ and Z⁶ is C(O) and the other is NH or NCH₃;
   R²¹ is hydrogen or -CH₃; and
   R²² and R²⁴ are each independently hydrogen, -NH₂, or -CH₃.
19. The compound of clause 17 or a pharmaceutically acceptable salt thereof, wherein B¹ and B² are independently selected from: 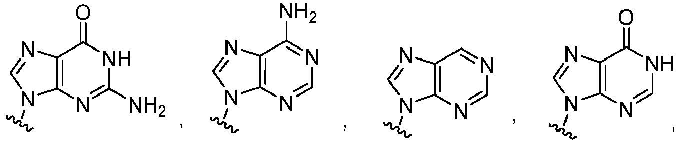 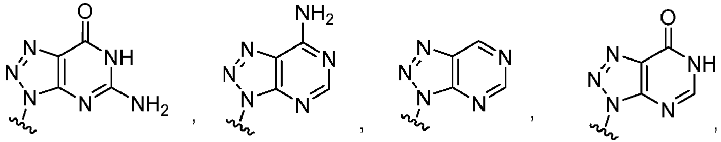 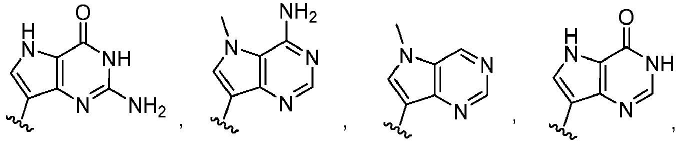 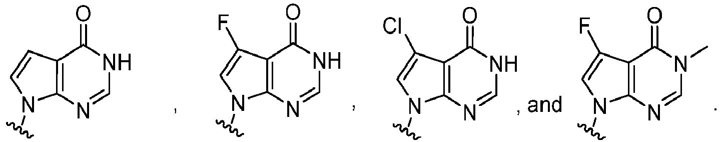
20. The compound of clause 19 or a pharmaceutically acceptable salt thereof, wherein B¹ is: 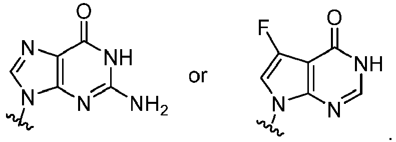
21. The compound of clause 20 or a pharmaceutically acceptable salt thereof, wherein B¹ is: 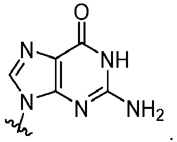
22. The compound of any one of clauses 19 to 21 or a pharmaceutically acceptable salt thereof, wherein B² is: 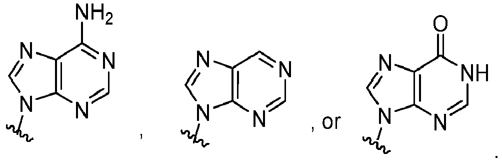
23. The compound of clause 22 or a pharmaceutically acceptable salt thereof, wherein B² is: 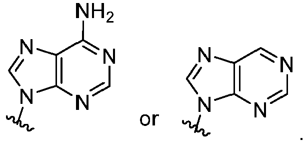
24. The compound of any one of clauses 1 to 23 or a pharmaceutically acceptable salt thereof, wherein when B¹, B², or B³ is substituted, the substituent is halogen, -OH, -CN, -NO₂, C₁-C₆alkyl, halo(C₁-C₆)alkyl, C₃-C₆cycloalkyl, -OR¹¹, -SR¹², -N(R¹²)₂, -NR¹²C(O)R¹², -C(O)R¹², -C(O)OR¹², -C(O)N(R¹²)₂, or-SO₂N(R¹²)₂; or two adjacent substituents, taken together with the intervening ring atoms, form a 4- to 8-membered ring;
   wherein R¹¹ is C₁-C₆alkyl, halo(C₁-C₆)alkyl, or C₃-C₆cycloalkyl; and
   R¹² is hydrogen, C₁-C₆alkyl, halo(C₁-C₆)alkyl, or C₃-C₆cycloalkyl.
25. The compound of clause 1 or a pharmaceutically acceptable salt thereof, wherein the compound is selected from Table 1.
26. The compound of clause 1 or a pharmaceutically acceptable salt thereof, wherein the compound is selected from: 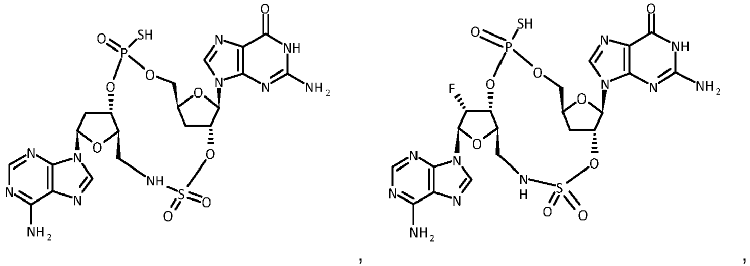 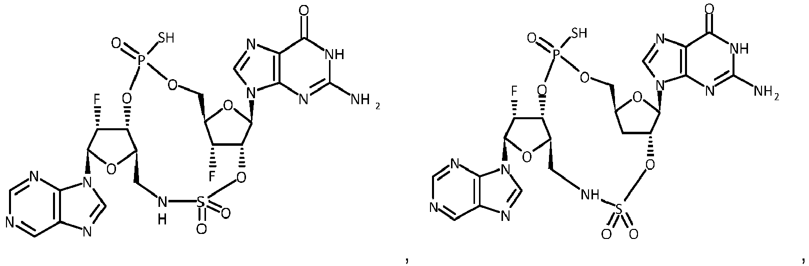 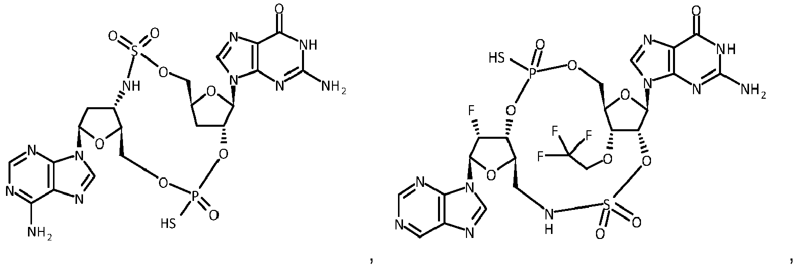 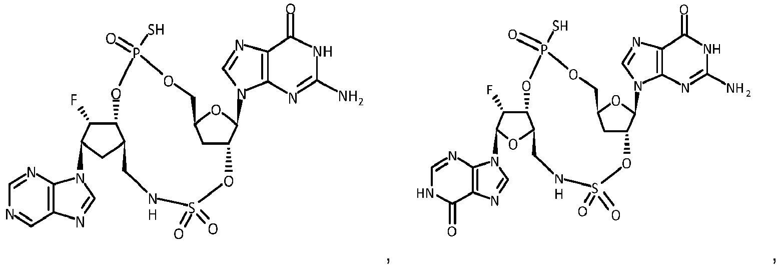 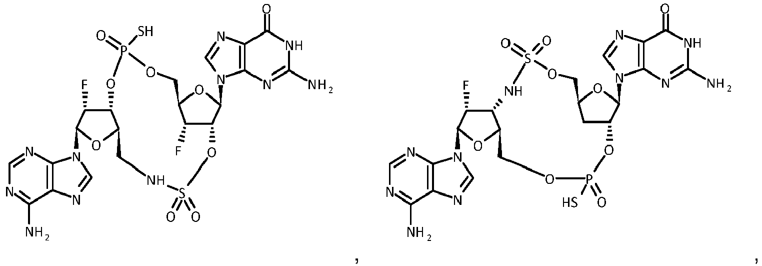 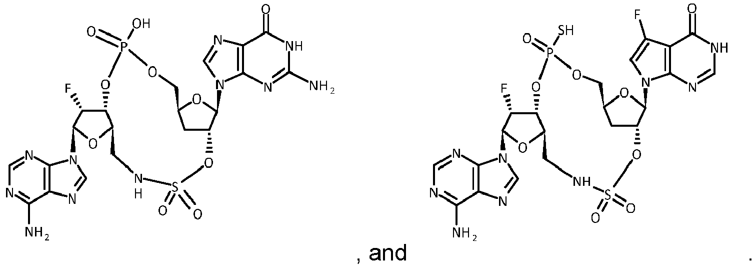
27. A pharmaceutical composition comprising (a) the compound of any one of clauses 1 to 26 or a pharmaceutically acceptable salt thereof, and (b) a pharmaceutically acceptable carrier.
28. A method of inducing an immune response in a subject, comprising administering to the subject in need thereof a therapeutically effective amount of the compound of any one of clauses 1 to 26 or a pharmaceutically acceptable salt thereof.
29. A method of inducing a STING-dependent type I interferon production in a subject, comprising administering to the subject in need thereof a therapeutically effective amount of the compound of any one of clauses 1 to 26 or a pharmaceutically acceptable salt thereof.
30. A method of treating a cell proliferation disorder in a subject, comprising administering to the subject in need thereof a therapeutically effective amount of the compound of any one of clauses 1 to 26 or a pharmaceutically acceptable salt thereof.
31. The method of clause 30, wherein the cell proliferation disorder is cancer.
32. The method of clause 30 or 31, further comprising administering to the subject a therapeutically effective amount of a second therapeutic agent.
33. A vaccine composition comprising an antigen and the compound of any one of clauses 1 to 26 or a pharmaceutically acceptable salt thereof.
34. A method of treating a disease in a subject, comprising administering to the subject in need thereof a therapeutically effective amount of the vaccine composition of clause 33.

## Claims

1. A compound of Formula I: 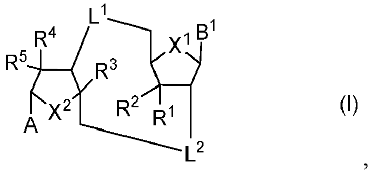 or a pharmaceutically acceptable salt thereof, wherein:
A is B² or -Y-B³, wherein Y is -O-, -S-, -NH-, or -NR^{a}-;
X¹ and X² are independently -O-, -S-, or -CH₂-;
at least one of L¹ and L² is -OS(O₂)NH-, or -NHS(O₂)-O-; and the other is -O-P(O)(R^{c})-O-, -OS(O₂)NH-, or -NHS(O₂)-O-;
R¹ is hydrogen, fluoro, -OH, -NH₂, -OR^{b}, or -NHR^{b};
R² is hydrogen or fluoro;
R³ is hydrogen or fluoro; R⁴ is hydrogen, fluoro, -OH, -NH₂, -OR^{b}, or -NHR^{b}; or R³ and R⁴ are taken together to form -CH₂O-;
R⁵ is hydrogen or fluoro;
R^{a} is C₁-C₄alkyl;
R^{b} is C₁-C₆alkyl, halo(C₁-C₆)alkyl, or C₃-C₆cycloalkyl;
R^{c} is -OH or -SH;
B¹ and B² are independently an optionally substituted 9- or 10-membered bicyclic heteroaryl ring containing 2-5 heteroatoms selected from N, O, or S; wherein B¹ and B² comprise at least two N atoms in the ring; and
B³ is an optionally substituted 5- or 6-membered monocyclic heteroaryl ring containing 1-4 heteroatoms selected from N, O, or S, or an optionally substituted 9- or 10-membered bicyclic heteroaryl ring containing 1-5 heteroatoms selected from N, O, or S; wherein B³ comprises at least one N atom in the ring, and wherein Y is attached to a carbon atom of B³.

2. The compound of claim 1, having Formula II: 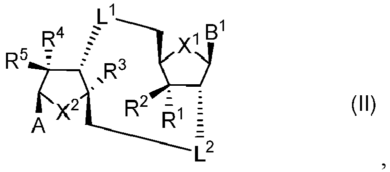 or a pharmaceutically acceptable salt thereof.

3. The compound of claim 1 or 2 or a pharmaceutically acceptable salt thereof, wherein:
a) L¹ is -O-P(O)(SH)-O- or -O-P(O)(OH)-O-; and L² is -OS(O₂)NH- or -NHS(O₂)-O-; or
b) L¹ is -OS(O₂)NH- or -NHS(O₂)-O-; and L² is -O-P(O)(SH)-O- or -O-P(O)(OH)-O-.

4. The compound of any one of claims 1 to 3 or a pharmaceutically acceptable salt thereof, wherein:
a) X¹ and X² are independently -O- or -CH₂-; and/or
b) R¹ is hydrogen, fluoro, -OH, or -OR^{b}, and R² is hydrogen or fluoro; and/or
c) R³ is hydrogen or fluoro; R⁴ is hydrogen, fluoro, or -OH; and R⁵ is hydrogen or fluoro; optionally wherein: R³ is hydrogen; R⁴ is fluoro; and R⁵ is hydrogen.

5. The compound of any one of claims 1 to 4, having Formula III: 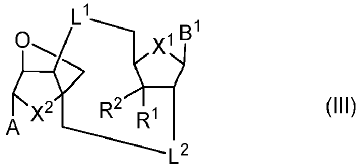 or a pharmaceutically acceptable salt thereof.

6. The compound of claim 1, having:
a) Formula (IV-A), (IV-B), (IV-C), (IV-D), (IV-E), or (IV-F): 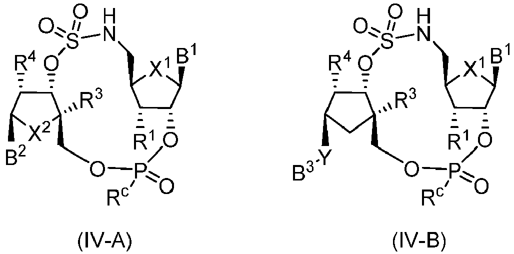 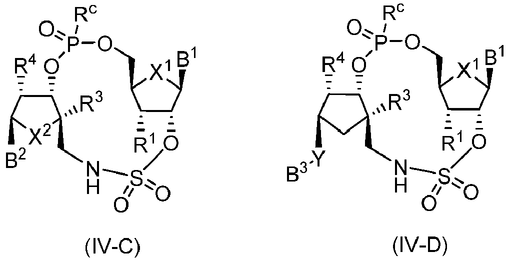 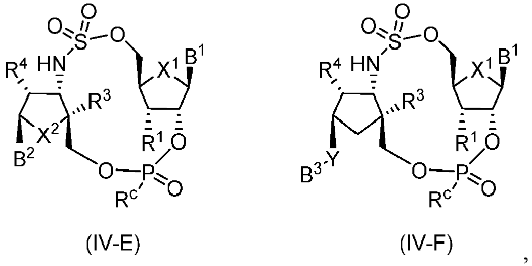 or a pharmaceutically acceptable salt thereof, wherein:
R¹ and R⁴ are each independently hydrogen, fluoro, -OH, or -OR^{b};
R³ is hydrogen or fluoro;
X¹ and X² are independently -O- or -CH₂-; and
Y is -O- or -NH-;
optionally having Formula (IV-C) or (IV-D): 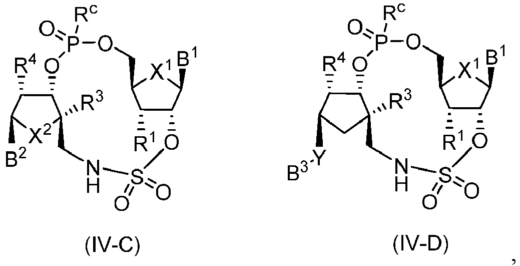
or a pharmaceutically acceptable salt thereof; or
b) Formula (VII-A), (VII-B), (VII-D), or (VII-D): 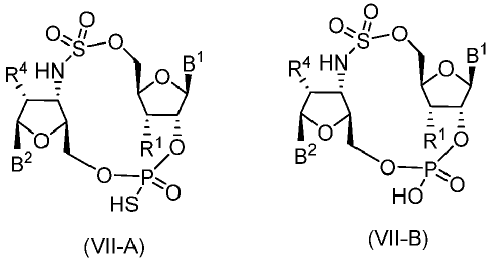 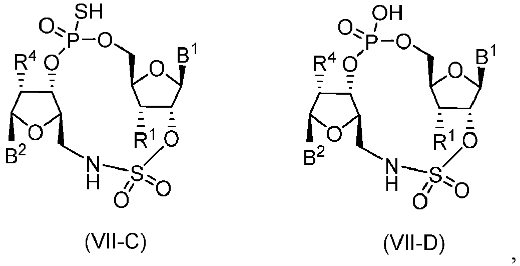 or a pharmaceutically acceptable salt thereof, wherein:
R¹ is hydrogen, fluoro, or -OR^{b}; and
R⁴ is hydrogen or fluoro;
optionally having Formula (VII-C) or (VII-D): 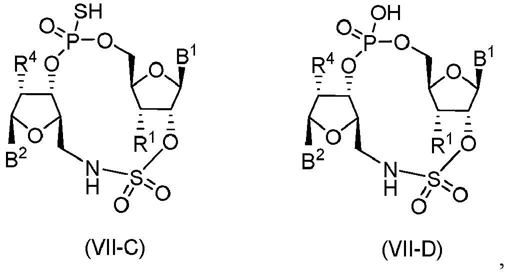
or a pharmaceutically acceptable salt thereof, wherein:
R¹ is hydrogen, fluoro, -OCH₂CF₃; and
R⁴ is fluoro.

7. The compound of any one of claims 1 to 6 or a pharmaceutically acceptable salt thereof, wherein:
a) B³ is an optionally substituted 6-membered monocyclic heteroaryl ring containing 1-3 N atoms; and/or
b) A is B²; and/or
c) B¹ and B² are independently an optionally substituted 9- membered bicyclic heteroaryl ring containing 3-5 N atoms.

8. The compound of claim 7 or a pharmaceutically acceptable salt thereof, wherein B¹ and B² are independently: 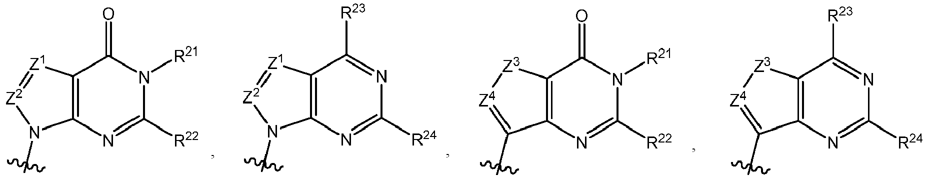 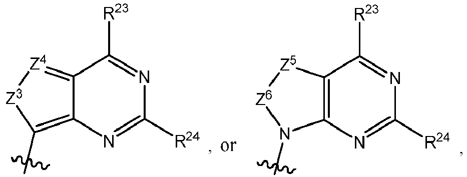
Z¹, Z², and Z⁴ are each independently N or CR²⁰;
Z³ is NR²¹ or CHR²⁰;
Z⁵ and Z⁶ are each independently C(O) or NR²¹;
R²¹ is hydrogen or C₁-C₆alkyl, halo(C₁-C₆)alkyl, or C₃-C₆cycloalkyl;
R²³ is hydrogen or -NH₂; and
R²⁰, R²², and R²⁴ are each independently hydrogen, halogen, -OH, -NH₂, -CN, C₁-C₆alkyl, halo(C₁-C₆)alkyl, or C₃-C₆cycloalkyl.

9. The compound of claim 8 or a pharmaceutically acceptable salt thereof, wherein:
a) Z¹, Z², and Z⁴ are each independently N, CH, or CF;
Z³ is NH or NCH₃;
one of Z⁵ and Z⁶ is C(O) and the other is NH or NCH₃;
R²¹ is hydrogen or -CH₃; and
R²² and R²⁴ are each independently hydrogen, -NH₂, or -CH₃; or
b) B¹ and B² are independently selected from: 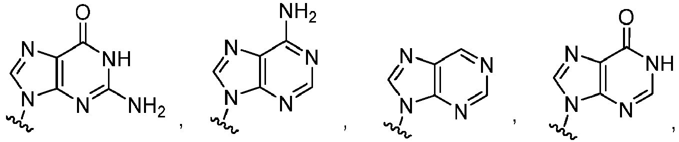 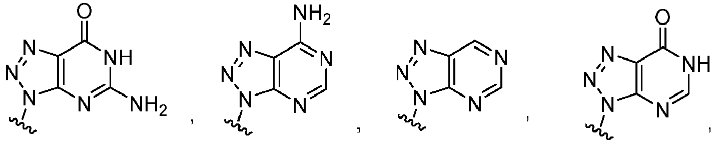 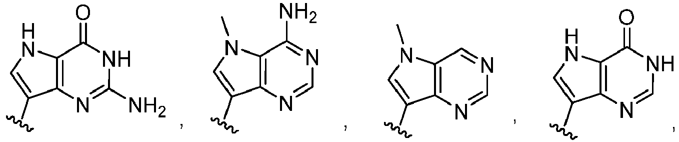 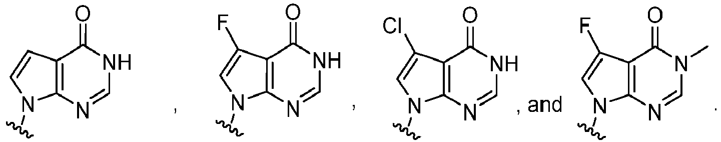
optionally wherein B¹ is: 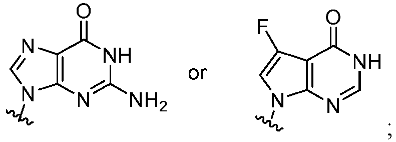 optionally: 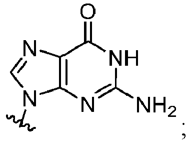 and/or
optionally wherein B² is: 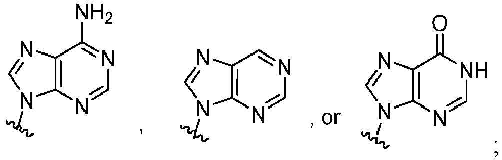 optionally:

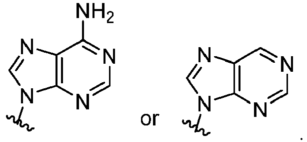

10. The compound of any one of claims 1 to 9 or a pharmaceutically acceptable salt thereof, wherein when B¹, B², or B³ is substituted, the substituent is halogen, -OH, -CN, -NO₂, C₁-C₆alkyl, halo(C₁-C₆)alkyl, C₃-C₆cycloalkyl, -OR¹¹, -SR¹², -N(R¹²)₂, -NR¹²C(O)R¹², -C(O)R¹², -C(O)OR¹², -C(O)N(R¹²)₂, or -SO₂N(R¹²)₂; or two adjacent substituents, taken together with the intervening ring atoms, form a 4- to 8-membered ring;
wherein R¹¹ is C₁-C₆alkyl, halo(C₁-C₆)alkyl, or C₃-C₆cycloalkyl; and
R¹² is hydrogen, C₁-C₆alkyl, halo(C₁-C₆)alkyl, or C₃-C₆cycloalkyl.

11. The compound of claim 1 or a pharmaceutically acceptable salt thereof, wherein the compound is selected from: 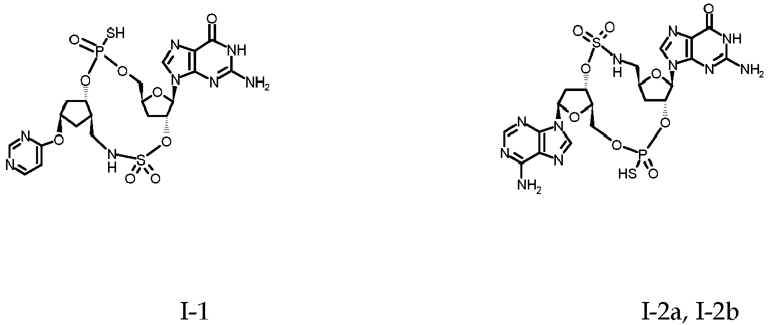 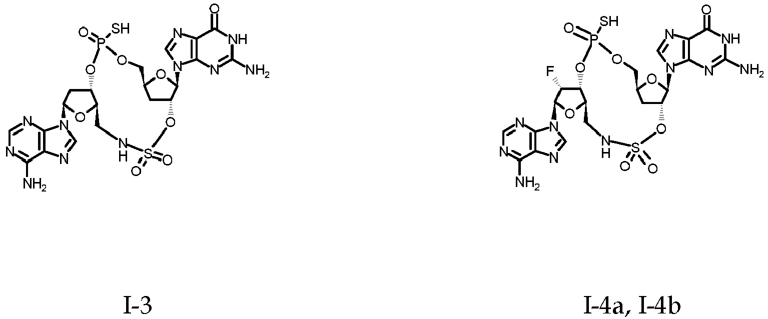 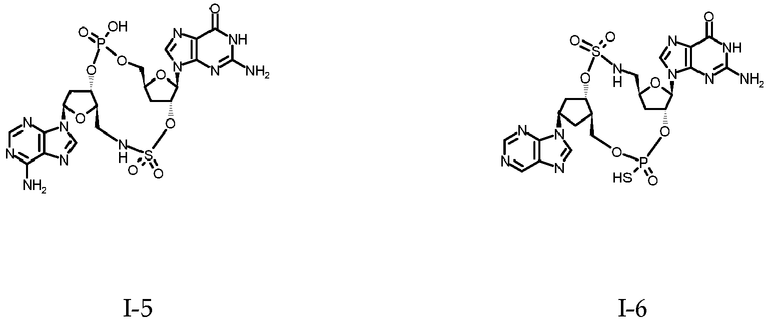 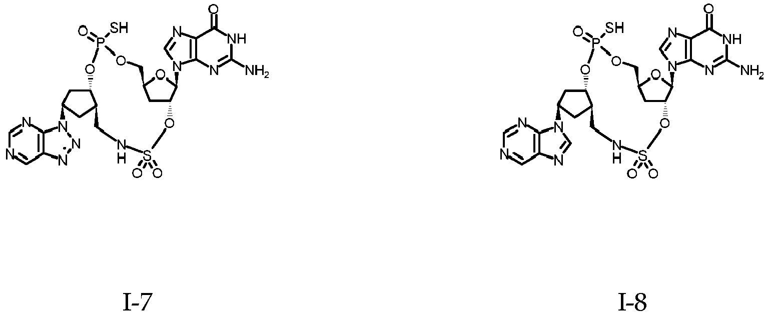 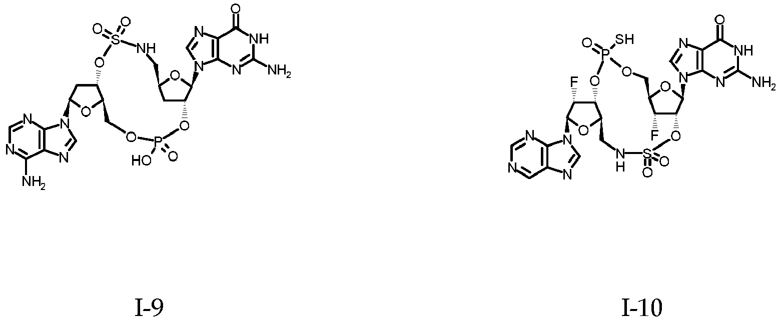 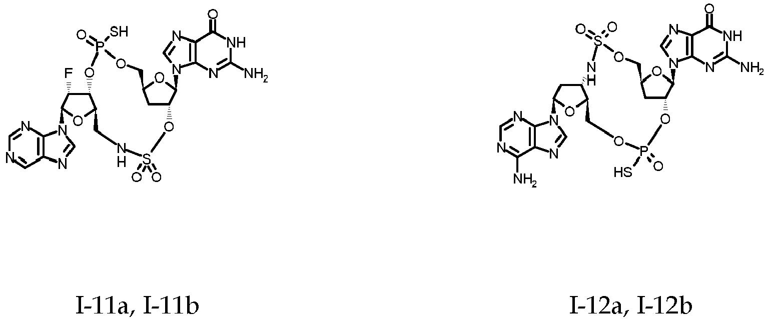 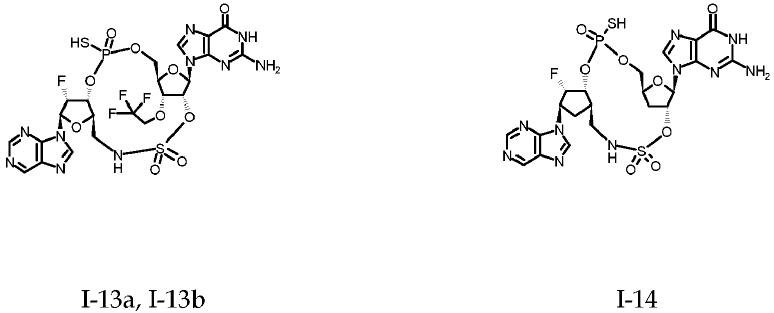 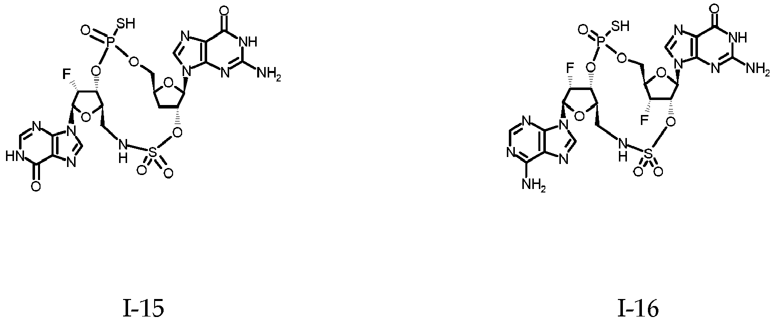 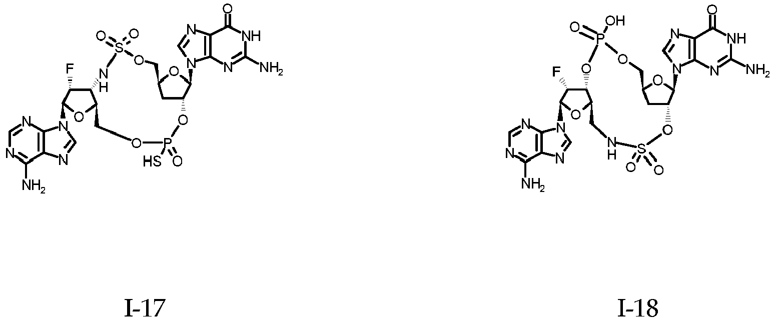 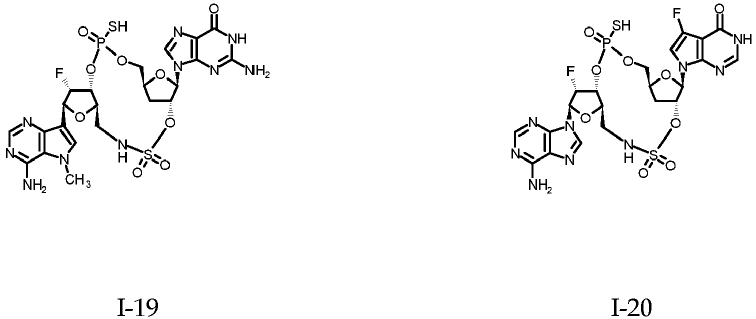 optionally wherein the compound is selected from:

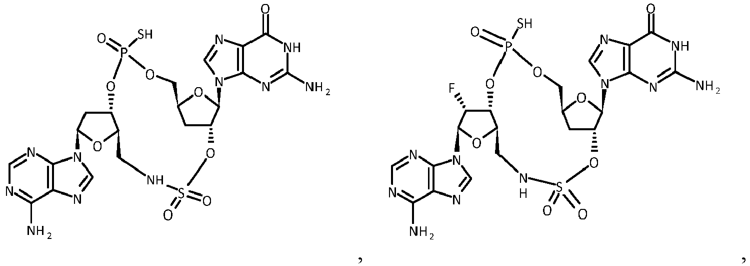

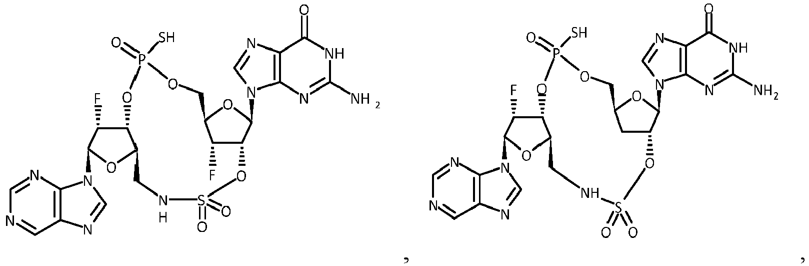

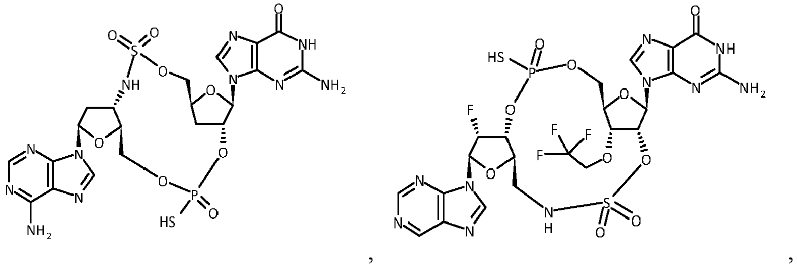

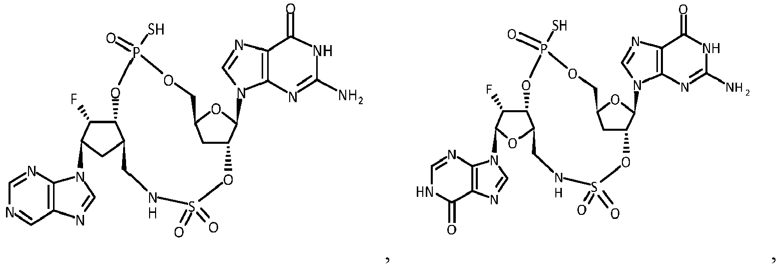

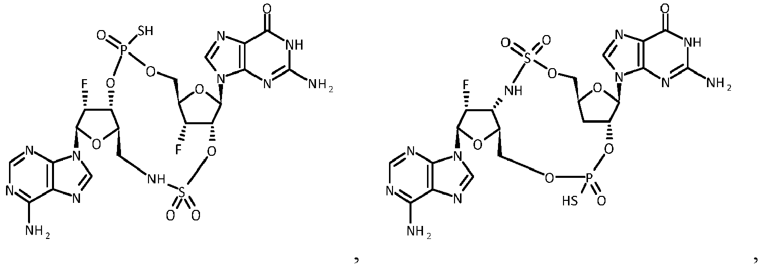

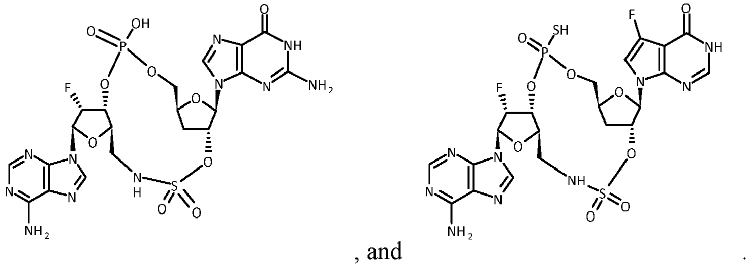

12. A pharmaceutical composition comprising (a) the compound of any one of claims 1 to 11 or a pharmaceutically acceptable salt thereof, and (b) a pharmaceutically acceptable carrier.

13. A compound of any one of claims 1 to 11 or a pharmaceutically acceptable salt thereof for use in a method of inducing an immune response, a method of inducing a STING-dependent type I interferon production, or a method of treating a cell proliferation disorder;
optionally wherein the cell proliferation disorder is cancer and/or the method further comprises administering a second therapeutic agent.

14. A vaccine composition comprising an antigen and the compound of any one of claims 1 to 11 or a pharmaceutically acceptable salt thereof.

15. A vaccine composition of claim 14 for use in a method of treating a disease.
